# EUROPEAN PATENT SPECIFICATION

(11) **EP 2 069 341 B1**
(45) Date of publication and mention of the grant of the patent: **29.12.2010**
(21) Application number: 07820377.5
(22) Date of filing: 20.09.2007
(51) Int. Cl.: C07D 471/04, A61K 31/437, A61P 29/00

(54) **PYRROLO[2, 3-B]PYRIDIN-4-YL-BENZENESULFONAMIDE COMPOUNDS AS IKK2 INHIBITORS**
PYRROLO[2, 3-B]PYRIDIN-4-YL-BENZOLSULFONAMIDVERBINDUNGEN ALS IKK2-HEMMER
COMPOSÉS DE PYRROLO[2, 3-B]PYRIDIN-4-YL-BENZÈNESULFONAMIDE UTILES COMME INHIBITEURS DE IKK2

(30) Priority: 22.09.2006 GB 0618776; 01.03.2007 GB 0704012; 04.09.2007 GB 0717170
(43) Date of publication of application: 17.06.2009
(73) Proprietor: Glaxo Group Limited, Greenford, Middlesex UB6 0NN (GB)
(72) Inventor: BAMBOROUGH, Paul, Stevenage Hertfordshire SG1 2NY (GB); BARKER, Michael, David, Stevenage Hertfordshire SG1 2NY (GB); CAMPOS, Sebastien, Andre, Stevenage Hertfordshire SG1 2NY (GB); COUSINS, Richard, Peter, Charles, Stevenage Hertfordshire SG1 2NY (GB); FAULDER, Paul, Stevenage Hertfordshire SG1 2NY (GB); HOBBS, Heather, Stevenage Hertfordshire SG1 2NY (GB); HOLMES, Duncan, Stuart, Stevenage Hertfordshire SG1 2NY (GB); JOHNSTON, Michael, John, Stevenage Hertfordshire SG1 2NY (GB); LIDDLE, John, Stevenage Hertfordshire SG1 2NY (GB); PAYNE, Jeremy, John, Stevenage Hertfordshire SG1 2NY (GB); PRITCHARD, John, Martin, Stevenage Hertfordshire SG1 2NY (GB); WHITWORTH, Caroline, Stevenage Hertfordshire SG1 2NY (GB)
(74) Representative: Gladwin, Amanda Rachel
(86) International application number: PCT/EP2007/059933
(87) International publication number: WO 2008/034860

(56) References cited:
- WO-A-03/035625
- WO-A-2004/089913

## Description

### FIELD OF THE INVENTION

The invention is directed to certain novel compounds which are inhibitors of kinase activity. More specifically, the compounds are IKK2 inhibitors. Compounds which are IKK2 inhibitors may be useful in the treatment of disorders associated with inappropriate IKK2 (also known as IKKβ) activity, in particular in the treatment and prevention of disorders mediated by IKK2 mechanisms including inflammatory and tissue repair disorders. Such disorders include rheumatoid arthritis, COPD (chronic obstructive pulmonary disease), asthma and rhinitis.

### BACKGROUND OF THE INVENTION

An important large family of enzymes is the protein kinase enzyme family. Currently, there are about 500 different known protein kinases. However, because three to four percent of the human genome is a code for the formation of protein kinases, there may be many thousands of distinct and separate kinases in the human body. Protein kinases serve to catalyze the phosphorylation of an amino acid side chain in various proteins by the transfer of the γ-phosphate of the ATP-Mg²⁺ complex to said amino acid side chain. These enzymes control the majority of the signaling processes inside cells, thereby governing cell function, growth, differentiation and destruction (apoptosis) through reversible phosphorylation of the hydroxyl groups of serine, threonine and tyrosine residues in proteins. Studies have shown that protein kinases are key regulators of many cell functions, including signal transduction, transcriptional regulation, cell motility, and cell division. Several oncogenes have also been shown to encode protein kinases, suggesting that kinases play a role in oncogenesis. These processes are highly regulated, often by complex intermeshed pathways where each kinase will itself be regulated by one or more kinases. Consequently, aberrant or inappropriate protein kinase activity can contribute to the rise of disease states associated with such aberrant kinase activity. Due to their physiological relevance, variety and ubiquitousness, protein kinases have become one of the most important and widely studied family of enzymes in biochemical and medical research.

The protein kinase family of enzymes is typically classified into two main subfamilies: Protein Tyrosine Kinases and Protein Serine/Threonine Kinases, based on the amino acid residue they phosphorylate. The serine/threonine kinases (PSTK) include cyclic AMP- and cyclic GMP-dependent protein kinases, calcium and phospholipid dependent protein kinase, calcium- and calmodulin-dependent protein kinases, casein kinases, cell division cycle protein kinases and others. These kinases are usually cytoplasmic or associated with the particulate fractions of cells, possibly by anchoring proteins. Aberrant protein serine/threonine kinase activity has been implicated or is suspected in a number of pathologies such as rheumatoid arthritis, psoriasis, septic shock, bone loss, many cancers and other proliferative diseases. Accordingly, serine/threonine kinases and the signal transduction pathways which they are part of are important targets for drug design. The tyrosine kinases phosphorylate tyrosine residues. Tyrosine kinases play an equally important role in cell regulation. These kinases include several receptors for molecules such as growth factors and hormones, including epidermal growth factor receptor, insulin receptor, platelet derived growth factor receptor and others. Studies have indicated that many tyrosine kinases are transmembrane proteins with their receptor domains located on the outside of the cell and their kinase domains on the inside. Much work is also in progress to identify modulators of tyrosine kinases as well.

Nuclear factor κB (NF-κB) represents a family of closely related dimeric transcription factor complexes composed of various combinations of the Rel/NF-κB family of polypeptides. The family consists of five individual gene products in mammals, RelA (p65), NF-κB1 (p50/ p105), NF-κB2 (p52/ p100), c-Rel, and RelB, all of which can form hetero- or homo-dimers. These proteins share a highly homologous 300 amino acid "Rel homology domain" which contains the DNA binding and dimerization domains. The NFkBs also carry a nuclear localisation sequence near the C-terminus of the Rel homology domain which is important in the transport of NF-κB from the cytoplasm to the nucleus. In addition, p65 and cRel possess potent transactivation domains at their C-terminal ends.

The activity of NF-κB is regulated by its interaction with a member of the inhibitor IκB family of proteins. This interaction effectively blocks the nuclear localization sequence on the NF-κB proteins, thus preventing migration of the dimer to the nucleus. A wide variety of stimuli activate NF-κB through what are likely to be multiple signal transduction pathways. Included are bacterial products (LPS), some viruses (HIV-1, HTLV-1), inflammatory cytokines (TNFα, IL-1), environmental and oxidative stress and DNA damaging agents. Apparently common to all stimuli however, is the phosphorylation and subsequent degradation of IκB. IκBα and β for example, are phosphorylated on two N-terminal serines by the recently identified IκB kinases (IKK-α and IKK-β), whilst NF-κB2, which carries an IkB-like C terminal region is phosphorylated on N and C terminal serines by IKK-α. IKK-β is also known as IKK2 and its now widely accepted that it is essential for rapid NFkB activation in response to pro-inflammatory stimuli. IKK2 is an example of a serine/threonine kinase. Site-directed mutagenesis studies indicate that these phosphorylations are critical for the subsequent activation of NF-κB in that once phosphorylated the protein is flagged for degradation via the ubiquitin-proteasome pathway. Free from IκB, the active NF-κB complexes are able to translocate to the nucleus where they bind in a selective manner to preferred gene-specific enhancer sequences. Included in the genes regulated by NF-κB are a number of cytokines and chemokines, cell adhesion molecules, acute phase proteins, immunoregualtory proteins, eicosanoid metabolizing enzymes and anti-apoptotic genes.

It is well-known that NF-κB plays a key role in the regulated expression of a large number of pro-inflammatory mediators including cytokines such as TNF, IL-1β, IL-6 and IL-8, cell adhesion molecules, such as ICAM and VCAM, and inducible nitric oxide synthase (iNOS). Such mediators are known to play a role in the recruitment of leukocytes at sites of inflammation and in the case of iNOS, may lead to organ destruction in some inflammatory and autoimmune diseases.

The importance of NF-κB in inflammatory disorders is further strengthened by studies of airway inflammation including asthma, in which NF-κB has been shown to be activated. This activation may underlie the increased cytokine production and leukocyte infiltration characteristic of these disorders. In addition, inhaled steroids are known to reduce airway hyperresponsiveness and suppress the inflammatory response in asthmatic airways. In light of the recent findings with regard to glucocorticoid inhibition of NF-κB, one may speculate that these effects are mediated through an inhibition of NF-κB.

Further evidence for a role of NF-κB in inflammatory disorders comes from studies of rheumatoid synovium. Although NF-κB is normally present as an inactive cytoplasmic complex, recent immunohistochemical studies have indicated that NF-κB is present in the nuclei, and hence active, in the cells comprising rheumatoid synovium. Furthermore, NF-κB has been shown to be activated in human synovial cells in response to stimulation with TNF-α or IL-1β. Such a distribution may be the underlying mechanism for the increased cytokine and eicosanoid production characteristic of this tissue. See Roshak, A. K., et al., J. Biol. Chem., 271, 31496-31501 (1996). Expression of IKK-β has been shown in synoviocytes of rheumatoid arthritis patients and gene transfer studies have demonstrated the central role of IKK-β in stimulated inflammatory mediator production in these cells. See Aupperele, K. R., et al., J. Immunology, 1999, 163:427-433 and Aupperle, K. R, et al., J. Immunology, 2001, 166:2705-11. More recently, the intra-articular administration of a wild type IKK-β adenoviral construct was shown to cause paw swelling while intra-articular administration of dominant-negative IKKβ inhibited adjuvant-induced arthritis in rat. See Tak, P. P., et al., Arthritis and Rheumatism, 2001, 44:1897-1907.

The NF-κB/Rel and IκB proteins are also likely to play a key role in neoplastic transformation and metastasis. Family members are associated with cell transformation *in vitro* and *in vivo* as a result of over expression, gene amplification, gene rearrangements or translocations. In addition, rearrangement and/or amplification of the genes encoding these proteins are seen in 20-25% of certain human lymphoid tumors. Further, NF-κB is activated by oncogenic ras, the most common defect in human tumors and blockade of NF-κB activation inhibits ras mediated cell transformation. In addition, a role for NF-κB in the regulation of apoptosis has been reported strengthening the role of this transcription factor in the regulation of tumor cell proliferation. TNF, ionizing radiation and DNA damaging agents have all been shown to activate NF-κB which in turn leads to the upregulated expression of several anti-apoptotic proteins. Conversely, inhibition of NF-κB has been shown to enhance apoptotic-killing by these agents in several tumor cell types. As this likely represents a major mechanism of tumor cell resistance to chemotherapy, inhibitors of NF-κB activation may be useful chemotherapeutic agents as either single agents or adjunct therapy. Recent reports have implicated NF-κB as an inhibitor of skeletal cell differentiation as well as a regulator of cytokine-induced muscle wasting (Guttridge, D. C., et al., Science, 2000, 289: 2363-2365) further supporting the potential of NFκB inhibitors as novel cancer therapies.

Several NF-κB and IKK inhibitors are described in Wahl, C., et al., J. Clin. Invest. 101(5), 1163-1174 (1998); Sullivan, R. W., et al., J. Med. Chem., 41, 413-419 (1998); Pierce, J. W., et al., J. Biol. Chem. 272, 21096-21103 (1997); and Colsh, P. D. G., et al., Expert Opin. Ther. Patents, 2006, vol 16(1) 1-12.

The marine natural product hymenialdisine is known to inhibit NF-κB. See Roshak, A., et al., JPET, 283, 955-961 (1997); and Breton, J. J., and Chabot-Fletcher, M. C., JPET, 282, 459-466 (1997).

WO2004/089913 discloses 2-aminopyrimidine derivatives as inhibitors of IKK activity. WO 2003/035625 discloses substituted indazole compounds for the treatment of inflammation.

Attempts have been made to prepare compounds that inhibit IKK2 activity and a number of such compounds have been disclosed in the art. However, in view of the number of pathological responses that are mediated by IKK2, there remains a continuing need for inhibitors of IKK2 which can be used in the treatment of a variety of conditions.

The present inventors have discovered novel compounds which are inhibitors of kinase activity, in particular IKK2 activity. Compounds which are IKK2 inhibitors may be useful in the treatment of disorders associated with inappropriate kinase activity, in particular inappropriate IKK2 activity, for example in the treatment and prevention of disorders mediated by IKK2 mechanisms. Such disorders include inflammatory and tissue repair disorders (including rheumatoid arthritis, inflammatory bowel disease, COPD (chronic obstructive pulmonary disease), asthma and rhinitis), fibrotic diseases, osteoarthritis, osteoporosis, dermatosis (including psoriasis, atopic dermatitis and ultraviolet radiation (UV)-induced skin damage), autoimmune diseases (including Sjogren's syndrome, systemic lupus eythematosus, multiple sclerosis, psoriatic arthritis, alkylosing spondylitis, tissue and organ rejection), Alzheimer's disease, stroke, atherosclerosis, restonosis, diabetes, glomerulonephritis, cancer (including Hodgkins disease), cachexia, inflammation associated with infection and certain viral infections (including acquired immune deficiency syndrome (AIDS)), adult respiratory distress syndrome, and Ataxia Telangiestasia.

In one embodiment, the compounds show selectivity for IKK2 over other kinases.

In one embodiment, the compounds are particularly suitable for development as a drug due to their pharmacokinetic profile. For example, compounds for oral administration show good oral bioavailability.

### SUMMARY OF THE INVENTION

The invention is directed to certain novel compounds. Specifically, the invention is directed to compounds according to formula (I): 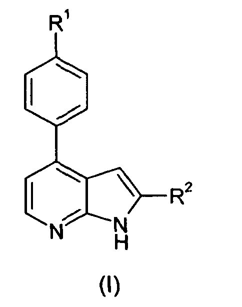 wherein R¹ and R² are as defined below, and salts thereof.

The compounds of the invention are inhibitors of IKK2 activity. Compounds which are IKK2 inhibitors may be useful in the treatment of disorders associated with inappropriate IKK2 (also known as IKKβ) activity, such as rheumatoid arthritis, COPD (chronic obstructive pulmonary disease), asthma and rhinitis (including seasonal rhinitis, allergic rhinitis and vasomotor rhinitis). Accordingly, the invention is further directed to pharmaceutical compositions comprising a compound of formula (I) or a pharmaceutically acceptable salt thereof. The invention is still further directed to methods of inhibiting IKK2 activity and treatment of disorders associated therewith using a compound of formula (I) or a pharmaceutically acceptable salt thereof, or a pharmaceutical composition comprising a compound of formula (I) or a pharmaceutically acceptable salt thereof.

### DETAILED DESCRIPTION OF THE INVENTION

In one embodiment, the invention is directed to compounds according to formula (I): 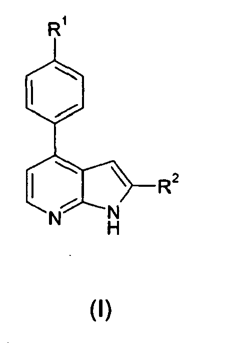 wherein
R¹ is -SO₂NR³R⁴ or -NR⁵SO₂CH₃;
R² is -CHR⁶R⁷, -CF₃ or -C(CH₃)₃;
R³ is hydrogen or methyl and R⁴ is hydrogen, C₁₋₆alkyl optionally substituted by hydroxy, -(CH₂)ₐC₃₋₆cycloalkyl wherein the cycloalkyl is optionally substituted by hydroxy or -NH₂, -(CH₂)_{b}NR⁸R⁹, piperidinyl optionally substituted by C₁₋₆alkyl, or 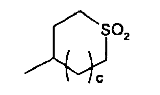 , or
R³ and R⁴ are linked to form pyrrolidinyl, or piperidinyl optionally substituted by - NH₂;
R⁵ is hydrogen or methyl;
R⁶ is hydrogen and R⁷ is hydrogen, C₁₋₆alkyl, -(CH₂)_{d}OR¹⁰, -NR¹¹R¹², -CO₂C₁₋₆alkyl, -CONR¹³R¹⁴, phenyl, or 5-membered heteroaryl containing from one to four nitrogen atoms wherein the heteroaryl is optionally substituted by one or two substituents independently selected from C₁₋₆alkyl, -COC₁₋₆alkyl, -(CH₂)ₑphenyl and thienyl,
R⁶ and R⁷ are each fluorine,
R⁶ is methyl and R⁷ is methyl or hydroxy, or
R⁶ and R⁷ are linked to form C₃₋₆cycloalkyl optionally substituted by methyl;
R⁸ is hydrogen;
R⁹ is hydrogen, C₁₋₆alkyl or -CO₂C₁₋₆alkyl;
R¹⁰ is hydrogen, phenyl optionally substituted by -(CH₂)_{f}CO₂R¹⁵, or pyridyl optionally substituted by one or two substituents independently selected from chlorine and C₁₋₆alkyl;
R¹¹ is hydrogen and R¹² is hydrogen, -(CH₂)₉NR¹⁶R¹⁷, -(CH₂)ₕNCOC₁₋₆alkyl, - (CH₂)ᵢC₃₋₆cycloalkyl, -(CH₂)ⱼphenyl, -(CH₂)ₖpyridyl, or -(CH₂)ₘheterocyclyl wherein the heterocyclyl is optionally substituted by C₁₋₆alkyl,
R¹¹ is C₁₋₆alkyl and R¹² is C₁₋₆alkyl or -SO₂phenyl,
R¹¹ and R¹² are linked to form a 6-membered heterocyclyl optionally containing one further nitrogen wherein the heterocyclyl is optionally substituted by -CO₂C₁₋₆alkyl or piperidinyl, or
R¹¹ and R¹² are linked to form 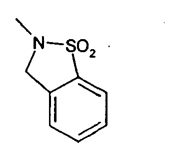
R¹³ is hydrogen and R¹⁴ is hydrogen, C₁₋₆alkyl, -(CH₂)ₙOR¹⁸, -(CH₂)ₚNR¹⁹R²⁰, - (CH₂)_{q}CO₂R²¹, -(CH₂)ᵣSO₂NH₂, C₃₋₆cycloalkyl, or phenyl optionally substituted by chlorine or -OC₁₋₆alkyl,
R¹³ and R¹⁴ are each independently C₁₋₆alkyl, or
R¹³ and R¹⁴ are linked to form pyrrolidinyl;
R¹⁵, R¹⁶, R¹⁷, R¹⁸, R¹⁹, R²⁰ and R²¹ are each independently hydrogen or C₁₋₆alkyl;
a, d, e, f, i, j, k and m are each independently an integer selected from 0 to 4;
b, g, h, n, p, q and r are each independently an integer selected from 1 to 4; and
c is 0 or 1;
and salts thereof (hereinafter "compounds of the invention").

In another embodiment, the invention is directed to compounds according to formula (IA): 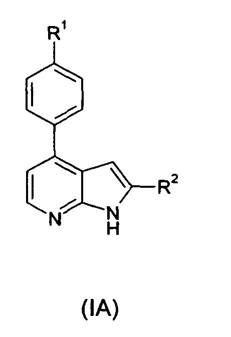 wherein
R¹ is -SO₂NR³R⁴ or -NR⁵SO₂CH₃;
R² is -CHR⁶R⁷, -CF₃ or -C(CH₃)₃;
R³ is hydrogen or methyl and R⁴ is hydrogen, C₁₋₆alkyl optionally substituted by hydroxy, -(CH₂)ₐC₃₋₆cycloalkyl wherein the cycloalkyl is optionally substituted by hydroxy or -NH₂, -(CH₂)_{b}NR⁸R⁹, piperidinyl optionally substituted by C₁₋₆alkyl, or 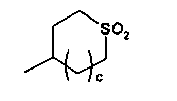 , or
R³ and R⁴ are linked to form pyrrolidinyl, or piperidinyl optionally substituted by - NH₂;
R⁵ is hydrogen or methyl;
R⁶ is hydrogen and R⁷ is hydrogen, methyl, -(CH₂)_{d}OR¹⁰, -NR¹¹R¹², -CO₂C₁₋₆alkyl, - CONR¹³R¹⁴, phenyl, or 5-membered heteroaryl containing from one to four nitrogen atoms wherein the heteroaryl is optionally substituted by one or two substituents independently selected from C₁₋₆alkyl, -COC₁₋₆alkyl, -(CH₂)ₑphenyl and thienyl,
R⁶ and R⁷ are each fluorine,
R⁶ is methyl and R⁷ is methyl or hydroxy, or
R⁶ and R⁷ are linked to form C₃₋₆cycloalkyl;
R⁸ is hydrogen;
R⁹ is hydrogen, C₁₋₆alkyl or -CO₂C₁₋₆alkyl;
R¹⁰ is hydrogen, phenyl optionally substituted by -(CH₂)_{f}CO₂R¹⁵, or pyridyl optionally substituted by one or two substituents independently selected from chlorine and C₁₋₆alkyl;
R¹¹ is hydrogen and R¹² is hydrogen, -(CH₂)_{g}NR¹⁶R¹⁷, -(CH₂)ₕNCOC₁₋₆alkyl, - (CH₂)ᵢC₃₋₆cycloalkyl, -(CH₂)ⱼphenyl, -(CH₂)ₖpyridyl, or -(CH₂)ₘheterocyclyl wherein the heterocyclyl is optionally substituted by C₁₋₆alkyl,
R¹¹ is C₁₋₆alkyl and R¹² is C₁₋₆alkyl or -SO₂phenyl,
R¹¹ and R¹² are linked to form a 6-membered heterocyclyl optionally containing one further nitrogen wherein the heterocyclyl is optionally substituted by -CO₂C₁₋₆alkyl or piperidinyl, or
R¹¹ and R¹² are linked to form 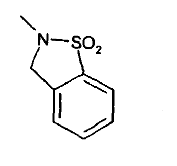
R¹³ is hydrogen and R¹⁴ is hydrogen, C₁₋₆alkyl, -(CH₂)ₙOR¹⁸, -(CH₂)ₚNR¹⁹R²⁰, - (CH₂)_{q}CO₂R²¹, -(CH₂)ᵣSO₂NH₂, C₃₋₆cycloalkyl, or phenyl optionally substituted by chlorine or -OC₁₋₆alkyl,
R¹³ and R¹⁴ are each independently C₁₋₆alkyl, or
R¹³ and R¹⁴ are linked to form pyrrolidinyl;
R¹⁵, R¹⁶, R¹⁷, R¹⁸, R¹⁹, R²⁰ and R²¹ are each independently hydrogen or C₁₋₆alkyl;
a, d, e, f, i, j, k and m are each independently an integer selected from 0 to 4;
b, g, h, n, p, q and r are each independently an integer selected from 1 to 4; and
c is 0 or 1;
and salts thereof.

In a further embodiment, the invention is directed to compounds according to formula (IB): 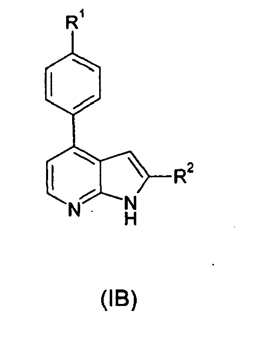 wherein
R¹ is -SO₂NR³R⁴ or -NR⁵SO₂CH₃;
R² is -CHR⁶R⁷, -CF₃ or -C(CH₃)₃;
R³ is hydrogen or methyl and R⁴ is hydrogen, C₁₋₆alkyl substituted by hydroxy, - (CH₂)ₐC₃₋₆cycloalkyl wherein the cycloalkyl is substituted by hydroxy or -NH₂, - (CH₂)_{b}NR⁸R⁹, piperidinyl optionally substituted by C₁₋₆alkyl, or 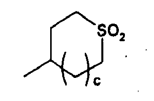 , or
R³ and R⁴ are linked to form pyrrolidinyl;
R⁵ is hydrogen or methyl;
R⁶ is hydrogen and R⁷ is hydrogen, methyl, -(CH₂)_{d}OR¹⁰, -NR¹¹R¹², -CO₂C₁₋₆alkyl, - CONR¹³R¹⁴, phenyl, or 5-membered heteroaryl containing from one to four nitrogen atoms wherein the heteroaryl is optionally substituted by one or two substituents independently selected from C₁₋₆alkyl, -COC₁₋₆alkyl, -(CH₂)ₑphenyl and thienyl,
R⁶ and R⁷ are each fluorine, or
R⁶ is methyl and R⁷ is methyl or hydroxy;
R⁸ is hydrogen;
R⁹ is hydrogen, C₁₋₆alkyl or -CO₂C₁₋₆alkyl;
R¹⁰ is hydrogen, phenyl optionally substituted by -(CH₂)_{f}CO₂R¹⁵, or pyridyl optionally substituted by one or two substituents independently selected from chlorine and C₁₋₆alkyl;
R¹¹ is hydrogen and R¹² is hydrogen, -(CH₂)_{g}NR¹⁶R¹⁷, -(CH₂)ₕNCOC₁₋₆alkyl, - (CH₂)ᵢC₃₋₆cycloalkyl, -(CH₂)ⱼphenyl, -(CH₂)ₖpyridyl, or -(CH₂)ₘheterocyclyl wherein the heterocyclyl is optionally substituted by C₁₋₆alkyl,
R¹¹ is C₁₋₆alkyl and R¹² is C₁₋₆alkyl or -SO₂phenyl,
R¹¹ and R¹² are linked to form a 6-membered heterocyclyl optionally containing one further nitrogen wherein the heterocyclyl is optionally substituted by -CO₂C₁₋₆alkyl or piperidinyl, or
R¹¹ and R¹² are linked to form 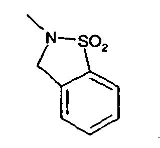
R¹³ is hydrogen and R¹⁴ is hydrogen, C₁₋₆alkyl, -(CH₂)ₙOR¹⁸, -(CH₂)ₚNR¹⁹R²⁰, - (CH₂)_{q}CO₂R²¹, -(CH₂)ᵣSO₂NH₂, C₃₋₆cycloalkyl, or phenyl optionally substituted by chlorine or -OC₁₋₆alkyl,
R¹³ and R¹⁴ are each independently C₁₋₆alkyl, or
R¹³ and R¹⁴ are linked to form pyrrolidinyl;
R¹⁵, R¹⁶, R¹⁷, R¹⁸, R¹⁹, R²⁰ and R²¹ are each independently hydrogen or C₁₋₆alkyl;
a, d, e, f, i, j, k and m are each independently an integer selected from 0 to 4;
b, g, h, n, p, q and r are each independently an integer selected from 1 to 4; and
c is 0 or 1;
and salts thereof.

In one embodiment, R¹ is -SO₂NR³R⁴. In a further embodiment, R¹ is -NR⁵SO₂CH₃.

In one embodiment, R² is -CHR⁶R⁷. In another embodiment, R² is -CF₃. In a further embodiment, R² is -C(CH₃)₃.

In one embodiment, R³ is hydrogen or methyl and R⁴ is hydrogen, C₁₋₆alkyl substituted by hydroxy, -(CH₂)ₐC₃₋₆cycloalkyl wherein the cycloalkyl is substituted by hydroxy or -NH₂, -(CH₂)_{b}NR⁸R⁹, piperidinyl optionally substituted by C₁₋₆alkyl, or 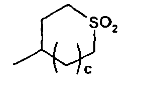 , or
R³ and R⁴ are linked to form pyrrolidinyl. In another embodiment, R³ is hydrogen or methyl and R⁴ is hydrogen, C₁₋₆alkyl substituted by hydroxy, -(CH₂)ₐC₃₋₆cycloalkyl wherein the cycloalkyl is substituted by hydroxy or -NH₂, piperidinyl optionally substituted by C₁₋₆alkyl, or 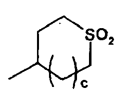 , or
R³ and R⁴ are linked to form pyrrolidinyl. In another embodiment, R³ is hydrogen and R⁴ is 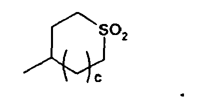 In another embodiment, R³ is hydrogen and R⁴ is C₁₋₆alkyl substituted by hydroxy, for example 2-hydroxyethyl. In a further embodiment, R³ is hydrogen and R⁴ is -(CH₂)_{b}NR⁸R⁹, for example 2-aminoethyl.

In one embodiment, R⁵ is hydrogen. In a further embodiment, R⁵ is methyl.

In one embodiment, R⁶ is hydrogen and R⁷ is hydrogen, methyl, -(CH₂)_{d}OR¹⁰,-NR¹¹R¹², -CO₂C₁₋₆alkyl, -CONR¹³R¹⁴, phenyl, or 5-membered heteroaryl containing from one to four nitrogen atoms wherein the heteroaryl is optionally substituted by one or two substituents independently selected from C₁₋₆alkyl, -COC₁₋₆alkyl, - (CH₂)ₑphenyl and thienyl, R⁶ and R⁷ are each fluorine, R⁶ is methyl and R⁷ is methyl or hydroxy, or R⁶ and R⁷ are linked to form C₃₋₆cycloalkyl. In another embodiment, R⁶ is hydrogen and R⁷ is hydrogen, methyl, -(CH₂)_{d}OR¹⁰, -NR¹¹R¹², - CO₂C₁₋₆alkyl, -CONR¹³R¹⁴, phenyl, or 5-membered heteroaryl containing from one to four nitrogen atoms wherein the heteroaryl is optionally substituted by one or two substituents independently selected from C₁₋₆alkyl, -COC₁₋₆alkyl, -(CH₂)ₑphenyl and thienyl, R⁶ and R⁷ are each fluorine, or R⁶ is methyl and R⁷ is methyl or hydroxyl. In another embodiment, R⁶ is hydrogen and R⁷ is hydrogen, methyl, -(CH₂)_{d}OR¹⁰, - NR¹¹R¹², -CO₂C₁₋₆alkyl, -CONR¹³R¹⁴, phenyl, or 5-membered heteroaryl containing from one to four nitrogen atoms wherein the heteroaryl is optionally substituted by one or two substituents independently selected from C₁₋₆alkyl, -COC₁₋₆alkyl, - (CH₂)ₑphenyl and thienyl, R⁶ and R⁷ are each fluorine, or R⁶ is methyl and R⁷ is methyl. In another embodiment, R⁶ is hydrogen and R⁷ is hydrogen, -CO₂C₁₋₆alkyl, or 5-membered heteroaryl containing from one to four nitrogen atoms wherein the heteroaryl is optionally substituted by one or two substituents independently selected from C₁₋₆alkyl, -COC₁₋₆alkyl, -(CH₂)ₑphenyl and thienyl. In another embodiment, R⁶ and R⁷ are each fluorine. In another embodiment, R⁶ and R⁷ are each methyl. In another embodiment, R⁶ is hydrogen and R⁷ is -NR¹¹R¹². In another embodiment, R⁶ is hydrogen and R⁷ is C₁₋₆alkyl. In another embodiment, R⁶ is hydrogen and R⁷ is methyl. In a further embodiment, R⁶ and R⁷ are linked to form C₃₋₆cycloalkyl, for example cyclopropyl or cyclobutyl, such as cyclopropyl.

In one embodiment, R⁸ is hydrogen.

In one embodiment, R⁹ is hydrogen.

In one embodiment, R¹⁰ is pyridyl optionally substituted by one or two substituents independently selected from chlorine and C₁₋₆alkyl.

In one embodiment, R¹¹ is hydrogen and R¹² is hydrogen, -(CH₂)₉NR¹⁶R¹⁷, - (CH₂)ₕNCOC₁₋₆alkyl, -(CH₂)ᵢC₃₋₆cycloalkyl, -(CH₂)ⱼphenyl, -(CH₂)ₖpyridyl, or - (CH₂)ₘheterocyclyl, for example -(CH₂)ₘpiperidinyl, wherein the heterocyclyl is optionally substituted by C₁₋₆alkyl,
R¹¹ is C₁₋₆alkyl and R¹² is C₁₋₆alkyl or -SO₂phenyl,
R¹¹ and R¹² are linked to form a 6-membered heterocyclyl optionally containing one further nitrogen wherein the heterocyclyl is optionally substituted by -CO₂C₁₋₆alkyl or piperidinyl, or
R¹¹ and R¹² are linked to form 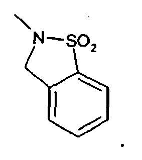 In another embodiment, R¹¹ is hydrogen and R¹² is hydrogen, - (CH₂)_{g}NR¹⁶R¹⁷, -(CH₂)ₕNCOC₁₋₆alkyl, -(CH₂)ᵢC₃₋₆cycloalkyl, -(CH₂)ⱼphenyl, - (CH₂)ₖpyridyl, or -(CH₂)ₘheterocyclyl wherein the heterocyclyl is optionally substituted by C₁₋₆alkyl. In a further embodiment, R¹¹ and R¹² are each independently C₁₋₆alkyl, for example R¹¹ and R¹² are each methyl.

In one embodiment, R¹³ is hydrogen and R¹⁴ is hydrogen, C₁₋₆alkyl, -(CH₂)ₙOR¹⁸, - (CH₂)ₚNR¹⁹R²⁰, -(CH₂)_{q}CO₂R²¹, -(CH₂)ᵣSO₂NH₂, C₃₋₆cycloalkyl, or phenyl optionally substituted by chlorine or -OC₁₋₆alkyl.

In one embodiment, R¹⁵ is hydrogen. In a further embodiment, R¹⁵ is methyl.

In one embodiment, R¹⁶ is methyl.

In one embodiment, R¹⁷ is methyl.

In one embodiment, R¹⁸ is hydrogen. In a further embodiment, R¹⁸ is methyl.

In one embodiment, R¹⁹ is hydrogen.

In one embodiment, R²⁰ is hydrogen.

In one embodiment, R²¹ is hydrogen.

In one embodiment, a is 0. In a further embodiment, a is 1.

In one embodiment, b is 2.

In one embodiment, c is 0. In a further embodiment, c is 1.

In one embodiment, d is 0. In another embodiment, d is 2. In a further embodiment d is 3.

In one embodiment, e is 1.

In one embodiment, f is 0. In a further embodiment, f is 1.

In one embodiment, g is 2.

In one embodiment, h is 2.

In one embodiment, i is 1.

In one embodiment, j is 1.

In one embodiment, k is 0. In a further embodiment, k is 1.

In one embodiment, m is 0.

In one embodiment, n is 3. In a further embodiment, n is 4.

In one embodiment, p is 2.

In one embodiment, q is 1.

In one embodiment, r is 3.

It is to be understood that the present invention covers all combinations of substituent groups described hereinabove.

In one embodiment, the invention is directed to compounds according to formula (I) wherein
R¹ is -SO₂NR³R⁴;
R² is -CHR⁶R⁷;
R³ is hydrogen and R⁴ is C₁₋₆alkyl substituted by hydroxyl, or -(CH₂)_{b}NR⁸R⁹;
R⁶ is hydrogen and R⁷ is C₁₋₆alkyl, or
R⁶ and R⁷ are linked to form C₃₋₆cycloalkyl;
R⁸ is hydrogen;
R⁹ is hydrogen; and
b is 2;
and salts thereof.

Compounds of the invention include the compounds of Examples 1 to 260 and salts thereof.

In one embodiment, the compound of the invention is:
2-[4-(4-{[(2-aminoethyl)amino]sulfonyl}phenyl)-1H-pyrrolo[2,3-b]pyridin-2-yl]acetamide;
2-[4-(4-{[(3-aminopropyl)amino]sulfonyl}phenyl)-1H-pyrrolo[2,3-b]pyridin-2-yl]acetamide;
1,1-dimethylethyl {3-[({4-[2-(2-amino-2-oxoethyl)-1H-pyrrolo[2,3-b]pyridin-4-yl]phenyl}sulfonyl)amino]propyl}carbamate;
1,1-dimethylethyl {2-[({4-[2-(2-amino-2-oxoethyl)-1H-pyrrolo[2,3-b]pyridin-4-yl]phenyl}sulfonyl)amino]ethyl}carbamate;
N-(2-aminoethyl)-4-[2-(hydroxymethyl)-1H-pyrrolo[2,3-b]pyridin-4-yl]benzenesulfonamide;
N-(2-aminoethyl)-4-[2-(3-hydroxypropyl)-1H-pyrrolo[2,3-b]pyridin-4-yl]benzenesulfonamide;
1,1-dimethylethyl [4-(4-{[(1,1-dioxidotetrahydro-3-thienyl)amino]sulfonyl}phenyl)-1H-pyrrolo[2,3-b]pyridin-2-yl]acetate;
1,1-dimethylethyl [4-(4-{[(1,1-dioxidotetrahydro-2H-thiopyran-4-yl)amino]sulfonyl}phenyl)-1H-pyrrolo[2,3-b]pyridin-2-yl]acetate;
2-(phenylmethyl)-4-[4-(1-pyrrolidinylsulfonyl)phenyl]-1H-pyrrolo[2,3-b]pyridine;
2-methyl-4-[4-(1-pyrrolidinylsulfonyl)phenyl]-1H-pyrrolo[2,3-b]pyridine;
{4-[4-(1-pyrrolidinylsulfonyl)phenyl]-1H-pyrrolo[2,3-b]pyridin-2-yl}methanol;
1,1-dimethylethyl (4-{4-[(methylsulfonyl)amino]phenyl}-1H-pyrrolo[2,3-b]pyridin-2-yl)acetate;
1,1-dimethylethyl {4-[4-(aminosulfonyl)phenyl]-1H-pyrrolo[2,3-b]pyridin-2-yl}acetate;
1,1-dimethylethyl {4-[4-(1-pyrrolidinylsulfonyl)phenyl]-1H-pyrrolo[2,3-b]pyridin-2-yl}acetate;
2-(difluoromethyl)-4-[4-(1-pyrrolidinylsulfonyl)phenyl]-1H-pyrrolo[2,3-b]pyridine;
N-methyl-2-(4-{4-[(methylsulfonyl)amino]phenyl}-1H-pyrrolo[2,3-b]pyridin-2-yl)acetamide;
N,N-dimethyl-2-(4-{4-[(methylsulfonyl)amino]phenyl}-1H-pyrrolo[2,3-b]pyridin-2-yl)acetamide;
N-methyl-2-(4-{4-[(methylsulfonyl)amino]phenyl}-1H-pyrrolo[2,3-b]pyridin-2-yl)acetamide;
N,N-dimethyl-2-(4-{4-[(methylsulfonyl)amino]phenyl}-1H-pyrrolo[2,3-b]pyridin-2-yl)acetamide;
N-(4-{2-[2-oxo-2-(1-pyrrolidinyl)ethyl]-1H-pyrrolo[2,3-b]pyridin-4-yl}phenyl)methanesulfonamide;
N-cyclohexyl-2-(4-{4-[(methylsulfonyl)amino]phenyl}-1H-pyrrolo[2,3-b]pyridin-2-yl)acetamide;
N-(3-chlorophenyl)-2-(4-{4-[(methylsulfonyl)amino]phenyl}-1H-pyrrolo[2,3-b]pyridin-2-yl)acetamide;
N-[3-(methyloxy)phenyl]-2-(4-{4-[(methylsulfonyl)amino]phenyl}-1H-pyrrolo[2,3-b]pyridin-2-yl)acetamide;
N-(3-hydroxypropyl)-2-(4-{4-[(methylsulfonyl)amino]phenyl}-1H-pyrrolo[2,3-b]pyridin-2-yl)acetamide;
N-(4-hydroxybutyl)-2-(4-{4-[(methylsulfonyl)amino]phenyl}-1H-pyrrolo[2,3-b]pyridin-2-yl)acetamide;
N-[3-(methyloxy)propyl]-2-(4-{4-[(methylsulfonyl)amino]phenyl}-1H-pyrrolo[2,3-b]pyridin-2-yl)acetamide;
N-[3-(aminosulfonyl)propyl]-2-(4-{4-[(methylsulfonyl)amino]phenyl}-1H-pyrrolo[2,3-b]pyridin-2-yl)acetamide;
N-[(4-{4-[(methylsulfonyl)amino]phenyl}-1H-pyrrolo[2,3-b]pyridin-2-yl)acetyl]glycine;
N-(2-aminoethyl)-2-(4-{4-[(methylsulfonyl)amino]phenyl}-1H-pyrrolo[2,3-b]pyridin-2-yl)acetamide;
N,N-dimethyl-N'-({4-[4-(1-pyrrolidinylsulfonyl)phenyl]-1H-pyrrolo[2,3-b]pyridin-2-yl}methyl)-1,2-ethanediamine;
1'-({4-[4-(1-pyrrolidinylsulfonyl)phenyl]-1H-pyrrolo[2,3-b]pyridin-2-yl}methyl)-1,4'-bipiperidine;
N-ethyl-N-({4-[4-(1-pyrrolidinylsulfonyl)phenyl]-1H-pyrrolo[2,3-b]pyridin-2-yl}methyl)-1-propanamine;
1-methyl-N-({4-[4-(1-pyrrolidinylsulfonyl)phenyl]-1H-pyrrolo[2,3-b]pyridin-2-yl}methyl)-4-piperidinamine;
(phenylmethyl)({4-[4-(1-pyrrolidinylsulfonyl)phenyl]-1H-pyrrolo[2,3-b]pyridin-2-yl}methyl)amine;
N-{2-[({4-[4-(1-pyrrolidinylsulfonyl)phenyl]-1H-pyrrolo[2,3-b]pyridin-2-yl}methyl)amino]ethyl}acetamide;
(cyclohexylmethyl)({4-[4-(1-pyrrolidinylsulfonyl)phenyl]-1H-pyrrolo[2,3-b]pyridin-2-yl}methyl)amine;
(cyclopropylmethyl)({4-[4-(1-pyrrolidinylsulfonyl)phenyl]-1H-pyrrolo[2,3-b]pyridin-2-yl}methyl)amine;
(3-pyridinylmethyl)({4-[4-(1-pyrrolidinylsulfonyl)phenyl]-1H-pyrrolo[2,3-b]pyridin-2-yl}methyl)amine;
1,1-dimethylethyl 4-({4-[4-(1-pyrrolidinylsulfonyl)phenyl]-1H-pyrrolo[2,3-b]pyridin-2-yl}methyl)-1-piperazinecarboxylate;
{3-[({4-[4-(1-pyrrolidinylsulfonyl)phenyl]-1H-pyrrolo[2,3-b]pyridin-2-yl}methyl)oxy]phenyl}acetic acid;
methyl {4-[({4-[4-(1-pyrrolidinylsulfonyl)phenyl]-1H-pyrrolo[2,3-b]pyridin-2-yl}methyl)oxy]phenyl}acetate;
2-[(3-pyridinyloxy)methyl]-4-[4-(1-pyrrolidinylsulfonyl)phenyl]-1H-pyrrolo[2,3-b]pyridine;
2-[(4-pyridinyloxy)methyl]-4-[4-(1-pyrrolidinylsulfonyl)phenyl]-1H-pyrrolo[2,3-b]pyridine;
methyl 3-[({4-[4-(1-pyrrolidinylsulfonyl)phenyl]-1H-pyrrolo[2,3-b]pyridin-2-yl}methyl)oxy]benzoate;
2-[(phenyloxy)methyl]-4-[4-(1-pyrrolidinylsulfonyl)phenyl]-1H-pyrrolo[2,3-b]pyridine; N-methyl-N-({4-[4-(1-pyrrolidinylsulfonyl)phenyl]-1H-pyrrolo[2,3-b]pyridin-2-yl}methyl)benzenesulfonamide;
2-({4-[4-(1-pyrrolidinylsulfonyl)phenyl]-1H-pyrrolo[2,3-b]pyridin-2-yl}methyl)-2,3-dihydro-1,2-benzisothiazole 1,1-dioxide;
2-{[2-(1-methylethyl)-1H-imidazol-1-yl]methyl}-4-[4-(1-pyrrolidinylsulfonyl)phenyl]-1H-pyrrolo[2,3-b]pyridine;
2-{[2-(phenylmethyl)-1H-imidazol-1-yl]methyl}-4-[4-(1-pyrrolidinylsulfonyl)phenyl]-1H-pyrrolo[2,3-b]pyridine;
1-[5-methyl-1-({4-[4-(1-pyrrolidinylsulfonyl)phenyl]-1H-pyrrolo[2,3-b]pyridin-2-yl}methyl)-1H-pyrazol-4-yl]ethanone;
1-[3-methyl-1-({4-[4-(1-pyrrolidinylsulfonyl)phenyl]-1H-pyrrolo[2,3-b]pyridin-2-yl}methyl)-1H-pyrazol-4-yl]ethanone;
2-[(3-methyl-1H-pyrazol-1-yl)methyl]-4-[4-(1-pyrrolidinylsulfonyl)phenyl]-1H-pyrrolo[2,3-b]pyridine;
2-[(5-methyl-1H-pyrazol-1-yl)methyl]-4-[4-(1-pyrrolidinylsulfonyl)phenyl]-1H-p yrrolo[2,3-b]pyridine;
4-[4-(1-pyrrolidinylsulfonyl)phenyl]-2-(1H-1,2,3-triazol-1-ylmethyl)-1H-pyrrolo[2,3-b]pyridine;
4-[4-(1-pyrrolidinylsulfonyl)phenyl]-2-{[3-(2-thienyl)-1H-pyrazol-1-yl]methyl}-1H-pyrrolo[2,3-b]pyridine;
2-(1H-imidazol-1-ylmethyl)-4-[4-(1-pyrrolidinylsulfonyl)phenyl]-1H-pyrrolo[2,3-b]pyridine;
2-(1H-pyrazol-1-ylmethyl)-4-[4-(1-pyrrolidinylsulfonyl)phenyl]-1H-pyrrolo[2,3-b]pyridine;
4-[4-(1-pyrrolidinylsulfonyl)phenyl]-2-(4H-1,2,4-triazol-4-ylmethyl)-1H-pyrrolo[2,3-b]pyridine;
2-[(3,5-dimethyl-1H-pyrazol-1-yl)methyl]-4-[4-(1-pyrrolidinylsulfonyl)phenyl]-1H-pyrrolo[2,3-b]pyridine;
4-[4-(1-pyrrolidinylsulfonyl)phenyl]-2-(1H-pyrrol-1-ylmethyl)-1H-pyrrolo[2,3-b]pyridine;
2-{[(5-chloro-3-pyridinyl)oxy]methyl}-4-[4-(1-pyrrolidinylsulfonyl)phenyl]-1H-pyrrolo[2,3-b]pyridine;
2-{[(6-methyl-3-pyridinyl)oxy]methyl}-4-[4-(1-pyrrolidinylsulfonyl)phenyl]-1H-pyrrolo[2,3-b]pyridine;
2-{[(2-methyl-3-pyridinyl)oxy]methyl}-4-[4-(1-pyrrolidinylsulfonyl)phenyl]-1H-pyrrolo[2,3-b]pyridine;
2-{[(2,6-dimethyl-3-pyridinyl)oxy]methyl}-4-[4-(1-pyrrolidinylsulfonyl)phenyl]-1H-pyrrolo[2,3-b]pyridine;
2-[(2-pyridinyloxy)methyl]-4-[4-(1-pyrrolidinylsulfonyl)phenyl]-1H-pyrrolo[2,3-b]pyridine;
N-(2-aminoethyl)-4-(2-methyl-1H-pyrrolo[2,3-b]pyridin-4-yl)benzenesulfonamide;
N-(1,1-dioxidotetrahydro-2H-thiopyran-4-yl)-4-(2-methyl-1H-pyrrolo[2,3-b]pyridin-4-yl)benzenesulfonamide;
2-methyl-4-[4-(1-pyrrolidinylsulfonyl)phenyl]-1H-pyrrolo[2,3-b]pyridine;
4-[2-(difluoromethyl)-1H-pyrrolo[2,3-b]pyridin-4-yl]-N-(1,1-dioxidotetrahydro-3-thienyl)benzenesulfonamide;
4-[2-(difluoromethyl)-1H-pyrrolo[2,3-b]pyridin-4-yl]-N-(1,1-dioxidotetrahydro-3-thienyl)benzenesulfonamide (Enantiomer 1);
4-[2-(difluoromethyl)-1H-pyrrolo[2,3-b]pyridin-4-yl]-N-(1,1-dioxidotetrahydro-3-thienyl)benzenesulfonamide (Enantiomer 2);
4-[2-(difluoromethyl)-1H-pyrrolo[2,3-b]pyridin-4-yl]-N-(1,1-dioxidotetrahydro-2H-thiopyran-4-yl)benzenesulfonamide;
2-(1-methylethyl)-4-[4-(1-pyrrolidinylsulfonyl)phenyl]-1H-pyrrolo[2,3-b]pyridine;
N-(1,1-dioxidotetrahydro-2H-thiopyran-4-yl)-4-[2-(1-methylethyl)-1H-pyrrolo[2,3-b]pyridin-4-yl]benzenesulfonamide;
2-ethyl-4-[4-(1-pyrrolidinylsulfonyl)phenyl]-1H-pyrrolo[2,3-b]pyridine;
N-(1,1-dioxidotetrahydro-2H-thiopyran-4-yl)-4-[2-(trifluoromethyl)-1H-pyrrolo[2,3-b]pyridin-4-yl]benzenesulfonamide;
4-[4-(1-pyrrolidinylsulfonyl)phenyl]-2-(trifluoromethyl)-1H-pyrrolo[2,3-b]pyridine;
N-(1,1-dioxidotetrahydro-3-thienyl)-4-[2-(trifluoromethyl)-1H-pyrrolo[2,3-b]pyridin-4-yl]benzenesulfonamide;
2-[4-(4-{[(1,1-dioxidotetrahydro-2H-thiopyran-4-yl)amino]sulfonyl}phenyl)-1H-pyrrolo[2,3-b]pyridin-2-yl]-N-[3-(methyloxy)phenyl]acetamide;
2-[4-(4-{[(1,1-dioxidotetrahydro-3-thienyl)amino]sulfonyl}phenyl)-1H-pyrrolo[2,3-b]pyridin-2-yl]-N-[3-(methyloxy)phenyl]acetamide;
N-(1,1-dioxidotetrahydro-3-thienyl)-4-{2-[2-oxo-2-(1-pyrrolidinyl)ethyl]-1H-pyrrolo[2,3-b]pyridin-4-yl}benzenesulfonamide;
N-(1,1-dioxidotetrahydro-2H-thiopyran-4-yl)-4-{2-[2-oxo-2-(1-pyrrolidinyl)ethyl]-1H-pyrrolo[2,3-b]pyridin-4-yl}benzenesulfonamide;
4-[4-(1-pyrrolidinylsulfonyl)phenyl]-2-(2H-1,2,3-tri,azol-2-ylmethyl)-1H-pyrrolo[2,3-b]pyridine;
4-[4-(1-pyrrolidinylsulfonyl)phenyl]-2-(1H-tetrazol-1-ylmethyl)-1H-pyrrolo[2,3-b]pyridine;
4-[4-(1-pyrrolidinylsulfonyl)phenyl]-2-(2H-tetrazol-2-ylmethyl)-1H-pyrrolo[2,3-b]pyridine;
4-{4-[4-(1-pyrrolidinylsulfonyl)phenyl]-1H-pyrrolo[2,3-b]pyridin-2-yl}-1-butanol;
4-[(3-{4-[4-(1-pyrrolidinylsulfonyl)phenyl]-1H-pyrrolo[2,3-b]pyridin-2-yl}-2-propyn-1-yl)oxy]benzoic acid;
N-[(2S)-2-hydroxypropyl]-4-(2-methyl-1H-pyrrolo[2,3-b]pyridin-4-yl)benzenesulfonamide;
4-(2-methyl-1H-pyrrolo[2,3-b]pyridin-4-yl)-N-4-piperidinylbenzenesulfonamide;
N-[2-(methylamino)ethyl]-4-(2-methyl-1H-pyrrolo[2,3-b]pyridin-4-yl)benzenesulfonamide;
N-[(2R)-2-hydroxypropyl]-4-(2-methyl-1H-pyrrolo[2,3-b]pyridin-4-yl)benzenesulfonamide;
N-[(1-aminocyclopentyl)methyl]-4-(2-methyl-1H-pyrrolo[2,3-b]pyridin-4-yl)benzenesulfonamide;
N-(2-hydroxy-2-methylpropyl)-4-(2-methyl-1H-pyrrolo[2,3-b]pyridin-4-yl)benzenesulfonamide;
N-(3-hydroxypropyl)-4-(2-methyl-1H-pyrrolo[2,3-b]pyridin-4-yl)benzenesulfonamide;
N-[(1S,2S)-2-hydroxycyclohexyl]-4-(2-methyl-1H-pyrrolo[2,3-b]pyridin-4-yl)benzenesulfonamide;
N-[(1S,2R)-2-hydroxycyclopentyl]-4-(2-methyl-1H-pyrrolo[2,3-b]pyridin-4-yl)benzenesulfonamide;
N-(3-aminopropyl)-4-(2-methyl-1H-pyrrolo[2,3-b]pyridin-4-yl)benzenesulfonamide;
N-(1,1-dioxidotetrahydro-2H-thiopyran-4-yl)-4-[2-(1-hydroxyethyl)-1H-pyrrolo[2,3-b]pyridin-4-yl]benzenesulfonamide;
N-(2-hydroxyethyl)-4-[2-(1-hydroxyethyl)-1H-pyrrolo[2,3-b]pyridin-4-yl]benzenesulfonamide;
1-{4-[4-(1-pyrrolidinylsulfonyl)phenyl]-1H-pyrrolo[2,3-b]pyridin-2-yl}ethanol;
N-(1,1-dioxidotetrahydro-2H-thiopyran-4-yl)-4-[2-(1H-1,2,3-triazol-1-ylmethyl)-1H-pyrrolo[2,3-b]pyridin-4-yl]benzenesulfonamide;
N-(2-hydroxyethyl)-4-[2-(1H-1,2,3-triazol-1-ylmethyl)-1H-pyrrolo[2,3-b]pyridin-4-yl]benzenesulfonamide;
N-(2-hydroxyethyl)-4-{2-[2-oxo-2-(1-pyrrolidinyl)ethyl]-1H-pyrrolo[2,3-b]pyridin-4-yl}benzenesulfonamide;
2-[2-oxo-2-(1-pyrrolidinyl)ethyl]-4-[4-(1-pyrrolidinylsulfonyl)phenyl]-1H-pyrrolo[2,3-b]pyridine;
N-(2-aminoethyl)-4-[2-(1H-1,2,3-triazol-1-ylmethyl)-1H-pyrrolo[2,3-b]pyridin-4-yl]benzenesulfonamide;
N-(2-aminoethyl)-4-{2-[2-oxo-2-(1-pyrrolidinyl)ethyl]-1H-pyrrolo[2,3-b]pyridin-4-yl}benzenesulfonamide;
N-(1,1-dioxidotetrahydro-2H-thiopyran-4-yl)-4-{2-[2-oxo-2-(1-pyrrolidinyl)ethyl]-1H-pyrrolo[2,3-b]pyridin-4-yl}benzenesulfonamide;
N-(2-aminoethyl)-4-[2-(1-hydroxyethyl)-1H-pyrrolo[2,3-b]pyridin-4-yl]benzenesulfonamide;
1-{4-[4-(1-pyrrolidinylsulfonyl)phenyl]-1H-pyrrolo[2,3-b]pyridin-2-yl}ethanol;
N-[4-(2-methyl-1H-pyrrolo[2,3-b]pyridin-4-yl)phenyl]methanesulfonamide;
N-methyl-N-[4-(2-methyl-1H-pyrrolo[2,3-b]pyridin-4-yl)phenyl]methanesulfonamide;
N-(1,1-dioxidotetrahydro-2H-thiopyran-4-yl)-N-methyl-4-(2-methyl-1H-pyrrolo[2,3-b]pyridin-4-yl)benzenesulfonamide;
({4-[4-(1-pyrrolidinylsulfonyl)phenyl]-1H-pyrrolo[2,3-b]pyridin-2-yl}methyl)amine;
1-{4-[4-(1-pyrrolidinylsulfonyl)phenyl]-1H-pyrrolo[2,3-b]pyridin-2-yl}methanamine;
N-(1,1-dioxidotetrahydro-3-thienyl)-4-(2-methyl-1H-pyrrolo[2,3-b]pyridin-4-yl)benzenesulfonamide;
N-(2-hydroxyethyl)-4-(2-methyl-1H-pyrrolo[2,3-b]pyridin-4-yl)benzenesulfonamide;
N-methyl-N-[4-(2-methyl-1H-pyrrolo[2,3-b]pyridin-4-yl)phenyl]methanesulfonamide;
4-[2-(1,1-dimethylethyl)-1H-pyrrolo[2,3-b]pyridin-4-yl]-N-(1,1-dioxidotetrahydro-3-thienyl)benzenesulfonamide;
4-[2-(1,1-dimethylethyl)-1H-pyrrolo[2,3-b]pyridin-4-yl]-N-(1,1-dioxidotetrahydro-2H-thiopyran-4-yl)benzenesulfonamide;
2-(1,1-dimethylethyl)-4-[4-(1-pyrrolidinylsulfonyl)phenyl]-1H-pyrrolo[2,3-b]pyridine; N-(2-aminoethyl)-4-[2-(1,1-dimethylethyl)-1H-pyrrolo[2,3-b]pyridin-4-yl]benzenesulfonamide;
4-[2-(1,1-dimethylethyl)-1H-pyrrolo[2,3-b]pyridin-4-yl]-N-(2-hydroxyethyl)benzenesulfonamide;
N-(2-aminoethyl)-4-[2-(difluoromethyl)-1H-pyrrolo[2,3-b]pyridin-4-yl]benzenesulfonamide;
N-(2-aminoethyl)-4-[2-(trifluoromethyl)-1H-pyrrolo[2,3-b]pyridin-4-yl]benzenesulfonamide;
N-(1-methylethyl)-2-(4-{4-[(methylsulfonyl)amino]phenyl}-1H-pyrrolo[2,3-b]pyridin-2-yl)acetamide;
N-({4-[4-(1-pyrrolidinylsulfonyl)phenyl]-1H-pyrrolo[2,3-b]pyridin-2-yl}methyl)-3-pyridinamine;
N-4-piperidinyl-4-[2-(trifluoromethyl)-1H-pyrrolo[2,3-b]pyridin-4-yl]benzenesulfonamide;
4-[2-(1-methylethyl)-1H-pyrrolo[2,3-b]pyridin-4-yl]-N-4-piperidinylbenzenesulfonamide;
1-({4-[2-(trifluoromethyl)-1H-pyrrolo[2,3-b]pyridin-4-yl]phenyl}sulfonyl)-4-piperidinamine;
1-({4-[2-(1-methylethyl)-1H-pyrrolo[2,3-b]pyridin-4-yl]phenyl}sulfonyl)-4-piperidinamine;
4-[2-(1,1-dimethylethyl)-1H-pyrrolo[2,3-b]pyridin-4-yl]-N-4-piperidinylbenzenesulfonamide;
4-(2-cyclopropyl-1H-pyrrolo[2,3-b]pyridin-4-yl)-N-(2-hydroxyethyl)-benzenesulfonamide;
N-(2-aminoethyl)-4-(2-ethyl-1H-pyrrolo[2,3-b]pyridin-4-yl)benzene-sulfonamide;
4-[2-(1,1-dimethylethyl)-1H-pyrrolo[2,3-b]pyridin-4-yl]-N-(1,1-dioxidotetrahydro-3-thienyl)benzenesulfonamide;
4-(2-cyclobutyl-1H-pyrrolo[2,3-b]pyridin-4-yl)-N-(1,1-dioxidotetrahydro-3-thienyl)benzenesulfonamide;
4-(2-cyclobutyl-1H-pyrrolo[2,3-b]pyridin-4-yl)-N-(1,1-dioxidotetrahydro-2H-thiopyran-4-yl)benzenesulfonamide;
2-cyclobutyl-4-[4-(1-pyrrolidinylsulfonyl)phenyl]-1H-pyrrolo[2,3-b]pyridine;
4-(2-cyclobutyl-1H-pyrrolo[2,3-b]pyridin-4-yl)-N-(2-hydroxyethyl)benzenesulfonamide;
N-(1,1-dioxidotetrahydro-3-thienyl)-4-[2-(1-methylethyl)-1H-pyrrolo[2,3-b]pyridin-4-yl]benzenesulfonamide;
N-(2-hydroxyethyl)-4-[2-(1-methylethyl)-1H-pyrrolo[2,3-b]pyridin-4-yl]benzenesulfonamide;
4-[2-(difluoromethyl)-1H-pyrrolo[2,3-b]pyridin-4-yl]-N-(2-hydroxyethyl)benzenesulfonamide;
N-(1,1-dioxidotetrahydro-3-thienyl)-4-(2-ethyl-1H-pyrrolo[2,3-b]pyridin-4-yl)benzenesulfonamide;
N-(1,1-dioxidotetrahydro-2H-thiopyran-4-yl)-4-(2-ethyl-1H-pyrrolo[2,3-b]pyridin-4-yl)benzenesulfonamide;
4-(2-ethyl-1H-pyrrolo[2,3-b]pyridin-4-yl)-N-(2-hydroxyethyl)benzenesulfonamide;
4-[2-(difluoromethyl)-1H-pyrrolo[2,3-b]pyridin-4-yl]-N-(2-hydroxyethyl)benzenesulfonamide;
N-(2-hydroxyethyl)-4-[2-(trifluoromethyl)-1H-pyrrolo[2,3-b]pyridin-4-yl]benzenesulfonamide;
4-(2-cyclopropyl-1H-pyrrolo[2,3-b]pyridin-4-yl)-N-(1,1-dioxidotetrahydro-3-thienyl)benzenesulfonamide;
4-(2-cyclopropyl-1H-pyrrolo[2,3-b]pyridin-4-yl)-N-(1,1-dioxidotetrahydro-2H-thiopyran-4-yl)benzenesulfonamide;
2-cyclopropyl-4-[4-(1-pyrrolidinylsulfonyl)phenyl]-1H-pyrrolo[2,3-b]pyridine;
N-4-piperidinyl-4-[2-(1H-1,2,3-triazol-1-ylmethyl)-1H-pyrrolo[2,3-b]pyridin-4-yl]benzenesulfonamide;
N-cyclohexyl-N-methyl-4-[2-(1H-1,2,3-triazol-1-ylmethyl)-1H-pyrrolo[2,3-b]pyridin-4-yl]benzenesulfonamide;
N-(2-hydroxyethyl)-N-methyl-4-[2-(1H-1,2,3-triazol-1-ylmethyl)-1H-pyrrolo[2,3-b]pyridin-4-yl]benzenesulfonamide;
N-methyl-N-(1-methyl-4-piperidinyl)-4-[2-(1H-1,2,3-triazol-1-ylmethyl)-1H-pyrrolo[2,3-b]pyridin-4-yl]benzenesulfonamide;
N-cyclohexyl-4-[2-(1H-1,2,3-triazol-1-ylmethyl)-1H-pyrrolo[2,3-b]pyridin-4-yl]benzenesulfonamide;
N-cyclopentyl-4-[2-(1H-1,2,3-triazol-1-ylmethyl)-1H-pyrrolo[2,3-b]pyridin-4-yl]benzenesulfonamide;
N-(1-methyl-4-piperidinyl)-4-[2-(1H-1,2,3-triazol-1-ylmethyl)-1H-pyrrolo[2,3-b]pyridin-4-yl]benzenesulfonamide;
N-(4-hydroxycyclohexyl)-4-[2-(1H-1,2,3-triazol-1-ylmethyl)-1H-pyrrolo[2,3-b]pyridin-4-yl]benzenesulfonamide;
N-(1-methylethyl)-4-[2-(1H-1,2,3-triazol-1-ylmethyl)-1H-pyrrolo[2,3-b]pyridin-4-yl]benzenesulfonamide;
N-(1,1-dimethylethyl)-4-[2-(1H-1,2,3-triazol-1-ylmethyl)-1H-pyrrolo[2,3-b]pyridin-4-yl]benzenesulfonamide;
N-butyl-4-[2-(1H-1,2,3-triazol-1-ylmethyl)-1H-pyrrolo[2,3-b]pyridin-4-yl]benzenesulfonamide;
N,N-dimethyl-4-[2-(1H-1,2,3-triazol-1-ylmethyl)-1H-pyrrolo[2,3-b]pyridin-4-yl]benzenesulfonamide;
N-(2-aminoethyl)-4-[2-(1,1-dimethylethyl)-1H-pyrrolo[2,3-b]pyridin-4-yl]benzenesulfonamide;
N-(2-aminoethyl)-4-(2-cyclobutyl-1H-pyrrolo[2,3-b]pyridin-4-yl)benzenesulfonamide;
N-(2-aminoethyl)-4-(2-cyclobutyl-1H-pyrrolo[2,3-b]pyridin-4-yl)benzenesulfonamide;
N-(2-aminoethyl)-4-[2-(1-methylethyl)-1H-pyrrolo[2,3-b]pyridin-4-yl]benzenesulfonamide;
N-(2-aminoethyl)-4-(2-cyclopropyl-1H-pyrrolo[2,3-b]pyridin-4-yl)benzenesulfonamide;
N-(3-aminopropyl)-4-[2-(1H-1,2,3-triazol-1-ylmethyl)-1H-pyrrolo[2,3-b]pyridin-4-yl]benzenesulfonamide;
N-[(1-aminocyclopentyl)methyl]-4-[2-(1H-1,2,3-triazol-1-ylmethyl)-1H-pyrrolo[2,3-b]pyridin-4-yl]benzenesulfonamide;
N-(3-aminopropyl)-N-methyl-4-[2-(1H-1,2,3-triazol-1-ylmethyl)-1H-pyrrolo[2,3-b]pyridin-4-yl]benzenesulfonamide;
N-(2-hydroxyethyl)-N-methyl-4-(2-methyl-1H-pyrrolo[2,3-b]pyridin-4-yl)benzenesulfonamide;
N-(2-hydroxyethyl)-N-methyl-4-(2-methyl-1H-pyrrolo[2,3-b]pyridin-4-yl)benzenesulfonamide;
N-methyl-N-(1-methyl-4-piperidinyl)-4-(2-methyl-1H-pyrrolo[2,3-b]pyridin-4-yl)benzenesulfonamide;
N-cyclohexyl-4-(2-methyl-1H-pyrrolo[2,3-b]pyridin-4-yl)benzenesulfonamide;
N-cyclopentyl-4-(2-methyl-1H-pyrrolo[2,3-b]pyridin-4-yl)benzenesulfonamide;
N-(1-methyl-4-piperidinyl)-4-(2-methyl-1H-pyrrolo[2,3-b]pyridin-4-yl)benzenesulfonamide;
N-cyclohexyl-N-methyl-4-(2-methyl-1H-pyrrolo[2,3-b]pyridin-4-yl)benzenesulfonamide;
N-(1-methylethyl)-4-(2-methyl-1H-pyrrolo[2,3-b]pyridin-4-yl)benzenesulfonamide;
N-(1,1-dimethylethyl)-4-(2-methyl-1H-pyrrolo[2,3-b]pyridin-4-yl)benzenesulfonamide;
N-butyl-4-(2-methyl-1H-pyrrolo[2,3-b]pyridin-4-yl)benzenesulfonamide;
N-cyclohexyl-4-[2-(difluoromethyl)-1H-pyrrolo[2,3-b]pyridin-4-yl]-N-methylbenzenesulfonamide;
4-[2-(difluoromethyl)-1H-pyrrolo[2,3-b]pyridin-4-yl]-N-[(1S,2S)-2-hydroxycyclohexyl]benzenesulfonamide;
4-[2-(difluoromethyl)-1H-pyrrolo[2,3-b]pyridin-4-yl]-N-(1-methylethyl)benzenesulfonamide;
4-[2-(difluoromethyl)-1H-pyrrolo[2,3-b]pyridin-4-yl]-N-(1,1-dimethylethyl)benzenesulfonamide;
N-butyl-4-[2-(difluoromethyl)-1H-pyrrolo[2,3-b]pyridin-4-yl]benzenesulfonamide;
4-[2-(difluoromethyl)-1H-pyrrolo[2,3-b]pyridin-4-yl]-N,N-dimethylbenzenesulfonamide;
N-(4-hydroxycyclohexyl)-4-(2-methyl-1H-pyrrolo[2,3-b]pyridin-4-yl)benzenesulfonamide;
N,N-dimethyl-4-(2-methyl-1H-pyrrolo[2,3-b]pyridin-4-yl)benzenesulfonamide;
4-[2-(difluoromethyl)-1H-pyrrolo[2,3-b]pyridin-4-yl]-N-(2-hydroxyethyl)-N-methylbenzenesulfonamide;
4-[2-(difluoromethyl)-1H-pyrrolo[2,3-b]pyridin-4-yl]-N-methyl-N-(1-methyl-4-piperidinyl)benzenesulfonamide;
N-cyclohexyl-4-[2-(difluoromethyl)-1H-pyrrolo[2,3-b]pyridin-4-yl]benzenesulfonamide;
N-cyclopentyl-4-[2-(difluoromethyl)-1H-pyrrolo[2,3-b]pyridin-4-yl]benzenesulfonamide;
4-[2-(difluoromethyl)-1H-pyrrolo[2,3-b]pyridin-4-yl]-N-(4-hydroxycyclohexyl)benzenesulfonamide;
N-cyclopentyl-4-[2-(difluoromethyl)-1H-pyrrolo[2,3-b]pyridin-4-yl]-N-methylbenzenesulfonamide;
4-[2-(difluoromethyl)-1H-pyrrolo[2,3-b]pyridin-4-yl]-N-(2-hydroxyethyl)benzenesulfonamide;
4-[2-(difluoromethyl)-1H-pyrrolo[2,3-b]pyridin-4-yl]-N-(1-methyl-4-piperidinyl)benzenesulfonamide;
N-(3-aminopropyl)-N-methyl-4-(2-methyl-1H-pyrrolo[2,3-b]pyridin-4-yl)benzenesulfonamide;
N-(3-aminopropyl)-4-[2-(difluoromethyl)-1H-pyrrolo[2,3-b]pyridin-4-yl]-N-methylbenzenesulfonamide;
N-(3-aminopropyl)-4-[2-(difluoromethyl)-1H-pyrrolo[2,3-b]pyridin-4-yl]benzenesulfonamide;
4-[2-(difluoromethyl)-1H-pyrrolo[2,3-b]pyridin-4-yl]-N-4-piperidinylbenzenesulfonamide;
N-[(1-aminocyclopentyl)methyl]-4-[2-(difluoromethyl)-1H-pyrrolo[2,3-b]pyridin-4-yl]benzenesulfonamide;
2-{[(5-methyl-3-pyridinyl)oxy]methyl}-4-[4-(1-pyrrolidinylsulfonyl)phenyl]-1H-pyrrolo[2,3-b]pyridine; .
1,1-dimethylethyl {2-[({4-[2-(difluoromethyl)-1H-pyrrolo[2,3-b]pyridin-4-yl]phenyl}sulfonyl)amino]ethyl}carbamate;
1,1-dimethylethyl [2-({[4-(2-ethyl-1H-pyrrolo[2,3-b]pyridin-4-yl)phenyl]sulfonyl}amino)ethyl]carbamate; or
a salt thereof.

In another embodiment, the compound of the invention is:
1,1-dimethylethyl [4-(4-{[(1,1-dioxidotetrahydro-3-thienyl)amino]sulfonyl}phenyl)-1*H-*pyrrolo[2,3-*b*]pyridin-2-yl]acetate;
1,1-dimethylethyl[4-(4-{[(1,1-dioxidotetrahydro-2*H*-thiopyran-4-yl)amino]sulfonyl}phenyl)-1H-pyrrolo[2,3-*b*]pyridin-2-yl]acetate;
N-(1,1-dioxidotetrahydro-2H-thiopyran-4-yl)-4-(2-methyl-1H-pyrrolo[2,3-b]pyridin-4-yl)benzenesulfonamide;
4-[2-(difluoromethyl)-1*H*-pyrrolo[2,3-*b*]pyridin-4-yl]-N-(1,1-dioxidotetrahydro-3-thienyl)benzenesulfonamide;
*N*-(1,1-dioxidotetrahydro-2*H*-thiopyran-4-yl)-4-[2-(1-methylethyl)-1*H*-pyrrolo[2,3-*b*]pyridin-4-yl]benzenesulfonamide;
*N*-(1,1-dioxidotetrahydro-2*H*-thiopyran-4-yl)-4-[2-(trifluoromethyl)-1*H*-pyrrolo[2,3-*b*]pyridin-4-yl]benzenesulfonamide;
*N*-(1,1-dioxidotetrahydro-3-thienyl)-4-[2-(trifluoromethyl)-1*H*-pyrrolo[2,3-*b*]pyridin-4-yl]benzenesulfonamide; or
a salt thereof.

In another embodiment, the compound of the invention is:
4-(2-cyclopropyl-1*H*-pyrrolo[2,3-*b*]pyridin-4-yl)-*N*-(2-hydroxyethyl)-benzenesulfonamide;
*N*-(2-aminoethyl)-4-(2-ethyl-1*H*-pyrrolo[2,3-*b*]pyridin-4-yl)benzenesulfonamide;
4-(2-cyclopropyl-1*H*-pyrrolo[2,3-*b*]pyridin-4-yl)-*N*-(1,1-dioxidotetrahydro-2*H-*thiopyran-4-yl)benzenesulfonamide;
*N*-[(1*S*)-2-hydroxy-1-methylethyl]-4-[2-(trifluoromethyl)-1*H*-pyrrolo[2,3-*b*]pyridin-4-yl]benzenesulfonamide;
4-(2-cyclopropyl-1*H*-pyrrolo[2,3-*b*]pyridin-4-yl)-N-(2-hydroxy-2-methylpropyl)benzenesulfonamide;
4-(2-cyclopropyl-1H-pyrrolo[2,3-b]pyridin-4-yl)-N-[(1S)-2-hydroxy-1-methylethyl]benzenesulfonamide;
4-{2-[(dimethylamino)methyl]-1*H*-pyrrolo[2,3-*b*]pyridin-4-yl}-*N*-[(3*R*)-1,1-dioxidotetrahydro-3-thienyl]benzenesulfonamide;
or a salt thereof.

In another embodiment, the compound of the invention is:
4-(2-cyclopropyl-1*H*-pyrrolo[2,3-*b*]pyridin-4-yl)-*N*-(2-hydroxyethyl)-benzenesulfonamide; or a salt thereof.

In another embodiment, the compound of the invention is:
4-(2-cyclopropyl-1*H*-pyrrolo[2,3-*b*]pyridin-4-yl)-*N*-(2-hydroxyethyl)-benzenesulfonamide.

In another embodiment, the compound of the invention is:
*N*-(2-aminoethyl)-4-(2-ethyl-1*H*-pyrrolo[2,3-*b*]pyridin-4-yl)benzene-sulfonamide; or a salt thereof.

In another embodiment, the compound of the invention is:
*N*-(2-aminoethyl)-4-(2-ethyl-1*H*-pyrrolo[2,3-*b*]pyridin-4-yl)benzene-sulfonamide.

In a further embodiment, the compound of the invention is:
*N*-(2-aminoethyl)-4-(2-ethyl-1*H*-pyrrolo[2,3-*b*]pyridin-4-yl)benzene-sulfonamide hydrochloride.

### Terms and Definitions

**"Alkyl"** refers to a saturated hydrocarbon chain having the specified number of member atoms. For example, C₁₋₆alkyl refers to an alkyl group having from 1 to 6 member atoms, for example 1 to 4 members. Alkyl groups may be optionally substituted with one or more substituents as defined herein. Alkyl groups may be straight or branched. Representative branched alkyl groups have one, two, or three branches. Alkyl includes methyl, ethyl, propyl (n-propyl and isopropyl), butyl (n-butyl, isobutyl, and t-butyl), pentyl (n-pentyl, isopentyl, and neopentyl), and hexyl. In one embodiment, alkyl is methyl. In a further embodiment, alkyl is ethyl.

**"Cycloalkyl"** refers to a saturated hydrocarbon ring having the specified number of member atoms. Cycloalkyl groups are monocyclic ring systems. For example, C₃₋₆ cycloalkyl refers to a cycloalkyl group having from 3 to 6 member atoms. In one embodiment, the cycloalkyl groups have 3 or 4 member atoms. In a further embodiment, the cycloalkyl groups have 5 or 6 member atoms. Cycloalkyl groups may be optionally substituted with one or more substituents as defined herein. It will be appreciated that the substituent may be at any position on the ring, including the carbon atom which is the point of attachment to the rest of the molecule. Cycloalkyl includes cyclopropyl, cyclobutyl, cyclopentyl, and cyclohexyl. In one embodiment, cycloalkyl is cyclopropyl.

**"Enantiomerically enriched"** refers to products whose enantiomeric excess is greater than zero. For example, enantiomerically enriched refers to products whose enantiomeric excess is greater than 50% ee, greater than 75% ee, and greater than 90% ee.

**"Enantiomeric excess"** or **"ee"** is the excess of one enantiomer over the other expressed as a percentage. As a result, since both enantiomers are present in equal amounts in a racemic mixture, the enantiomeric excess is zero (0% ee). However, if one enantiomer was enriched such that it constitutes 95% of the product, then the enantiomeric excess would be 90% ee (the amount of the enriched enantiomer, 95%, minus the amount of the other enantiomer, 5%).

**"Enantiomerically pure"** refers to products whose enantiomeric excess is 99% ee or greater.

**"Half-life"** (or "half-lives") refers to the time required for half of a quantity of a substance to be converted to another chemically distinct species *in vitro* or *in vivo*.

**"Heteroaryl",** unless otherwise defined, refers to an aromatic ring containing from 1 to 4 heteroatoms as member atoms in the ring. Heteroaryl groups containing more than one heteroatom may contain different heteroatoms. Heteroaryl groups may be optionally substituted with one or more substituents as defined herein. Heteroaryl groups are monocyclic ring systems or are fused bicyclic ring systems. Monocyclic heteroaryl rings have 5 or 6 member atoms. Bicyclic heteroaryl rings have from 7 to 11 member atoms. Bicyclic heteroaryl rings include those rings wherein phenyl and a monocyclic heterocyclyl ring are attached forming a fused bicyclic ring system, and those rings wherein a monocyclic heteroaryl ring and a monocyclic cycloalkyl, cycloalkenyl, heterocyclyl, or heteroaryl ring are attached forming a fused bicyclic ring system. Heteroaryl includes pyrrolyl, pyrazolyl, imidazolyl, oxazolyl, isoxazolyl, thiazolyl, isothiazolyl, furanyl, furazanyl, thienyl, triazolyl, pyridinyl, pyrimidinyl, pyridazinyl, pyrazinyl, triazinyl, tetrazinyl, indolyl, isoindolyl, indolizinyl, indazolyl, purinyl, quinolinyl, isoquinolinyl, quinoxalinyl, quinazolinyl, pteridinyl, cinnolinyl, benzimidazolyl, benopyranyl, benzoxazolyl, benzofuranyl, isobenzofuranyl, benzothiazolyl, benzothienyl, furopyridinyl, and napthyridinyl. For example, 5-membered heteroaryl groups having from 1 to 4 nitrogen atoms include pyrrolyl, pyrazolyl, imidazolyl, triazolyl (including 1,2,3-triazolyl and 1,2,4-triazolyl) and tetrazolyl.

**"Heteroatom"** refers to a nitrogen, sulphur, or oxygen atom.

**"Heterocyclyl",** unless otherwise defined, refers to a saturated or unsaturated ring containing from 1 to 4 heteroatoms as member atoms in the ring. However, heterocyclyl rings are not aromatic. Heterocyclyl groups containing more than one heteroatom may contain different heteroatoms. Heterocyclyl groups may be optionally substituted with one or more substituents as defined herein. Heterocyclyl groups are monocyclic ring systems having from 4 to 7 member atoms. In certain embodiments, heterocyclyl is saturated. In other embodiments, heterocyclyl is unsaturated but not aromatic. Heterocyclyl includes pyrrolidinyl, tetrahydrofuranyl, dihydrofuranyl, pyranyl, tetrahydropyranyl, dihydropyranyl, tetrahydrothienyl, pyrazolidinyl, oxazolidinyl, thiazolidinyl, piperidinyl, homopiperidinyl, piperazinyl, morpholinyl, thiamorpholinyl, 1,3-dioxolanyl, 1,3-dioxanyl, 1,4-dioxanyl, 1,3-oxathiolanyl, 1,3-oxathianyl, 1,3-dithianyl, and azetidinyl. In one embodiment, heterocyclyl is piperidinyl. In a further embodiment, heterocyclyl is piperazinyl.

**"Member atoms"** refers to the atom or atoms that form a chain or ring. Where more than one member atom is present in a chain and within a ring, each member atom is covalently bound to an adjacent member atom in the chain or ring. Atoms that make up a substituent group on a chain or ring are not member atoms in the chain or ring.

**"Optionally substituted"** indicates that a group, such as heterocyclyl, may be unsubstituted or substituted with one or more substituents as defined herein.

**"Substituted"** in reference to a group indicates that a hydrogen atom attached to a member atom within a group is replaced. It should be understood that the term "substituted" includes the implicit provision that such substitution be in accordance with the permitted valence of the substituted atom and the substituent and that the substitution results in a stable compound (i.e. one that does not spontaneously undergo transformation such as by rearrangement, cyclization, or elimination). In certain embodiments, a single atom may be substituted with more than one substituent as long as such substitution is in accordance with the permitted valence of the atom. Suitable substituents are defined herein for each substituted or optionally substituted group.

**"Pharmaceutically acceptable"** refers to those compounds, materials, compositions, and dosage forms which are, within the scope of sound medical judgment, suitable for use in contact with the tissues of human beings and animals without excessive toxicity, irritation, or other problem or complication, commensurate with a reasonable benefit/risk ratio.

As used herein the symbols and conventions used in these processes, schemes and examples are consistent with those used in the contemporary scientific literature, for example, the *Journal of the American Chemical Society* or the *Journal of Biological Chemistry.* Standard single-letter or three-letter abbreviations are generally used to designate amino acid residues, which are assumed to be in the L-configuration unless otherwise noted. Unless otherwise noted, all starting materials were obtained from commercial suppliers and used without further purification. Specifically, the following abbreviations may be used in the examples and throughout the specification:
Ac (acetyl);
Aq (aqueous);
ATP (adenosine triphosphate);
BOC (*tert*-butyloxycarbonyl);
BSA (bovine serum albumin);
Bu (butyl);
nBu (n-butyl);
tBu (t-butyl);
CHAPS (3[(3-Cholamidopropyl)dimethylammonio]-propanesulfonic acid);
DCE (dichloroethane);
DCM (dichloromethane);
DIAD (diisopropyl azodicarboxylate);
DIPEA (diisopropylethylamine);
DMF (*N,N*-dimethylformamide);
DMSO (dimethylsulfoxide);
dppf (1,1'-bis(diphenylphosphino)ferrocene);
DTT (1,4-dithiothreitol);
EDTA (ethylenediaminetetraacetic acid);
Et (ethyl);
EtOAc (ethyl acetate);
g (grams);
HATU (O-(7azabenzobenzotriazol-1-yl)-N,N,N',N'-tetramethyluronium hexafluorophosphate);
HOBT (1-hydroxybenzotriazole);
HPLC (high pressure liquid chromatography);
H, hr or hrs (hours);
Hz (Hertz);
IMS (industrial methylated spirits);
L (liters);
LDA (lithium diisopropylamide);
M (molar);
MCPBA (meta-chloroperbenzoic acid);
MDAP (mass directed autopreparative HPLC);
Me (methyl);
MeOH (methanol);
mg (milligrams);
MHz (megahertz);
Min or mins (minutes);
ml or mL(milliliters);
mw (microwave);
µl (microliters);
mM (millimolar);
mmol (millimoles);
mol (moles);
mp (melting point);
MTBE (methyl tertiary butyl ether);
Ph (phenyl);
ⁱPr (isopropyl);
rt (retention time);
SPE (solid phase extraction);
TBAF (tetra-n-butylammonium fluoride);
TBTU (O-(benzotriazol-1-yl)-N,N,N',N'-tetramethyluronium tetrafluoroborate);
TEA (triethylamine);
TFA (trifluoroacetic acid);
THF (tetrahydrofuran);
TLC (thin layer chromatography);
TMS (trimethylsilyl);
Tos (tosyl or p-toluenesulfonyl);
*p*TSA (p-toluenesulfonic acid); and
WSCDI (water soluble carbodiimide).

All references to ether are to diethyl ether and brine refers to a saturated aqueous solution of NaCl.

Included within the scope of the "compounds of the invention" are all solvates, hydrates, complexes, polymorphs, prodrugs, radiolabelled derivatives, stereoisomers and optical isomers of the compounds of formula (I) and salts thereof.

The compounds of the invention may exist in solid or liquid form. In the solid state, the compounds of the invention may exist in crystalline or noncrystalline form, or as a mixture thereof. For compounds of the invention that are in crystalline form, the skilled artisan will appreciate that pharmaceutically acceptable solvates may be formed wherein solvent molecules are incorporated into the crystalline lattice during crystallization. Solvates may involve nonaqueous solvents such as ethanol, isopropanol, DMSO, acetic acid, ethanolamine, and EtOAc, or they may involve water as the solvent that is incorporated into the crystalline lattice. Solvates wherein water is the solvent that is incorporated into the crystalline lattice are typically referred to as "hydrates." Hydrates include stoichiometric hydrates as well as compositions containing variable amounts of water. The invention includes all such solvates.

The skilled artisan will further appreciate that certain compounds of the invention that exist in crystalline form, including the various solvates thereof, may exhibit polymorphism (i.e. the capacity to occur in different crystalline structures). These different crystalline forms are typically known as "polymorphs." The invention includes all such polymorphs. Polymorphs have the same chemical composition but differ in packing, geometrical arrangement, and other descriptive properties of the crystalline solid state. Polymorphs, therefore, may have different physical properties such as shape, density, hardness, deformability, stability, and dissolution properties. Polymorphs typically exhibit different melting points, IR spectra, and X-ray powder diffraction patterns, which may be used for identification. The skilled artisan will appreciate that different polymorphs may be produced, for example, by changing or adjusting the reaction conditions or reagents, used in making the compound. For example, changes in temperature, pressure, or solvent may result in polymorphs. In addition, one polymorph may spontaneously convert to another polymorph under certain conditions.

In one aspect, the present invention provides 4-(2-cyclopropyl-1*H*-pyrrolo[2,3-*b*]pyridin-4-yl)-*N*-(2-hydroxyethyl)-benzenesulfonamide or a salt thereof in crystalline form.

In one embodiment, the present invention provides 4-(2-cyclopropyl-1*H*-pyrrolo[2,3-*b*]pyridin-4-yl)-*N*-(2-hydroxyethyl)-benzenesulfonamide in crystalline form.

In another embodiment, the present invention provides crystalline 4-(2-cyclopropyl-1*H*-pyrrolo[2,3-*b*]pyridin-4-yl)-*N*-(2-hydroxyethyl)-benzenesulfonamide characterised in that it provides:
(i) a DSC (differential scanning calorimetry) thermogram having an endotherm with an onset temperature of about 188°C to about 195°C, and/or
(ii) an XRPD (X-ray powder diffraction) pattern having peaks (°2θ) at about 9.5, about 11.1, about 12.0, about 14.4 and about 22.6.

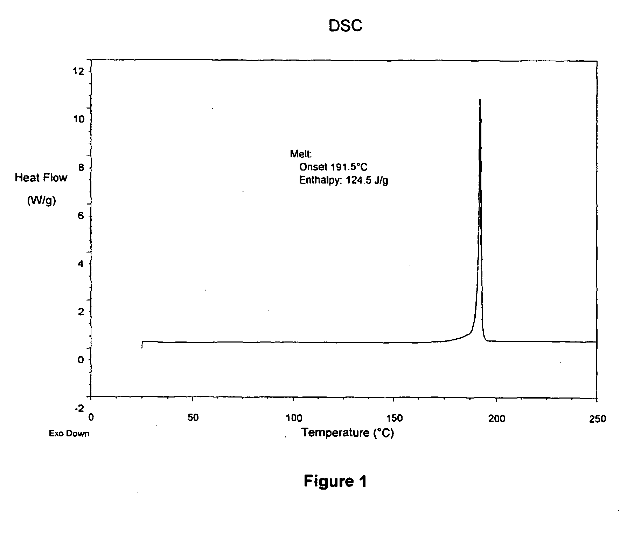

In another embodiment, the present invention provides crystalline 4-(2-cyclopropyl-1*H*-pyrrolo[2,3-*b*]pyridin-4-yl)-*N*-(2-hydroxyethyl)-benzenesulfonamide characterised in that it provides a DSC thermogram substantially in accordance with Figure 1.

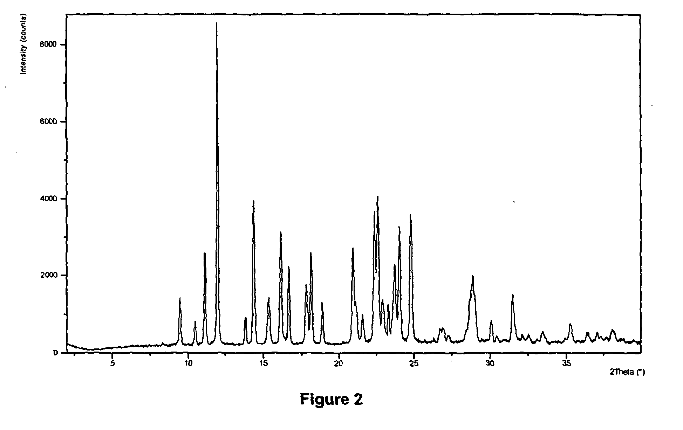

In another embodiment, the present invention provides crystalline 4-(2-cyclopropyl-1*H*-pyrrolo[2,3-*b*]pyridin-4-yl)-*N*-(2-hydroxyethyl)-benzenesulfonamide characterised in that it provides an XRPD pattern substantially in accordance with Figure 2.

In a further embodiment, the present invention provides crystalline 4-(2-cyclopropyl-1*H*-pyrrolo[2,3-*b*]pyridin-4-yl)-*N*-(2-hydroxyethyl)-benzenesulfonamide characterised in that it provides an XRPD pattern comprising peaks substantially as set out in Table 1.

In a further aspect, the present invention provides *N*-(2-aminoethyl)-4-(2-ethyl-1*H-*pyrrolo[2,3-*b*]pyridin-4-yl)benzenesulfonamide or a salt thereof in crystalline form.

In one embodiment, the present invention provides *N*-(2-aminoethyl)-4-(2-ethyl-1*H-*pyrrolo[2,3-*b*]pyridin-4-yl)benzenesulfonamide hydrochloride in crystalline form.

In another embodiment, the present invention provides crystalline *N*-(2-aminoethyl)-4-(2-ethyl-1*H*-pyrrolo[2,3-b]pyridin-4-yl)benzenesulfonamide hydrochloride characterised in that it provides:
(i) a DSC (differential scanning calorimetry) thermogram having an endotherm with an onset temperature of about 284°C to about 290°C, and/or
(ii) an XRPD (X-ray powder diffraction) pattern having peaks (°2θ) at about 7.2, about 15.2, about 18.3, about 21.2 and about 23.3.

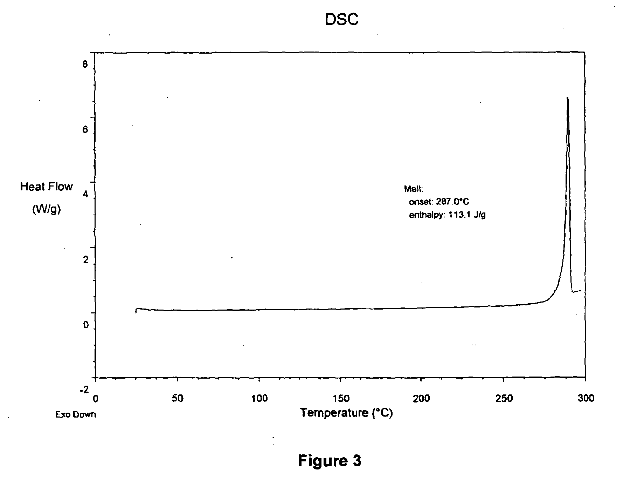

In another embodiment, the present invention provides crystalline *N*-(2-aminoethyl)-4-(2-ethyl-1*H*-pyrrolo[2,3-*b*]pyridin-4-yl)benzenesulfonamide hydrochloride characterised in that it provides a DSC thermogram substantially in accordance with Figure 3.

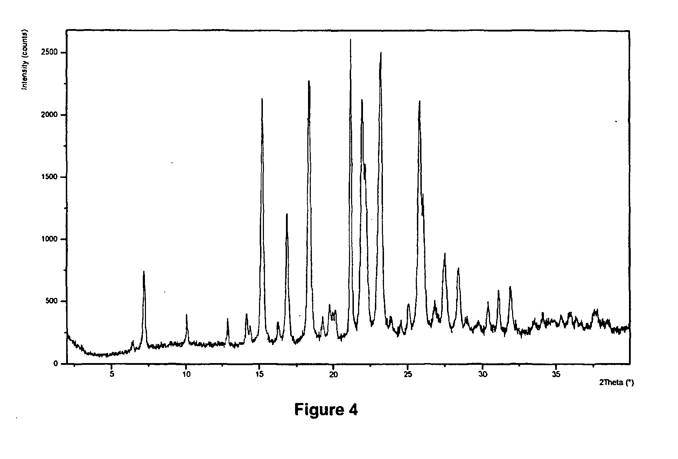

In another embodiment, the present invention provides crystalline *N*-(2-aminoethyl)-4-(2-ethyl-1*H*-pyrrolo[2,3-*b*]pyridin-4-yl)benzenesulfonamide hydrochloride characterised in that it provides an XRPD pattern substantially in accordance with Figure 4.

In a further embodiment, the present invention provides crystalline *N*-(2-aminoethyl)-4-(2-ethyl-1*H*-pyrrolo[2,3-*b*]pyridin-4-yl)benzenesulfonamide hydrochloride characterised in that it provides an XRPD pattern comprising peaks substantially as set out in Table 2.

In one embodiment, the present invention provides *N*-(2-aminoethyl)-4-(2-ethyl-1*H-*pyrrolo[2,3-*b*]pyridin-4-yl)benzenesulfonamide in crystalline form.

In another embodiment, the present invention provides crystalline *N*-(2-aminoethyl)-4-(2-ethyl-1*H*-pyrrolo[2,3-*b*]pyridin-4-yl)benzenesulfonamide characterised in that it provides:
(i) a DSC (differential scanning calorimetry) thermogram having an endotherm with an onset temperature of about 192°C to about 199°C, and/or
(ii) an XRPD (X-ray powder diffraction) pattern having peaks (°2θ) at about 8.8, about 9.0, about 15.4, about 21.2 and about 21.4.

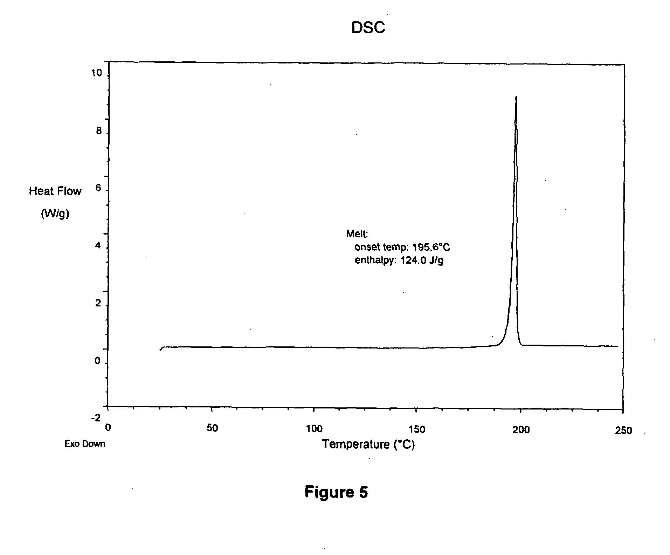

In another embodiment, the present invention provides crystalline *N*-(2-aminoethyl)-4-(2-ethyl-1*H*-pyrrolo[2,3-*b*]pyridin-4-yl)benzenesulfonamide characterised in that it provides a DSC thermogram substantially in accordance with Figure 5.

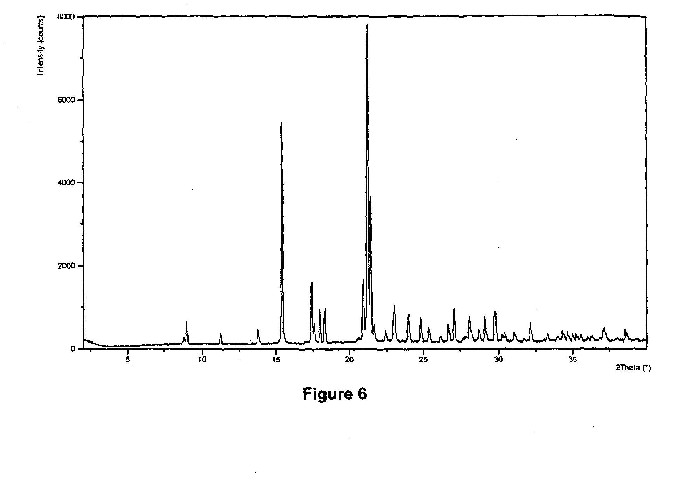

In another embodiment, the present invention provides crystalline *N*-(2-aminoethyl)-4-(2-ethyl-1*H*-pyrrolo[2,3-*b*]pyridin-4-yl)benzenesulfonamide characterised in that it provides an XRPD pattern substantially in accordance with Figure 6.

In a further embodiment, the present invention provides crystalline *N*-(2-aminoethyl)-4-(2-ethyl-1*H*-pyrrolo[2,3-*b*]pyridin-4-yl)benzenesulfonamide characterised in that it provides an XRPD pattern comprising peaks substantially as set out in Table 3.

When it is indicated herein that there is an onset temperature at a given value, it is typically meant that the temperature is within ± 1.5°C of the value quoted.

When it is indicated herein that there is a peak in an XRPD pattern at a given value, it is typically meant that the peak is within ± 0.2 of the value quoted.

The invention also includes isotopically-labelled compounds, which are identical to the compounds of formula (I) and salts thereof, but for the fact that one or more atoms are replaced by an atom having an atomic mass or mass number different from the atomic mass or mass number most commonly found in nature. Examples of isotopes that can be incorporated into the compounds of the invention include isotopes of hydrogen, carbon, nitrogen, oxygen and fluorine, such as 3H, 11C, 14C and 18F.

The compounds according to formula (I) may contain one or more asymmetric center (also referred to as a chiral center) and may, therefore, exist as individual enantiomers, diastereomers, or other stereoisomeric forms, or as mixtures thereof. Chiral centers, such as chiral carbon atoms, may also be present in a substituent such as an alkyl group. Where the stereochemistry of a chiral center present in formula (I), or in any chemical structure illustrated herein, is not specified the structure is intended to encompass any stereoisomer and all mixtures thereof. Thus, compounds according to formula (I) containing one or more chiral center may be used as racemic mixtures, enantiomerically enriched mixtures, or as enantiomerically pure individual stereoisomers.

Individual stereoisomers of a compound according to formula (I) which contain one or more asymmetric center may be resolved by methods known to those skilled in the art. For example, such resolution may be carried out (1) by formation of diastereoisomeric salts, complexes or other derivatives; (2) by selective reaction with a stereoisomer-specific reagent, for example by enzymatic oxidation or reduction; or (3) by gas-liquid or liquid chromatography in a chiral enviornment, for example, on a chiral support such as silica with a bound chiral ligand or in the presence of a chiral solvent. The skilled artisan will appreciate that where the desired stereoisomer is converted into another chemical entity by one of the separation procedures described above, a further step is required to liberate the desired form. Alternatively, specific stereoisomers may be synthesized by asymmetric synthesis using optically active reagents, substrates, catalysts or solvents, or by converting one enantiomer to the other by asymmetric transformation.

The terms enantiomer 1 and enantiomer 2 are used herein to refer to the enantiomers of a compound of the invention based on the order of their elution using the chiral chromatography methodology described herein. Enantiomer 1 refers to the first enantiomer to elute, and enantiomer 2 refers to the second enantiomer to elute.

It will be appreciated by those skilled in the art that although the absolute retention time on chromatography can be variable, the order of elution remains the same when the same column and conditions are employed. However, the use of a different chromatography column and conditions may alter the order of elution.

The compounds according to formula (I) may also contain centers of geometric asymmetry. Where the stereochemistry of a center of geometric asymmetry present in formula (I), or in any chemical structure illustrated herein, is not specified, the structure is intended to encompass the trans geometric isomer, the cis geometric isomer, and all mixtures thereof. Likewise, all tautomeric forms are also included in formula (I) whether such tautomers exist in equilibrium or predominately in one form.

It is to be understood that the references herein to compounds of formula (I) and salts thereof covers the compounds of formula (I) as the free base or as salts thereof, for example as a pharmaceutically acceptable salt thereof.

The skilled artisan will appreciate that pharmaceutically acceptable salts of the compounds according to formula (I) may be prepared. Indeed, in certain embodiments of the invention, pharmaceutically acceptable salts of the compounds according to formula (I) may be preferred over the respective free base or free acid because such salts impart greater stability or solubility to the molecule thereby facilitating formulation into a dosage form. Accordingly, the invention is further directed to compounds of formula (I) and pharmaceutically acceptable salts thereof.

As used herein, the term "pharmaceutically acceptable salts" refers to salts that retain the desired biological activity of the subject compound and exhibit minimal undesired toxicological effects. These pharmaceutically acceptable salts may be prepared *in situ* during the final isolation and purification of the compound, or by separately reacting the purified compound in its free acid or free base form with a suitable base or acid, respectively.

Salts and solvates having non-pharmaceutically acceptable counter-ions or associated solvents are within the scope of the present invention, for example, for use as intermediates in the preparation of other compounds of formula (I) and their pharmaceutically acceptable salts. Thus one embodiment of the invention embraces compounds of formula (I) and salts thereof.

In certain embodiments, compounds according to formula (I) may contain an acidic functional group. Suitable pharmaceutically-acceptable salts include salts of such acidic functional groups. Representative salts include pharmaceutically acceptable metal salts such as sodium, potassium, lithium, calcium, magnesium, aluminum, and zinc salts; carbonates and bicarbonates of a pharmaceutically acceptable metal cation such as sodium, potassium, lithium, calcium, magnesium, aluminum, and zinc; pharmaceutically acceptable organic primary, secondary, and tertiary amines including aliphatic amines, aromatic amines, aliphatic diamines, and hydroxy alkylamines such as methylamine, ethylamine, 2-hydroxyethylamine, diethylamine, TEA, ethylenediamine, ethanolamine, diethanolamine, and cyclohexylamine.

In certain embodiments, compounds according to formula (I) may contain a basic functional group and are therefore capable of forming pharmaceutically acceptable acid addition salts by treatment with a suitable acid. Suitable acids include pharmaceutically acceptable inorganic acids and pharmaceutically acceptable organic acids. Representative pharmaceutically acceptable acid addition salts include hydrochloride, hydrobromide, nitrate, methylnitrate, sulfate, bisulfate, sulfamate, phosphate, acetate, hydroxyacetate, phenylacetate, propionate, butyrate, isobutyrate, valerate, maleate, hydroxymaleate, acrylate, fumarate, malate, tartrate, citrate, salicylate, *p*-aminosalicyclate, glycollate, lactate, heptanoate, phthalate, oxalate, succinate, benzoate, o-acetoxybenzoate, chlorobenzoate, methylbenzoate, dinitrobenzoate, hydroxybenzoate, methoxybenzoate, naphthoate, hydroxynaphthoate, mandelate, tannate, formate, stearate, ascorbate, palmitate, oleate, pyruvate, pamoate, malonate, laurate, glutarate, glutamate, estolate, methanesulfonate (mesylate), ethanesulfonate (esylate), 2-hydroxyethanesulfonate, benzenesulfonate (besylate), p-aminobenzenesulfonate, p-toluenesulfonate (tosylate), and napthalene-2-sulfonate. In one embodiment, the pharmaceutically acceptable acid addition salt is a hydrochloride.

### Compound Preparation

The compounds of this invention may be made by a variety of methods, including standard chemistry. Any previously defined variable will continue to have the previously defined meaning unless otherwise indicated. Illustrative general synthetic methods are set out below and then specific compounds of the invention are prepared in the Examples section.

In one embodiment of the invention, the compounds of formula (I), and salts thereof, may be prepared by a process comprising reacting a compound of formula (IIA) or (IIB) 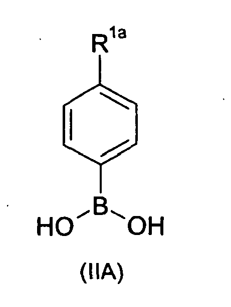 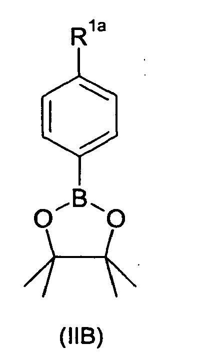 wherein R^{1a} is R¹ as defined above or a group convertible to R¹, with a compound of formula (IIIA) 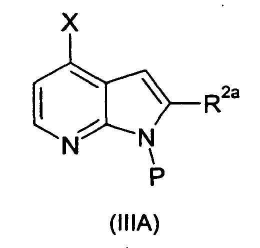 wherein P is hydrogen or a protecting group, R^{2a} is R² as defined above or a group convertible to R², and X is halogen, for example bromine or chlorine, in the presence of a catalyst, for example a palladium (II) complex.

Alternatively, the compounds of formula (I), and salts thereof, may be prepared by a process comprising reacting a compound of formula (IIC) 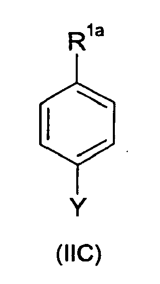 wherein R^{1a} is R¹ as defined above or a group convertible to R¹ and Y is chlorine, bromine, iodine or triflate, with a compound of formula (IIIB) or (IIIC) 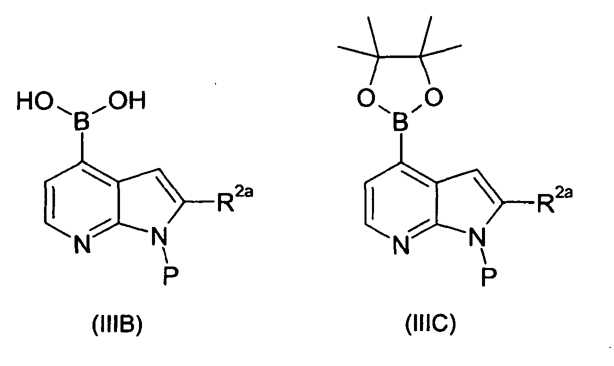 wherein P is hydrogen or a protecting group and R^{2a} is R² as defined above or a group convertible to R², in the presence of a catalyst, for example a palladium (II) complex.

The above processes may be followed, if required, by subjecting the resulting compound to one or more of the following operations:
i) removal of the protecting group P,
ii) conversion of R^{1a} to R¹,
iii) conversion of R^{2a} to R², and
iv) conversion of the resultant compound of formula (I) into a salt thereof.

A comprehensive discussion of the ways in which groups may be protected and methods for cleaving the resulting protected derivatives is given by for example T.W. Greene and P.G.M Wuts in Protective Groups in Organic Synthesis 2nd ed., John Wiley & Son, Inc., 1991 and by P.J. Kocienski in Protecting Groups, Georg Thieme Verlag, 1994. Examples of suitable protecting groups, P, include phenylsulfone and 4-methylphenylsulfone. Such protecting groups may be removed under basic conditions, for example using sodium hydroxide or potassium hydroxide.

In a further embodiment of the invention, the compounds of formula (I), and salts thereof, may be prepared by conversion of one compound of formula (I) into another compound of formula (I).

Suitable functional group transformations for converting one compound of formula (I) into another compound of formula (I), or converting R^{1a} to R¹ or R^{2a} to R², are well known in the art and are described in, for instance, Comprehensive Heterocyclic Chemistry II, eds. A. R. Katritzky, C. W. Rees and E. F. V. Scriven (Pergamon Press, 1996), Comprehensive Organic Functional Group Transformations, eds. A.R. Katritzky, O. Meth-Cohn and C.W. Rees (Elsevier Science Ltd., Oxford, 1995), Comprehensive Organic Chemistry, eds. D. Barton and W.D. Ollis (Pergamon Press, Oxford, 1979), and Comprehensive Organic Transformations, R.C. Larock (VCH Publishers Inc., New York, 1989).

Compounds of formula (IIA) or (IIB) may be prepared from compounds of formula (IV) wherein R^{1a} is as defined above and Z is a halogen, for example bromine, by reaction with bis(pinacolato)diboron in the presence of a catalyst, for example a palladium (II) complex, optionally followed by aqueous hydrolysis.

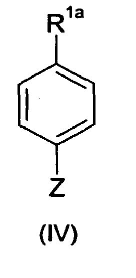

In compounds of formula (IV) wherein R^{1a} is a group convertible to R¹, suitable groups that may enable this conversion include sulfonyl chloride and sulfonylpentafluorophenyl ester. Such groups may be reacted with the required amine under suitable conditions, for example in the presence of a hindered organic base, for example TEA, and in an inert solvent, for example 1,4-dioxane. Sulfonylpentafluorophenyl ester may be obtained from sulfonyl chloride by reaction with pentafluorophenol in the presence of a hindered organic base, for example TEA, and in an inert solvent, for example DCM. Sulfonyl chloride groups may be obtained from sulfonic acids using a chlorinating reagent such as thionyl chloride.

Compounds of formula (III) wherein P is a protecting group may be obtained by reacting compounds of formula (V) wherein P is a protecting group, X is as defined above and L is a leaving group such as a halogen, for example iodine, with a suitable terminal alkyne in the presence of a catalyst, for example a palladium (II) complex. In a further embodiment, compounds of formula (III) may also be obtained by reacting compounds of formula (V) wherein L is a leaving group such as a halogen, for example iodine, with a Grignard reagent, for example isopropylmagnesium chloride, and subsequent reaction with a suitable electrophile, for example paraformaldehyde.

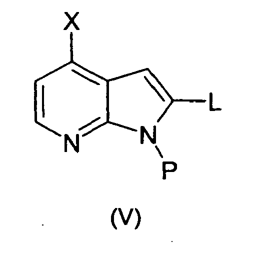

Compounds of formula (V) may be obtained from the reaction of compounds of formula (VI), wherein P is a protecting group and X is as defined above, via generation of an anion at the 2-position with a strong hindered base, for example lithum di-isopropylamide, at low temperature, for example -78°C, and subsequent reaction with an electrophile such as an alkyl halide, for example methyl iodide, or an acid chloride, for example acetyl chloride or a formylating reagent, for example dimethylformamide.

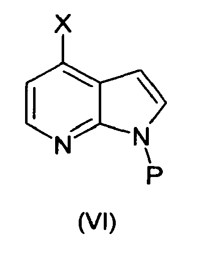

Compounds of formula (V) may also be obtained from compounds of formula (VI) by reaction comprising deprotonation with a strong hindered base, for example lithum di-isopropylamide, at low temperature, for example -78°C and subsequent reaction with an electrophile, for example iodine.

Compounds of formula (VI) may be obtained by reacting compounds of formula (VII), wherein X is as defined above, in the presence of aryl sulphonyl chloride, for example benzene sulfonyl chloride, under phase transfer conditions with biphasic solvents such as water and DCM and in the presence of a phase transfer catalyst such as tetrabutylammonium sulphate.

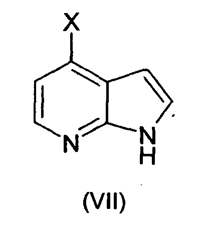

Compounds of formula (VII) may be obtained by reacting from compounds of formula (VIII) in the presence of a suitable halogenating reagent, for example tetrabutylammonium bromide, with methansulfonic anhydride in a suitable solvent, for example dimethylformamide.

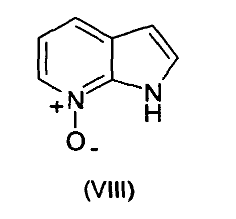

Compounds of formula (VIII) may be obtained by reacting compounds of formula (IX) with a suitable oxidising agent, for example meta-chloroperbenzoic acid, in a suitable solvent, for example EtOAc.

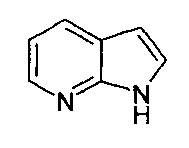

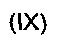

Compounds of formula (III) wherein P is hydrogen may be prepared by reacting a compound of formula (X), wherein R^{2a} is as defined above, with a suitable halogenating reagent, for example methane sulfonyl chloride, in a suitable suitable solvent, for example dimethylformamide, and at elevated temperatures, for example 50-70°C.

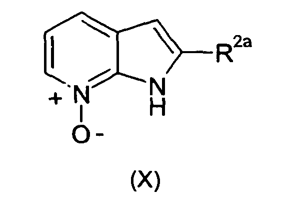

Compounds of formula (X) may be prepared by reacting a compound of formula (XI), wherein R^{2a} is as defined above, with a suitable oxidising agent, for example meta-chloroperbenzoic acid, in a suitable solvent, for example EtOAc.

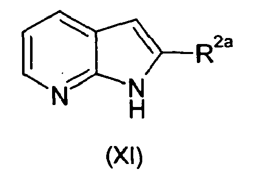

Compounds of formula (XI) may be prepared by reacting a compound of formula (XII) with a suitable strong base, for example butyl lithium, at low temperature, for example -4 to 0°C, in an inert solvent, for example THF, and subsequently reacting with a N,N-dimethylamide or N-methyl-N-methoxy (Weinreb) amide at low temperature, for example 0 to 10°C. The preparation of compounds of formula (XI) may be completed by acidification with a strong mineral acid, for example hydrochloric acid, at low temperature, for example 0 to 5°C, followed by heating at an elevated temperature, for example 50 to 90°C.

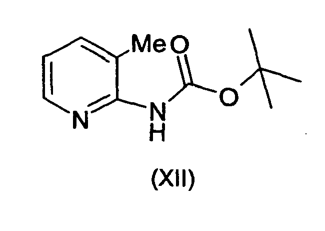

Compounds of formula (I) can be prepared, for example, according to Schemes 1 to 28 below:

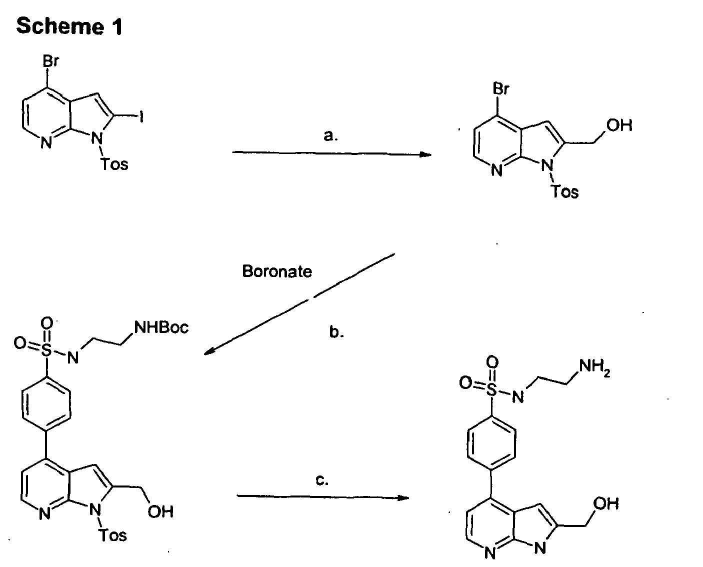

### Conditions

a. (i) ⁱPrMgCl / THF (ii) (HCHO)ₙ / THF
b. (i) H₂N(CH₂)₂NHBoc / Et₃N (ii) PdCl₂(dppf)₂ / Na₂CO_{3 (s)} / Dioxan : Water
c. (i) *p*TSA / CHCl₃ / mw 150W (ii) 5%KOH / MeOH

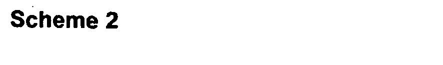

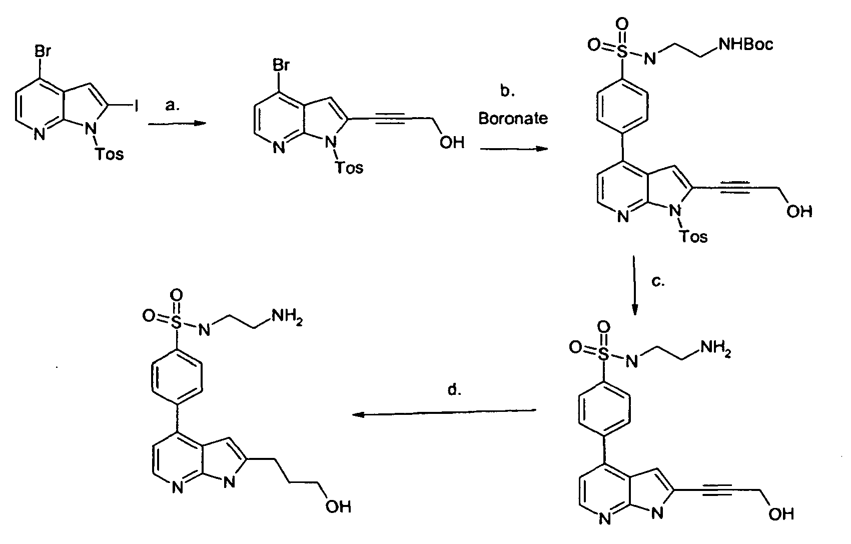

### Conditions

a. 2-Propyn-1-ol / PdCl₂(PPh₃)₂ / Cul / Et₃N / THF
b. H₂N(CH₂)₂NHBoc / Et₃N (ii) PdCl₂(dppf)₂ / Na₂CO_{3 (s)} / Dioxan : Water
c. *p*TSA / CHCl₃ (ii) 5%KOH / MeOH
d. H₂ / Pd-C / MeOH

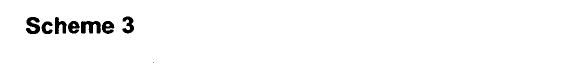

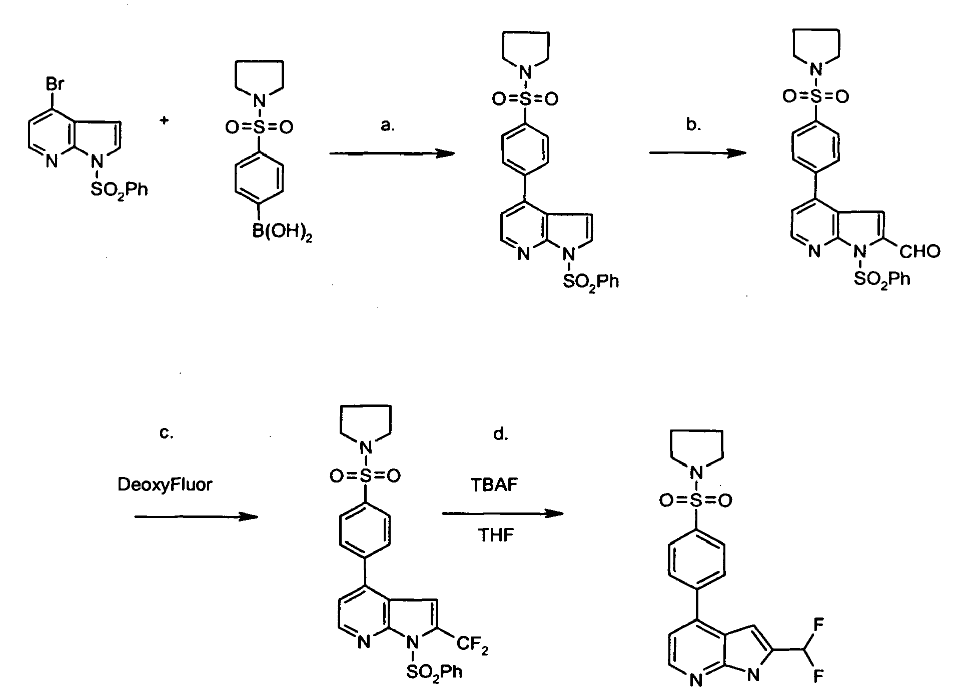

### Conditions

a. PdCl₂(dppf)₂ / Na₂CO_{3 (s)} / Dioxan : Water
b. (i) LDA / THF (ii) DMF
c. DeoxyFluor / DCM
d. TBAF / THF

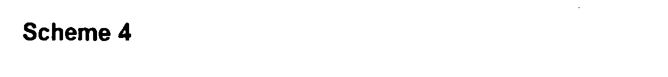

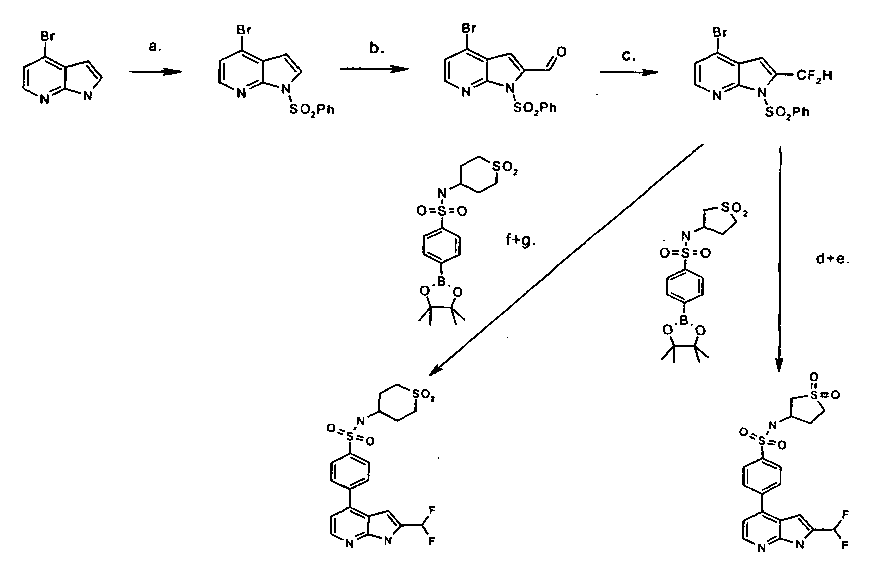

### Conditions

a. PhSO₂Cl / NaOH / NaBuHSO₄ / DCM / Dioxan
b. (i) LDA / THF (ii) DMF
c. DeoxyFluor / DCM
d. PdCl₂(dppf)₂ / Na₂CO_{3 (s)} / Dioxan : Water / Biotage Initiator
e. TBAF / THF
f. PdCl₂(dppf)₂ / Na₂CO_{3 (s)} / Dioxan : Water / Biotage Initiator
g. TBAF / THF

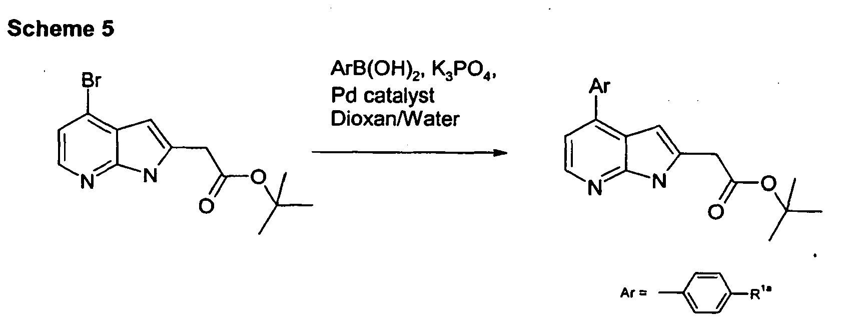

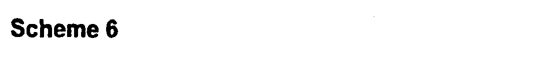

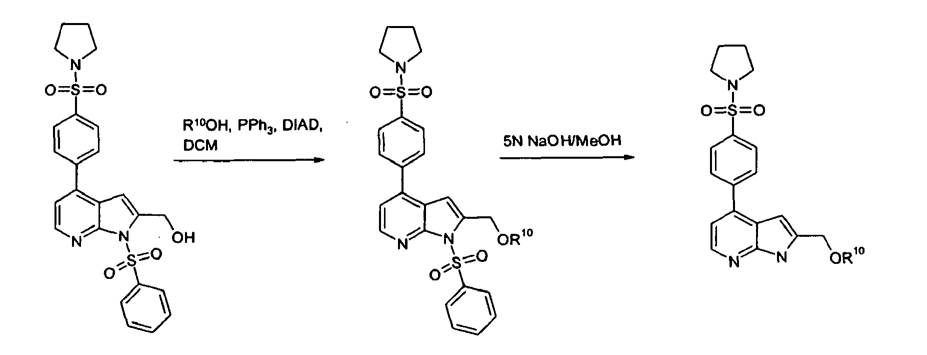

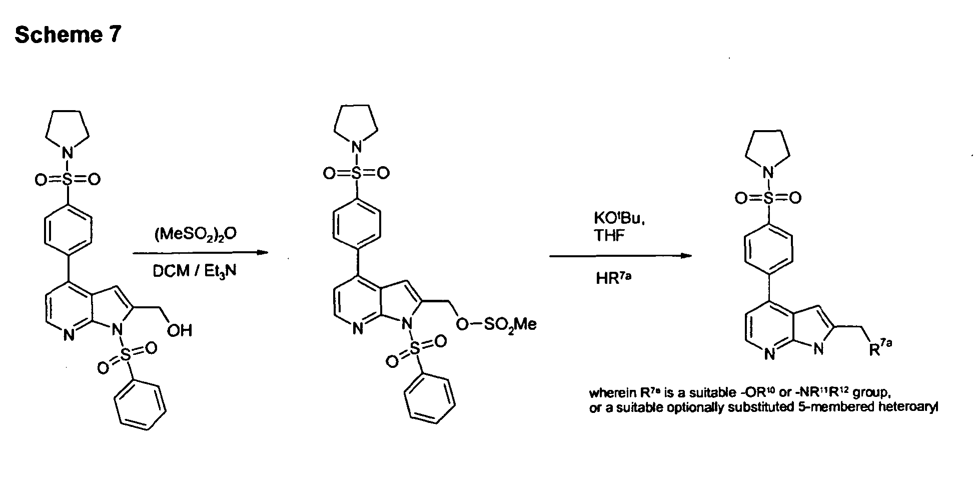

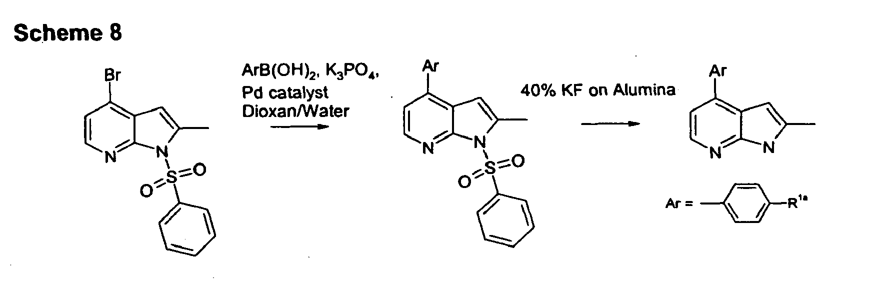

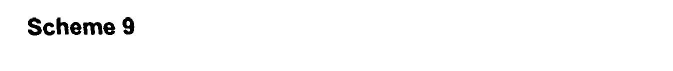

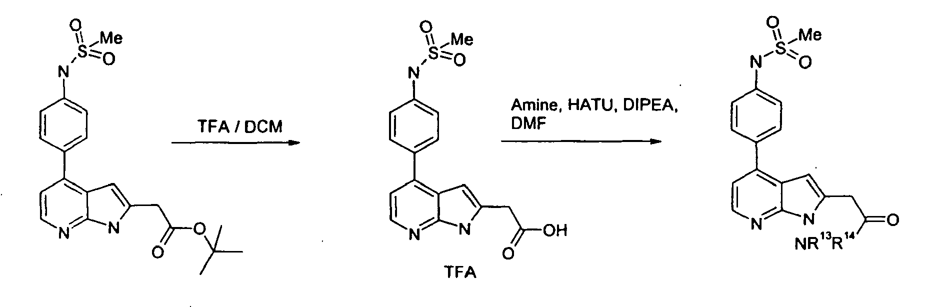

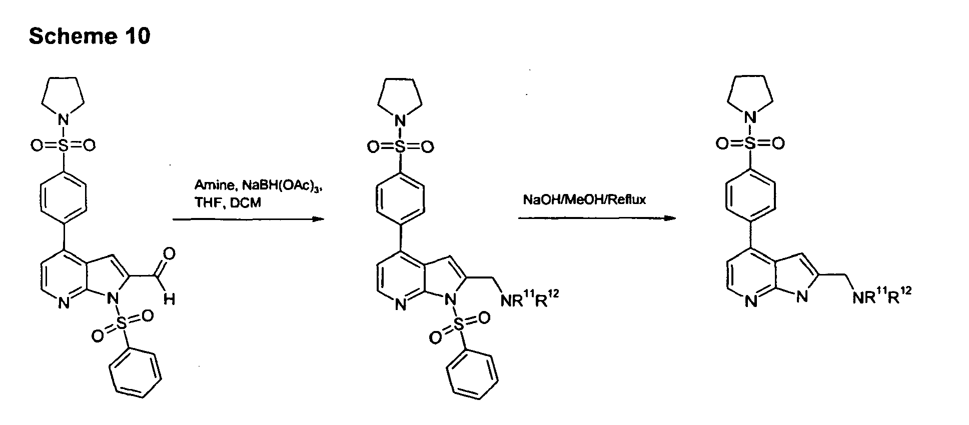

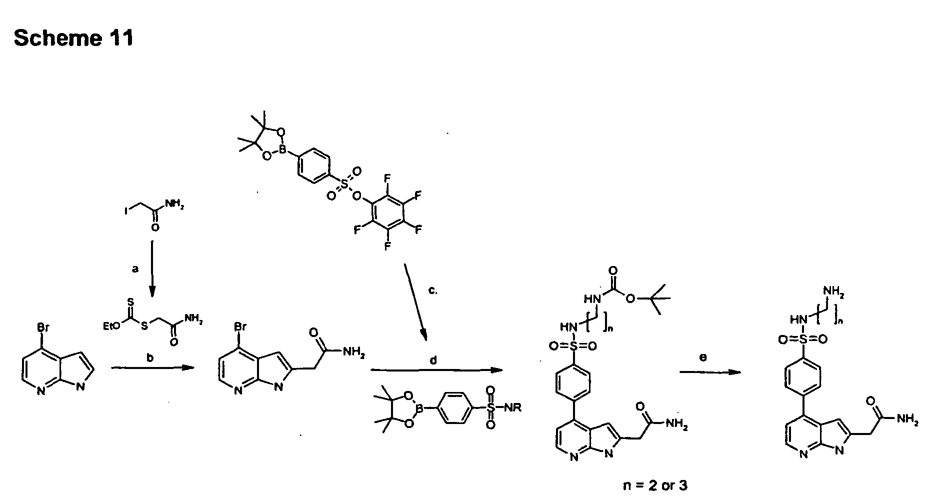

### Conditions

a. Ethylxanthic acid / acetone
b. Lauroyl peroxide / 1,2-dichloroethane
c. Et₃N / Biotage Initiator, amine
d. PdCl₂(dppf)₂ / / Na₂CO_{3 (s)} / Dioxan : Water (5:1) / Biotage Initiator
e. TFA / DCM

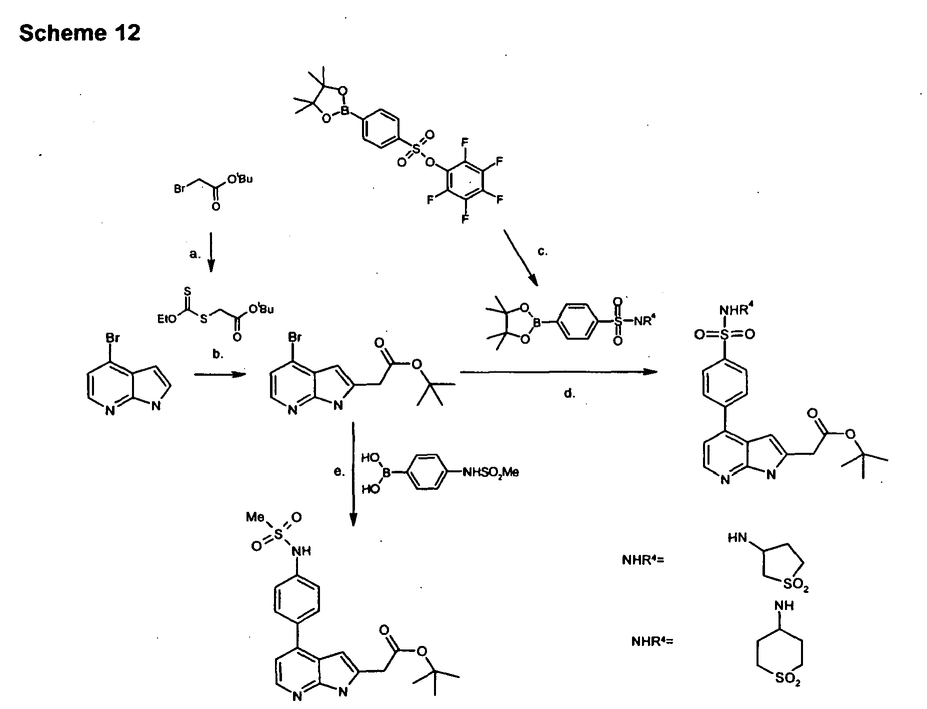

### Conditions

a. Ethylxanthic acid / acetone
b. Lauroyl peroxide / 1,2-dichloroethane
c. Et₃N / Biotage Initiator
d. PdCl₂(dppf)₂ / Na₂CO_{3 (s)} / Dioxan : Water (5:1) / Biotage Initiator
e. PdCl₂(dppf)₂ / Na₂CO_{3 (s)} / Dioxan : Water (5:1) / Biotage Initiator

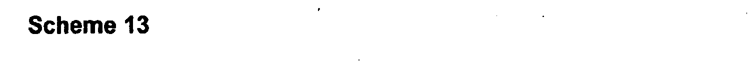

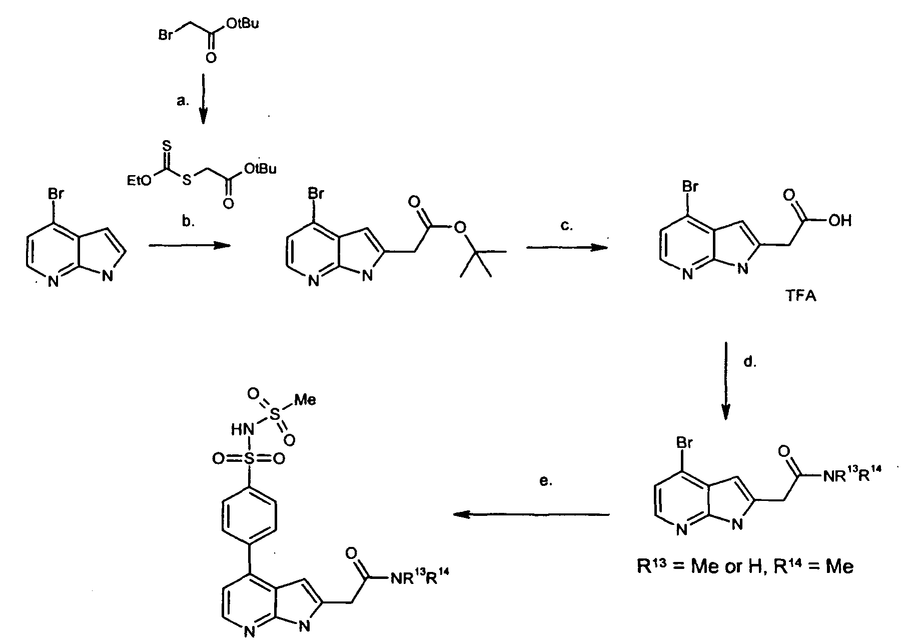

### Conditions

a. Ethylxanthic acid / acetone
b. Lauroyl peroxide / 1,2-dichloroethane
c. TFA : DCM (1:1)
d. (i) DIPEA / TBTU / DCM / room temperature (ii) R¹³NHR¹⁴
e. Boronate ester / PdCl₂(dppf)₂ / Na₂CO_{3 (s)} / Dioxan : Water (5: 1) / Biotage Initiator

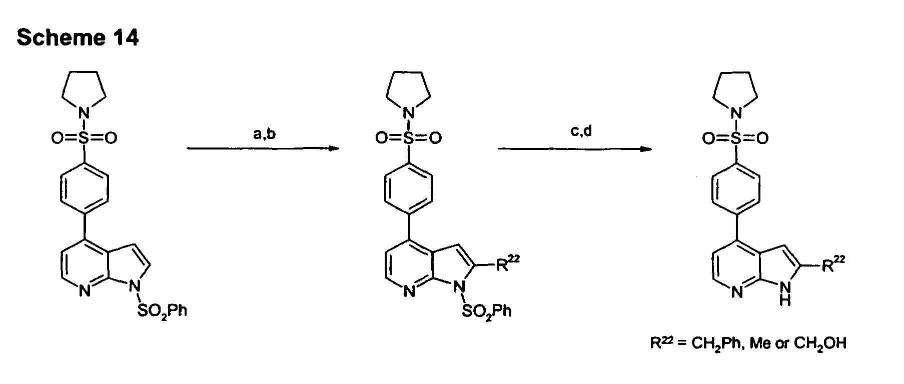

### Conditions

| R²² = | Reagents |
|---|---|
| 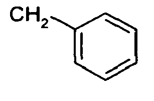 | (a.) LDA / THF |
| | (b.) Benzyl bromide |
| | (c.) NaOH / Dioxan / H₂O |
| | (d.) NaOH |
| Me | (a.) LDA / THF |
| | (b.) Mel |
| | (c.) NaOH / 150 °C / Dioxan / H₂O |
| CH₂OH | (a.) LDA / THF |
| | (b.) HCHO |
| | (c.) NaOH / MeOH |

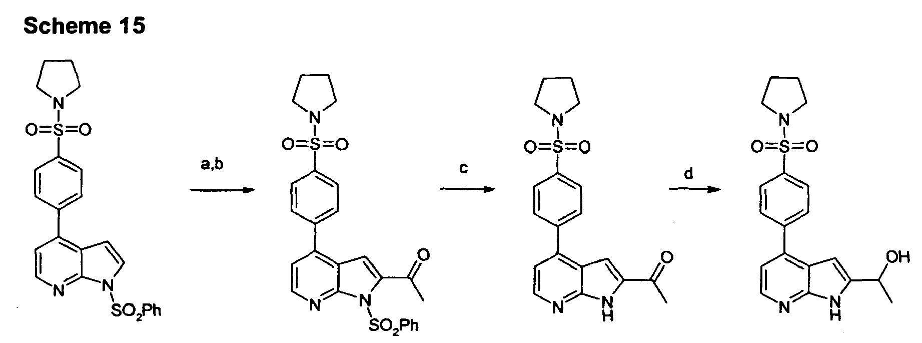

### Conditions

a. LDA / THF
b. Acetic anhydride
c. NaOH / 140 °C / Dioxan / H₂O / mw
d. Sodium borohydride / THF / H₂O

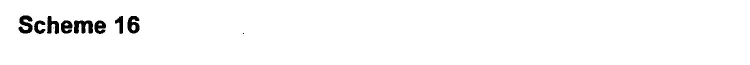

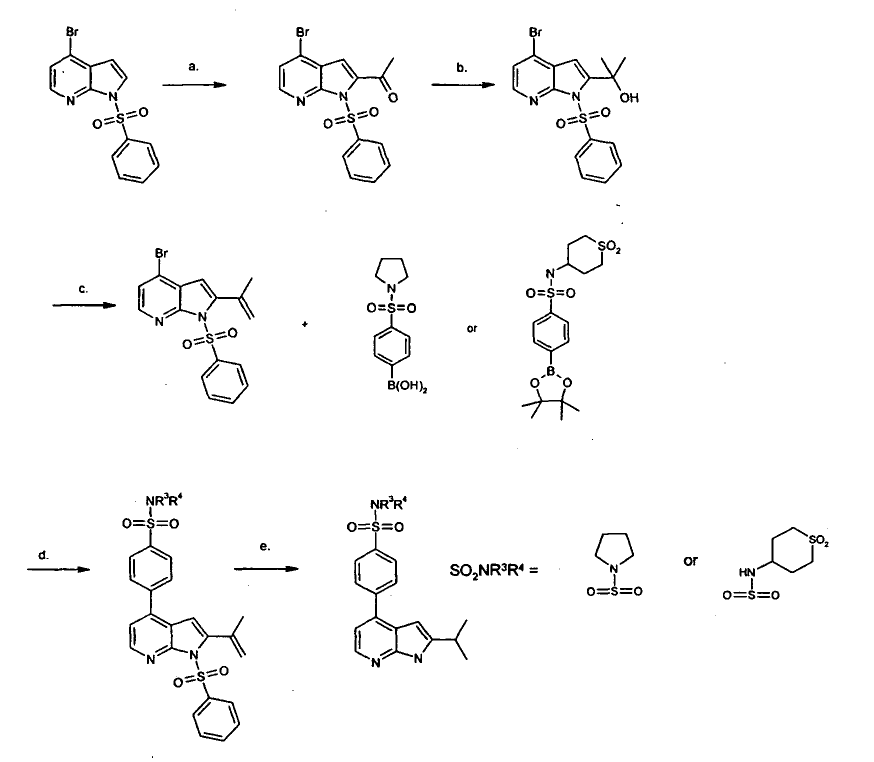

### Conditions

a. (i) LDA / THF (ii) Ac₂O
b. (i) MeMgCl / THF (ii) AcOH
c. AcOH / c.H₂SO₄ / THF
d. Pd(II) / K₃PO₄ / Dioxan : Water / Biotage Initiator
e. (i) H₂ / Pd-C / DMF / MeOH / H-Cube (ii) KOH / THF (iii) 5MNaOH / MeOH

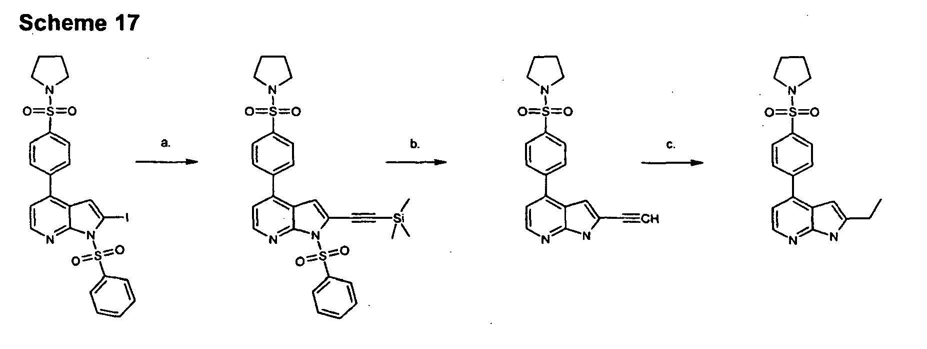

### Conditions

a. TMS acetylene / PdCl₂(PPh₃)₂ / Cul / Et₃N / THF
b. 1M TBAF in THF / THF
c. H₂ / Pd-C / DMF / MeOH / H-Cube

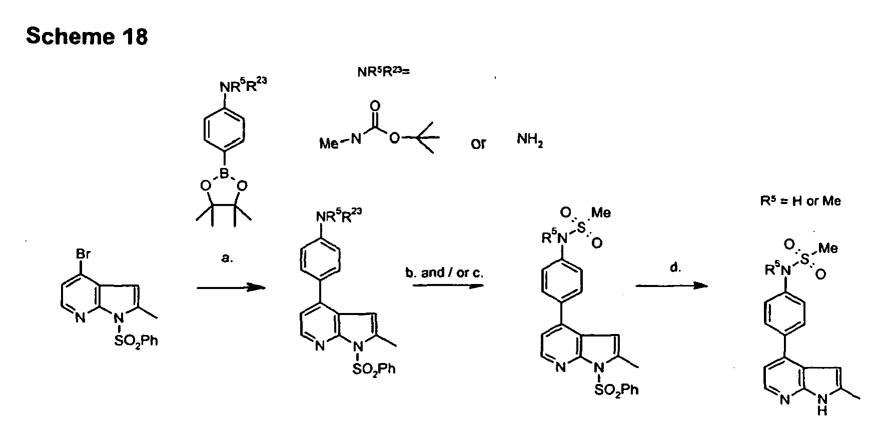

### Conditions

a. PdCl₂(dppf)₂ / Na₂CO_{3 (s)} / Dioxan : Water / Biotage Initiator mw
b. TFA / DCM
c. MeSO₂Cl / TEA
d. 10M NaOH / Dioxan / H₂O

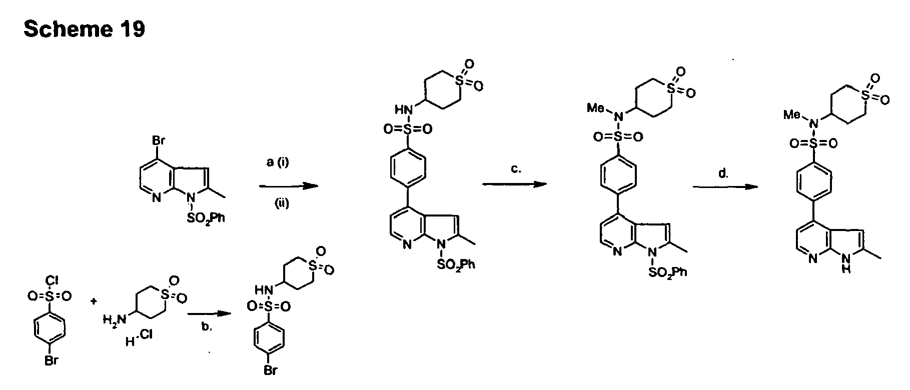

### Conditions

a. (i) PdCl₂(dppf)₂ / Pinacoldiborane / KOAc / DMF (ii) Aryl Bromide / H₂O /
b. TEA / DCM
c. Mel / Cs₂CO₃ / DCM
d. 5M NaOH / Dioxan / Biotage Initiator mw

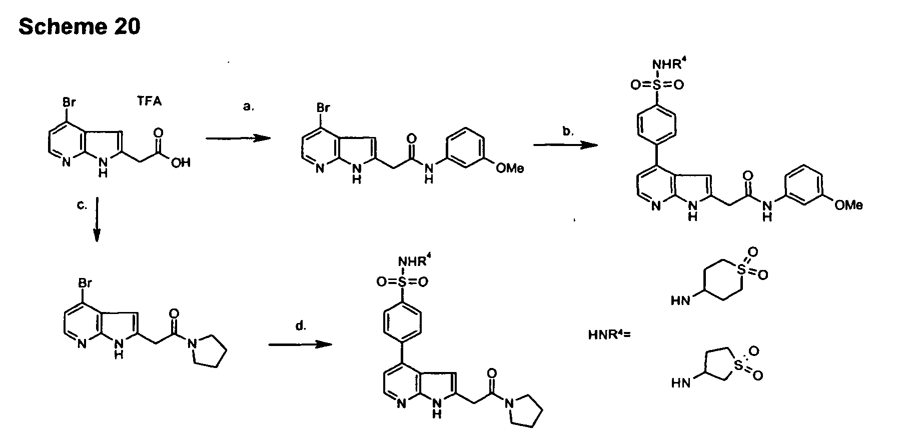

### Conditions

a. (i) WSCDI / HOBT / DIPEA / DMF (ii) m-anisidine
b. Boronate ester / PdCl₂(dppf)₂ / Na₂CO_{3 (s)} / Dioxan : Water / Biotage Initiator mw
c. TBTU/ pyrrolidine / DCM
d. Boronate ester / PdCl₂(dppf)₂ / Na₂CO_{3 (s)} / Dioxan : Water / Biotage Initiator mw

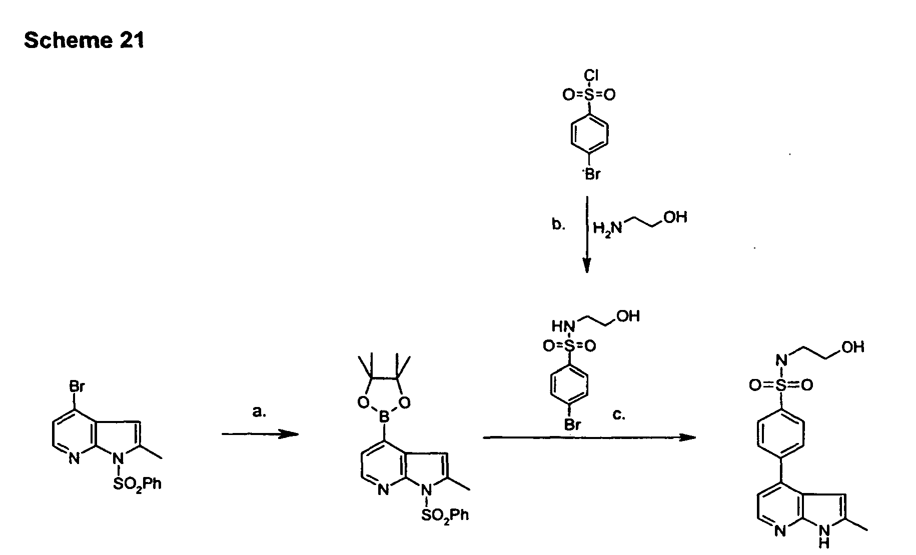

### Conditions

a. Pd(OAc)₂ / Pinacoldiborane / KOAc / DMF
b. TEA / DCM
c.
   (i) Pd II / K₃PO₄ / Dioxan / Water / Biotage Initiator mw
   (ii) NaOH / Biotage Initiator

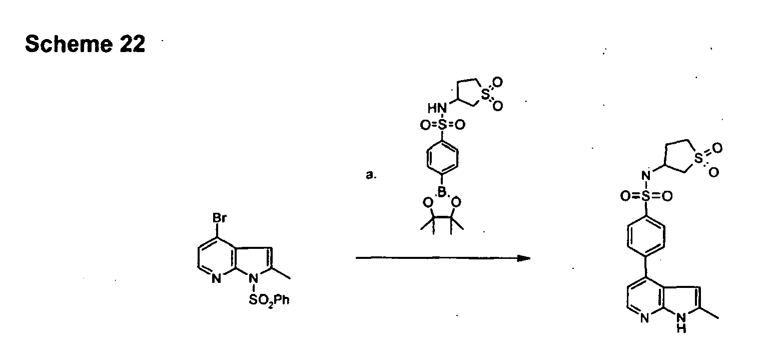

### Conditions

a. (i) Pd II / K₃PO₄ / Dioxan / Water / Biotage Initiator mw, (ii) NaOH / Biotage Initiator

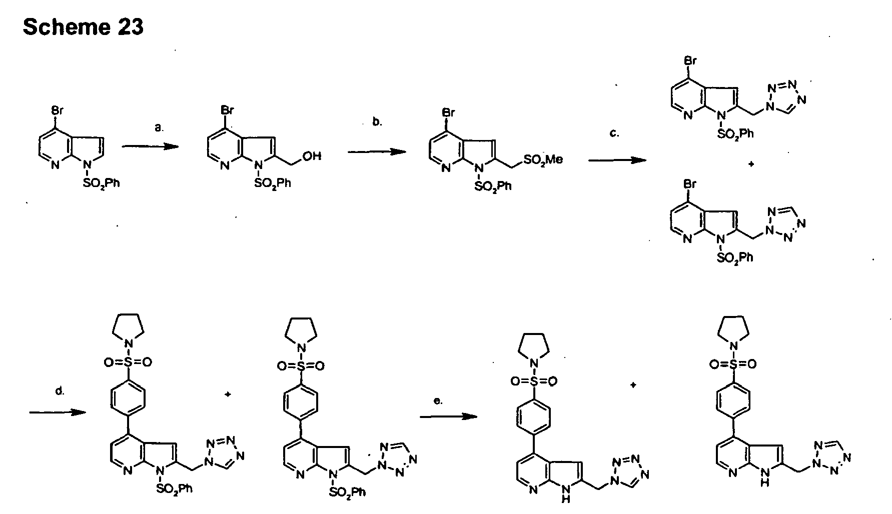

### Conditions

a. (i) LDA / THF (ii) paraformaldehyde
b. (MeSO₂)₂O / TEA / DCM
c. NaH / tetrazole / DMF
d. Boronate acid / PdCl₂(dppf)₂ / Na₂CO_{3 (s)} / Dioxan : Water / Biotage Initiator mw
e. 3M NaOH in MeOH / THF

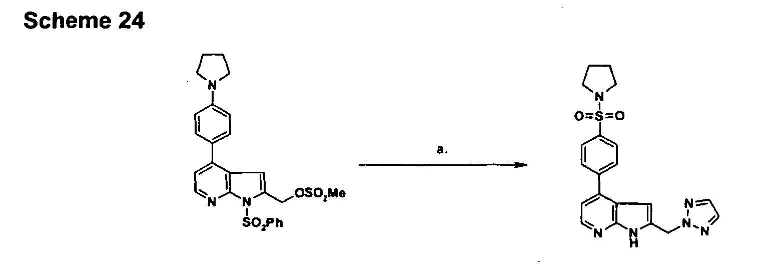

### Conditions

a. (i) Triazole / NaH / DMF (ii) 2M NaOH / MeOH / Biotage Initiator mw

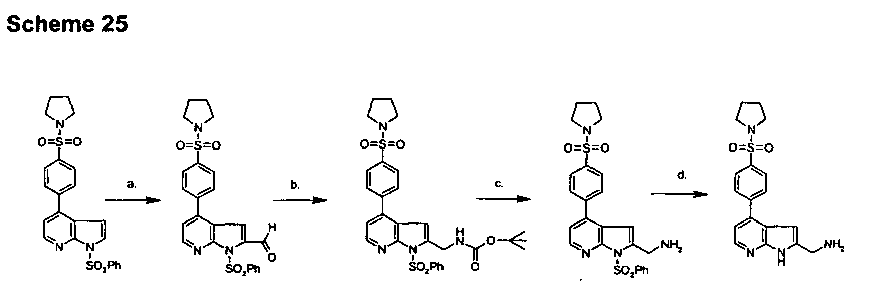

### Conditions

a. (i) LDA / THF (ii) DMF
b. t-Butyl carbamate / TFA / triethylsilane / MeCN
c. TFA / DCM
d. 5M NaOH / Dioxan / Biotage Initiator mw

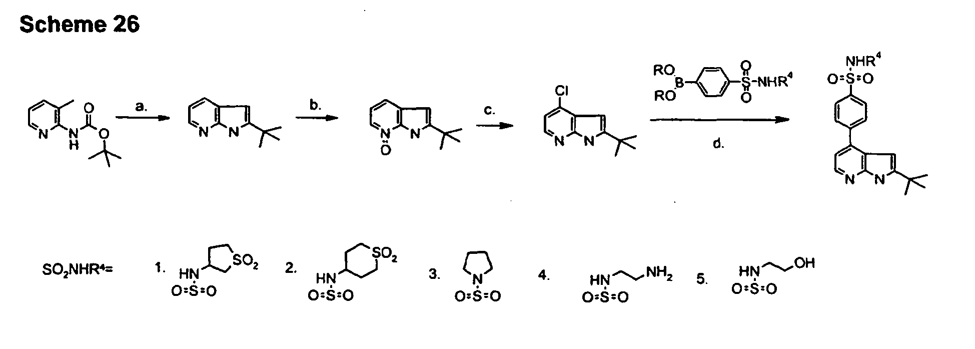

### Conditions

a. (i) nBuLi / THF / N,N'-Dimethylpivalamide (ii) 5M HCl_{(aq)}
b. MCPBA / EtOAc
c. CH₃SO₂Cl / DMF
d. Pd(II) / K₃PO₄ / Dioxan / Water / Biotage Initiator Microwave

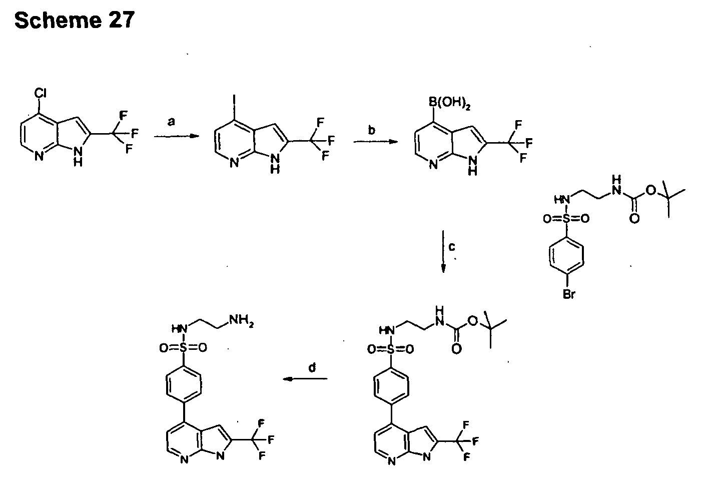

### Conditions

a. 4M HCl / dioxane then Nal / acetonitrile
b. NaH / THF then n-butyl-lithium then triisopropylborate
c. PdCl₂ (dppf)₂ / 1M NaHCO₃ / IPA / Biotage Initiator mw
d. TFA / DCM

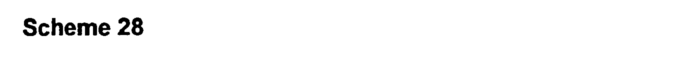

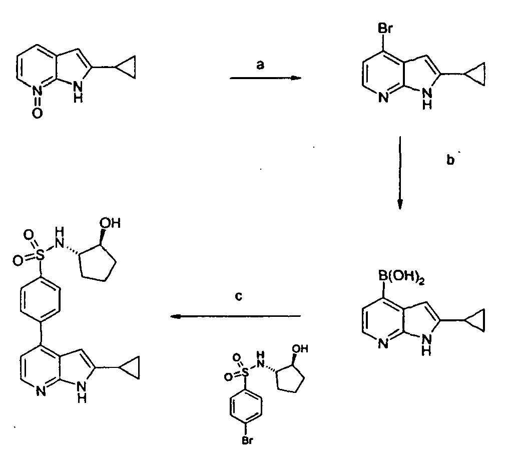

### Conditions

a. methane sulfonic anhydride / tetramethylammonium bromide / DMF
b. NaH / THF then n-butyl-lithium then triisopropylborate
c. Potassium phosphate / Pd catalyst / dioxane / water

### Methods of Use

The compounds of the invention are inhibitors of IKK2. Compounds which are IKK2 inhibitors may be useful in the treatment of disorders wherein the underlying pathology is (at least in part) attributable to inappropriate IKK2 (also known as IKKβ) activity such as rheumatoid arthritis, COPD (chronic obstructive pulmonary disease), asthma and rhinitis. "Inappropriate IKK2 activity" refers to any IKK2 activity that deviates from the normal IKK2 activity expected in a particular patient. Inappropriate IKK2 activity may take the form of, for instance, an abnormal increase in activity, or an aberration in the timing and or control of IKK2 activity. Such inappropriate activity may result then, for example, from overexpression or mutation of the protein kinase leading to inappropriate or uncontrolled activation. Accordingly, in another aspect the invention is directed to methods of treating such disorders.

Such disorders include inflammatory and tissue repair disorders (including rheumatoid arthritis, inflammatory bowel disease, COPD (chronic obstructive pulmonary disease), asthma and rhinitis), fibrotic diseases, osteoarthritis, osteoporosis, dermatosis (including psoriasis, atopic dermatitis and ultraviolet radiation (UV)-induced skin damage), autoimmune diseases (including Sjogren's syndrome, systemic lupus eythematosus, multiple sclerosis, psoriatic arthritis, alkylosing spondylitis, tissue and organ rejection), Alzheimer's disease, stroke, atherosclerosis, restonosis, diabetes, glomerulonephritis, cancer (including Hodgkins disease), cachexia, inflammation associated with infection and certain viral infections (including acquired immune deficiency syndrome (AIDS)), adult respiratory distress syndrome, and Ataxia Telangiestasia.

The methods of treatment of the invention comprise administering a safe and effective amount of a compound of formula (I) or a pharmaceutically acceptable salt thereof to a patient in need thereof. Individual embodiments of the invention include methods of treating any one of the above-mentioned disorders by administering a safe and effective amount of a compound of formula (I) or a pharmaceutically acceptable salt thereof to a patient in need thereof.

As used herein, "treat" in reference to a disorder means: (1) to ameliorate or prevent the disorder or one or more of the biological manifestations of the disorder, (2) to interfere with (a) one or more points in the biological cascade that leads to or is responsible for the disorder or (b) one or more of the biological manifestations of the disorder, (3) to alleviate one or more of the symptoms or effects associated with the disorder, or (4) to slow the progression of the disorder or one or more of the biological manifestations of the disorder.

As indicated above, "treatment" of a disorder includes prevention of the disorder. The skilled artisan will appreciate that "prevention" is not an absolute term. In medicine, "prevention" is understood to refer to the prophylactic administration of a drug to substantially diminish the likelihood or severity of a disorder or biological manifestation thereof, or to delay the onset of such disorder or biological manifestation thereof.

As used herein, "safe and effective amount" in reference to a compound of formula (I) or a pharmaceutically acceptable salt thereof or other pharmaceutically-active agent means an amount of the compound sufficient to treat the patient's condition but low enough to avoid serious side effects (at a reasonable benefit/risk ratio) within the scope of sound medical judgment. A safe and effective amount of a compound will vary with the particular compound chosen (e.g. consider the potency, efficacy, and half-life of the compound); the route of administration chosen; the disorder being treated; the severity of the disorder being treated; the age, size, weight, and physical condition of the patient being treated; the medical history of the patient to be treated; the duration of the treatment; the nature of concurrent therapy; the desired therapeutic effect; and like factors, but can nevertheless be routinely determined by the skilled artisan.

As used herein, "patient" refers to a human (including adults and children) or other animal.

The compounds of formula (I) or pharmaceutically acceptable salts thereof may be administered by any suitable route of administration, including both systemic administration and topical administration. Systemic administration includes oral administration, parenteral administration, transdermal administration and rectal administration. Parenteral administration refers to routes of administration other than enteral or transdermal, and is typically by injection or infusion. Parenteral administration includes intravenous, intramuscular, and subcutaneous injection or infusion. Topical administration includes application to the skin as well as intraocular, otic, intravaginal, inhaled and intranasal administration. Inhalation refers to administration into the patient's lungs whether inhaled through the mouth or through the nasal passages. In one embodiment, the compounds of formula (I) or pharmaceutically acceptable salts thereof may be administered orally. In another embodiment, the compounds of formula (I) or pharmaceutically acceptable salts thereof may be administered by inhalation. In a further embodiment, the compounds of formula (I) or pharmaceutically acceptable salts thereof may be administered intranasally.

The compounds of formula (I) or pharmaceutically acceptable salts thereof may be administered once or according to a dosing regimen wherein a number of doses are administered at varying intervals of time for a given period of time. For example, doses may be administered one, two, three, or four times per day. In one embodiment, a dose is administered once per day. In a further embodiment, a dose is administered twice per day. Doses may be administered until the desired therapeutic effect is achieved or indefinitely to maintain the desired therapeutic effect. Suitable dosing regimens for a compound of formula (I) or a pharmaceutically acceptable salt thereof depend on the pharmacokinetic properties of that compound, such as absorption, distribution, and half-life, which can be determined by the skilled artisan. In addition, suitable dosing regimens, including the duration such regimens are administered, for a compound of formula (I) or a pharmaceutically acceptable salt thereof depend on the disorder being treated, the severity of the disorder being treated, the age and physical condition of the patient being treated, the medical history of the patient to be treated, the nature of concurrent therapy, the desired therapeutic effect, and like factors within the knowledge and expertise of the skilled artisan. It will be further understood by such skilled artisans that suitable dosing regimens may require adjustment given an individual patient's response to the dosing regimen or over time as individual patient needs change.

Typical daily dosages may vary depending upon the particular route of administration chosen. Typical daily dosages for oral administration range from 0.001 mg to 50mg per kg of total body weight, for example from 1 mg to 10mg per kg of total body weight. For example, daily dosages for oral administration may be from 0.5mg to 2g per patient, such as 10mg to 1g per patient.

Additionally, the compounds of formula (I) may be administered as prodrugs. As used herein, a "prodrug" of a compound of formula (I) is a functional derivative of the compound which, upon administration to a patient, eventually liberates the compound of formula (I) *in vivo.* Administration of a compound of formula (I) as a prodrug may enable the skilled artisan to do one or more of the following: (a) modify the onset of the activity of the compound *in vivo*; (b) modify the duration of action of the compound *in vivo*; (c) modify the transportation or distribution of the compound *in vivo*; (d) modify the solubility of the compound *in vivo*; and (e) overcome a side effect or other difficulty encountered with the compound. Typical functional derivatives used to prepare prodrugs include modifications of the compound that are chemically or enzymatically cleavable *in vivo.* Such modifications, which include the preparation of phosphates, amides, esters, thioesters, carbonates, and carbamates, are well known to those skilled in the art.

The invention thus provides a method of treating a disorder mediated by inappropriate IKK2 activity comprising administering a safe and effective amount of a compound of formula (I) or a pharmaceutically acceptable salt thereof to a patient in need thereof.

In one embodiment, the disorder mediated by inappropriate IKK2 activity is selected from the group consisting of inflammatory and tissue repair disorders (including rheumatoid arthritis, inflammatory bowel disease, COPD (chronic obstructive pulmonary disease), asthma and rhinitis), fibrotic diseases, osteoarthritis, osteoporosis, dermatosis (including psoriasis, atopic dermatitis and ultraviolet radiation (UV)-induced skin damage), autoimmune diseases (including Sjogren's syndrome, systemic lupus eythematosus, multiple sclerosis, psoriatic arthritis, alkylosing spondylitis, tissue and organ rejection), Alzheimer's disease, stroke, atherosclerosis, restonosis, diabetes, glomerulonephritis, cancer (including Hodgkins disease), cachexia, inflammation associated with infection and certain viral infections (including acquired immune deficiency syndrome (AIDS)), adult respiratory distress syndrome, and Ataxia Telangiestasia.

In another embodiment, the disorder mediated by inappropriate IKK2 activity is an inflammatory or tissue repair disorder. In another embodiment, the disorder mediated by inappropriate IKK2 activity is rheumatoid arthritis, COPD, asthma or rhinitis. In another embodiment, the disorder mediated by inappropriate IKK2 activity is rheumatoid arthritis. In another embodiment, the disorder mediated by inappropriate IKK2 activity is COPD. In another embodiment, the disorder mediated by inappropriate IKK2 activity is asthma. In a further embodiment, the disorder mediated by inappropriate IKK2 activity is rhinitis (including seasonal rhinitis, allergic rhinitis and vasomotor rhinitis).

In another embodiment, the disorder mediated by inappropriate IKK2 activity is an autoimmune disease. In a further embodiment, the disorder mediated by inappropriate IKK2 activity is Sjogren's syndrome, systemic lupus eythematosus, multiple sclerosis, psoriatic arthritis, or alkylosing spondylitis.

In another embodiment, the disorder mediated by inappropriate IKK2 activity is selected from the group consisting of Alzheimer's disease, stroke atherosclerosis, restenosis, diabetes, glomerulonephritis, osteoarthritis, osteoporosis, and Ataxia Telangiestasia.

In another embodiment, the disorder mediated by inappropriate IKK2 activity is cancer or cachexia. In a further embodiment, the disorder mediated by inappropriate IKK2 activity is cancer.

In one embodiment, the present invention provides a method of treating rhinitis comprising administering a safe and effective amount of N-(2-aminoethyl)-4-(2-ethyl-1*H*-pyrrolo[2,3-*b*]pyridin-4-yl)benzenesulfonamide or a pharmaceutically acceptable salt thereof to a patient in need thereof.

In one embodiment, the present invention provides a method of treating rheumatoid arthritis comprising administering a safe and effective amount of 4-(2-cyclopropyl-1*H*-pyrrolo[2,3-*b*]pyridin-4-yl)-*N*-(2-hydroxyethyl)-benzenesulfonamide or a pharmaceutically acceptable salt thereof to a patient in need thereof.

In one embodiment, the present invention provides a method of treating COPD comprising administering a safe and effective amount of 4-(2-cyclopropyl-1*H-*pyrrolo[2,3-*b*]pyridin-4-yl)-*N*-(2-hydroxyethyl)-benzenesulfonamide or a pharmaceutically acceptable salt thereof to a patient in need thereof.

The term "rhinitis" is used herein to refer to all types of rhinitis including allergic rhinitis such as seasonal rhinitis (for example hayfever) or perennial rhinitis, and non-allergic rhinitis or vasomotor rhinitis.

The invention also provides a compound of formula (I) of a pharmaceutically acceptable salt thereof for use in medical therapy, particularly in the treatment of disorders mediated by IKK2 activity. Thus, in a further aspect, the invention is directed to the use of a compound of formula (I) or a pharmaceutically acceptable salt thereof in the manufacture of a medicament for use in the treatment of a disorder characterized by inappropriate IKK2 activity.

### Compositions

The compounds of formula (I) and pharmaceutically acceptable salts thereof will normally, but not necessarily, be formulated into pharmaceutical compositions prior to administration to a patient. Accordingly, in another aspect the invention is directed to pharmaceutical compositions comprising a compound of formula (I) or a pharmaceutically acceptable salt thereof and one or more pharmaceutically-acceptable excipients.

The pharmaceutical compositions of the invention may be prepared and packaged in bulk form wherein a safe and effective amount of a compound of formula (I) or a pharmaceutically acceptable salt thereof can be extracted and then given to the patient such as with powders or syrups. Alternatively, the pharmaceutical compositions of the invention may be prepared and packaged in unit dosage form wherein each physically discrete unit contains a compound of formula (I) or a pharmaceutically acceptable salt thereof. When prepared in unit dosage form, the pharmaceutical compositions of the invention typically may contain, for example, from 0.5mg to 1g, or from 1 mg to 700mg, or from 5mg to 100mg of a compound of formula (I) or a pharmaceutically acceptable salt thereof.

The pharmaceutical compositions of the invention typically contain one compound of formula (I) or a pharmaceutically acceptable salt thereof.

As used herein, "pharmaceutically-acceptable excipient" means a pharmaceutically acceptable material, composition or vehicle involved in giving form or consistency to the pharmaceutical composition. Each excipient must be compatible with the other ingredients of the pharmaceutical composition when commingled such that interactions which would substantially reduce the efficacy of the compound of formula (I) or a pharmaceutically acceptable salt thereof when administered to a patient and interactions which would result in pharmaceutical compositions that are not pharmaceutically acceptable are avoided. In addition, each excipient must of course be pharmaceutically-acceptable eg of sufficiently high purity.

The compound of formula (I) or a pharmaceutically acceptable salt thereof and the pharmaceutically-acceptable excipient or excipients will typically be formulated into a dosage form adapted for administration to the patient by the desired route of administration. For example, dosage forms include those adapted for (1) oral administration such as tablets, capsules, caplets, pills, troches, powders, syrups, elixers, suspensions, solutions, emulsions, sachets, and cachets; (2) parenteral administration such as sterile solutions, suspensions, and powders for reconstitution; (3) transdermal administration such as transdermal patches; (4) rectal administration such as suppositories; (5) inhalation such as aerosols, solutions, and dry powders; and (6) topical administration such as creams, ointments, lotions, solutions, pastes, sprays, foams, and gels.

Suitable pharmaceutically acceptable excipients will vary depending upon the particular dosage form chosen. In addition, suitable pharmaceutically acceptable excipients may be chosen for a particular function that they may serve in the composition. For example, certain pharmaceutically acceptable excipients may be chosen for their ability to facilitate the production of uniform dosage forms. Certain pharmaceutically acceptable excipients may be chosen for their ability to facilitate the production of stable dosage forms. Certain pharmaceutically acceptable excipients may be chosen for their ability to facilitate the carrying or transporting of the compound or compounds of formula (I) or pharmaceutically acceptable salts thereof once administered to the patient from one organ, or portion of the body, to another organ, or portion of the body. Certain pharmaceutically acceptable excipients may be chosen for their ability to enhance patient compliance.

Suitable pharmaceutically-acceptable excipients include the following types of excipients: Diluents, fillers, binders, disintegrants, lubricants, glidants, granulating agents, coating agents, wetting agents, solvents, co-solvents, suspending agents, emulsifiers, sweetners, flavoring agents, flavor masking agents, coloring agents, anticaking agents, hemectants, chelating agents, plasticizers, viscosity increasing agents, antioxidants, preservatives, stabilizers, surfactants, and buffering agents. The skilled artisan will appreciate that certain pharmaceutically-acceptable excipients may serve more than one function and may serve alternative functions depending on how much of the excipient is present in the formulation and what other excipients are present in the formulation.

Skilled artisans possess the knowledge and skill in the art to enable them to select suitable pharmaceutically-acceptable excipients in appropriate amounts for use in the invention. In addition, there are a number of resources that are available to the skilled artisan which describe pharmaceutically-acceptable excipients and may be useful in selecting suitable pharmaceutically-acceptable excipients. Examples include Remington's Pharmaceutical Sciences (Mack Publishing Company), The Handbook of Pharmaceutical Additives (Gower Publishing Limited), and The Handbook of Pharmaceutical Excipients (the American Pharmaceutical Association and the Pharmaceutical Press).

The pharmaceutical compositions of the invention are prepared using techniques and methods known to those skilled in the art. Some of the methods commonly used in the art are described in Remington's Pharmaceutical Sciences (Mack Publishing Company).

Accordingly, in another aspect the invention is directed to process for the preparation of a pharmaceutical composition comprising a compound of formula (I) or a pharmaceutically acceptable salt thereof and one or more pharmaceutically-acceptable excipients which comprises mixing the ingredients. A pharmaceutical composition comprising a compound of formula (I) or a pharmaceutically acceptable salt thereof may be prepared by, for example, admixture at ambient temperature and atmospheric pressure.

In one embodiment, the compounds of formula (I) or pharmaceutically acceptable salts thereof will be formulated for oral administration. In another embodiment, the compounds of formula (I) or pharmaceutically acceptable salts thereof will be formulated for inhaled administration. In a further embodiment, the compounds of formula (I) or pharmaceutically acceptable salts thereof will be formulated for intranasal administration.

In one aspect, the invention is directed to a solid oral dosage form such as a tablet or capsule comprising a safe and effective amount of a compound of formula (I) or a pharmaceutically acceptable salt thereof and a diluent or filler. Suitable diluents and fillers include lactose, sucrose, dextrose, mannitol, sorbitol, starch (e.g. corn starch, potato starch, and pre-gelatinized starch), cellulose and its derivatives (e.g. microcrystalline cellulose), calcium sulfate, and dibasic calcium phosphate. The oral solid dosage form may further comprise a binder. Suitable binders include starch (e.g. corn starch, potato starch, and pre-gelatinized starch), gelatin, acacia, sodium alginate, alginic acid, tragacanth, guar gum, povidone, and cellulose and its derivatives (e.g. microcrystalline cellulose). The oral solid dosage form may further comprise a disintegrant. Suitable disintegrants include crospovidone, sodium starch glycolate, croscarmelose, alginic acid, and sodium carboxymethyl cellulose. The oral solid dosage form may further comprise a lubricant. Suitable lubricants include stearic acid, magnesuim stearate, calcium stearate, and talc.

Where appropriate, dosage unit formulations for oral administration can be microencapsulated. The composition can also be prepared to prolong or sustain the release as for example by coating or embedding particulate material in polymers, wax or the like.

The compounds of formula (I) or pharmaceutically acceptable salts thereof may also be coupled with soluble polymers as targetable drug carriers. Such polymers can include polyvinylpyrrolidone, pyran copolymer, polyhydroxypropylmethacrylamide -phenol, polyhydroxyethylaspartamidephenol, or polyethyleneoxidepolylysine substituted with palmitoyl residues. Furthermore, the compounds of formula (I) or pharmaceutically acceptable salts thereof may be coupled to a class of biodegradable polymers useful in achieving controlled release of a drug, for example, polylactic acid, polepsilon caprolactone, polyhydroxy butyric acid, polyorthoesters, polyacetals, polydihydropyrans, polycyanoacrylates and cross-linked or amphipathic block copolymers of hydrogels.

In another aspect, the invention is directed to a liquid oral dosage form. Oral liquids such as solution, syrups and elixirs can be prepared in dosage unit form so that a given quantity contains a predetermined amount of a compound of formula (I) or a pharmaceutically acceptable salt thereof. Syrups can be prepared by dissolving the compound of formula (I) or a pharmaceutically acceptable salt thereof in a suitably flavored aqueous solution, while elixirs are prepared through the use of a non-toxic alcoholic vehicle. Suspensions can be formulated by dispersing the compound of formula (I) or a pharmaceutically acceptable salt thereof in a non-toxic vehicle. Solubilizers and emulsifiers such as ethoxylated isostearyl alcohols and polyoxy ethylene sorbitol ethers, preservatives, flavor additive such as peppermint oil or natural sweeteners or saccharin or other artificial sweeteners, and the like can also be added.

In another aspect, the invention is directed to a dosage form adapted for administration to a patient by inhalation. For example, as a dry powder, an aerosol, a suspension, or a solution composition.

Dry powder compositions for delivery to the lung by inhalation typically comprise a compound of formula (I) or a pharmaceutically acceptable salt thereof as a finely divided powder together with one or more pharmaceutically-acceptable excipients as finely divided powders. Pharmaceutically-acceptable excipients particularly suited for use in dry powders are known to those skilled in the art and include lactose, starch, mannitol, and mono-, di-, and polysaccharides. The finely divided powder may be prepared by, for example, micronisation and milling. Generally, the size-reduced (eg micronised) compound can be defined by a D₅₀ value of about 1 to about 10 microns (for example as measured using laser diffraction).

The dry powder may be administered to the patient via a reservoir dry powder inhaler (RDPI) having a reservoir suitable for storing multiple (un-metered doses) of medicament in dry powder form. RDPIs typically include a means for metering each medicament dose from the reservoir to a delivery position. For example, the metering means may comprise a metering cup, which is movable from a first position where the cup may be filled with medicament from the reservoir to a second position where the metered medicament dose is made available to the patient for inhalation.

Alternatively, the dry powder may be presented in capsules (e.g. gelatin or plastic), cartridges, or blister packs for use in a multi-dose dry powder inhaler (MDPI). MDPIs are inhalers wherein the medicament is comprised within a multi-dose pack containing (or otherwise carrying) multiple defined doses (or parts thereof) of medicament. When the dry powder is presented as a blister pack, it comprises multiple blisters for containment of the medicament in dry powder form. The blisters are typically arranged in regular fashion for ease of release of the medicament therefrom. For example, the blisters may be arranged in a generally circular fashion on a disc-form blister pack, or the blisters may be elongate in form, for example comprising a strip or a tape. Each capsule, cartridge, or blister may, for example, contain between 20µg-10mg of the compound of formula (I) or a pharmaceutically acceptable salt thereof.

Aerosols may be formed by suspending or dissolving a compound of formula (I) or a pharmaceutically acceptable salt thereof in a liquified propellant. Suitable propellants include halocarbons, hydrocarbons, and other liquified gases. Representative propellants include: trichlorofluoromethane (propellant 11), dichlorofluoromethane (propellant 12), dichlorotetrafluoroethane (propellant 114), tetrafluoroethane (HFA-134a), 1,1-difluoroethane (HFA-152a), difluoromethane (HFA-32), pentafluoroethane (HFA-12), heptafluoropropane (HFA-227a), perfluoropropane, perfluorobutane, perfluoropentane, butane, isobutane, and pentane. Aerosols comprising a compound of formula (I) or a pharmaceutically acceptable salt thereof will typically be administered to a patient via a metered dose inhaler (MDI). Such devices are known to those skilled in the art.

The aerosol may contain additional pharmaceutically-acceptable excipients typically used with MDIs such as surfactants, lubricants, cosolvents and other excipients to improve the physical stability of the formulation, to improve valve performance, to improve solubility, or to improve taste.

There is thus provided as a further aspect of the invention a pharmaceutical aerosol formulation comprising a compound of formula (I) or a pharmaceutically acceptable salt thereof and a fluorocarbon or hydrogen-containing chlorofluorocarbon as propellant, optionally in combination with a surfactant and/or a cosolvent.

According to another aspect of the invention, there is provided a pharmaceutical aerosol formulation wherein the propellant is selected from 1,1,1,2-tetrafluoroethane, 1,1,1,2,3,3,3-heptafluoro-n-propane and mixtures thereof.

The formulations of the invention may be buffered by the addition of suitable buffering agents.

Capsules and cartridges for use in an inhaler or insufflator, of for example gelatine, may be formulated containing a powder mix for inhalation of a compound of formula (I) or a pharmaceutically acceptable salt thereof and a suitable powder base such as lactose or starch. Each capsule or cartridge may generally contain from 20µg to 10mg of the compound of formula (I) or pharmaceutically acceptable salt thereof. Alternatively, the compound of formula (I) or pharmaceutically acceptable salt thereof may be presented without excipients such as lactose.

The proportion of the active compound of formula (I) or pharmaceutically acceptable salt thereof in the local compositions according to the invention depends on the precise type of formulation to be prepared but will generally be within the range of from 0.001 to 10% by weight. Generally, for most types of preparations, the proportion used will be within the range of from 0.005 to 1%, for example from 0.01 to 0.5%. However, in powders for inhalation or insufflation the proportion used will normally be within the range of from 0.1 to 5%.

Aerosol formulations are preferably arranged so that each metered dose or "puff" of aerosol contains from 20µg to 10mg, preferably from 20µg to 2000µg, more preferably from about 20µg to 500µg of a compound of formula (I). Administration may be once daily or several times daily, for example 2, 3, 4 or 8 times, giving for example 1, 2 or 3 doses each time. The overall daily dose with an aerosol will be within the range from 100µg to 10mg, preferably from 200µg to 2000µg. The overall daily dose and the metered dose delivered by capsules and cartridges in an inhaler or insufflator will generally be double that delivered with aerosol formulations.

In the case of suspension aerosol formulations, the particle size of the particulate (e.g., micronised) drug should be such as to permit inhalation of substantially all the drug into the lungs upon administration of the aerosol formulation and will thus be less than 100 microns, desirably less than 20 microns, and in particular in the range of from 1 to 10 microns, such as from 1 to 5 microns, more preferably from 2 to 3 microns.

The formulations of the invention may be prepared by dispersal or dissolution of the medicament and a compound of formula (I) or a pharmaceutically acceptable salt thereof in the selected propellant in an appropriate container, for example, with the aid of sonication or a high-shear mixer. The process is desirably carried out under controlled humidity conditions.

The chemical and physical stability and the pharmaceutical acceptability of the aerosol formulations according to the invention may be determined by techniques well known to those skilled in the art. Thus, for example, the chemical stability of the components may be determined by HPLC assay, for example, after prolonged storage of the product. Physical stability data may be gained from other conventional analytical techniques such as, for example, by leak testing, by valve delivery assay (average shot weights per actuation), by dose reproducibility assay (active ingredient per actuation) and spray distribution analysis.

The stability of the suspension aerosol formulations according to the invention may be measured by conventional techniques, for example, by measuring flocculation size distribution using a back light scattering instrument or by measuring particle size distribution by cascade impaction or by the "twin impinger" analytical process. As used herein reference to the "twin impinger" assay means "Determination of the deposition of the emitted dose in pressurised inhalations using apparatus A" as defined in British Pharmacopaeia 1988, pages A204-207, Appendix XVII C. Such techniques enable the "respirable fraction" of the aerosol formulations to be calculated. One method used to calculate the "respirable fraction" is by reference to "fine particle fraction" which is the amount of active ingredient collected in the lower impingement chamber per actuation expressed as a percentage of the total amount of active ingredient delivered per actuation using the twin impinger method described above.

The term "metered dose inhaler" or MDI means a unit comprising a can, a secured cap covering the can and a formulation metering valve situated in the cap. MDI system includes a suitable channelling device. Suitable channelling devices comprise for example, a valve actuator and a cylindrical or cone-like passage through which medicament may be delivered from the filled canister via the metering valve to the nose or mouth of a patient such as a mouthpiece actuator.

MDI canisters generally comprise a container capable of withstanding the vapour pressure of the propellant used such as a plastic or plastic-coated glass bottle or preferably a metal can, for example, aluminium or an alloy thereof which may optionally be anodised, lacquer-coated and/or plastic-coated (for example incorporated herein by reference WO96/32099 wherein part or all of the internal surfaces are coated with one or more fluorocarbon polymers optionally in combination with one or more non-fluorocarbon polymers), which container is closed with a metering valve. The cap may be secured onto the can via ultrasonic welding, screw fitting or crimping. MDIs taught herein may be prepared by methods of the art (e.g. see Byron, above and WO96/32099). Preferably the canister is fitted with a cap assembly, wherein a drug-metering valve is situated in the cap, and said cap is crimped in place.

In one embodiment of the invention the metallic internal surface of the can is coated with a fluoropolymer, more preferably blended with a non-fluoropolymer. In another embodiment of the invention the metallic internal surface of the can is coated with a polymer blend of polytetrafluoroethylene (PTFE) and polyethersulfone (PES). In a further embodiment of the invention the whole of the metallic internal surface of the can is coated with a polymer blend of polytetrafluoroethylene (PTFE) and polyethersulfone (PES).

The metering valves are designed to deliver a metered amount of the formulation per actuation and incorporate a gasket to prevent leakage of propellant through the valve. The gasket may comprise any suitable elastomeric material such as, for example, low density polyethylene, chlorobutyl, bromobutyl, EPDM, black and white butadiene-acrylonitrile rubbers, butyl rubber and neoprene. Suitable valves are commercially available from manufacturers well known in the aerosol industry, for example, from Valois, France (e.g. DF10, DF30, DF60), Bespak plc, UK (e.g. BK300, BK357) and 3M-Neotechnic Ltd, UK (e.g. Spraymiser^{™}).

In various embodiments, the MDIs may also be used in conjunction with other structures such as, without limitation, overwrap packages for storing and containing the MDIs, including those described in U.S. Patent Nos. 6,119,853; 6,179,118; 6,315,112; 6,352,152; 6,390,291; and 6,679,374, as well as dose counter units such as, but not limited to, those described in U.S. Patent Nos. 6,360,739 and 6,431,168.

Conventional bulk manufacturing methods and machinery well known to those skilled in the art of pharmaceutical aerosol manufacture may be employed for the preparation of large-scale batches for the commercial production of filled canisters. Thus, for example, in one bulk manufacturing method for preparing suspension aerosol formulations a metering valve is crimped onto an aluminium can to form an empty canister. The particulate medicament is added to a charge vessel and liquefied propellant together with the optional excipients is pressure filled through the charge vessel into a manufacturing vessel. The drug suspension is mixed before recirculation to a filling machine and an aliquot of the drug suspension is then filled through the metering valve into the canister. In one example bulk manufacturing method for preparing solution aerosol formulations a metering valve is crimped onto an aluminium can to form an empty canister. The liquefied propellant together with the optional excipients and the dissolved medicament is pressure filled through the charge vessel into a manufacturing vessel.

In an alternative process, an aliquot of the liquefied formulation is added to an open canister under conditions which are sufficiently cold to ensure the formulation does not vaporise, and then a metering valve crimped onto the canister.

Typically, in batches prepared for pharmaceutical use, each filled canister is check-weighed, coded with a batch number and packed into a tray for storage before release testing.

Suspensions and solutions comprising a compound of formula (I) or a pharmaceutically acceptable salt thereof may also be administered to a patient via a nebulizer. The solvent or suspension agent utilized for nebulization may be any pharmaceutically-acceptable liquid such as water, aqueous saline, alcohols or glycols, e.g., ethanol, isopropylalcohol, glycerol, propylene glycol, polyethylene glycol, etc. or mixtures thereof. Saline solutions utilize salts which display little or no pharmacological activity after administration. Both organic salts, such as alkali metal or ammonium halogen salts, e.g., sodium chloride, potassium chloride or organic salts, such as potassium, sodium and ammonium salts or organic acids, e.g., ascorbic acid, citric acid, acetic acid, tartaric acid, etc. may be used for this purpose.

Other pharmaceutically-acceptable excipients may be added to the suspension or solution. The compound of formula (I) or pharmaceutically acceptable salt thereof may be stabilized by the addition of an inorganic acid, e.g., hydrochloric acid, nitric acid, sulphuric acid and/or phosphoric acid; an organic acid, e.g., ascorbic acid, citric acid, acetic acid, and tartaric acid, etc., a complexing agent such as EDTA or citric acid and salts thereof; or an antioxidant such as antioxidant such as vitamin E or ascorbic acid. These may be used alone or together to stabilize the compound of formula (I) or pharmaceutically acceptable salt thereof. Preservatives may be added such as benzalkonium chloride or benzoic acid and salts thereof. Surfactant may be added particularly to improve the physical stability of suspensions. These include lecithin, disodium dioctylsulphosuccinate, oleic acid and sorbitan esters.

In a further aspect, the invention is directed to a dosage form adapted for intranasal administration.

Formulations for administration to the nose may include pressurised aerosol formulations and aqueous formulations administered to the nose by pressurised pump. Formulations which are non-pressurised and adapted to be administered topically to the nasal cavity are of particular interest. Suitable formulations contain water as the diluent or carrier for this purpose. Aqueous formulations for administration to the lung or nose may be provided with conventional excipients such as buffering agents, tonicity modifying agents and the like. Aqueous formulations may also be administered to the nose by nebulisation.

The compounds of formula (I) or pharmaceutically acceptable salts thereof may be formulated as a fluid formulation for delivery from a fluid dispenser, for example a fluid dispenser having a dispensing nozzle or dispensing orifice through which a metered dose of the fluid formulation is dispensed upon the application of a user-applied force to a pump mechanism of the fluid dispenser. Such fluid dispensers are generally provided with a reservoir of multiple metered doses of the fluid formulation, the doses being dispensable upon sequential pump actuations. The dispensing nozzle or orifice may be configured for insertion into the nostrils of the user for spray dispensing of the fluid formulation into the nasal cavity. A fluid dispenser of the aforementioned type is described and illustrated in WO05/044354, the entire content of which is hereby incorporated herein by reference. The dispenser has a housing which houses a fluid discharge device having a compression pump mounted on a container for containing a fluid formulation. The housing has at least one finger-operable side lever which is movable inwardly with respect to the housing to cam the container upwardly in the housing to cause the pump to compress and pump a metered dose of the formulation out of a pump stem through a nasal nozzle of the housing. In one embodiment, the fluid dispenser is of the general type illustrated in Figures 30-40 of WO05/044354.

Pharmaceutical compositions adapted for intranasal administration wherein the carrier is a solid include a coarse powder having a particle size for example in the range 20 to 500 microns which is administered by rapid inhalation through the nasal passage from a container of the powder held close up to the nose. Suitable compositions wherein the carrier is a liquid, for administration as a nasal spray or as nasal drops, include aqueous or oil solutions of the compound of formula (I) or a pharmaceutically acceptable salt thereof.

Pharmaceutical compositions adapted for transdermal administration may be presented as discrete patches intended to remain in intimate contact with the epidermis of the patient for a prolonged period of time. For example, the active ingredient may be delivered from the patch by iontophoresis as generally described in Pharmaceutical Research, 3(6), 318 (1986).

Pharmaceutical compositions adapted for topical administration may be formulated as ointments, creams, suspensions, lotions, powders, solutions, pastes, gels, sprays, aerosols or oils.

Ointments, creams and gels, may, for example, be formulated with an aqueous or oily base with the addition of suitable thickening and/or gelling agent and/or solvents. Such bases may thus, for example, include water and/or an oil such as liquid paraffin or a vegetable oil such as arachis oil or castor oil, or a solvent such as polyethylene glycol. Thickening agents and gelling agents which may be used according to the nature of the base include soft paraffin, aluminium stearate, cetostearyl alcohol, polyethylene glycols, woolfat, beeswax, carboxypolymethylene and cellulose derivatives, and/or glyceryl monostearate and/or non-ionic emulsifying agents.

Lotions may be formulated with an aqueous or oily base and will in general also contain one or more emulsifying agents, stabilising agents, dispersing agents, suspending agents or thickening agents.

Powders for external application may be formed with the aid of any suitable powder base, for example, talc, lactose or starch. Drops may be formulated with an aqueous or non-aqueous base also comprising one or more dispersing agents, solubilising agents, suspending agents or preservatives.

Topical preparations may be administered by one or more applications per day to the affected area; over skin areas occlusive dressings may advantageously be used. Continuous or prolonged delivery may be achieved by an adhesive reservoir system.

For treatments of the eye or other external tissues, for example mouth and skin, the compositions may be applied as a topical ointment or cream. When formulated in an ointment, the compound of formula (I) or a pharmaceutically acceptable salt thereof may be employed with either a paraffinic or a water-miscible ointment base. Alternatively, the compound of formula (I) or pharmaceutically acceptable salt thereof may be formulated in a cream with an oil-in-water cream base or a water-in-oil base.

Pharmaceutical compositions adapted for parenteral administration include aqueous and non-aqueous sterile injection solutions which may contain antioxidants, buffers, bacteriostats and solutes which render the formulation isotonic with the blood of the intended recipient; and aqueous and non-aqueous sterile suspensions which may include suspending agents and thickening agents. The compositions may be presented in unit-dose or multi-dose containers, for example sealed ampoules and vials, and may be stored in a freeze-dried (lyophilized) condition requiring only the addition of the sterile liquid carrier, for example water for injections, immediately prior to use. Extemporaneous injection solutions and suspensions may be prepared from sterile powders, granules and tablets.

The compound and pharmaceutical formulations according to the invention may be used in combination with or include one or more other therapeutic agents, for example selected from anti-inflammatory agents, anticholinergic agents (particularly an M₁/M₂/M₃ receptor antagonist), β₂-adrenoreceptor agonists, antiinfective agents, such as antibiotics or antivirals, or antihistamines. The invention thus provides, in a further aspect, a combination comprising a compound of formula (I) or a pharmaceutically acceptable salt thereof together with one or more other therapeutically active agents, for example selected from an anti-inflammatory agent, such as a corticosteroid or an NSAID, an anticholinergic agent, a β₂-adrenoreceptor agonist, an antiinfective agent, such as an antibiotic or an antiviral, or an antihistamine. One embodiment of the invention encompasses combinations comprising a compound of formula (I) or a pharmaceutically acceptable salt thereof together with a β₂-adrenoreceptor agonist, and/or an anticholinergic, and/or a PDE-4 inhibitor, and/or an antihistamine.

One embodiment of the invention encompasses combinations comprising one or two other therapeutic agents.

It will be clear to a person skilled in the art that, where appropriate, the other therapeutic ingredient(s) may be used in the form of salts, for example as alkali metal or amine salts or as acid addition salts, or prodrugs, or as esters, for example lower alkyl esters, or as solvates, for example hydrates to optimise the activity and/or stability and/or physical characteristics, such as solubility, of the therapeutic ingredient. It will be clear also that, where appropriate, the therapeutic ingredients may be used in optically pure form.

In one embodiment, the invention encompasses a combination comprising a compound of formula (I) or a pharmaceutically acceptable salt thereof together with a β₂-adrenoreceptor agonist.

Examples of β₂-adrenoreceptor agonists include salmeterol (which may be a racemate or a single enantiomer such as the *R*-enantiomer), salbutamol (which may be a racemate or a single enantiomer such as the *R*-enantiomer), formoterol (which may be a racemate or a single duastereomer such as the *R,R-*diastereomer), salmefamol, fenoterol carmoterol, etanterol, naminterol, clenbuterol, pirbuterol, flerbuterol, reproterol, bambuterol, indacaterol, terbutaline and salts thereof, for example the xinafoate (1-hydroxy-2-naphthalenecarboxylate) salt of salmeterol, the sulphate salt or free base of salbutamol or the fumarate salt of formoterol. In one embodiment, long-acting β₂-adrenoreceptor agonists, for example, compounds which provide effective bronchodilation for about 12 hrs or longer, are preferred.

Other β₂-adrenoreceptor agonists include those described in WO 02/066422, WO 02/070490, WO 02/076933, WO 03/024439, WO 03/072539, WO 03/091204, WO 04/016578, WO 2004/022547, WO 2004/037807, WO 2004/037773, WO 2004/037768, WO 2004/039762, WO 2004/039766, WO01/42193 and WO03/042160.

Examples of β₂-adrenoreceptor agonists include:
3-(4-{[6-({(2*R*)-2-hydroxy-2-[4-hydroxy-3-(hydroxymethyl)phenyl]ethyl}amino) hexyl] oxy} butyl) benzenesulfonamide;
3-(3-{[7-({(2R)-2-hydroxy-2-[4-hydroxy-3-hydroxymethyl) phenyl] ethyl}-amino) heptyl] oxy} propyl) benzenesulfonamide;
4-{(1*R*)-2-[(6-{2-[(2, 6-dichlorobenzyl) oxy] ethoxy} hexyl) amino]-1-hydroxyethyl}-2-(hydroxymethyl) phenol;
4-{(1*R*)-2-[(6-{4-[3-(cyclopentylsulfonyl)phenyl]butoxy}hexyl)amino]-1-hydroxyethyl}-2-(hydroxymethyl)phenol;
N-[2-hydroxyl-5-[(1*R*)-1-hydroxy-2-[[2-4-[[(2*R*)-2-hydroxy-2-phenylethyl]amino]phenyl]ethyl]amino]ethyl]phenyl]formamide;
N-2{2-[4-(3-phenyl-4-methoxyphenyl)aminophenyl]ethyl}-2-hydroxy-2-(8-hydroxy-2(1*H*)-quinolinon-5-yl)ethylamine; and
5-[(*R*)-2-(2-{4-[4-(2-amino-2-methyl-propoxy)-phenylamino]-phenyl}-ethylamino)-1-hydroxy-ethyl]-8-hydroxy-1H-quinolin-2-one.

The β₂-adrenoreceptor agonist may be in the form of a salt formed with a pharmaceutically acceptable acid selected from sulphuric, hydrochloric, fumaric, hydroxynaphthoic (for example 1- or 3-hydroxy-2-naphthoic), cinnamic, substituted cinnamic, triphenylacetic, sulphamic, sulphanilic, naphthaleneacrylic, benzoic, 4-methoxybenzoic, 2- or 4-hydroxybenzoic, 4-chlorobenzoic and 4-phenylbenzoic acid.

Suitable anti-inflammatory agents include corticosteroids. Suitable corticosteroids which may be used in combination with the compounds of formula (I) or pharmaceutically acceptable salts thereof are those oral and inhaled corticosteroids and their pro-drugs which have anti-inflammatory activity. Examples include methyl prednisolone, prednisolone, dexamethasone, fluticasone propionate, 6α,9α-difluoro-11β-hydroxy-16α-methyl-17α-[(4-methyl-1,3-thiazole-5-carbonyl)oxy]-3-oxo-androsta-1,4-diene-17β-carbothioic acid *S*-fluoromethyl ester, 6α,9α-difluoro-17α-[(2-furanylcarbonyl)oxy]-11β-hydroxy-16α-methyl-3-oxo-androsta-1,4-diene-17β-carbothioic acid *S*-fluoromethyl ester (fluticasone furoate), 6α,9α-difluoro-11β-hydroxy-16α-methyl-3-oxo-17α-propionyloxy- androsta-1,4-diene-17β-carbothioic acid *S*-(2-oxo-tetrahydro-furan-3S-yl) ester, 6α,9α-difiuoro-11β-hydroxy-16α-methyl-3-oxo-17α-(2,2,3,3- tetramethycyclopropylcarbonyl)oxy-androsta-1,4-diene-177β-carbothioic acid *S*-cyanomethyl ester and 6α,9-difluoro-11β-hydroxy-16α-methyl-17α-(1-methycyclopropylcarbonyl)oxy-3-oxo-androsta-1,4-diene-17β-carbothioic acid *S*-fluoromethyl ester, beclomethasone esters (for example the 17-propionate ester or the 17,21-dipropionate ester), budesonide, flunisolide, mometasone esters (for example mometasone furoate), triamcinolone acetonide, rofleponide, ciclesonide (16α,17-[[(*R*)-cyclohexylmethylene]bis(oxy)]-11β,21-dihydroxy-pregna-1,4-diene-3,20-dione), butixocort propionate, RPR-106541, and ST-126. Preferred corticosteroids include fluticasone propionate, 6α,9α-difluoro-11β-hydroxy-16α-methyl-17α-[(4-methyl-1,3-thiazole-5-carbonyl)oxy]-3-oxo-androsta-1,4-diene-17β-carbothioic acid *S*-fluoromethyl ester, 6α,9α-difluoro-17α-[(2-furanylcarbonyl)oxy]-11β-hydroxy-16α-methyl-3-oxo-androsta-1,4-diene-17β-carbothioic acid *S*-fluoromethyl ester, 6α,9α-difluoro-11β-hydroxy-16α-methyl-3-oxo-17α-(2,2,3,3- tetramethycyclopropylcarbonyl)oxy-androsta-1,4-diene-17β-carbothioic acid *S*-cyanomethyl ester and 6α,9α-difluoro-11β-hydroxy-16α-methyl-17α-(1-methycyclopropylcarbonyl)oxy-3-oxo-androsta-1,4-diene-17β-carbothioic acid *S*-fluoromethyl ester. In one embodiment the corticosteroid is 6α,9α-difluoro-17α-[(2-furanylcarbonyl)oxy]-11β-hydroxy-16α-methyl-3-oxo-androsta-1,4-diene-17β-carbothioic acid *S*-fluoro,methyl ester.

Examples of corticosteroids may include those described in WO2002/088167, WO2002/100879, WO2002/12265, W02002/12266, W02005/005451, WO2005/005452, WO2006/072599 and WO2006/072600.

Non-steroidal compounds having glucocorticoid agonism that may possess selectivity for transrepression over transactivation and that may be useful in combination therapy include those covered in the following patents: WO03/082827, WO98/54159, WO04/005229, WO04/009017, WO04/018429, WO03/104195, WO03/082787, WO03/082280, WO03/059899, WO03/101932, WO02/02565, WO01/16128, WO00/66590, WO03/086294, WO04/026248, WO03/061651 and WO03/08277. Further non-steroidal compounds are covered in: W02006/000401, W02006/000398 and WO2006/015870.

Examples of anti-inflammatory agents include non-steroidal anti-inflammatory drugs (NSAID's).

Examples of NSAID's include sodium cromoglycate, nedocromil sodium, phosphodiesterase (PDE) inhibitors (for example, theophylline, PDE4 inhibitors or mixed PDE3/PDE4 inhibitors), leukotriene antagonists, inhibitors of leukotriene synthesis (for example montelukast), iNOS inhibitors, tryptase and elastase inhibitors, beta-2 integrin antagonists and adenosine receptor agonists or antagonists (e.g. adenosine 2a agonists), cytokine antagonists (for example chemokine antagonists, such as a CCR3 antagonist) or inhibitors of cytokine synthesis, or 5-lipoxygenase inhibitors. An iNOS (inducible nitric oxide synthase inhibitor) is preferably for oral administration. Examples of iNOS inhibitors include those disclosed in WO93/13055, WO98/30537, WO02/50021, WO95/34534 and WO99/62875. Examples of CCR3 inhibitors include those disclosed in WO02/26722.

In one embodiment, the invention provides the use of the compounds of formula (I) in combination with a phosphodiesterase 4 (PDE4) inhibitor, especially in the case of a formulation adapted for inhalation. The PDE4-specific inhibitor useful in this aspect of the invention may be any compound that is known to inhibit the PDE4 enzyme or which is discovered to act as a PDE4 inhibitor, and which are only PDE4 inhibitors, not compounds which inhibit other members of the PDE family, such as PDE3 and PDE5, as well as PDE4.

Compounds include *cis*-4-cyano-4-(3-cyclopentyloxy-4-methoxyphenyl)cyclohexan-1-carboxylic acid, 2-carbomethoxy-4-cyano-4-(3-cyclopropylmethoxy-4-difluoromethoxyphenyl)cyclohexan-1-one and *cis*-[4-cyano-4-(3-cyclopropylmethoxy-4-difluoromethoxyphenyl)cyclohexan-1-ol]. Also, *cis*-4-cyano-4-[3-(cyclopentyloxy)-4-methoxyphenyl]cyclohexane-1-carboxylic acid (also known as cilomilast) and its salts, esters, pro-drugs or physical forms, which is described in U.S. patent 5,552,438 issued 03 September, 1996; this patent and the compounds it discloses are incorporated herein in full by reference.

Other compounds include AWD-12-281 from Elbion (Hofgen, N. et al. 15th EFMC Int Symp Med Chem (Sept 6-10, Edinburgh) 1998, Abst P.98; CAS reference No. 247584020-9); a 9-benzyladenine derivative nominated NCS-613 (INSERM); D-4418 from Chiroscience and Schering-Plough; a benzodiazepine PDE4 inhibitor identified as CI-1018 (PD-168787) and attributed to Pfizer; a benzodioxole derivative disclosed by Kyowa Hakko in WO99/16766; K-34 from Kyowa Hakko; V-11294A from Napp (Landells, L.J. et al. Eur Resp J [Annu Cong Eur Resp Soc (Sept 19-23, Geneva) 1998] 1998, 12 (Suppl. 28): Abst P2393); roflumilast (CAS reference No 162401-32-3) and a pthalazinone (WO99/47505, the disclosure of which is hereby incorporated by reference) from Byk-Gulden; Pumafentrine, (-)-p-[(4aR*,10bS*)-9-ethoxy-1,2,3,4,4a,10b-hexahydro-8-methoxy-2-methylbenzo[c][1,6]naphthyridin-6-yl]-N,N-diisopropylbenzamide which is a mixed PDE3/PDE4 inhibitor which has been prepared and published on by Byk-Gulden, now Altana; arofylline under development by Almirall-Prodesfarma; VM554/UM565 from Vernalis; or T-440 (Tanabe Seiyaku; Fuji, K. et al. J Pharmacol Exp Ther,1998, 284(1): 162), and T2585.

Further compounds are disclosed in the published international patent application WO04/024728 (Glaxo Group Ltd), WO04/056823 (Glaxo Group Ltd) and WO04/103998 (Glaxo Group Ltd) (e.g. Example 399 or 544 disclosed therein). Further compounds are also disclosed in WO2005/058892, WO2005/090348, W02005/090353, and W02005/090354, all in the name of Glaxo Group Limited.

Examples of anticholinergic agents are those compounds that act as antagonists at the muscarinic receptors, in particular those compounds which are antagonists of the M₁ or M₃ receptors, dual antagonists of the M₁/M₃ or M₂/M₃, receptors or pan-antagonists of the M₁/M₂/M₃ receptors. Exemplary compounds for administration via inhalation include ipratropium (for example, as the bromide, CAS 22254-24-6, sold under the name Atrovent), oxitropium (for example, as the bromide, CAS 30286-75-0) and tiotropium (for example, as the bromide, CAS 136310-93-5, sold under the name Spiriva). Also of interest are revatropate (for example, as the hydrobromide, CAS 262586-79-8) and LAS-34273 which is disclosed in WO01/04118. Exemplary compounds for oral administration include pirenzepine (CAS 28797-61-7), darifenacin (CAS 133099-04-4, or CAS 133099-07-7 for the hydrobromide sold under the name Enablex), oxybutynin (CAS 5633-20-5, sold under the name Ditropan), terodiline (CAS 15793-40-5), tolterodine (CAS 124937-51-5, or CAS 124937-52-6 for the tartrate, sold under the name Detrol), otilonium (for example, as the bromide, CAS 26095-59-0, sold under the name Spasmomen), trospium chloride (CAS 10405-02-4) and solifenacin (CAS 242478-37-1, or CAS 242478-38-2 for the succinate also known as YM-905 and sold under the name Vesicare).

Additional compounds are disclosed in WO 2005/037280, WO 2005/046586 and WO 2005/104745, incorporated herein by reference. The present combinations include, but are not limited to:
(3-*endo*)-3-(2,2-di-2-thienylethenyl)-8,8-dimethyl-8-azoniabicyclo[3.2.1]octane iodide;
(3-*endo*)-3-(2-cyano-2,2-diphenylethyl)-8,8-dimethyl-8-azoniabicyclo[3.2.1]octane bromide;
4-[hydroxy(diphenyl)methyl]-1-{2-[(phenylmethyl)oxy]ethyl}-1-azoniabicyclo[2.2.2]octane bromide; and
(1*R*,5*S*)-3-(2-cyano-2,2-diphenylethyl)-8-methyl-8-{2-[(phenylmethyl)oxy]ethyl}-8-azoniabicyclo[3.2.1]octane bromide.

Other anticholinergic agents include compounds which are disclosed in US patent application 60/487981 including, for example:
(3-*endo*)-3-(2,2-di-2-thienylethenyl)-8,8-dimethyl-8-azoniabicyclo[3.2.1]octane bromide;
(3-*endo*)-3-(2,2-diphenylethenyl)-8,8-dimethyl-8-azoniabicyclo[3.2.1]octane bromide;
(3-*endo*)-3-(2,2-diphenylethenyl)-8,8-dimethyl-8-azoniabicyclo[3.2.1]octane 4-methylbenzenesulfonate;
(3-endo)-8,8-dimethyl-3-[2-phenyl-2-(2-thienyl)ethenyl]-8-azoniabicyclo[3.2.1]octane bromide; and/or
(3-*endo*)-8,8-dimethyl-3-[2-phenyl-2-(2-pyridinyl)ethenyl]-8-azoniabicyclo[3.2.1]octane bromide.

Further anticholinergic agents include compounds which are disclosed in US patent application 60/511009 including, for example:
(*endo*)-3-(2-methoxy-2,2-di-thiophen-2-yl-ethyl)-8,8-dimethyl-8-azonia-bicyclo[3.2.1]octane iodide;
3-((*endo*)-8-methyl-8-aza-bicyclo[3.2.1]oct-3-yl)-2,2-diphenyl-propionitrile;
(*endo*)-8-methyl-3-(2,2,2-triphenyl-ethyl)-8-aza-bicyclo[3.2.1]octane;
3-((*endo*)-8-methyl-8-aza-bicyclo[3.2.1]oct-3-yl)-2,2-diphenyl-propionamide;
3-((*endo*)-8-methyl-8-aza-bicyclo[3.2.1]oct-3-yl)-2,2-diphenyl-propionic acid;
(*endo*)-3-(2-cyano-2,2-diphenyl-ethyl)-8,8-dimethyl-8-azonia-bicyclo[3.2.1]octane iodide;
(*endo*)-3-(2-cyano-2,2-diphenyl-ethyl)-8,8-dimethyl-8-azonia-bicyclo[3.2.1]octane bromide;
3-((*endo*)-8-methyl-8-aza-bicyclo[3.2.1]oct-3-yl)-2,2-diphenyl-propan-1-ol; N-benzyl-3-((*endo*)-8-methyl-8-aza-bicyclo[3.2.]oct-3-yl)-2,2-diphenyl-propionamide;
(*endo*)-3-(2-carbamoyl-2,2-diphenyl-ethyl)-8,8-dimethyl-8-azonia-bicyclo[3.2.1]octane iodide;
1-benzyl-3-[3-((*endo*)-8-methyl-8-aza-bicyclo[3.2.1]oct-3-yl)-2,2-diphenyl-propyl]-urea;
1-ethyl-3-[3-((*endo*)-8-methyl-8-aza-bicyclo[3.2.1]oct-3-yl)-2,2-diphenyl-propyl]-urea;
*N*-[3-((*endo*)-8-methyl-8-aza-bicyclo[3.2.1]oct-3-yl)-2,2-diphenyl-propyl]-acetamide;
*N*-[3-((*endo*)-8-methyl-8-aza-bicyclo[3.2.1]oct-3-yl)-2,2-diphenyl-propyl]-benzamide;
3-((*endo*)-8-methyl-8-aza-bicyclo[3.2.1]oct-3-yl)-2,2-di-thiophen-2-yl-propionitrile;
(*endo*)-3-(2-cyano-2,2-di-thiophen-2-yl-ethyl)-8,8-dimethyl-8-azonia-bicyclo[3.2.1]octane iodide;
*N*-[3-((*endo*)-8-methyl-8-aza-bicyclo[3.2.1]oct-3-yl)-2,2-diphenyl-propyl]-benzenesulfonamide;
[3-((*endo*)-8-methyl-8-aza-bicyclo[3.2.1]oct-3-yl)-2,2-diphenyl-propyl]-urea;
*N*-[3-((*endo*)-8-methyl-8-aza-bicyclo[3.2.1]oct-3-yl)-2,2-diphenyl-propyl]-methanesulfonamide; and/or
(*endo*)-3-{2,2-diphenyl-3-[(1-phenyl-methanoyl)-amino]-propyl}-8,8-dimethyl-8-azonia-bicyclo[3.2.1]octane bromide.

Further compounds include:
(*endo*)-3-(2-methoxy-2,2-di-thiophen-2-yl-ethyl)-8,8-dimethyl-8-azonia-bicyclo[3.2.1]octane iodide;
(*endo*)-3-(2-cyano-2,2-diphenyl-ethyl)-8,8-dimethyl-8-azonia-bicyclo[3.2.1]octane iodide;
(*endo*)-3-(2-cyano-2,2-diphenyl-ethyl)-8,8-dimethyl-8-azonia-bicyclo[3.2.1]octane bromide;
(*endo*)-3-(2-carbamoyl-2,2-diphenyl-ethyl)-8,8-dimethyl-8-azonia-bicyclo[3.2.1]octane iodide;
(*endo*)-3-(2-cyano-2,2-di-thiophen-2-yl-ethyl)-8,8-dimethyl-8-azonia-bicyclo[3.2.1]octane iodide; and/or
(*endo*)-3-{2,2-diphenyl-3-[(1-phenyl-methanoyl)-amino]-propyl}-8,8-dimethyl-8-azonia-bicyclo[3.2.1]octane bromide.

In one embodiment the invention provides a combination comprising a compound of formula (I) or a pharmaceutically acceptable salt thereof together with an H1 antagonist. Examples of H1 antagonists include, without limitation, amelexanox, astemizole, azatadine, azelastine, acrivastine, brompheniramine, cetirizine, levocetirizine, efletirizine, chlorpheniramine, clemastine, cyclizine, carebastine, cyproheptadine, carbinoxamine, descarboethoxyloratadine, doxylamine, dimethindene, ebastine, epinastine, efletirizine, fexofenadine, hydroxyzine, ketotifen, loratadine, levocabastine, mizolastine, mequitazine, mianserin, noberastine, meclizine, norastemizole, olopatadine, picumast, pyrilamine, promethazine, terfenadine, tripelennamine, temelastine, trimeprazine and triprolidine, particularly cetirizine, levocetirizine, efletirizine and fexofenadine. In a further embodiment the invention provides a combination comprising a compound of formula (I) or a pharmaceutically acceptable salt thereof together with an H3 antagonist (and/or inverse agonist). Examples of H3 antagonists include, for example, those compounds disclosed in WO2004/035556 and in W02006/045416. Other histamine receptor antagonists which may be used in combination with the compounds of the present invention include antagonists (and/or inverse agonists) of the H4 receptor, for example, the compounds disclosed in Jablonowski et al., J. Med. Chem. 46:3957-3960 (2003).

The invention thus provides, in a further aspect, a combination comprising a compound of formula (I) or a pharmaceutically acceptable salt thereof together with a PDE4 inhibitor.

The invention thus provides, in a further aspect, a combination comprising a compound of formula (I) or a pharmaceutically acceptable salt thereof together with a β₂-adrenoreceptor agonist.

The invention thus provides, in a further aspect, a combination comprising a compound of formula (I) or a pharmaceutically acceptable salt thereof together with a corticosteroid.

The invention thus provides, in a further aspect, a combination comprising a compound of formula (I) or a pharmaceutically acceptable salt thereof together with a non-steroidal GR agonist.

The invention thus provides, in a further aspect, a combination comprising a compound of formula (I) or a pharmaceutically acceptable salt thereof together with an anticholinergic.

The invention thus provides, in a further aspect, a combination comprising a compound of formula (I) or a pharmaceutically acceptable salt thereof together with an antihistamine.

The invention thus provides, in a further aspect, a combination comprising a compound of formula (I) or a pharmaceutically acceptable salt thereof together with a PDE4 inhibitor and a β₂-adrenoreceptor agonist.

The invention thus provides, in a further aspect, a combination comprising a compound of formula (I) or a pharmaceutically acceptable salt thereof together with an anticholinergic and a PDE-4 inhibitor.

The combinations referred to above may conveniently be presented for use in the form of a pharmaceutical composition and thus pharmaceutical compositions comprising a combination as defined above together with a pharmaceutically acceptable diluent or carrier represent a further aspect of the invention.

The individual compounds of such combinations may be administered either sequentially or simultaneously in separate or combined pharmaceutical formulations. In one embodiment, the individual compounds will be administered simultaneously in a combined pharmaceutical formulation. Appropriate doses of known therapeutic agents will readily be appreciated by those skilled in the art.

The invention thus provides, in a further aspect, a pharmaceutical composition comprising a combination of a compound of formula (I) or a pharmaceutically acceptable salt thereof together with another therapeutically active agent.

The invention thus provides, in a further aspect, a pharmaceutical composition comprising a combination of a compound of formula (I) or a pharmaceutically acceptable salt thereof together with a PDE4 inhibitor.

The invention thus provides, in a further aspect, a pharmaceutical composition comprising a combination of a compound of formula (I) or a pharmaceutically acceptable salt thereof together with a β₂-adrenoreceptor agonist.

The invention thus provides, in a further aspect, a pharmaceutical composition comprising a combination of a compound of formula (I) or a pharmaceutically acceptable salt thereof together with a corticosteroid.

The invention thus provides, in a further aspect, a pharmaceutical composition comprising a combination of a compound of formula (I) or a pharmaceutically acceptable salt thereof together with a non-steroidal GR agonist.

The invention thus provides, in a further aspect, a pharmaceutical composition comprising a combination of a compound of formula (I) or a pharmaceutically acceptable salt thereof together with an anticholinergic.

The invention thus provides, in a further aspect, a pharmaceutical composition comprising a combination of a compound of formula (I) or a pharmaceutically acceptable salt thereof together with an antihistamine.

The invention thus provides, in a further aspect, a pharmaceutical composition comprising a combination of a compound of formula (I) or a pharmaceutically acceptable salt thereof together with a PDE4 inhibitor and a β₂-adrenoreceptor agonist.

The invention thus provides, in a further aspect, a pharmaceutical composition comprising a combination of a compound of formula (I) or a pharmaceutically acceptable salt thereof together with an anticholinergic and a PDE4 inhibitor.

The invention will now be illustrated by way of the following non-limiting examples.

### EXAMPLES

The following examples illustrate the invention. These examples are not intended to limit the scope of the present invention, but rather to provide guidance to the skilled artisan to prepare and use the compounds, compositions, and methods of the present invention. While particular embodiments of the present invention are described, the skilled artisan will appreciate that various changes and modifications can be made without departing from the spirit and scope of the invention.

Unless otherwise noted, all starting materials were obtained from commercial suppliers and used without further purification. Unless otherwise indicated, all temperatures are expressed in °C (degrees Centigrade). All reactions are conducted under an inert atmosphere at room temperature unless otherwise noted. All references to ether are to diethyl ether; brine refers to a saturated aq. solution of NaCl.

¹H NMR spectra were recorded using a Bruker DPX 400MHz, referenced to tetramethylsilane.

LC/MS was conducted using either Method A or Method B:
Method A: LC/MS (5min system) was conducted on a Supelcosil LCABZ+PLUS column (3.3 cm x 4.6 mm ID) eluting with 0.1% HCO₂H and 0.01 M ammonium acetate in water (solvent A) and 0.05% HCO₂H 5% water in acetonitrile (solvent B), using the following elution gradient 0.0-0.7min 0%B, 0.7-4.2min 0-100%B, 4.2-4.6min 100%B, 4.6-4.8min 100-0%B at a flow rate of 3ml/min. The mass spectra were recorded on a Waters ZQ Mass spectrometer using electrospray positive and negative mode (ES+ve and ES-ve)
Method B: LC/MS (2min system) was conducted on a Acquity UPLC BEH C₁₈ column (5.0 cm x 2.1 mm) at 40°C, eluting with 0.1% HCO₂H and 0.01 M ammonium acetate in water (solvent A) and 0.05% HCO₂H 5% water in acetonitrile (solvent B), using the following elution gradient 0.0-0.1min 3%B, 0.1-1.4min 3-100%B, 1.4-1.9min 100%B, 1.9-2min 3%B at a flow rate of 1ml/min. The mass spectra were recorded on a Waters ZQ Mass spectrometer using electrospray with pos negative switching (ES+ve and ES-ve).

In the LCMS data reported herein, the mass ion was mathematically rounded to the nearest integer.

"Mass directed autoprep" / "MDAP" / "preparative mass directed HPLC" was conducted on a system such as; a Waters *FractionLynx* system comprising of a Waters 600 pump with extended pump heads, Waters 2700 autosampler, Waters 996 diode array and Gilson 202 fraction collector on a 10 cm 2.54 cm ID ABZ+ column, eluting with either 0.1% formic acid or TFA in water (solvent A) and 0.1 % formic or TFA in acetonitrile (solvent B) using the appropriate elution gradient. Mass spectra were recorded on Micromass ZMD mass spectrometer using electrospray positive and negative mode, alternate scans. The software used was *MassLynx* 3.5 with *OpenLynx* and *FractionLynx* optio or using equivalent alternative systems.

"Hydrophobic frits" refers to filtration tubes sold by Whatman. SPE (solid phase extraction, SCX-2 and aminopropyl) refers to the use of cartridges sold by International Sorbent Technology Ltd. The Flashmaster II is an automated multi-user flash chromatography system, available from Argonaut Technologies Ltd, which utilises disposable, normal phase, SPE cartridges (2 g to 100 g). It provides quaternary on-line solvent mixing to enable gradient methods to be run. Samples are queued using the multi-functional open access software, which manages solvents, flow-rates, gradient profile and collection conditions. The system is equipped with a Knauer variable wavelength uv-detector and two Gilson FC204 fraction-collectors enabling automated peak cutting, collection and tracking.

Silica chromatography techniques include either automated (Flashmaster) techniques or manual chromatography on pre-packed cartridges (SPE) or manually-packed flash columns.

Microwave chemistry was typically performed in sealed vessels, irradiating with a suitable microwave reactor system, such as a Biotage Initiator^{™} Microwave Synthesiser.

When the name of a commercial supplier is given after the name of a compound or a reagent, for instance "compound X (Aldrich)" or "compound X / Aldrich", this means that compound X is obtainable from a commercial supplier, such as the commercial supplier named. For example, 1,1'-bis(diphenylphosphino) ferrocenedichloro palladium(II), complex with dichloromethane may be purchased from Acros, and tetrabutylammonium fluoride (1M solution in tetrahydrofuran) and trifuoroacetic acid may be purchased from Aldrich. H cubes are commercially available from, for example, Asynt.

Similarly, when a literature or a patent reference is given after the name of a compound, for instance compound Y (EP 0 123 456), this means that the preparation of the compound is described in the named reference.

The names of the Examples have been obtained from the structures using the compound naming programme "ACD Name Pro 6.02".

### Intermediate 1

### S-(2-Amino-2-oxoethyl) O-ethyl dithiocarbonate

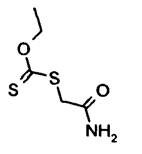

Ethylxanthic acid (17.6g, 110mmol) was stirred in acetone (200ml) and treated with a solution of iodoacetamide (18.5g, 100mmol) in acetone (50ml). The reaction was stirred at room temperature for 3 hrs. The suspension was filtered and the filtrate was evaporated. The cream solid was dissolved in EtOAc (200ml) and washed with water (2x50ml). The organic phase was evaporated to give the title compound as a white solid (17.2g).
MH+180, rt= 2.06 mins

### Intermediate 2

### 1,1-Dimethylethyl {[(ethyloxy)carbonothloyl]thio}acetate

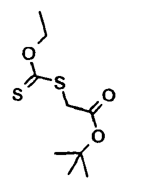

To ethylxanthic acid (90.3g, 564mmol) in acetone (800ml) was added bromoacetate (100g, 512mmol) in acetone (200ml) over 20 mins. After stirring overnight, the reaction was concentrated *in vacuo* to afford the title compound (114.8g, 95%).
MH-235, rt= 3.33 mins

### Intermediate 3

### 2-(4-Bromo-1H-pyrrolo[2,3-b]pyridin-2-yl)acetamide

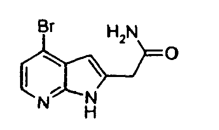

4-Bromoazaindole (which may be prepared, for example, as described in Org Lett, 2003, 5(26), 5023-5026) (1.0g, 5mmol) and *S*-(2-amino-2-oxoethyl) O-ethyl dithiocarbonate (1.1g, 6mmol) were dissolved in DCE (10ml) and the mixture was degassed then heated at reflux. The solution was treated by a slow addition of lauroyl peroxide (2.4g, 6mmol) in DCE (20ml) over 4 hrs. The brown solid was collected and purified by MDAP. The main fraction was evaporated to give the title compound as a white solid (0.123g).
¹H NMR (400MHz; CDCl₃) δ: 3.6 (2H, s), 6.24 (1H, s), 7.06 (1H, br s), 7.27 (1H, d), 7.49 (1H, br s), 8.00 (1H, d), 11.90 (1H, br s).

### Intermediate 4

### 4-Bromo-2-iodo-1-[(4-methylphenyl)sulfonyl]-1H-pyrrolo[2,3-b]pyridine

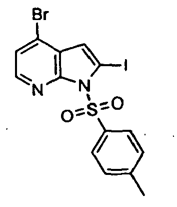

n-Bulithium (63.2 mL of a 2.5 M solution in hexanes, 0.158 mol) was added by syringe to a solution of diisopropylamine (23.8 mL, 0.17 mol) in anhydrous THF (717 mL) at 0°C and upon complete addition, the mixture was cooled to -78°C. 4-Bromo-1-[(4-methylphenyl)sulfonyl]-1*H*-pyrrolo[2,3-*b*]pyridine (52.8 g, 0.15 mol) was added, the resultant reaction mixture was stirred at -78°C for 1.5 h, and iodine (50.13 g, 0.196 mol) was then added. After being allowed to stir at -78°C for a further 20 min, the reaction mixture was allowed to warm to -20°C and was quenched by the addition of saturated NH₄Cl solution (226 mL). Upon reaching room temperature, the organic phase was separated, washed with saturated NH₄Cl solution, dried (Na₂SO₄), and the solvent was removed under reduced pressure to afford the crude product. This was purified by repeated slurrying at 5°C in MeOH (3 x 190 mL) to give 4-bromo-2-iodo-1-[(4-methylphenyl)sulfonyl]-1*H*-pyrrolo[2,3-*b*]pyridine (50.23 g, 70%) as a brown solid, mp 140-143°C.

### Intermediate 5

### {4-Bromo-1-[(4-methylphenyl)sulfonyl]-1H-pyrrolo[2,3-b]pyridin-2-yl}methanol

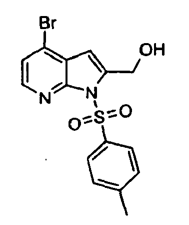

A solution of 4-bromo-2-iodo-1-[(4-methylphenyl)sulfonyl]-1*H*-pyrrolo[2,3-*b*]pyridine (0.300g,0.63mmol) in THF (1.2ml) was cooled to -20°C (ice/salt) under nitrogen and was treated dropwise with isopropyl magnesium chloride (2M, 0.33ml, 0.66mmol). The solution was stirred at -20°C for 1 hour and was added dropwise to a suspension of paraformaldehyde in THF (1ml) at -20°C. The mixture was warmed to room temperature and stirred for 16 hrs. Aq. ammonium chloride (10ml) was added and the mixture extracted with EtOAc (2x10ml). The dried (MgSO₄) extract was evaporated and the residue was purified on a column of silica (10g) eluted with DCM to 50% EtOAc in DCM to give the title compound as a colourless gum (0.160g, 67%).
TLC, SiO₂, DCM, Rf=0.05, det=uv and KMnO₄

### Intermediate 6

### Pentafluorophenyl 4-(4,4,5,5-tetramethyl-1,3,2-dioxaborolan-2-yl)benzenesulfonate

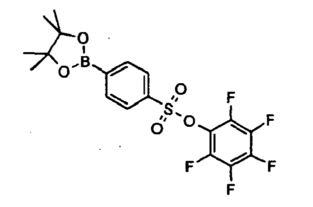

To a solution of 4-bromo-pentafluorphenylsulphonate ester (which may be prepared, for example, as described in Org Letts, 2005, 7, 843) (25.00g, 62 mmol) in 1,4-dioxane (875ml) were added pinacolato diboron (17.27g, 68.2 mmol, 1.1 eq), PdCl₂(dppf) catalyst (1.52g, 1.86 mmol, 0.03 eq), dppf (1.03g, 1.86 mmol, 0.03 eq) and NaOAc (30.52g, 372 mmol, 6 eq) to form a orange/red suspension. The reaction was stirred at reflux (105°C) for 16 hrs. After this period hplc analysis showed the reaction had gone to completion, with no start material observed. The reaction mixture was allowed to cool to room temperature and the dark inorganic material removed by filtration, washing the material with DCM (3 x 200ml). The combined organic filtrate was concentrated *in vacuo* to yield a black tar like residue (approx. 45g). The material was suspended/washed in water and vigorously stirred to help break up the solid material. The suspension was then extracted with Et₂O (3 x 300ml). The combined Et₂O extracts were combined, washed with brine, dried on MgSO₄ and filtered. The organic filtrate was concentrated *in vacuo* to yield a orange / red solid residue. The residue was boiled in hexane (3 x 300ml) and filtered through fluted filter paper while still hot (the product is soluble in hot hexane). The combined hexane filtrates were concentrated *in vacuo,* and the residue re-crystallised using a minimum volume of hot MTBE (approx. 200 ml). Upon allowing the MTBE to cool to room temperature a fine precipitate formed, which was filtered, washed with cold MTBE and dried under vacuum to yield the product as a pale cream solid (3.579g).

¹H NMR (400MHz; CDCl₃) δ ppm: 8.03 (2H, d), 7.96 (2H, d), 1.39 (12H, s).

The MTBE filtrate was concentrated *in vacuo* and re-crystallised again using a minimum volume of hot MTBE to yield a further 0.518g of title compound. The re-crystallisation procedure was repeated twice more, this time using 50:50 Hexane : Et₂O. These procedures accumulated a further 6.149g of title compound. Batches were combined to afford the title compound as a solid (10.21g).

### Intermediate 7

### 1,1-Dimethylethyl (2-{[(4-{2-(hydroxymethyl)-1-[(4-methylphenyl)sulfonyl]-1H-pyrrolo[2,3-b]pyridin-4-yl}phenyl)sulfonyl]amino}ethyl)carbamate

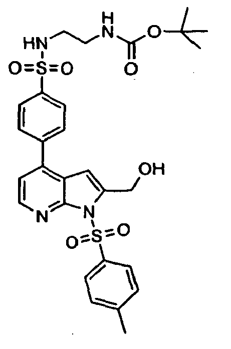

A mixture of pentafluorophenyl 4-(4,4,5,5-tetramethyl-1,3,2-dioxaborolan-2-yl)benzenesulfonate (0.330g, 0.75mmol), 1,1-dimethylethyl(2-aminoethyl)carbamate (0.130g, 0.8mmol) and TEA (0.5ml) was heated in a sealed vessel at 120°C for 10 mins. The dark solution was treated with a solution of {4-bromo-1-[(4-methylphenyl)sulfonyl]-1*H-*pyrrolo[2,3-*b*]pyridin-2-yl}methanol (0.160g, 0.42mmol) in dioxan / water (2.5ml), 1,1'-bis(diphenylphosphino)ferrocenedichloropalladium(II) (0.020g, 0.027mmol) and sodium carbonate (0.085g, 0.8mmol) and the mixture heated in the mw at 150°C (130W Max power) for 20 mins. The mixture was added to water (150ml) and extracted with EtOAc (100ml) and EtOAc / methanol (19:1, 100ml). The dried (MgSO₄) extract was evaporated and the residue was purified on a silica cartridge (10g) eluted with DCM to 50% EtOAc in DCM to give the title compound as a foam (0.215g, 71%).
MH+601, rt= 3.32 mins

### Intermediate 8

### 3-{4-Bromo-1-[(4-methylphenyl)sulfonyl]-1H-pyrrolo[2,3-b]pyridin-2-yl}-2-propyn-1-ol

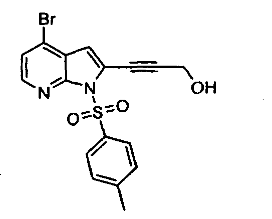

A mixture of 4-bromo-2-iodo-1-[(4-methylphenyl)sulfonyl]-1*H*-pyrrolo[2,3-*b*]pyridine (0.300g,0.63mmol), 2-propyn-1-ol (0.039g, 0.7mmol), bis(triphenylphosphine)palladium(II) chloride (0.008g, 0.011mmol), copper (I) iodide (0.008g, 0.042mmol) and TEA (1ml) in THF (2ml) was stirred under nitrogen for 16 hrs and evaporated. The residue was partitioned between DCM (2x25ml) and aq. sodium bicarbonate (50ml). The dried (Na₂SO₄) organic phase was evaporated and the residue purified on a silica cartridge (10g) eluted with DCM to 50% EtOAc in DCM to give the title compound as a white foam that later solidified (0.165g, 65%).
MH+405 / 407, rt= 3.3 mins

### Intermediate 9

### 1,1-Dimethylethyl (2-{[(4-{2-(3-hydroxy-1-propyn-1-yl)-1-[(4-methylphenyl)sulfonyl]-1H-pyrrolo[2,3-b]pyridin-4-yl}phenyl)sulfonyl]amino}ethyl)carbamate

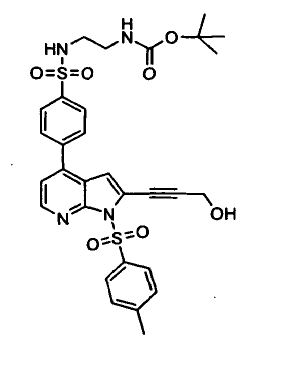

A mixture of pentafluorophenyl 4-(4,4,5,5-tetramethyl-1,3,2-dioxaborolan-2-yl)benzenesulfonate (0.330g, 0.73mmol), 1,1-dimethylethyl(2-aminoethyl)carbamate (0.130g, 0.8mmol) and TEA (0.5ml) was heated in a sealed vessel at 120°C for 10 mins. The resulting dark solution was treated with a solution of 3-{4-bromo-1-[(4-methylphenyl)sulfonyl]-1*H*-pyrrolo[2,3-*b*]pyridin-2-yl}-2-propyn-1-ol (0.160g, 0.4mmol) in dioxan : water (5:1, 2.5ml), 1,1'-bis(diphenylphosphino)ferrocenedichloropalladium(II) (0.020g, 0.027mmol) and sodium carbonate (0.085g, 0.8mmol) and the mixture heated in the mw at 150°C for 20 mins. The mixture was added to water / brine (1:1, 150ml) and extracted with EtOAc / methanol (9:1, 2x100ml). The dried (MgSO₄) extract was evaporated and the residue was purified on a silica cartridge (10g) eluted with DCM to 50% EtOAc in DCM to give the title compound as a foam (0.140g, 56%).
MH+625, rt= 3.32 mins

### Intermediate 10

### Formic acid - N-(2-aminoethyl)-4-[2-(3-hydroxy-1-propyn-1-yl)-1H-pyrrolo[2,3-b]pyridin-4-yl]benzenesulfonamide (1:1)

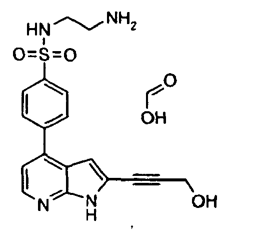

1,1-Dimethylethyl(2-{[(4-{2-(3-hydroxy-1-propyn-1-yl)-1-[(4-methylphenyl)sulfonyl]-1*H-*pyrrolo[2,3-*b*]pyridin-4-yl}phenyl)sulfonyl]amino}ethyl)carbamate (0.160g, 0.26mmol) in chloroform (2ml) was treated with p-toluenesulphonic acid (0.100g, 0.5mmol) and heated in a sealed mw vessel at 100°C for 10 mins (150W). The suspension was evaporated and the residue was dissolved in 5% potassium hydroxide in methanol (4ml) and heated in a sealed mw vessel at 120°C for 5 mins. The mixture was evaporated and the residue purified by MDAP (90mg run x2) to give the title compound as a yellow solid (0.0024g, 25%).
MH+371, rt= 2.12 mins

### Intermediate 11

### 1,1-Dimethylethyl (4-bromo-1H-pyrrolo[2,3-b]pyridin-2-yl)acetate

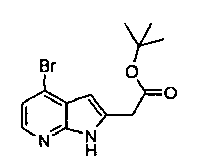

To the 4-bromo-1*H*-pyrrolo[2,3-*b*]pyridine (which may be prepared, for example, as described in Org Letts, 2003, 5, 5023) (20g) in dry DCM (700ml) was added the 1,1-dimethylethyl {[(ethyloxy)carbonothioyl]thio}acetate (59g), the mixture was stirred and heated to reflux. Lauroyl peroxide (139.3g) was added in portions over 5 hrs, 6.95g every 15mins. After the addition the mixture was left at reflux for a further 113 hour. The reaction was cooled and quenched into 10% aq. sodium metabisulphite solution (400ml) and stirred until not peroxide remained by Merckoquant peroxide test strip. The DCM was extracted from the 10% aq. sodium metabisulphite solution with a separating funnel and was then washed with 2M NaOH (3x300ml). This resulted in precipitation of a lot of solid. The DCM extracts were dried with magnesium sulphate and concentrated on the evaporator to give a dark oily solid (>100%). The oily solid was purified on a glass column of 3000ml of silica, initially eluting with DCM and collecting 500ml fractions. At fraction 17 the solvent system was changed to 0.5% Methanol in DCM, then to 1 % Methanol in DCM at fraction 26, then to 1.5% Methanol in DCM at fraction 33, then to 2% Methanol in DCM at fraction 37, then to 5% Methanol in DCM at fraction 41, then to 10% Methanol in DCM at fraction 60. Product eluted in fraction 35 onwards. The product containing fractions were concentrated to give the title compound as a dark oil (29.9g, 96%). The impure title compound (25g) was columned on a 400g Biotage 75 reverse-phase C-18 column eluting initially with 2:1 water:acetonitrile, both containing 0.1% TFA. The compound was loaded by dissolving in DMSO (∼40ml). After collecting about twenty 250ml fractions, a further 2.5 litres of each solvent were added, giving an estimated 40% acetonitrile mixture. Fractions containing product (by hplc analysis) were combined and the acetonitrile removed on the evaporator. The resulting aq. suspension was treated with saturated sodium bicarbonate to pH8 and extracted with DCM. The organics were washed with brine, dried (magnesium sulphate) and evaporated to give title compound as a cream solid, 4.9g.
MH+=311 / 313, RT = 3.32 min

### Intermediate 12

### 4-Bromo-1-(phenylsulfonyl)-1H-pyrrolo[2,3-b]pyridine

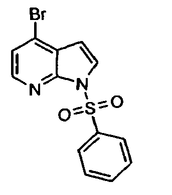

4-Bromo-1*H*-pyrrolo[2,3-*b*]pyridine (which may be prepared, for example, as described in Org Letts, 2003, 5, 5023) (15.0g, 76mmol) was suspended in DCM (300ml) and 1,4-dioxane (100ml) and 50% aq. sodium hydroxide (23ml) was added followed by NaBu₄HSO₄ solution (7.5ml). The mixture was vigorously stirred and cooled in an ice bath whilst benzene sulfonyl chloride (14.6ml, 115mmol) was added dropwise. The reaction mixture was left stirring vigorously for 5 days. It was evaporated to dryness and the resulting solid was washed with water (100ml) followed by methanol (50ml). The yellow solid was dried *in vacuo.* The title compound was obtained as a pale yellow solid (23.5g, 92%).
MH+ 337 / 339, rt= 3.35 mins

### Intermediate 13

### 1-(Phenylsulfonyl)-4-[4-(1-pyrrolidinylsulfonyl)phenyl]-1H-pyrrolo[2,3-b]pyridine

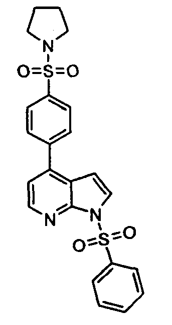

To a mixture of 4-bromo-1-(phenylsulfonyl)-1*H*-pyrrolo[2,3-*b*]pyridine (12g, 35.61mmol), [4-(1-pyrrolidinylsulfonyl)phenyl]boronic acid (10g, 39.22mmol) and sodium carbonate (7.5g, 71 mmol) in 1,4-dioxane (300ml) and water (100ml) was added bis(diphenylphosphino)ferrocene palladium II chloride (1.5g, 1.84mmol). The reaction mixture was heated and stirred at 100 °C under a nitrogen atmosphere for 4 hrs. The reaction mixture was cooled to room temperature, concentrated *in vacuo* and partitioned between DCM (400ml) and water (100ml). The aq. layer was extracted with DCM (2x100ml). The combined organic extracts were evaporated to give a crude brown foam (22g). 10g of this material was purified by silica SPE cartridge (50g) using DCM to 50:1 DCM:EtOAc. The title compound was obtained as a white foam (6g, 36%). The remainder of the material was purified as above to give more of the title compound as a cream solid (7.3g, 44%).
MH+468, rt= 3.44 mins

### Intermediate 14

### 1-(Phenylsulfonyl)-4-[4-(1-pyrrolidinylsulfonyl)phenyl]-1H-pyrrolo[2,3-b]pyridine-2-carbaldehyde

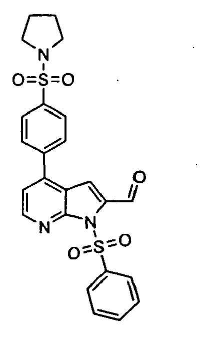

A stirred solution of 1-(phenylsulfonyl)-4-[4-(1-pyrrolidinylsulfonyl)phenyl]-1*H*-pyrrolo[2,3-*b*]pyridine (10.3g, 22mmol) in THF (180ml) at -40°C under nitrogen was treated with 2M LDA in heptane/THF/ethylbenzene (22.0ml, 44.0mmol). The reaction was maintained at - 35°C(+/-5°C) for 35 min. DMF (6.82ml, 88.1mmol) was added and the reaction allowed to warm to room temperature over 40 min. The reaction was quenched by addition of 2M hydrochloric acid (250ml) and extracted with DCM (3x250ml). The combined organic layers were reduced *in vacuo* to give (13g) which was purified using silica SPE cartridges (2x70g) eluting with DCM to 2% EtOAc in DCM to 5% EtOAc in DCM to 10% EtOAc in DCM to afford the title compound (8.0g, 73%).
MH+496, rt= 3.43 min

### Intermediate 15

### 4-Bromo-1-(phenylsulfonyl)-1H-pyrrolo[2,3-b]pyridine-2-carbaldehyde

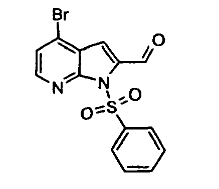

To a solution of 4-bromo-1-(phenylsulfonyl)-1*H*-pyrrolo[2,3-*b*]pyridine (3.37g, 10.0mmol) in dry THF (50ml) at -25 °C to -40 °C under nitrogen was added 2M LDA in heptane/THF/ethylbenzene (10ml, 20.0mmol). The reaction was stirred at this temperature for 40 mins. DMF (2ml) was added dropwise at -30 °C and allowed to warm up to room temperature. The reaction was poured into 2M aq. HCl, extracted with DCM (3x50ml) and evaporated to give the crude material which was purified using a silica SPE cartridge (50g) eluting with DCM. Fractions containing the product were combined and evaporated to give the title compound as a cream foam (0.8g, 22%).
MH+365 / 367, rt= 3.31 mins

### Intermediate 16

### Tetrahydro-4H-thiopyran-4-one oxime

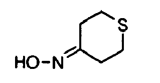

Anhydrous sodium acetate (16.25g, 18.86mmol) and hydroxylamine hydrochloride (7.72g, 10.66mmol) were added to a solution of 4-oxothiane (10.02g, 8.2mmol) in ethanol (100mi) and water (100ml). The reaction mixture was stirred and heated at reflux for 56 hrs. The cooled reaction mixture was diluted with water (200ml) and extracted with ether (2x150ml). The combined organic extracts were washed with brine (100ml), dried (hydrophobic frit) and evaporated to dryness to give the title compound as a colourless solid (12.8g).
MH+132, rt= 1.56 mins

### Intermediate 17

### Tetrahydro-2H-thiopyran-4-amine

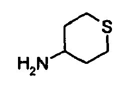

2.3M Lithium aluminium hydride in THF (100ml) was transferred under nitrogen to a 1 litre 3-necked flask and diluted with dry THF (130ml) to make a 1 M lithium aluminium hydride solution. The solution was stirred under nitrogen whilst a solution of tetrahydro-4*H-*thiopyran-4-one oxime (12.8g, 97.6mmol) in dry THF (90ml) was added drop wise. The addition was exothermic and was done slowly whilst cooling the reaction in a water bath so that the reaction stayed at or below 25°C. Addition was over 2 hrs. After the addition the mixture was stirred at ambient temperature for a further 0.5 to 1 hour and cautiously warmed to reflux. Stirring at reflux was continued overnight. The reaction was allowed to cool, diluted by addition of dry THF (130ml) and cooled to below 10°C in an ice/water bath. It was quenched by slowly adding water (10ml) keeping the temperature below 20°C (very exothermic and mixture became quite thick). Aq. sodium hydroxide solution (15% w/v, 10ml) was added slowly drop wise keeping the temperature below 20°C. Water (26ml) was added drop wise. The mixture was stirred for a while longer then the solid was filtered off washing the flask and solid with THF. The solvent was removed and the remaining yellow oil was dried under *vacuum* overnight to give the title compound as a colourless solid (6.3g).
MH+118, rt= 0.32 mins

### Intermediate 18

### 1,1-Dimethylethyl tetrahydro-2H-thiopyran-4-ylcarbamate

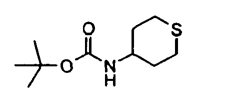

Tetrahydro-2*H*-thiopyran-4-amine (5.8g, 49.5mmol) was dissolved in dioxan (55ml). Aq. 2M sodium hydroxide was added. Di-tert-buty dicarbonate (21.6g, 99mmol) was added portion wise keeping the temperature below 30°C (ice/water bath). The last portion was washed in with a little dioxan. The mixture was stirred for a further 2 hrs at ambient temperature. It was diluted with water (100ml) and extracted with EtOAc (3x200ml). The combined EtOAc extracts were washed with water (100ml), brine (100ml), dried (MgSO₄), filtered, evaporated and dried (high *vacuum*) to give crude material (18g). This was dissolved in DCM (60ml) and applied to a 9cm diameter glass column. The column was packed in 9:1 cyclohexane:EtOAc. It was eluted with 9:1 cyclohexane:EtOAc. Product containing fractions were pooled and evaporated to give the title compound (9.5g).
MH+218, rt= 2.81 mins

### Intermediate 19

### 1,1-Dimethylethyl (1,1-dioxidotetrahydro-2H-thiopyran-4-yl)carbamate

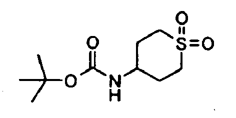

A solution of 1,1-dimethylethyl tetrahydro-2*H*-thiopyran-4-ylcarbamate (9.5g, 44mmol) in methanol (300ml) was stirred and cooled to 0-5°C in an ice/IMS bath. A solution of oxone (43.6g, 70.9mmol) in water (300ml) was added drop wise over 2 hrs keeping the temperature below 10°C. After the addition was complete, water was added to the cooling bath. The mixture was stirred overnight whilst slowly warming to ambient temperature. The mixture was poured, with stirring, onto stirred aq. potassium carbonate solution (10% w/v, 650ml). Water (200ml) was added and the mixture extracted with EtOAc (3x500ml). The combined EtOAc extracts were washed with water (500ml), brine (300ml), dried (MgSO₄), filtered, evaporated and dried (high *vacuum*) to give the title compound as a white solid (9.9g).
LCMS MH+250, MNH₄+267 seen at rt= 2.14 mins

### Intermediate 20

### (1,1-Dioxidotetrahydro-2H-thiopyran-4-yl)amine hydrochloride

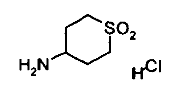

1,1-Dimethylethyl (1,1-dioxidotetrahydro-2*H*-thiopyran-4-yl)carbamate (9.9g, 40mmol) was dissolved in 1,4 dioxan (210ml) and the solution stirred under nitrogen. 5M Aq. HCl (105ml, 525mmol) was added dropwise keeping the temperature below 25°C (ice/water bath). Stirring was continued at ambient temperature overnight. The solvent was removed *in vacuo.* The residue was evaporated down again from dioxan to azeotrope off the water and HCl. The residual white powder was dried (high vacuum) and overnight in the vac oven (40°C ) to give the title compound (7.2g).
ELSD MH+150, rt= 0.31 mins

### Intermediate 21

### 4-Bromo-N-(1,1-dioxidotetrahydro-2H-thiopyran-4-yl)benzenesulfonamide

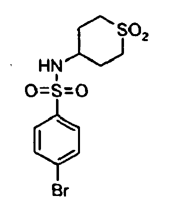

(1,1-Dioxidotetrahydro-2*H*-thiopyran-4-yl)amine hydrochloride (3.7g, 20mmol), TEA (15ml) and DCM (100ml) were mixed together and stirred at room temperature for 2 hrs. 4-Bromobenzenesulfonyl chloride (5g, 19.57mmol) in DCM (25ml) was added to the mixture slowly. The reaction was stirred at room temperature under a nitrogen atmosphere for 60 hrs. The mixture was reduced under *vacuum,* diluted with 2N HCl, filtered and washed with water and 2N HCl to give the title compound as a pale brown solid (5.96g, 82%).
M-H+368, rt= 2.58 mins

### Intermediate 22

### N-(1,1-Dioxidotetrahydro-2H-thlopyran-4-yl)-4-(4,4,5,5-tetramethyl-1,3,2-dioxaborolan-2-yl)benzenesulfonamide

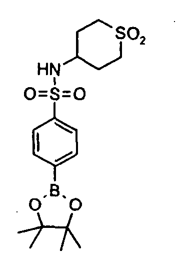

4-Bromo-*N*-(1,1-dioxidotetrahydro-2*H*-thiopyran-4-yl)benzenesulfonamide (3g, 8.15mmol), potassium acetate (2.4g, 24.45mmol) and palladium acetate (0.091g, 0.4mmol) were mixed together in dimethylformamide (100ml) and stirred at 25°C. Bis(pinacolato)diboron (9g, 40mmol) was introduced and the mixture stirred at room temperature for 4 hrs. The mixture was reduced under *vacuum,* diluted with water and filtered. The aq. filtrate was removed. The filter cake was washed with DCM and the organic filtrate dried using a phase separator. The DCM was removed *in vacuo* and the residue treated with a further portion of DCM (20ml). The suspension was filtered again. The organic filtrate was concentrated *in vacuo* and the residue triturated in cyclohexane. Filtration yielded the title compound as a white solid (1.28g).
MH+ 416, rt= 2.95 mins

### Intermediate 23

### 4-[2-(Difluoromethyl)-1-(phenylsulfonyl)-1H-pyrrolo[2,3-b]pyridin-4-yl]-N-(1,1-dioxidotetrahydro-2H-thiopyran-4-yl)benzenesulfonamide

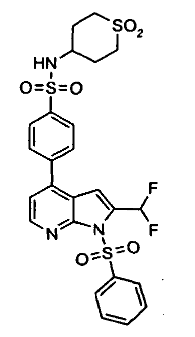

A mixture of 4-bromo-2-(difluoromethyl)-1-(phenylsulfonyl)-1*H*-pyrrolo[2,3-*b*]pyridine (0.100g, 0.26mmol), *N*-(1,1-dioxidotetrahydro-2H-thiopyran-4-yl)-4-(4,4,5,5-tetramethyl-1,3,2-dioxaborolan-2-yl)benzenesulfonamide (0.140g, 0.33mmol), bis(diphenylphosphino)ferrocene palladium II chloride (0.015g) and sodium carbonate (0.055g, 0.52mmol) in 1,4-dioxane (2.5ml) and water (1ml) were stirred in the Biotage Initiator mw at 120°C for 40 mins. DCM (50ml) and brine (20ml) were added. The DCM layer was evaporated to dryness and the product purified by chromatography (10g silica SPE cartridge) using DCM to 20% EtOAc in DCM. The appropriate fractions were combined and evaporated to give the title compound as a cream solid (0.090g, 45%).
MH+596, rt= 1.12 mins

### Intermediate 24

### 1-{4-[4-(1-Pyrrolidinylsulfonyl)phenyl]-1H-pyrrolo[2,3-b]pyridin-2-yl}ethanone

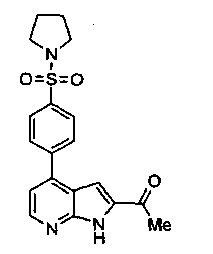

A solution of 1-(phenylsulfonyl)-4-[4-(1-pyrrolidinylsulfonyl)phenyl]-1*H*-pyrrolo[2,3-*b*]pyridine (0.200g, 0.43mmol) in anhydrous THF (6ml) was stirred at -30°C under nitrogen. It was treated dropwise with 2M LDA in heptane/THF/ethylbenzene (0.428ml, 0.855mmol). The reaction was stirred for 40 mins at -30°C, cooled to -40°C and acetic anhydride (0.243ml, 2.56mmol) in THF (1ml) was added dropwise. The reaction was allowed to warm to room temperature and stirred for 2 hrs. The reaction was quenched with addition of aq. ammonium chloride (20ml) and extracted with EtOAc (3x20ml). The organic layers were combined and reduced under *vacuum* to give the crude material (0.460g) which was purified using 20g silica / FlashMaster II eluting with 0-100%, EtOAc to cyclohexane over 20 mins. This gave 1-{1-(phenylsulfonyl)-4-[4-(1-pyrrolidinylsulfonyl)phenyl]-1*H*-pyrrolo[2,3-*b*]pyridin-2-yl}ethanone (0.240g, impure) which was dissolved in dioxan (2ml) and water (0.2ml) and then treated with sodium hydroxide (0.120g). The reaction was heated in the Biotage Initiator mw at 140°C for 40 mins. The reaction was reduced under *vacuum,* diluted with water (20ml), acidified to pH9 using aq. HCl and extracted using DCM. There was poor solubility with the DCM and a precipitate formed in the organic layers. The organic layers were combined and reduced under *vacuum* to give the crude material (0.150g) which was triturated in methanol to give (0.058g). This material was re-triturated with methanol to afford the title compound as a solid (0.045g).
MH+370, rt= 2.95 mins

### Intermediate 25

### 1-[4-Bromo-1-(phenylsulfonyl)-1H-pyrrolo[2,3-b]pyridin-2-yl]ethanone

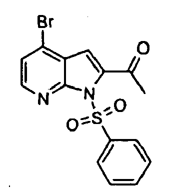

A solution of 4-bromo-1-(phenylsulfonyl)-1*H*-pyrrolo[2,3-*b*]pyridine (1g, 2.97mmol) in THF (21ml) at -35°C under nitrogen was treated with 2M LDA in heptane/THF/ethylbenzene (2.97ml, 5.93mmol). The reaction was stirred for 35 mins at -30°C to -35°C. The reaction was cooled to -40°C and treated with acetic anhydride (1.12ml, 11.86mmol). The ice-bath was removed and the reaction allowed to warm to room temperature over a further 30 mins. The reaction was quenched with aq. ammonium chloride (70ml) and extracted with DCM (3x70ml). The organic layers were combined and reduced under *vacuum* to give crude material (2g) which was purified by 50g silica FlashMaster (II) eluting with DCM. The first 3 fractions gave (0.520g) which contained the desired product and impurities. 4-Bromo-1-(phenylsulfonyl)-1*H*-pyrrolo[2,3-*b*]pyridine (6g, 17.8mmol) in THF (130ml) was treated with 2M LDA in heptane/THF/ethylbenzene (17.8ml, 35.6mmol) at -30°C under nitrogen. The reaction was stirred for 35 mins at or below -30°C. The reaction was quenched with acetic anhydride (6.7ml, 71.2mmol) at -40°C. The reaction was maintained at -30°C for 40 mins, and slowly allowed to warm to room temperature over a further 30 mins. The reaction was quenched by addition of ammonium chloride (200ml) and extracted with DCM (3x170ml). The organic layers were combined and reduced under *vacuum* to afford the crude material (11g). This was purified using silica SPE cartridges (2x70g) eluting with cyclohexane:DCM (50:50) to cyclohexane:DCM (25:75) to neat DCM to afford still impure material (1.52g). This was combined with the earlier batch of material (0.520g) and purified using a silica SPE cartridge (70g) eluting with cyclohexane:DCM (50:50) to cyclohexane:DCM (25:75) to neat DCM. This afforded the title compound (1.09g).
MH+379 / 381, rt= 3.24 mins

### Intermediate 26

### 2-[4-Bromo-1-(phenylsulfonyl)-1H-pyrrolo[2,3-b]pyridin-2-yl]-2-propanol

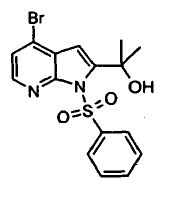

1-[4-Bromo-1-(phenylsulfonyl)-1*H*-pyrrolo[2,3-*b*]pyridin-2-yl]ethanone (0.7g, 1.85mmol) in anhydrous THF (14ml) under nitrogen was treated with methylmagnesium chloride (3M in THF, 0.677ml, 2.03mmol). There was a noticeable exotherm which was controlled using a water bath. The mixture was stirred for 1 hour at room temperature. The reaction was treated with acetic acid (2.1ml) and refluxed for 30 mins.The reaction was diluted with water (50ml) and extracted with DCM (2x50ml). The organic layers were combined and reduced under *vacuum* to afford the crude material (0.8g) which was purified using a 20g silica SPE cartridge eluting with DCM / cyclohexane (50:50) to DCM to 5% EtOAc in DCM to 20% EtOAc in DCM. This afforded the title compound (0.518g).
MH+395 / 397, rt= 3.35 mins

### Intermediate 27

### 4-Bromo-2-(1-methylethenyl)-1-(phenylsulfonyl)-1H-pyrrolo[2,3-b]pyridine

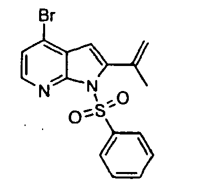

2-[4-Bromo-1-(phenylsulfonyl)-1*H*-pyrrolo[2,3-*b*]pyridin-2-yl]-2-propanol (0.220g, 0.56mmol) in THF (2ml), acetic acid (1ml) and concentrated sulphuric acid (1ml) were stirred for 15 mins. The exotherm was controlled using a water bath when the sulphuric acid was added. Additional concentrated sulphuric acid (0.2ml) and acetic acid (0.2ml) were added. LC/MS after 20 mins showed no real change. Additional concentrated sulphuric acid (0.2ml) was added. After an additional 1 hour the reaction was neutralized by addition of aq. sodium hydrogen carbonate and extracted with DCM (3x40ml). The organic layers were combined, dried (phase separator) and reduced under *vacuum* to afford the crude material (0.250g) which was purified by 10g silica FlashMaster (II) eluting with 0-50% EtOAc to cyclohexane over 20 mins to afford the title compound (0.162g).
MH+377 / 379, rt= 3.64 mins

### Intermediate 28

### 2-(1-Methylethenyl)-1-(phenylsulfonyl)-4-[4-(1-pyrrolidinylsulfonyl)phenyl]-1H-pyrrolo[2,3-b]pyridine

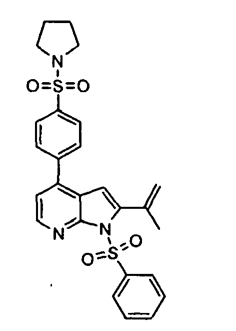

4-Bromo-2-(1-methylethenyl)-1-(phenylsulfonyl)-1*H*-pyrrolo[2,3-*b*]pyridine (0.080g, 0.212mmol), 2-(dimethylamino)-2-biphenyl palladium (II) chloride dinorbornyl phosphine complex (0.006g, 0.01mmol), potassium phosphate tribasic (0.135g, 0.636mmol) and [4-(1-pyrrolidinylsulfonyl)phenyl]boronic acid (0.060g, 0.233mmol) in dioxan (1.5ml) and water (0.3ml). The reaction was heated at 120°C in the Biotage Initiator mw for 40 mins. The reaction was poured into water (20ml) and extracted with DCM (2x20ml). The organic layers were combined and reduced under *vacuum* to give the crude material (0.150g). This was purified by 20g silica FlashMaster II, eluting with 0-100% EtOAc to cyclohexane over 20 mins to afford the title compound (0.103g).
MH+508, rt= 3.63 mins

### Intermediate 29

### N-(1,1-Dioxidotetrahydro-2H-thiopyran-4-yl)-4-[2-(1-methylethenyl)-1-(phenylsulfonyl)-1H-pyrrolo[2,3-b]pyridin-4-yl]benzenesulfonamide

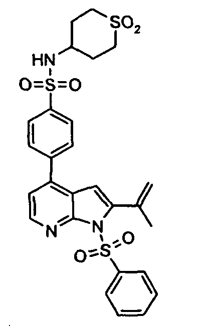

4-Bromo-2-(1-methylethenyl)-1-(phenylsulfonyl)-1*H*-pyrrolo[2,3-*b*]pyridine (0.080g, 0.212mmol), 2-(dimethylamino)-2-biphenyl palladium (II) chloride dinorbornyl phosphine complex (0.006g, 0.01 mmol), potassium phosphate tribasic (0.135g, 0.636mmol) and *N-*(1,1-dioxidotetrahydro-2*H*-thiopyran-4-yl)-4-(4,4,5,5-tetramethyl-1,3,2-dioxaborolan-2-yl)benzenesulfonamide (0.102g) in dioxan (1.5ml) and water (0.3ml) were heated at 120°C for 40 mins in the Biotage Initiator mw, mostly product but some starting material remains. Additional *N*-(1,1-dioxidotetrahydro-2*H*-thiopyran-4-yl)-4-(4,4,5,5-tetramethyl-1,3,2-dioxaborolan-2-yl)benzenesulfonamide (0.025g) was added and the reaction heated for a further 25 mins at 120°C. The reaction was poured into water (20ml) and extracted with DCM (2x20ml). The organic layers were combined and reduced under *vacuum* to afford the crude material (0.200g). The crude material was purified using 20g silica FlashMaster II, eluting with 0-100% EtOAc to cyclohexane over 20 mins to afford the title compound (0.119g).
MH+586, rt= 3.26 mins

### Intermediate 30

### 1-(Phenylsulfonyl)-4-[4-(1-pyrrolidinylsulfonyl)phenyl]-2-[(trimethylsilyl)ethynyl]-1H-pyrrolo[2,3-b]pyridine

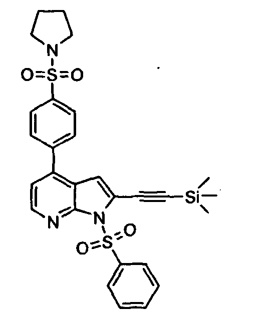

2-lodo-1-(phenylsulfonyl)-4-[4-(1-pyrrolidinylsulfonyl)phenyl]-1*H*-pyrrolo[2,3-*b*]pyridine (0.102g, 0.17mmol), copper (I) iodide (0.0033g, 0.017mmol), bis(triphenylphosphine)palladium(II) chloride (0.006g, 0.008mmol) and TEA (0.075ml) in THF (3ml) under nitrogen at room temperature were treated with trimethylsilylacetylene (0.036ml, 0.25mmol) and the reaction stirred for 18 hrs. The reaction was poured into water (50ml) and extracted with DCM (2x30ml). The organic layers were combined and reduced under *vacuum* to afford the crude material which was purified by FlashMaster (II) / normal phase silica eluting with 0-100% EtOAc / cyclohexane over 15 mins to afford the title compound (0.076g, 79%).
MH+564, rt= 3.95 mins

### Intermediate 31

### 2-Ethynyl-4-[4-(1-pyrrolidinylsulfonyl)phenyl]-1H-pyrrolo[2,3-b]pyridine

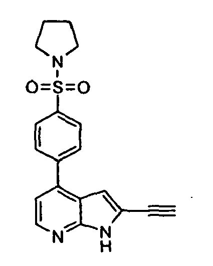

1-(Phenylsulfonyl)-4-[4-(1-pyrrolidinylsulfonyl)phenyl]-2-[(trimethylsilyl)ethynyl]-1*H-*pyrrolo[2,3-*b*]pyridine (0.076g, 0.135mmol) in THF (1ml) was treated with TBAF (1M solution in THF, 3.9ml, 3.90mmol) and the reaction stirred for 1 hour under nitrogen. After stirring for an additional 30 mins, the reaction was quenched by addition of 2M HCl (40ml) and extracted with DCM (3x30ml). The organic layers were combined and reduced under *vacuum* to afford material that was purified by eluting with cyclohexane to 50:50 cyclohexane:EtOAc to EtOAc using a 10g silica SPE cartridge. Fractions containing clean product were combined to afford a brown solid. The brown solid was washed with methanol to give solid (0.013g). The filtrate (methanol) from above was reduced under *vacuum* to afford product plus impurity (0.030g). The impure fractions from the SPE described earlier were combined and reduced to afford (0.070g) of material which was washed with water yielding (0.020g). The three batches of material (0.013g, 0.030g and 0.020g) were combined and purified by preparative TLC eluting with EtOAc to afford the title compound (0:017g).
MH+352, rt= 3.20 mins

### Intermediate 32

### 4-Bromo-2-methyl-1-(phenylsulfonyl)-1H-pyrrolo[2,3-b]pyridine

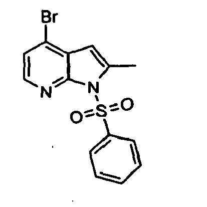

To the solution of 4-bromo-1-(phenylsulfonyl)-1*H*-pyrrolo[2,3-*b*]pyridine (9.4g, 28.0mmol) in dry THF (100ml) stirred at -35°C was added 2M LDA in heptane/THF/ethylbenzene (28.0ml, 56.0mmol) and the reaction was stirred at -35°C for 30 min. Methyl iodide (10.5ml, 168.0mmol) was added drop wise to the solution and the mixture was allowed to warm to room temperature over 2 hrs. The reaction was quenched with aqueous ammonium chloride solution (100ml) and extracted with EtOAc (3x60ml). The combined organic layers were dried (phase separator) and concentrated *in vacuo.* Purification was by FlashMaster on silica gel (2x70g) using EtOAc - cyclohexane (0-100% gradient) to give the title compound as a white solid (7.85g, 80%).
MH+351 / 353, rt= 3.45 min

### Intermediate 33

### {1-(Phenylsulfonyl)-4-[4-(1-pyrrolidinylsulfonyl)phenyl]-1H-pyrrolo[2,3-b]pyridin-2-yl}methanol

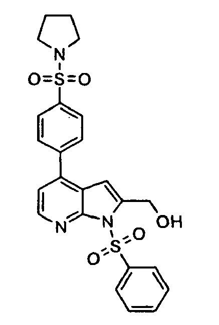

To a solution of 1-(phenylsulfonyl)-4-[4-(1-pyrrolidinylsulfonyl)phenyl]-1*H*-pyrrolo[2,3-*b*]pyridine (3.0g,6.42mmol) in dry THF (30ml) at -60°C under nitrogen was added 2M LDA in heptane/THF/ethylbenzene (6.5ml, 13.0mmol) dropwise. The reaction was allowed to warm up to -30°C and stirred at -30°C to -40°C for 30 mins. Paraformaldehyde (1.5g, 38.0mmol) was added. The reaction was allowed to warm to room temperature. The reaction was quenched by adding to 2M HCl (100ml), keeping the solution acidic. This was extracted with DCM (3x50ml). The organic layers were washed with 2M HCl, brine and evaporated to give a brown foam (3g) which was purified by silica SPE cartridge (50g) eluting with DCM to 20:1 DCM : EtOAc to 10:1 DCM : EtOAc to neat EtOAc to give the title compound as a cream foam (1 g, 31%).
MH+498, rt= 3.21 mins

### Intermediate 34

### (4-{4-[(Methylsulfonyl)amino]phenyl}-1H-pyrrolo[2,3-b]pyridin-2-yl)acetic acid trifluoroacetate

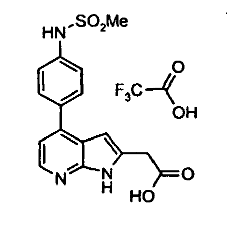

1,1-Dimethylethyl (4-{4-[(methylsulfonyl)amino]phenyl}-1*H*-pyrrolo[2,3-*b*]pyridin-2-yl)acetate (0.730g, 1.8mmol) was dissolved in DCM (60ml) and TFA (20ml). The reaction was stirred at room temperature for 4 hrs. The solution was evaporated and recrystallised from acetonitrile to give the title compound as a buff solid (0.578g).
MH+346, rt= 2.32 mins

### Intermediate 35

### (4-Bromo-1H-pyrrolo[2,3-b]pyridin-2-yl)acetic acid trifluoroacetate

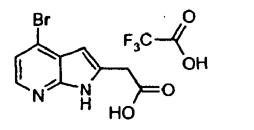

1,1-Dimethylethyl (4-bromo-1*H*-pyrrolo[2,3-*b*]pyridin-2-yl)acetate (1g, 3.3mmol) was treated with 1:1 TFA : DCM (20ml). The reaction was stirred for 2 hrs and then evaporated. The orange gum was triturated with cyclohexane and evaporated *in vacuo* to give the title compound as a pale orange solid (1.1g).
MH+255, rt= 2.66 mins

### Intermediate 36

### 2-(4-Bromo-1H-pyrrolo[2,3-b]pyridin-2-yl)-N,N-dimethylacetamide

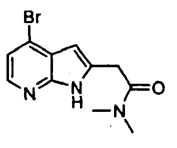

(4-Bromo-1*H*-pyrrolo[2,3-*b*]pyridin-2-yl)acetic acid trifluoroacetate (0.800g, 2.16mmol), TBTU (0.847g, 2.59mmol) and *N*,*N*-diisopropylethylamine (1.14ml, 6.48mmol) were dissolved in DCM (50ml). The reaction was stirred at room temperature. The reaction was divided into 2 flasks. One portion was treated with excess 2M dimethylamine in THF (2ml) and the reaction stirred at room temperature for 3 days. The mixture was diluted with DCM (50ml), washed with 10% citric acid and evaporated. The gum was purified by MDAP to afford the title compound (0.048g).
MH+282 / 284, rt= 2.49 mins

### Intermediate 37

### 2-(4-Bromo-1H-pyrrolo[2,3-b]pyridin-2-yl)-N-methylacetamide

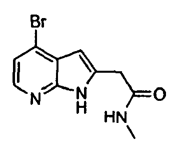

(4-Bromo-1*H*-pyrrolo[2,3-*b*]pyridin-2-yl)acetic acid trifluoroacetate (0.800g, 2.16mmol), TBTU (0.847g, 2.59mmol) and *N*,*N*-diisopropylethylamine (1.14ml, 6.48mmol) were dissolved in DCM (50ml). The reaction was stirred at room temperature. The reaction was divided into 2 flasks. One portion was treated with excess 40% methylamine in water (2ml) and the reaction stirred at room temperature for 3 days. The mixture was diluted with DCM (50ml), washed with 10% citric acid and evaporated. The gum was purified by MDAP to afford the title compound (0.040g).
MH+268 / 270, rt= 2.43 mins

### Intermediate 38

### {1-(Phenylsulfonyl)-4-[4-(1-pyrrolidinylsulfonyl)phenyl]-1H-pyrrolo[2,3-b]pyridin-2-yl}methyl methanesulfonate

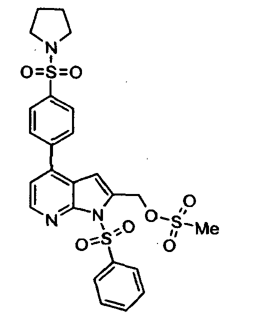

To a solution of {1-(phenylsutfonyl)-4-[4-(1-pyrrolidinylsulfonyl)phenyl]-1*H*-pyrrolo[2,3-*b*]pyridin-2-yl}methanol (1.4g, 2.82mmol) in DCM (10ml) containing TEA (0.5ml, 3.6mmol) was added methanesulfonic anhydride (0.600g, 3.45mmol) portion wise under nitrogen. The reaction was stirred at room temperature for 2 hrs. DCM (50ml) was added, it was washed with 0.1M aq. HCl followed by saturated sodium bicarbonate solution (50ml), dried (hydrophobic frit) and evaporated to give the title compound as cream foam (1.4g, 84%).
¹H NMR (400MHz; CDCl₃) δ: 1.84 (4H, m), 3.10 (3H, s), 3.32 (4H, m), 5.74 (2H, s), 6.96 (1H, s), 7.30 (1H, d), 7.54 (2H, t), 7.63 (1H, t), 7.72 (2H, d), 7.98 (2H, d), 8.31 (2H, d), 8.57 (1H, d).

### Intermediate 39

### 3-Amino-1-propanesulfonamide hydrochloride

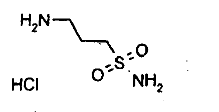

A mixture of 3-amino-1-propanesulphonic acid (9.96g, 71.55mmol), potassium acetate (7.01 g, 71.55mmol) and glacial acetic acid (30ml) was heated to reflux, with stirring for 10 mins. Phthalic anhydride (10.6g, 71.55mmol) was added to the suspension and the mixture refluxed for a further 24 hrs. The reaction was cooled and left to stand for a further 2 days. No precipitation occurred so ether (400ml) was added, resulting in a white solid. This was filtered off under vacuum and washed well with ether (200ml) to afford potassium 3-(1,3-dioxo-1,3-dihydro-2*H*-isoindol-2-yl)-1-propanesulfonate as a white solid (22.9g, 100%). Potassium 3-(1,3-dioxo-1,3-dihydro-2*H*-isoindol-2-yl)-1-propanesulfonate (20g,65mmol) was heated with stirring under reflux with toluene (100ml) using Dean-Stark apparatus for 24 hrs. Phosphorous pentachloride (9.8g, 47.2mmol) was added portion wise to the suspension and heated at reflux for 1 hour, resulting in a green suspension. A further quantity of phosphorous pentachloride (10.5g, 50.42mmol) was added portion wise under nitrogen and the resulting solution refluxed for a further 5 hrs. The solution was left to stand overnight, distilled at atmospheric pressure and evaporated to give an oil. Crushed ice (100ml) was added giving a sticky solid which hardened and dried on filtering off under vacuum. The filtrate was decanted off, redissolved in toluene and evaporated again to give an oil. A further quantity of crushed ice (100ml) was added, the solid filtered off and washed with water. The 2 batches of solid were combined and dried (60°C) to give 3-(1,3-dioxo-1,3-dihydro-2*H*-isoindol-2-yl)-1-propanesulfonyl chloride as a beige solid (14.77g, 79%). 3-(1,3-Dioxo-1,3-dihydro-2*H*-isoindol-2-yl)-1-propanesulfonyl chloride (5.0g, 17.4mmol) was added in portions, with stirring to liquid ammonia. Stirring at -28 °C was continued for 30 mins. The cooling bath was removed, the reaction mixture warmed to room temperature and the ammonia allowed to boil off. The residue was treated with acetic acid (glacial, 4ml) in water (10ml). The resulting brown solution was evaporated to dryness giving viscous orange/brown oil (4g). This failed to solidify on treating with ether so it was applied in the minimum volume of methanol to a flash column of silica (Merck 9385, 230ml, 4.2cm wide). Elution with DCM-methanol-0.88 ammonia (890:100:10) afforded on evaporation 3-(1,3-dioxo-1,3-dihydro-2*H*-isoindol-2-yl)-1-propanesulfonamide as a cream foam (1.52g, 33%). A solution of 3-(1,3-dioxo-1,3-dihydro-2*H*-isoindol-2-yl)-1-propanesulfonamide (1.52g, 5.7mmol) in ethanol (21 ml) and water (0.6ml) was treated with hydrazine hydrate (0.2ml, 0.2g, 6.23mmol) and heated under reflux under nitrogen for 4 hrs. The mixture was cooled and the suspension filtered off under vacuum. The filtrate was acidified to pH4.0, evaporated to dryness, taken up in water (20ml), re-filtered and evaporated again. The solid residue was re-dissolved in water, acidified to pH4.0 and the resulting solid filtered off. The filtrate was evaporated to dryness to afford the title compound as a white solid (0.728g, 74%).
TLC, SiO₂ (DCM-MeOH-).88NH₃, 890:100:10) Rf=baseline det uv/KMnO₄ (Literature reference: J. Chem. Soc., 1952, 3334-3337 and J.Am.Chem.Soc., 62, 1940, 2099-2101)

### Intermediate 40

### 1,1-Dimethylethyl [2-({[4-(4,4,5,5-tetramethyl-1,3,2-dioxaborolan-2-yl)phenyl]sulfonyl}amino)ethyl]carbamate

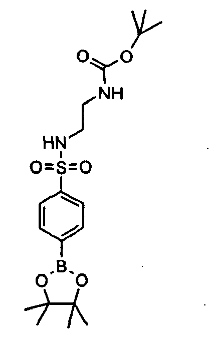

### Method A

Pentafluorophenyl 4-(4,4,5,5-tetramethyl-1,3,2-dioxaborolan-2-yl)benzenesulfonate (0.450g, 1mmol) was placed in a mw vessel together with N-boc ethylene diamine (0.160ml, 1mmol) and triethylamine (0.700ml, 5mmol). The reaction was heated at 120°C for 10 mins in the Biotage Initiator mw. The reaction mixture was reduced under vacuum to afford the title compound (0.100g, 23%).
LC/MS rt= 2.49 mins does not ionise

### Method B

1,1-Dimethylethyl (2-{[(4-bromophenyl)sulfonyl]amino}ethyl)carbamate (5g, 13.2mmol), palladium acetate (0.150g, 0.66mmol) and potassium acetate (3.9g, 39.5mmol) in DMF (175ml) under nitrogen were treated with 4,4,4',4',5,5,5',5'-octamethyl-2,2'-bi-1,3,2-dioxaborolane (8.07g, 31.6mmol). The reaction was stirred at 50°C for 2 hr. The reaction was cooled, filtered and the filtrate reduced *in vacuo.* The resulting crude material was diluted with water (100ml) and extracted with DCM (2x180ml). The combined organic extracts were reduced *in vacuo* to give (10g). This material was triturated in ether (∼100ml). The ether filtrate was reduced *in vacuo* to give (8.5g). This material was diluted with water (80ml) and extracted with cyclohexane (3x80ml). The aqueous phase was reduced *in vacuo* to give impure material (7.5g) which was purified by silica SPE cartridge (100g) eluting with cyclohexane to 20% EtOAc in cyclohexane to 50% EtOAc in cyclohexane to EtOAc. The fractions containing product were combined and reduced *in vacuo* to afford the title compound (5.8g).
MH-425, rt= 3.32 min

### Intermediate 41

### 4-Bromo-N-(1,1-dioxidotetrahydro-3-thienyl)benzenesulfonamide

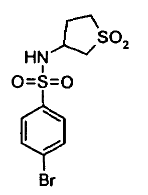

4-Bromobenzenesulfonyl chloride (7g, 27.4mmol) was added to a solution of tetrahydro-3-thiophenamine 1,1-dioxide (4.07g, 30.1mmol) and TEA (19ml, 137mmol) in DCM (150ml). The reaction was stirred at room temperature for 3 hrs. The mixture was washed with 2M hydrochoric acid (2x60ml) and a solid crashed out of the organic layer. The solid was filtered and washed with water to afford the title compound as a white solid (10.6g).
M-H+354, rt= 2.59 mins

### Intermediate 42

### N-(1,1-Dioxidotetrahydro-3-thienyl)-4-(4,4,5,5-tetramethyl-1,3,2-dioxaborolan-2-yl)benzenesulfonamide

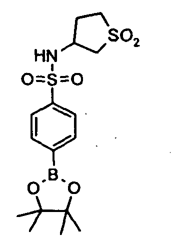

4-Bromo-*N*-(1,1-dioxidotetrahydro-3-thienyl)benzenesulfonamide (3g, 8.45mmol), potassium acetate (2.5g, 25.35mmol) and palladium acetate (0.095g, 0.422mmol) were mixed together in dimethylformamide (100ml) and stirred at 25°C. 4,4,4',4',5,5,5',5'-Octamethyl-2,2'-bi-1,3,2-dioxaborolane (5g, 20.3mmol) was introduced and the mixture stirred at room temperature for 2.5 hrs. The mixture was heated at 85°C for 1.5 hrs. The reaction mixture was reduced under vacuum, diluted with water (60ml) and filtered. The solid was washed with DCM (50ml). The DCM filtrate was dried (phase separator) to give crude material. This was triturated with cyclohexane (60ml) and filtered to afford the title compound as a white solid (1.82g, 53%).
MH+ 400, rt= 1.92 mins

### Intermediate 43

### 2-Iodo-1-(phenylsulfonyl)-4-[4-(1-pyrrolidinylsulfonyl)phenyl]-1H-pyrrolo[2,3-b]pyridine

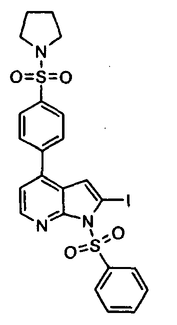

1-(Phenylsulfonyl)-4-[4-(1-pyrrolidinylsulfonyl)phenyl]-1*H*-pyrrolo[2,3-*b*]pyridine (3g, 6.42mmol) was dissolved in THF (80ml) and cooled to -70°C under nitrogen. 2M LDA in heptane/THF/ethylbenzene (7.06ml, 14.12mmol) was added and the reaction was stirred at -70°C for 1 hour. A solution of iodine (4.86g, 19.25mmol) in THF (18ml) was added drop wise whilst maintaining the temperature below -60 °C . The solution became very thick and stirring became difficult but was maintained due to a large stirrer bar. After 15 mins the ice-bath was removed and the reaction was allowed to warm to room temperature. The reaction was quenched with aq. ammonium chloride (150ml) and extracted with EtOAc (3x150ml). The combined organic layers were washed with brine (150ml) and reduced under *vacuum* to give crude material (7g, Material A). This was washed with chloroform. The chloroform was reduced under vacuum to afford (1.5g) of material which was purified by 100g silica FlashMaster (II) eluting with EtOAc-cyclohexane over 30 mins to afford (0.461g, Material B, impure). The remaining crude material (Material A) was dissolved in 50:50 DCM: methanol, reduced onto silica and purified using 2x 50g SPE cartridges eluting with DCM to 5% EtOAc in DCM to 10% EtOAc in DCM. The product containing fractions were reduced under *vacuum* to afford impure material (3.5g, Material C). This material (3.5g, Material C) was triturated using methanol to afford a solid (3g, Material D). The filtrate was also kept (Material D). The filtrate (Material D) was purified using 2x100g silica FlashMaster (II) eluting with 0-100% EtOAc-cyclohexane. The product containing fractions were combined and reduced under vacuum to afford (2.3g, Material E, minor impurity). Material E (2.3g) and Material B (0.461g) were combined, washed with methanol and dried to afford the title compound (2.52g, 66%).
MH+594, rt= 3.63 mins

### Intermediate 44

### 2-(Trifluoromethyl)-1H-pyrrolo[2,3-b]pyridine

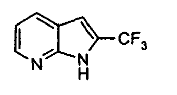

1,1-Dimethylethyl (3-methyl-2-pyridinyl)carbamate (Synthesis, 1996, 7, 877) (5.2g, 25mmol) was stirred in dry THF (60ml) and cooled in an ice bath (ice/salt) to -4°C. The mixture was treated with 2M-Bu lithium in cyclohexane (25ml, 50mmol) under nitrogen in a drop wise fashion over 45 mins while maintaining the temperature below 0°C. The red suspension was stirred for an hour at -3°C then treated with N-methoxy-N-methyl trifluoro acetamide (4.71g, 30mmol). There was a temperature rise to 20°C. The dark red solution was cooled to 2°C and then allowed to warm to 10°C over an hour. The dark orange solution was added to 5M HCl (55ml) over 30 mins at 3°C. The mixture was then heated at 60°C for an hour. The reaction was heated at 80°C for a further hour. The phases were separated and the aq. phase was made alkaline with 10M sodium hydroxide. The mixture was extracted with EtOAc (2x50ml). The organic phase was dried then evaporated to give a pale orange solid which was filtered through silica (70g) eluting with DCM to DCM: ether 9:1. The main fraction was evaporated to give the title compound as a pale yellow crystalline solid (2.66g, 57%).
MH+187, rt= 2.73 mins

### Intermediate 45

### 2-(Trifluoromethyl)-1H-pyrrolo[2,3-b]pyridine 7-oxide

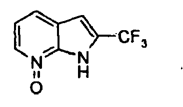

A stirred, cooled (0°C ice/salt) solution of 2-(trifluoromethyl)-1*H*-pyrrolo[2,3-*b*]pyridine (1.86g, 10mmol) in EtOAc (35ml) was treated drop wise with a solution of m-chloroperoxybenzoic acid (2.78g, 12.2mmol) in EtOAc (35ml) over 30 mins. The reaction temperature was maintained below 5°C during the addition. The reaction was warmed to 10°C over 2 hrs. The reaction was cooled to 0°C and treated with an additional portion of MCPBA (0.700g, 4mmol) in EtOAc (10ml). The reaction was warmed to room temperature over 2 hrs. The solid precipitate was collected to give the title compound as a white solid (0.950g, 47%).
MH+203, rt= 0.68 mins

### Intermediate 46

### 4-Chloro-2-(trifluoromethyl)-1H-pyrrolo[2,3-b]pyridine

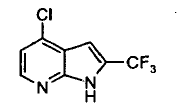

2-(Trifluoromethyl)-1*H*-pyrrolo[2,3-*b*]pyridine 7-oxide (1.26g, 6.25mmol) was suspended in dimethylformamide (7.5ml) and heated to 50°C. The mixture was treated with methanesulfonyl chloride (2.5ml) drop wise. The solid went into solution on addition of the sulphonyl chloride and there was a temperature increase to 60°C. The reaction was heated at 70°C for 2 hrs and cooled to room temperature. The reaction was poured into water (50ml) and neutralised with 10M sodium hydroxide. The solid was collected and dried under air to give (1.24g). This material was triturated with hot aq. ethanol, collected and was dried *in vacuo* (50°C) to afford the title compound as a cream solid (1.1g, 80%).
MH+221, rt= 3.22 mins

### Intermediate 47

### 4-{4-[4-(1-Pyrrolidinyisulfonyl)phenyl]-1H-pyrrolo[2,3-b]pyridin-2-yl}-3-butyn-1-ol trifluoroacetate (salt)

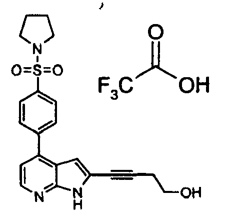

3-Butyn-1-ol (280mg, 0.4mmol) was treated with copper iodide (10mg, 0.05mmol) and Bis(triphenylphosphine)palladium (II) dichloride (10mg, 0.014mmol). A suspension of 2-iodo-1-(phenylsulfonyl)-4-[4-(1-pyrrolidinyfsulfonyl)phenyl]-1*H*-pyrrolo[2,3-*b*]pyridine (178mg, 0.3mmol) in dry THF (1.5mL), treated with TEA (0.5mL) was added and the reaction mixture stirred at 22°C for 18h. Purification by flash chromatography affords 4-{1-(phenylsulfonyl)-4-[4-(1-pyrrolidinylsulfonyl)phenyl]-1H-pyrrolo[2,3-b]pyridin-2-yl}-3-butyn-1-ol. (LCMS RT= 3.27min ES+ve 536 m/z (MH)⁺). A suspension of 4-{1-(phenylsulfonyl)-4-[4-(1-pyrrolidinylsulfonyl)phenyl]-1H-pyrrolo[2,3-b]pyridin-2-yl}-3-butyn-1-ol (0.064g, 0.12mmol) in dioxan:water, 5:1 (1mL) was treated with 40% KF supported on Alumina (220mg, 0.15mmol) and heated at 120°C in the mw for 10 mins. The reaction mixture was applied directly to a C18 cartridge (0.5g) and eluted with 0.1% TFA in acetonitrile (3 x 1 mL). Concentration by blow down and purification by mass directed HPLC gave the title compound.
LCMS RT= 2.99min MH+ 396

Intermediate 48 was similarly prepared:

| **Intermediate** | Compound | LEMS rt, | m/z |
|---|---|---|---|
| | | min | (MH+) |
| **48** | 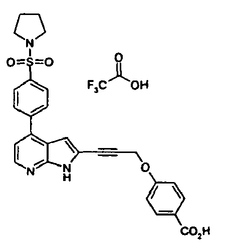 | 3.48 | 502 |
| | 4-[(3-{4-[4-(1-pyrrolidinylsulfonyl)phenyl]-1*H*-pyrrolo[2,3-*b*]pyridin-2-yl}-2-propyn-1-yl)oxy]benzoic acid trifluoroacetate | | |

### Intermediate 49

### {4-[2-Methyl-1-(phenylsulfonyl)-1H-pyrrolo[2,3-b]pyridin-4-yl]phenyl}amine

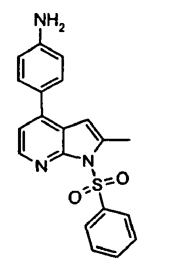

4-Bromo-2-methyl-1-(phenylsulfonyl)-1*H*-pyrrolo[2,3-*b*]pyridine (0.1.00g, 0.284mmol) [4-(4,4,5,5-tetramethyl-1,3,2-dioxaborolan-2-yl)phenyl]amine (0.093g, 0.426mmol), bis(diphenylphosphino)ferrocene palladium (II) chloride (0.023g, 0.0284mmol) and sodium carbonate (0.090g, 0.852mmol) in dioxan (3.5ml) and water (0.7ml) were heated in the Biotage Initiator mw at 150°C for 30 min. The reaction mixture was partitioned between DCM : EtOAc (1:1, 15ml) and saturated citric acid : water (1:1, 10ml). The aqueous phase was extracted with DCM : EtOAc (1:1, 10ml). The combined organic extracts were concentrated and the dark residue purified by FlashMaster using EtOAc - cyclohexane (0-100%). The desired fractions were combined and concentrated *in vacuo* to give the title compound as a beige solid (0.088g, 85%).
MH+364, rt= 1.11 min

### Intermediate 50

### 1,1-Dimethylethyl methyl{4-[2-methyl-1-(phenylsulfonyl)-1H-pyrrolo[2,3-b]pyridin-4-yl]phenyl}carbamate

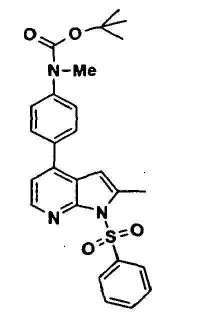

4-Bromo-2-methyl-1-(phenylsulfonyl)-1*H*-pyrrolo[2,3-*b*]pyridine (0.100g, 0.284mmol) 1,1-dimethylethyl methyl[4-(4,4,5,5-tetramethyl-1,3,2-dioxaborolan-2-yl)phenyl]carbamate (0.144g, 0.427mmol), bis(diphenylphosphino)ferrocene palladium (II) chloride (0.023g, 0.0284mmol) and sodium carbonate (0.090g, 0.852mmol) in dioxan (3.5ml) and water (0.7ml) were heated in the Biotage Initiator mw at 150°C for 30 min. The reaction mixture was partitioned between DCM (10ml) and saturated citric acid : water (1:2, 15ml). The aqueous phase was extracted with DCM (10ml). The combined organic extracts were concentrated and the dark residue purified by FlashMaster on silica using EtOAc - cyclohexane (0-100%). The desired fractions were combined and concentrated *in vacuo* to give the title compound as a white solid (0.132g, 97%).
MH+478, rt= 1.41 min

### Intermediate 51

### Methyl{4-[2-methyl-1-(phenylsulfonyl)-1H-pyrrolo[2,3-b]pyridin-4-yl]phenyl}amine

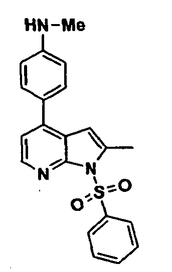

A mixture of 1,1-dimethylethyl methyl{4-[2-methyl-1-(phenylsulfonyl)-1*H*-pyrrolo[2,3-*b*]pyridin-4-yl]phenyl}carbamate (0.138g, 0.289mmol) and TFA (0.045ml) in DCM (5ml) was stirred at room temperature for 20 hrs. The reaction was quenched with saturated sodium carbonate solution (10ml). DCM (15ml) and water (10ml) were added and the layers separated. The aqueous phase was extracted with DCM (15ml). The combined organic extracts were dried (hydrophobic frit) and concentrated *in vacuo* to give the title compound as a beige glass (0.106g, 97%).
MH+378, rt= 1.24 min

### Intermediate 52

### N-{4-[2-Methyl-1-(phenylsulfonyl)-1H-pyrrolo[2,3-b]pyridin-4-yl]phenyl}methanesulfonamide

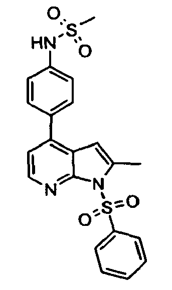

A mixture of {4-[2-methyl-1-(phenylsulfonyl)-1*H*-pyrrolo[2,3-*b*]pyridin-4-yl]phenyl}amine (0.086g, 0.237mmol), methanesulfonyl chloride (0.020ml, 0.261 mmol) and TEA (0.036ml, 0.261 mmol) in THF (5ml) was stirred at room temperature for 3 hrs. Methanesulfonyl chloride (0.020ml, 0.261mmol) and TEA (0.036ml, 0.261mmol) were added to push the reaction to completion and stirring was continued for 1 hour. The reaction mixture was partitioned between EtOAc (25ml) and water (25ml). A few drops of ammonium chloride were added to the aqueous phase to reach pH6-7. After extraction and separation of the 2 phases, the aqueous phase was extracted with EtOAc (20ml). The combined organic phases were dried (hydrophobic frit), the solvent removed and the yellow residue purified by FlashMaster on silica using EtOAc - cyclohexane (0-100%) as eluant. The desired fractions were combined and concentrated *in vacuo* to give the title compound as a colourless glass (0.092g, 88%).
MH+442, rt= 1.11 min

### Intermediate 53

### N-Methyl-N-{4-[2-methyl-1-(phenylsulfonyl)-1H-pyrrolo[2,3-b]pyridin-4-yl]phenyl}methanesulfonamide

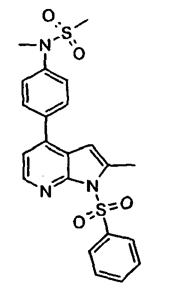

A mixture of methyl{4-[2-methyl-1-(phenylsulfonyl)-1*H*-pyrrolo[2,3-*b*]pyridin-4-yl]phenyl}amine (0.104g, 0.275mmol), methanesulfonyl chloride (0.032ml, 0.414mmol) and TEA (0.056ml, 0.414mmol) in dry THF (5ml) was stirred at room temperature under nitrogen for 3 hrs. Methanesulfonyl chloride (0.032ml, 0.414mmol) and TEA (0.056ml, 0.414mmol) were added and stirring was continued for 1 hour. DCM : EtOAc (1:1, 25ml) and water (25ml) were added. The aqueous layer was extracted with DCM : EtOAc (1:1, 20ml). The combined organic phases were dried (hydrophobic frit), the solvent removed in *vacuo* and the yellow oil purified by FlashMaster on silica using EtOAc - cyclohexane (0-100%). The desired fractions were combined and concentrated *in vacuo* to give the title compound (0.091 g, 72%).

### Intermediate 54

### N-(1,1-Dioxidotetrahydro-2H-thiopyran-4-yl)-4-[2-methyl-1-(phenylsulfonyl)-1H-pyrrolo[2,3-b]pyridin-4-yl]benzenesulfonamide

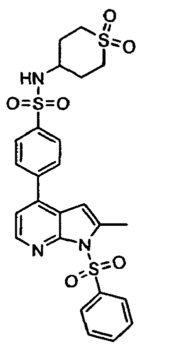

A mixture of 4-bromo-2-methyl-1-(phenylsulfonyl)-1*H*-pyrrolo[2,3-*b*]pyridine (0.300g, 0.854mmol), bis(pinacolato)diborane (1.084g, 4.27mmol), bis(diphenylphosphino)ferrocene palladium (II) chloride (0.070g, 0.0854mmol) and potassium acetate (0.418g, 4.27mmol) was stirred in dry DMF (15ml) under nitrogen for 30 min, at 50°C for 3 hrs and at 90°C for 1 hour. Water (5ml) was added and the reaction mixture heated at 90°C for 30 min. 4-Bromo-*N*-(1,1-dioxidotetrahydro-2*H*-thiopyran-4-yl)benzenesulfonamide (0.377g, 1.02mmol) was added and the reaction mixture was heated at 90°C for 1 hour. The cooled reaction mixture was partitioned between DCM (50ml) and water (50ml). The DCM extract was dried (hydrophobic frit), concentrated *in vacuo* and purified by FlashMaster on silica using EtOAc - cyclohexane (0-100%). The desired fractions were combined and concentrated *in vacuo* to give the title compound as a beige solid (0.194g, 40%).
MH+560, rt= 1.08 min

### Intermediate 55

### N-(1,1-Dioxidotetrahydro-2H-thiopyran-4-yl)-N-methyl-4-[2-methyl-1-(phenylsulfonyl)-1H-pyrrolo[2,3-b]pyridin-4-yl]benzenesulfonamide

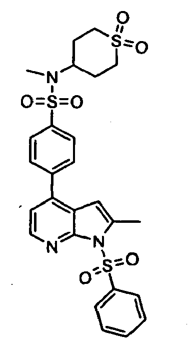

A mixture of *N*-(1,1-dioxidotetrahydro-2*H*-thiopyran-4-yl)-4-[2-methyl-1-(phenylsulfonyl)-1*H*-pyrrolo[2,3-*b*]pyridin-4-yl]benzenesulfonamide (0.050g, 0.089mmol), cesium carbonate (0.045g, 0.134mmol) and methyl iodide (0.0084ml, 0.134mmol) was stirred in DCM (5ml) at room temperature under nitrogen for 14 hrs. Methyl iodide (0.055ml, 0.89mmol) was added and the reaction mixture heated at 45°C under nitrogen for 5 hrs. The reaction mixture was partitioned between water (10ml) and DCM (10ml). The organic layer was dried (hydrophobic frit) and concentrated *in vacuo* to give the title compound (0.053g, 100%).
MH+574, rt= 1.13min

### Intermediate 56

### 2-(4-Bromo-1H-pyrrolo[2,3-b]pyridin-2-yl)-N-[3-(methyloxy)phenyl]acetamide

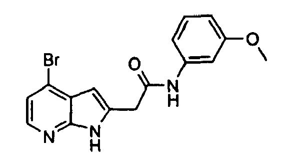

(4-Bromo-1*H*-pyrrolo[2,3-*b*]pyridin-2-yl)acetic acid trifluoroacetate (1.0g, 2.7mmol) was dissolved in DMF (10ml) and treated with 1-hydroxybenzotriazole (0.600g, 4mmol), *N*-[3-(dimethylamino)propyl]-*N*'-ethylcarbodiimide (0.777g, 4mmol) and *N*,*N-*diisopropylethylamine (1.52ml, 9mmol). The solution was stirred at room temperature for 20 min. The solution was divided into two and one portion treated with m-anisidine (0.370g, 5.4mmol). The reaction was stirred at room temperature for 3 days. The reaction was evaporated and purified by SPE eluting with DCM to DCM : MeOH (99:1 to 9:1) to give the title compound as a brown solid (0.109g. 11%).
MH+360, rt= 1.04 min

### Intermediate 57

### 4-Bromo-2-[2-oxo-2-(1-pyrrolidinyl)ethyl]-1H-pyrrolo[2,3-b]pyridine

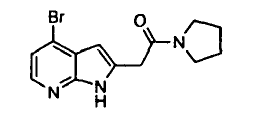

(4-Bromo-1*H*-pyrrolo[2,3-*b*]pyridin-2-yl)acetic acid trifluoroacetate (1.11g, 3.0mmol) was stirred in DCM (25ml) and cooled in an ice bath. The suspension was treated with pyrrolidine (0.254ml, 3mmol), stirred for 5 min, treated with *N*-[(1*H*-1,2,3-benzotriazol-1-yloxy)(dimethylamino)methylidene]-*N*-methylmethanaminium tetrafluoroborate (1.2g, 3.6mmol) and pyrrolidine (0.508ml, 6mmol). The reaction was allowed to warm to room temperature over 1 hour. The reaction was washed with water (20ml), 10% citric acid (10ml) and 2M sodium hydroxide (10ml) and evaporated to give the title compound as a buff solid (0.843g, 91%).
MH+310, rt= 0.92 min

### Intermediate 58

### 2-Methyl-1-(phenylsulfonyl)-4-(4,4,5,5-tetramethyl-1,3,2-dioxaborolan-2-yl)-1H-pyrrolo[2,3-b]pyridine

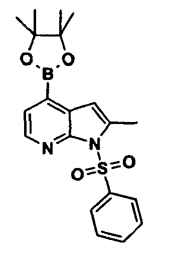

4-Bromo-2-methyl-1-(phenylsulfonyl)-1*H*-pyrrolo[2,3-*b*]pyridine (0.600g, 1.7mmol), potassium acetate (0.500g, 5.1 mmol) and palladium acetate (0.020g, 0.085mmol) were mixed together in DMF (30ml) and stirred at room temperature for 5 min. Bis(pinacolato)diboron (1.08g, 4.25mmol) in DMF (5ml) was added to the mixture and it was stirred at room temperature for 2 hrs and at 65°C for 2 hrs. The mixture was reduced *in vacuo,* diluted with water (60ml) and extracted with DCM (3x60ml). The organic layers were filtered (phase separator) and reduced *in vacuo* to give a solid which was triturated in cyclohexane. The solid was removed by filtration and the filtrate was reduced *in vacuo* to give impure material as a pale grey solid (0.450g).
MH+399, rt= 1.37 min

### Intermediate 59

### 4-Bromo-N-(2-hydroxyethyl)benzenesulfonamide

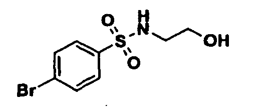

Ethanolamine (12.99ml, 215.25mmol) and TEA (81.82ml, 587.04mmol) were combined in DCM (200ml) and cooled to 0°C. 4-Bromobenzenesulfonyl chloride (50g, 195.68mmol) in DCM (50ml) was added slowly. The reaction was stirred under nitrogen at room temperature overnight. The reaction mixture was concentrated and diluted with EtOAc (750ml). The organic layer was washed with 1 M sodium hydroxide (250ml), sodium hydrogen carbonate, brine, dried (MgSO₄), filtered, concentrated and dried on the vacuum line overnight. The title compound was obtained as a white solid (43.64g).
MH+280 / 282, rt= 2.39 min

### Intermediate 60

### [4-Bromo-1-(phenylsulfonyl)-1H-pyrrolo[2,3-b]pyridin-2-yl]methanol

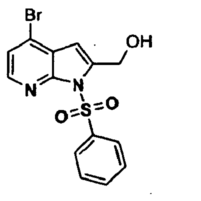

To a solution of 4-bromo-2-methyl-1-(phenylsulfonyl)-1*H*-pyrrolo[2,3-*b*]pyridine (10.0g, 29.7mmol) in dry THF (150ml) at -40°C under nitrogen was added 2M LDA in heptane/THF/ethylbenzene (30.0ml, 60.0mmol) drop wise. The reaction was stirred -40°C for 30 min then cooled to -60°C. Paraformaldehyde (7g, 23.3mmol) was added in one portion. After 3 hrs the reaction was cooled to -60°C and saturated ammonium chloride solution (100ml) was added to pH5-6. It was allowed to warm to room temperature and extracted with DCM (2x100ml). The combined extracts were washed with 2M hydrochloric acid, brine, dried (MgSO₄) and evaporated to give a brown oil (10g). Purification was by silica SPE cartridge (50g) eluting with DCM to 90% DCM / EtOAc to give the title compound as a yellow foam (2.5g, 23%).
MH+369, rt= 1.03 min

### Intermediate 61

### [4-Bromo-1-(phenylsulfonyl)-1H-pyrrolo[2,3-b]pyridin-2-yl]methyl methanesulfonate

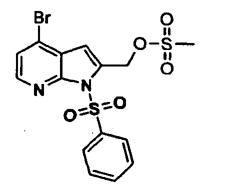

To a solution of [4-bromo-1-(phenylsulfonyl)-1*H*-pyrrolo[2,3-*b*]pyridin-2-yl]methanol (2.5g, 6.81mmol) in dry DCM (30ml) at room temperature under nitrogen was added TEA (1.1ml, 8.0mmol) followed by methanesulfonic anhydride (1.4g, 8.05mmol) in one portion. The reaction was left standing at room temperature over the weekend. DCM (50ml) was added and washed with 2M hydrochloric acid (50ml), brine (50ml), dried (MgSO₄) and evaporated to give the title compound as a brown foam (2.8g, 89%).
¹H NMR (400MHz; CDCl₃) δ: 3.10 (3H, s), 5.72 (2H, s), 6.86 (1H, s), 7.40 (1H, d), 7.52 (2H, t), 7.61 (1H, t), 8.27 (3H, m).

### Intermediate 62

### 4-Bromo-1-(phenylsulfonyl)-2-(1H-1,2,3-triazol-1-ylmethyl)-1H-pyrrolo[2,3-b]pyridine

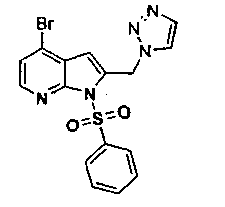

Triazole (1.150g, 16.54mmol) in THF was added to a solution of potassium t-butoxide (1.0g, 9.1mmol) in THF (total volume 50ml) at room temperature. After stirring for 30 mins [4-Bromo-1-(phenylsulfonyl)-1*H*-pyrrolo[2,3-*b*]pyridin-2-yl]methyl methanesulfonate (3.68g, 8.27mmol) was added and left to stir for 2 hrs. The reaction mixture was poured onto aqueous sodium bicarbonate (100ml), organic layer was separated and the aqueous fractionw as extracted with DCM (2 x 100ml). The cobined organic fractions were passed through a phase separator and soulvent removed under vacuum. Crude product was purfied with a Flashmaster Si II cartridge (100g) in a gradient 0-100% EtOAc in cyclohexane over 40 minute to afford the title compound as a pale yellow solid (1.48g).
Rt = 2.98 min, MH+ = 420

### Intermediate 63

### 4-Bromo-2-(1H-1,2,3-triazol-1-ylmethyl)-1H-pyrrolo[2,3-b]pyridine

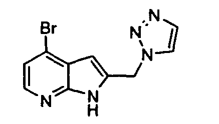

4-Bromo-1-(phenylsulfonyl)-2-(1*H*-1,2,3-triazol-1-ylmethyl)-1*H*-pyrrolo[2,3-*b*]pyridine (1.0g, 2.4mmol) was diluted in THF (15ml) and mixed with TBAF (1M in THF) (3.1ml, 3.1mmol) and stirred at room temperature for 2 hrs. The reaction mixture was applied to a SCX cartridge (50g) and eluted with MeOH followed by 2M ammonia in MeOH. Appropriate fractions were combined and solvent was removed *in vacuo* to afford the title compound as yellow solid (0.76g).
Rt = 0.87 min, M-H+ = 276 / 278

### Intermediate 64

### 1-[4-Bromo-1-(phenylsulfonyl)-1H-pyrrolo[2,3-b]pyridin-2-yl]ethanol

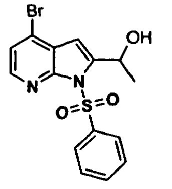

A solution of 1-[4-bromo-1-(phenylsulfonyl)-1*H*-pyrrolo[2,3-*b*]pyridin-2-yl]ethanone (0.56g, 1.48mmol) in THF (8.4ml) and water (3.4ml) was treated with sodium borohydride (0.168g, 4.43mmol) after stirring at room temperature for 90 mins the reaction was diluted with water (50ml) and extracted with DCM (2 x 50ml). Organic layers were combined and reduced under vacuum whereupon the crude product was purified on Si SPE (20g) column eluting with DCM, DCM / 2% EtOAc, DCM / 5% ETOAc to afford the title compound (0.443g).
Rt = 3.11min, MH+ = 383

### Intermediate 65

### 1,1-Dimethylethyl ({1-(phenylsulfonyl)4-[4-(1-pyrrolidinylsulfonyl)phenyl]-1H-pyrrolo[2,3-b]pyridin-2-yl}methyl)carbamate

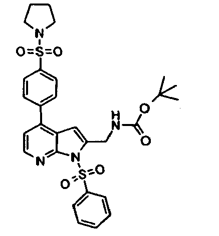

1-(Phenylsulfonyl)-4-[4-(1-pyrrolidinyisulfonyl)phenyl]-1*H*-pyrrolo[2,3-*b*]pyridine-2-carbaldehyde (0.300g, 0.61 mmol), t-Bu carbamate (0.213g, 1.82mmol), TFA (0.090ml, 1.21mmol) and triethylsiliane (0.290ml, 1.83mmol) in acetonitrile (4ml) were stirred at room temperature under nitrogen overnight. The reaction was poured into aqueous sodium hydrogen carbonate (50ml) and extracted with DCM (2x50ml). The organic layers were combined and reduced *in vacuo* to give crude material (0.600g) which was partially dissolved in DCM and loaded onto a silica SPE cartridge (50g). It was eluted with DCM to 1% EtOAc in DCM to 2% EtOAc in DCM to 3% EtOAc in DCM to 5% EtOAc in DCM to give the title compound (0.198g, 54%).
MH+597, rt= 3.57 min

### Intermediate 66

### ({1-(Phenylsulfonyl)-4-[4-(1-pyrrolidinylsulfonyl)phenyl]-1H-pyrrolo[2,3-b]pyridin-2-yl}methyl)amine

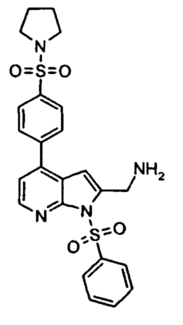

1,1-Dimethylethyl ({1-(phenylsulfonyl)-4-[4-(1-pyrrolidinylsulfonyl)phenyl]-1*H*-pyrrolo[2,3-*b*]pyridin-2-yl}methyl)carbamate (0.188g, 0.315mmol) in DCM (1ml) and TFA (1ml) was stirred at room temperature under nitrogen for 2 hrs. The reaction was reduced *in vacuo,* the residue diluted with aqueous sodium hydrogen carbonate (30ml) and extracted with DCM (2x30ml). The combined organic layers were dried (phase separator) and reduced in *vacuo* to give the title compound (0.127g, 81%).
MH+497, rt= 2.79 min

### Intermediate 67

### N-Methyl-4-(2-methyl-1H-pyrrolo[2,3-b]pyridin-4-yl)aniline

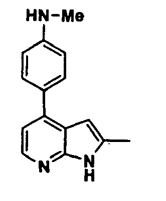

A mixture of methyl{4-[2-methyl-1-(phenylsulfonyl)-1*H*-pyrrolo[2,3-*b*]pyridin-4-yl]phenyl}amine (1.5g) and NaOH (6M, 30mL) in 1,4-dioxane (75ml) was heated at 85C for 4 days. Upon cooling the reaction mixture was partitioned between DCM (100ml) and HCl (2M, to reach pH -9). The 2 phases were separated and the organic layer was dried through a hydrophobic fritand concentrated *in vacuo* to afford the title compound as a brown solid (0.955g)
MH+238, rt= 2.43 min

### Intermediate 68

### 1,1-Dimethylethyl (2-{[(4-bromophenyl)sulfonyl]amino}ethyl)carbamate

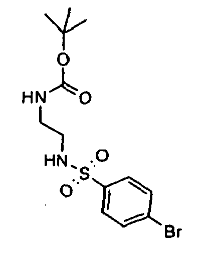

1,1-Dimethylethyl (2-aminoethyl)carbamate (34.08ml, 215.25mmol), TEA (81.82ml, 587.04mmol) and DCM (250ml) were stirred for 10 min. 4-Bromobenzenesulfonyl chloride (50g, 195.68mmol) in DCM (250ml) was added. Exothermic reaction occured (DCM refluxing, recommend slow addition with cooling). The reaction mixture was stirred at room temperature overnight. The reaction mixture was diluted with 0.5M hydrochloric acid (500 ml). A white solid crashed out, it was filtered and left in the oven for 4 hr. The title compound was obtained as a white fluffy solid (55.73g).
MH+ 381, rt= 3.10 min

### Intermediate 69

### N-(2-Hydroxyethyl)-4-(4,4,5,5-tetramethyl-1,3,2-dioxaborolan-2-yl)benzenesulfonamide

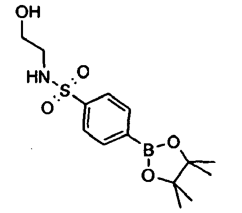

### Method A

4-Bromo-*N*-(2-hydroxyethyl)benzenesulfonamide (5.0g, 17.86mmol), potassium acetate (5.28g, 53.6mmol) and palladium acetate (0.207g, 0.89mmol) were dissolved in DMF (100ml) and heated to 60°C before addition of a solution of 4,4,4',4',5,5,5',5'-octamethyl-2,2'-bi-1,3,2-dioxaborolane (10.9g, 42.86mmol) in DMF (50ml). The mixture was stirred at 60°C for 2 hr. The stirring was stopped and the liquid portion decanted and concentrated *in vacuo*. The residue was triturated in water, the grey solid filtered and dried *in vacuo* to give dimer (5.01g). The product containing aqueous phase was quickly re-filtered and extracted with DCM (2x100ml). The combined organic extracts were dried (hydrophobic frit), concentrated *in vacuo* to give a very pale yellow oil (2.33g) and dried further to give the title compound (1.84g, 31.5%).
MH+328, rt= 1.02 min

### Method B

To a degassed suspension of 4-bromo-N-(2-hydroxyethyl)benzenesulfonamide (50 g, 178 mmol), bispinacolatodiboron (68.0 g, 268 mmol) and potassium acetate (52.6 g, 535 mmol) in N,N-dimethylformamide (DMF) (500 mL) stirred under nitrogen at ambient temperature was added solid palladium acetate (2.00 g, 8.91 mmol) in one charge and the reaction degassed for a further 10 mins. The reaction was warmed to 55°C at which point an exotherm raised the temperature to 70°C before cooling to 55°C. The reaction was stirred for a total of 4 hr. The reaction was allowed to cool to ambient temperature and filtered through a pad of celite. The cake was washed with methanol (500 mL) and the combined filtrates concentrated *in vacuo* to a yellow sludge. Trituration with dichloromethane (2 L) gave a white solid which was removed by filtration. The filtrate was washed with water (2 L). The organic suspension was separated and dried over magnesium sulphate, filtered and concentrated *in vacuo* to a yellow oil. This oil was treated with cyclohexane (200 mL) and stirred vigourously until a white solid formed. This solid was collected by filtration and washed with cyclohexane (ca. 500 mL) before drying in a vacuum oven to give the required product N-(2-hydroxyethyl)-4-(4,4,5,5-tetramethyl-1,3,2-dioxaborolan-2-yl)benzenesulfonamide as an off white solid (42.19 g, 129 mmol, 72.2 %).
LCMS: rt = 2.80 mins, MH+ = 328

### Intermediate 70

### 2-(1,1-Dimethylethyl)-1H-pyrrolo[2,3-b]pyridine

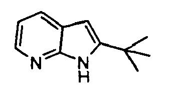

1,1-Dimethylethyl (3-methyl-2-pyridinyl)carbamate (5.2g, 25mmol) was suspended in anhydrous THF (60ml). This suspension was cooled to -5°C under nitrogen. n-Butyllitium (ca 1.25M in hexanes; 19.5ml, 24mmol) was added via dropping funnel until the suspension became red (45 min drop wise). A further portion of n-butyllithium (19.5ml) was added over 20 min at -5°C. The suspension was stirred for a further 40 min. *N,N'-*Dimethylpivalamide (3.87g, 30mmol) was added in one portion. There was a colour change to bright orange. After 30 min the reaction was allowed to warm to room temperature. After a further 30 min 5M hydrochloric acid (55ml) was added and the biphasic mixture was heated to 60°C for 2 hr. The organic layer was removed, the aqueous treated with 10M aqueous sodium hydroxide (40ml) and extracted with EtOAc (2x55ml). The combined organic extracts were dried and concentrated *in vacuo* to give (4.35g). This solid was pre-absorbed onto Florosil and purified by chromatography on silica (100g) eluting with 0 to 100% EtOAc in DCM over 40 min to give the title compound as a very pale yellow crystalline solid (2.34g, 54%).
MH+175, rt= 0.99 min

### Intermediate 71

### 2-(1,1-Dimethylethyl)-1H-pyrrolo[2,3-b]pyridine 7-oxide

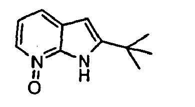

A solution of 2-(1,1-dimethylethyl)-1*H*-pyrrolo[2,3-*b*]pyridine (2.34g, 13.4mmol) in EtOAc (50ml) was cooled to 0°C. A solution of MCPBA (5.2g, 22.8mmol) in EtOAc (50ml) was added drop wise over 30 min. Once addition was complete, the reaction was allowed to warm up to 13°C over 2 hr. The reaction was washed sequentially with saturated aqueous sodium hydrogen carbonate (100ml) followed by saturated sodium metabisulphate (100ml), dried (MgSO₄), filtered and concentrated *in vacuo* to give an orange gum. Purification was by chromatography on silica gel (100g) eluting with 0-25% MeOH in DCM to give impure material as a yellow gum/glass (2.7g). Trituration with cyclohexane gave the title compound as a white solid (0.572g, 22%). The remainder was purified again (100g, silica, 0-25% MeOH/DCM, 60 min) to give a viscous oil (1.729g) which was triturated in a mixture of water (10ml) and saturated aqueous potassium carbonate (7ml) with heating. The solid was filtered and dried *in vacuo* to give a further batch of the title compound as a very pale yellow solid (0.864g, 33.5%).
MH+191, rt= 0.84 min

### Intermediate 72

### 4-Chloro-2-(1,1-dimethylethyl)-1H-pyrrolo[2,3-b]pyridine

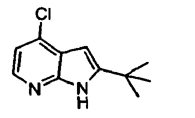

2-(1,1-Dimethylethyl)-1*H*-pyrrolo[2,3-*b*]pyridine 7-oxide (1.425g, 7.5mmol) was suspended in anhydrous DMF (10ml) under nitrogen and heated to 50°C. Methanesulfonyl chloride (3.2ml, 42mmol) was added drop wise over 5 min. The reaction was heated at 70°C for 3 hr. The reaction was poured onto water (50ml) was basified with 10M sodium hydroxide. The solid was collected by filtration and dried *in vacuo* to give the title compound as a yellow solid (1.418g, 90.5%).
MH+209 / 211, rt= 1.25 min

### Intermediate 73

### N-(2-Aminoethyl)-4-[2-(difluoromethyl)-1-(phenylsulfonyl)-1H-pyrrolo[2,3-b]pyridin-4-yl]benzenesulfonamide

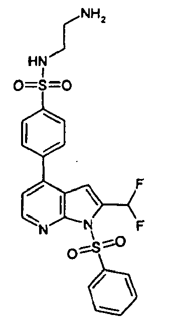

1,1-Dimethylethyl [2-({[4-(4,4,5,5-tetramethyl-1,3,2-dioxaborolan-2-yl)phenyl]sulfonyl}amino)ethyl]carbamate (660mg, 1.55mmol), 4-bromo-2-(difluoromethyl)-1-(phenylsulfonyl)-1H-pyrrolo[2,3-b]pyridine (500mg, 1.29mmol), bis(diphenylphosphino)ferrocene palladium (II) chloride (105mg, 0.129mmol) and a solution of 1 M sodium hydrogenocarbonate (3.87mL, 3.87mmol) in isopropanol (10mL) were heated in the Biotage Initiator mw at 120°C for 30 min in a sealed vial. The reaction mixture was partitioned between DCM (25ml) and water (25ml). After separation, the aqueous phase was extracted with DCM (25mL). The combined organic extracts were dried (hydrophobic frit) and concentrated *in vacuo.* The crude residue was dissolved in DCM (10mL) and treated with trifluoroacetic acid (1mL). The reaction mixture was left into solution for 64h. A saturated solution of sodium carbonate was added (20mL) and the phases separated. The organic extract was dried (hydrophobic frit), concentrated *in vacuo* and the residue purified by FlashMaster using a gradient of MeOH - DCM (0-50%) over 30mins. The desired fractions were combined and concentrated *in vacuo* to give the title compound as a brown solid (350mg).
LCMS rt= 2.93 min, MH+=507

### Intermediate 74

### 1,1-Dimethylethyl {2-[({4-[2-(difluoromethyl)-1-(phenylsulfonyl)-1H-pyrrolo[2,3-b]pyridin-4-yl]phenyl}sulfonyl)amino]ethyl}carbamate

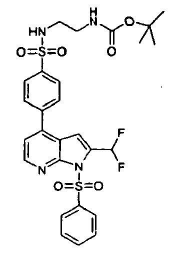

1,1-Dimethylethyl [2-({[4-(4,4,5,5-tetramethyl-1,3,2-dioxaborolan-2-yl)phenyl]-sulfonyl}amino)ethyl]carbamate (443 mg, 1.034 mmol), 4-bromo-2-(difluoromethyl)-1-(phenylsulfonyl)-1H-pyrrolo[2,3-b]pyridine (258mg, 0.677 mmol), sodium carbonate (139 mg, 1.311 mmol) and 1,1'-bis(diphenylphosphino) ferrocenedichloro palladium(II), complex with dichloromethane (30mg, 0.036 mmol) in dioxane:water (5:1) (10 mL) were heated in the Biotage Initiator mw at 150°C for 15 min in a sealed vial. The reaction mixture was partitioned between dichloromethane (250 mL) and a saturated solution of sodium carbonate (50mL). The organic extract was separated, dried (hydrophobic frit) and concentrated under vacuum. The product was purified by chromatography on silica (FlashMaster) eluting with an ethyl acetate/cyclohexane gradient (0-100%) to afford, after evaporation of the solvents, the title compound (312 mg).
LCMS: rt = 3.58 mins, MH+ = 607

### Intermediate 75

### [2-(Difluoromethyl)-1-(phenylsulfonyl)-1H-pyrrolo[2,3-b]pyridin-4-yl]boronic acid

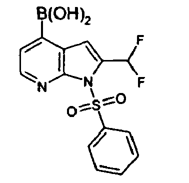

To a solution of 4-bromo-2-(difluoromethyl)-1-(phenylsulfonyl)-1*H*-pyrrolo[2,3-*b*]pyridine (2.0g, 5.17 mmol) in dry THF (50mL) at -78°C under nitrogen whilst stirring was added tris(1-methylethyl) borate (1.9 mL, 8.3 mmol) followed by n-butyl lithium (4.1ml, 1.6M in hexanes, 6.6 mmol) dropwise over 10 mins. Stirred at -78°C for 1h then allowed to warm up to ambient temperature over 3h. Cooled to -78°C and added 2M aqueous hydrochloric acid (100 ml) under nitrogen over 5 min then added 100 ml DCM. Separated organic layer then extracted aqueous layer with ethyl acetate (50 ml). Combined both organic extracts, dried through phase sep. cartridge and evaporated to give a pale brown foam. The foam was triturated with diethyl ether (100 mL). The resulting pale brown solid (900mg) was dried *in vacuo* at 60°C for 2h.
LCMS rt=3.06mins, MH+=353

### Intermediate 76

### 4-Iodo-2-(trifluoromethyl)-1H-pyrrolo[2,3-b]pyridine

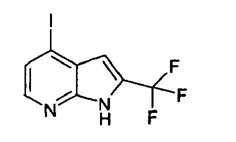

To 4-chloro-2-(trifluoromethyl)-1*H*-pyrrolo[2,3-*b*]pyridine (23.92g, 0.108mole, 1eq) dissolved in hot 1,4-dioxane (120ml, 5 volumes) was added 4M HCl in dioxane (30ml, 0.119mole, 1.1eq). The resulting suspension was cooled to room temperature and the solid collected by filtration washing well with diethyl ether. The solid was then suspended in anhydrous acetonitrile (480ml, 20 volumes) and then sodium iodide (97.7g, 0.652mole, 6eq) was added. The mixture was then heated to 80°C and maintained at that temperature overnight. It was then cooled to room temperature and 2M NaOH added till mixture was basic. The layers were then separated and the organic layer was washed with brine, dried over magnesium sulphate, filtered then concentrated *in vacuo* to a yield the title compound as a brown solid (9.98g, 29%).
LCMS rt = 1.19 mins, MH⁺= 313

The magnesium sulfate was then suspended in ethyl acetate and the mixture heated to 65°C. The mixture was filtered and the filtrate concentrated *in vacuo* to yield a second crop of the title compound as a cream solid (15.8g, 47%).
LCMS rt = 1.19 mins, MH⁺= 313

### Intermediate 77

### [2-(Trifluoromethyl)-1H-pyrrolo[2,3-b]pyridin-4-yl]boronic acid

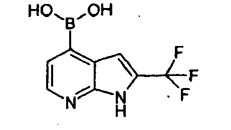

To a degassed solution of 4-iodo-2-(trifluoromethyl)-1*H*-pyrrolo[2,3-*b*]pyridine (1.0g, 3.2mmol) in anhydrous tetrahydrofuran (20mL) at 20°C was added sodium hydride as a 60% dispersion on mineral oil (160mg 4mmol) and stirred at 20°C for 75 mins. The mixture was degassed and cooled to -78°C before addition of n-butyl-lithium 1.5M in hexanes (4.91 mL, 7.36mmol) over 10 mins. Reaction stirred at -78°C for 20 mins. Triisopropylborate (2.26mL, 9.6mmol) was added over 5 mins. Reaction was warmed to 20°C over 1.5 h and water (20mL) added. The aqueous was extracted with ethyl acetate. The aqueous was adjusted to pH = 7 (citric acid) and further extracted with ethyl acetate. The combined extracts were dried (hydrophobic frit) and concentrated *in vacuo* to a yellow solid. Purification by aminopropyl cartridge (10g, eluent 4M ammonia in methanol) gave the title compound as a pale yellow solid (343mg, 47%).
LCMS rt = 0.76mins, MH⁺ = 231

### Intermediate 78

### 1,1-Dimethylethyl 4-[({4-[2-(1,1-dimethylethyl)-1H-pyrrolo[2,3-b]pyridin-4-yl]phenyl}-sulfonyl)amino]-1-piperidinecarboxylate

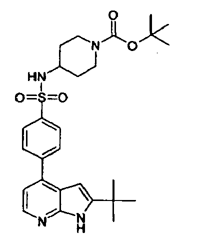

A mixture of 4-bromo-2-(1,1-dimethylethyl)-1*H*-pyrrolo[2,3-*b*]pyridine (177mg, 0.0007mole), 10% sodium carbonate (0.3ml), 1,1-dimethylethyl 4-({[4-(4,4,5,5-tetramethyl-1,3,2-dioxaborolan-2-yl)phenyl]sulfonyl}amino)-1-piperidinecarboxylate (359mg, 0.00077mole), 1,1-bis(diphenylphosphino)ferrocenedichloro palladium(II) (20mg), in 1,2-dimethoxyethane (3ml) was heated in a microwave at 140°C for 1 hr. The reaction mixture was poured into a mixture of dichloromethane (100ml) and water (20ml) and stirred for 5 minutes. The organic solution was separated and dried by passing through a phase separator cartridge, and evaporated to dryness to leave a gum. The crude product was purified by chromatography (bond elut cartridge 20g) eluting with cyclohexane : ethyl acetate = 20:1, 10:1, 5:1, 3:1, 2:1, and 1:1 (100ml of each). The appropriate fractions were evaporated to dryness to afford the title compound as a pale yellow solid (310mg, 86%)
LCMS rt = 1.27 min, MH+ = 513

### Intermediate 79

### 1,1-Dimethylethyl 4-[({4-[2-(trifluoromethyl)-1H-pyrrolo[2,3-b]pyridin-4-yl]phenyl}-sulfonyl)amino]-1-piperidinecarboxylate

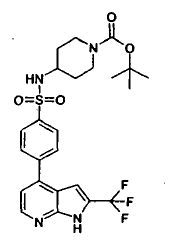

A mixture of 4-iodo-2-(trifluoromethyl)-1*H*-pyrrolo[2,3-*b*]pyridine (156mg, 0.0005mole), 10% sodium carbonate (0.2ml), 1,1-dimethylethyl 4-({[4-(4,4,5,5-tetramethyl-1,3,2-dioxaborolan-2-yl)phenyl]sulfonyl}amino)-1-piperidinecarboxylate (256mg, 0.00055mole), 1,1-bis(diphenylphosphino)ferrocenedichloro palladium(II) (15mg), in 1,2-dimethoxyethane (2ml) was heated in a microwave at 130°C, for 1h. The reaction mixture was poured into a mixture of dichloromethane (50ml) and water (20ml) and stirred for 5 minutes. The organic solution was separated and dried by passing through a phase separator cartridge, and evaporated to dryness to leave a gum. The crude product was purified by chromatography (bond elut cartridge 20g) eluting with cyclohexane : ethyl acetate = 20:1, 10:1, 5:1, 2:1, ethyl acetate and ethyl acetate:methanol = 20:1 (100ml, of each). The appropriate fractions were evaporated to dryness to afford the title compound as a foam/gum (170mg, 65%).
LCMS rt = 1.22 min, MH+ = 525

### Intermediate 80

### N-Methyl-N-(methyloxy)cyclopropanecarboxamide

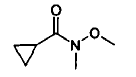

To a cooled (0°C) stirred solution of N,O-dimethylhydroxylamine HCl salt (5.87g, 0.06mole) in dry dichloromethane (40ml) was added triethylamine (15ml) and to this stirred mixture was added, under an atmosphere of nitrogen, a solution of cyclopropanecarbonyl chloride (6.27g, 0.06mole) in dichloromethane (20ml) over 45 minutes, keeping the reaction temperature below 0°C by means of an ice/water bath. After the addition was complete the reaction mixture was stirred at 0 to 5°C for 2h and then left to warm to 20°C over 18h. The reaction mixture was poured into dichloromethane (200ml) and water (50ml). The aqueous layer was separated and extracted with dichloromethane (2x25ml), the combined organic extract was washed with water (3x50ml), saturated brine (25ml), dried (phase separator cartridge) and evaporated to dryness to leave the title compound as a mobile oil (7.75g,).
LCMS rt = 0.67 min, MH+ = 130. Used without purification.

### Intermediate 81

### 2-Cyclopropyl-1H-pyrrolo[2,3-b]pyridine

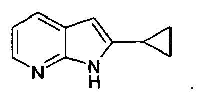

To a stirred, cooled (-4°C ice/salt bath) mixture of 1,1-dimethylethyl (3-methyl-2-pyridinyl)carbamate (10.4g, 0.05mole) in dry tetrahydrofuran (70ml) was added, under an atmosphere of nitrogen, n-butyllithium (2M solution in cyclohexane; 50ml, 0.1mole) dropwise over 45 minutes keeping the temperature between -5 and 0°C. The resulting red suspension was stirred between 0 to -5°C for 1 h, and then a solution of N-methyl-N-(methyloxy)cyclopropanecarboxamide (7.75g, 0.05mole) in tetrahydrofuran (20ml) added in a single portion at -3°C. The temperature rose to 20°C and the reaction mixture stirred 0°C for 2hr and then allowed to warm to 10°C and poured into 5M hydrochloric acid (100ml) with stirring over 5 minutes. The mixture was the heated at 60°C for 2h, cooled and the aqueous layer separated. The aqueous solution was basified by the addition of 10M sodium hydroxide, with ice/water cooling, until pH 10 /12 was obtained. The resulting mixture was extracted with ethyl acetate (3x100ml), the combined organic extract was washed with water (3x75ml) and saturated brine (50ml), dried (phase separator cartridge) and evaporated to dryness to leave an orange oil which solidified on standing. The solid was triturated under methanol:water = 4:1, filtered and air dried to provide the title compound as a pale orange solid (7.11g, 90%).
LCMS rt = 0.94 min, MH+ = 159

### Intermediate 82

### 2-Cyclopropyl-1H-pyrrolo[2,3-b]pyridine 7-oxide

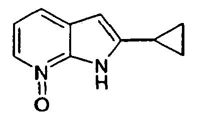

To a stirred solution of 2-cyclopropyl-1H-pyrrolo[2,3-b]pyridine (8.1g, 0.051mole) in 1,2-dimethoxyethane (150ml) was added a solution of m-chloroperoxybenzoic acid (17.9g, 1.22equiv of 60%) in 1,2-dimethoxyethane (50ml), under an atmosphere of nitrogen, keeping the temperature between 20 and 25°C (ice / water bath). The resulting suspension was stirred at 20°C for 1 hr, filtered, the solid washed with ethyl acetate and air dried to afford the title compound as a colourless solid (2.3g).
LCMS m/z = 175 (M+1), rt = 0.75 min

The filtrate was concentrated to ∼30ml *in vacuo* without heat, the oily residue was taken to pH8 to 9 by the addition of 30% potassium carbonate solution. The mixture was diluted with water (50ml) and extracted with chloroform (3x100ml). The combined organic extract was washed with water (2x50ml), dried (hydrophobic frit) and evaporated to dryness to yield a yellow solid. Trituration under ether afforded after filtration and air drying the title compound as a yellow solid (4.1g, 49%).
LCMS rt = 0.74 min, MH+ = 174, 176

### Intermediate 83

### 4-Chloro-2-cyclopropyl-1H-pyrrolo[2,3-b]pyridine

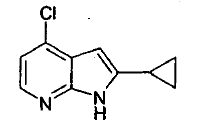

To a stirred solution of 2-cyclopropyl-1*H*-pyrrolo[2,3-*b*]pyridine 7-oxide (6.27g, 0.036mole) in dry N,N-dimethylformamide (50ml) was added methanesulphonyl chloride (30 ml, xs) in a single portion at 50°C. The resulting mixture was stirred at 70°C for 3h. The cooled black solution was poured into water (400ml), cooled to 10°C (ice / water). The solution was stirred at 10 to -5 °C and 10M sodium hydroxide added carefully until pH 9 to 10 was reached. The resulting suspension was stirred at 20°C for 2h and filtered and the solid washed well with water and air dried to furnish the title compound as a brown solid (5.1g, 74%).
LCMS rt = 1.11 min, MH+ = 193

Purification of 4-chloro-2-cyclopropyl-1*H*-pyrrolo[2,3-*b*]pyridine:

The crude solid (2.04g) was preabsorbed onto Florosil^{™} and purified by chromatography (silica 100g, 0-25% methanol in dichloromethane 60 mins). This gave the title compound as a pale yellow solid (1.0g).
LCMS rt = 3.18mins, MH⁺ = 193

### Intermediate 84

### 2-Ethyl-1H-pyrrolo[2,3-b]pyridine

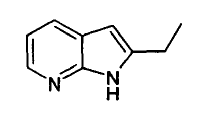

### Method A

To a stirred suspension of 1,1-dimethylethyl (3-methyl-2-pyridinyl)carbamate (5.2g, 25mmol) in anhydrous tetrahydrofuran (60mL) at 0°C under an atmosphere of nitrogen was added n-butyl-lithium (1M, 50mL) over 45 mins. After stirring for 1 h at 0°C N,N-dimethylpropionamide (3.3mL, 30mmol) was added. After stirring for a further 1 h at 0°C the reaction was allowed to warm to ambient temperature and was added to 5M aqueous hydrochloric acid (55mL) and heated to 70°C for 1.5 h. The organic was separated. The aqueous was basified with 10M aqueous sodium hydroxide and extracted with ethyl acetate (2x75mL). The combined extracts were dried (hydrophobic frit) and concentrated *in vacuo* to give an orange oil. Purification by chromatography (silica 100g, 0 to 25% ethylacetate in dichloromethane) followed by trituration with ca 9:1 methanol:water, filtration and drying *in vacuo* gave the title compound as a very pale yellow solid (0.819g, 23%).
LCMS rt = 0.79 min, MH+ = 147

### Method B

A suspension of 1,1-dimethylethyl (3-methyl-2-pyridinyl)carbamate (40.1g, 190mmol) in tetrahydrofuran (400mL) was stirred for 10 mins to give a solution. The solution was cooled to -3°C. n-Hexyllithium (2.3M, 185mL, 228mmol) was slowly added over 36 mins (temperature mostly in the -4 to 4°C range with a brief peak at 8°C). The mixture was stirred for an additional hour at -3 to -1°C to give a deep red mixture. N-Methyl-N-(methyloxy)propanamide (30.2g, 90.2% assay, 228mmol) was added dropwise over 17 minutes at -1 to 4°C. The contents of the dropping funnel were rinsed with THF (8mL) and added to the reaction. After a further 1 h and 45 minutes at 1 ± 1°C the reaction mixture was transferred over 21 minutes to a second vessel containing aqueous sulfuric acid (170mL, 15% v/v) stirred at stirred at 1 to 7°C. The reaction mixture was stirred at 3 ± 2°C for 10 mins. The stirred reaction mixture was then heated to 47°C over 30 mins and stirred at 47 to 49°C under nitrogen for 2.5 h (NOTE: gas evolution). The reaction was cooled to 20°C and stirred overnight. The phases were separated and the organic phase extracted with 25% w/w aqueous sulfuric acid (80mL). The combined aqueous layers were washed with tert-butylmethylether (160mL) and cooled to 14°C. Aqueous sodium hydroxide (10M, 120mL) was slowly added over 25 minutes at 14 to 22°C and the resulting basic solution (pH ∼ 13) extracted with tert-butylmethylether (160mL) and washed with 20% w/w aqueous sodium chloride (160mL). The basic solution was extracted with further tert-butylmethylether (80mL). The combined extracts were filtered, diluted with IMS (80mL) and concentrated *in vacuo* to 80mL. This process was repeated three more times. The resulting mixture was then heated to 55°C with stirring and water (80mL) added slowly above 45°C. The mixture was cooled to 36°C, seeded*, and then stirred at 36 ± 1°C for 20 minutes. Water (80mL) was slowly added over 7 mins at 37 to 35°C and the mixture allowed to cool to ambient temperature over 1 h. The title compound was isolated by filtration, washed with 1:2 IMS : water (40mL) and water (40mL), and then dried *in vacuo* at 40°C (18.57g, 66%).

* The seed was prepared by a similar method except without seeding.

### Intermediate 85

### 2-Ethyl-1H-pyrrolo[2,3-b]pyridine 7-oxide

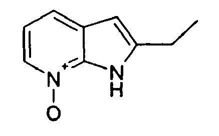

### Method A

To a stirred solution of 2-ethyl-1*H*-pyrrolo[2,3-*b*]pyridine (1.79g, 12.2mmol) in ethylacetate (40mL) at 0°C was added, dropwise via hydrophobic frit, a solution of meta-chloroperbenzoic acid (4.14g, 18.4mmol) in ethylacetate (40mL). After stirring for 2h the reaction was washed with saturated aqueous sodium metabisulphite (50mL) and concentrated *in vacuo* to a yellow solid. Treatment with saturated aqueous potassium carbonate (30 mL) gave a brown solution. This was extracted with dichloromethane (2x50mL), the combined extracts were dried (hydrophobic frit) and concentrated *in vacuo* to yield the title compound as a yellow / brown solid (1.322g, 67%).
LCMS rt = 0.71 mins, MH+ = 163

### Method B

To a stirred solution of 2-ethyl-1*H*-pyrrolo[2,3-*b*]pyridine (14.0g, 96.0mmol) in ethyl acetate (70mL) at -1°C was added a solution of meta-chloroperbenzoic acid (41.5g, 168.0mmol) in ethyl acetate (84mL) over 41mins with a maximum temperature of 9°C (most of addition ≤ 5°C). The reaction was stirred at ca 0°C for 2 hours 35 mins. The reaction was treated with 2M aq HCl (35mL) and warmed to 20°C. After stirring for 5 mins, the phases were separated and the organic phase extracted with further 2M aq HCl (3 x 14mL). The acidic layers were combined and cooled to 14°C. 10M aq NaOH (20mL) was added in 4 portions (temperature had risen to 17°C) and the reaction recooled to 4°C and stirred at 2 to 4°C for 20 minutes. The resulting suspension was filtered and the cake washed with cold water (2 x 14mL) and the isolated product dried *in vacuo* at 40°C overnight (6.77g, 43%).

### Intermediate 86

### 4-Chloro-2-ethyl-1H-pyrrolo[2,3-b]pyridine

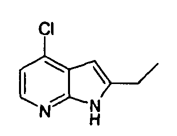

To a stirred suspension of 2-ethyl-1*H*-pyrrolo[2,3-*b*]pyridine 7-oxide (1.319g, 8.13mmol) in anhydrous dimethylformamide (10mL) under an atmosphere of nitrogen at 50°C was added dropwise over 5 mins methanesulfonylchloride (3.15mL, 40.7mmol) and the reaction heated to 70°C for 2h. The reaction was poured onto water (50mL) and basified to pH≥10 with aqueous sodium hydroxide. The resultant solid was collected by filtration and dried overnight *in vacuo* to reveal the title compound as a brown solid (1.1g, 74.5%).
LCMS rt = 1.10mins, MH+= 181

### Intermediate 87

### 2-Cyclobutyl-1H-pyrrolo[2,3-b]pyridine

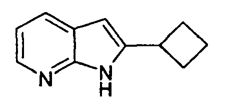

To a stirred suspension of 1,1-dimethylethyl (3-methyl-2-pyridinyl)carbamate (12.495g, 60mmol) in tetrahydrofuran (125mL) at 0°C under an atmosphere of nitrogen was added n-butyl-lithium (1.5M, 90mL) over 90 mins. After 45 mins at 0°C *N*-methyl-*N-*(methyloxy)cyclobutanecarboxamide (10.3g, 72mmol) was added. After a further 45 mins at 0°C the reaction was allowed to warm to ambient temperature and was added to 5M aqueous hydrochloric acid (110mL) and heated to 60°C for 1h. The organic was separated. The aqueous was basified with 10M aqueous sodium hydroxide and extracted with ethyl acetate. The combined extracts were dried (hydrophobic frit) and concentrated *in vacuo* to give an orange oil. Recrystalisation from methanol/water furnished the title compound as a yellow solid (8.06g, 78%).
LCMS rt = 0.97mins, MH+ = 173

### Intermediate 88

### 2-Cyclobutyl-1H-pyrrolo[2,3-b]pyridine7-oxide

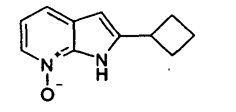

To a stirred solution of 2-cyclobutyl-1*H*-pyrrolo[2,3-*b*]pyridine (4.0g, 23.2mmol) in dichloromethane (60mL) at 0°C was added a solution of meta-chloroperbenzoic acid (7.84g, 34.8mmol) in dichloromethane (100mL). After 2.5 h meta-chloroperbenzoic acid (3.5g, 15.5mmol) was added. After 1 h the reaction was filtered and the filtrate washed with saturated aqueous sodium metabisulphite (300mL), saturated aqueous potassium carbonate (4x250mL), dried (hydrophobic frit) and concentrated *in vacuo* to give an orange foam. Purification by chromatography (Silica 100g, 0 to 25% methanol in dichloromethane over 60 mins) gave the title compound as a yellow solid (0.99g, 23%).
LCMS rt = 0.83mins, MH+ = 189

### Intermediate 89

### 4-Chloro-2-cyclobutyl-1H-pyrrolo[2,3-b]pyridine

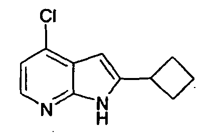

To a stirred suspension of 2-cyclobutyl-1*H*-pyrrolo[2,3-*b*]pyridine 7-oxide (2.26g, 12mmol) in dimethylformamide (15mL) under an atmosphere of nitrogen at 50°C was added methanesulfonylchloride (4.64mL, 60mmol) and the reaction heated to 70°C for 2 h. The reaction was poured onto water (70mL) and basified to pH>10 with aqueous sodium hydroxide the resultant solid was collected by filtration and triturated with methanol/water, filtered and dried *in vacuo* to furnish the title compound as a beige solid (1.32g, 53%).
LCMS rt = 1.23mins MH+ = 207

### Intermediate 90

### 2-(1-Methylethyl)-1H-pyrrolo[2,3-b]pyridine

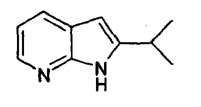

To a stirred suspension of 1,1-dimethylethyl (3-methyl-2-pyridinyl)carbamate (30g, 144mmol) in tetrahydrofuran (300mL) at 0°C under an atmosphere of nitrogen was added n-butyl-lithium (2.3M, 123mL) over 90 mins. After 30 mins at 0°C N,2-dimethyl-N-(methyloxy)propanamide (22.7g, 173mmol) was added. After a further 60 mins at 0°C the reaction was allowed to warm to ambient temperature and was added to 5M aqueous hydrochloric acid (300mL) and heated to 60°C for 1.5 h. The organic was separated. The aqueous was basified with 10M aqueous sodium hydroxide and extracted with ethyl acetate. The combined extracts were dried (MgSO₄) and concentrated *in vacuo* to give the title compound as an orange crystalline solid (22.04g, 95%).
LCMS rt = 2.38mins, MH+= 161

### Intermediate 91

### 2-(1-Methylethyl)-1H-pyrrolo[2,3-b]pyridine 7-oxide

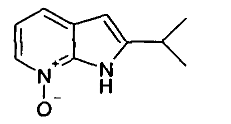

To a stirred solution of 2-(1-methylethyl)-1*H*-pyrrolo[2,3-*b*]pyridine (28.59g, 0.178mol) in dichloromethane (500mL) at 0°C was added a solution of meta-chloroperbenzoic acid (46.25g, 0.268mol) in dichloromethane (500mL). After 1h 45mins metachloroperbenzoic acid (5g, 0.03mol) was added. The reaction was warmed to ambient temperature over a period of 16 h. Metachloroberbenzoic acid (10g, 0.06mol) was added and the reaction stirred at 0°C for 1 h. The reaction was washed with saturated aqueous sodium metabisulphite (600mL), saturated aqueous potassium carbonate (1 L), dried (hydrophobic frit) and concentrated *in vacuo* to a dark red oil. Trituration with diethyl ether and filtration gave title compound (5.8g, 18.5%). Concentration of the filtrate *in vacuo* follow by chromatography (silica 200g, 0 to 50% methanol in dichloromethane) gave an orange solid. Trituration with diethyl ether furnished a second batch of the title compound (3.02g, 10%).
LCMS rt = 2.29mins, MH+ = 177

### Intermediate 92

### 4-Bromo-2-(1-methylethyl)-1H-pyrrolo[2,3-b]pyridine

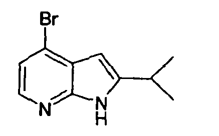

To a stirred solution of 2-(1-methylethyl)-1*H*-pyrrolo[2,3-*b*]pyridine 7-oxide (0.75g, 4.26mmol) in anhydrous dimethylformamide (7.5mL) was added tetramethylammoniumbromide [TMAB] (0.94g, 6.13mmol) the resultant suspension was cooled to 0°C. The reaction was treated with methane-sulfonic-anhydride (1.48g, 8.52mmol) and the reaction was allowed to warm to ambient temperature over 16 h. The reaction was treated further with TMAB (0.33g, 2.13mmol) and methane-sulfonic-anhydride (0.37g, 2.13mmol). After 1 h the reaction was poured onto water (10mL) and basified with 10M aqueous sodium hydroxide. The resultant yellow solid was collected by filtration. Purification by chromatography (silica 50g, 0-25% ethylacetate in cyclohexane) gave the title compound as a white solid (0.498g, 48%).
LCMS rt = 3.34mins, MH+ = 239

### Intermediate 93

### 1,1-Dimethylethyl 4-{[(4-bromophenyl)sulfonyl]amino}-1-piperidinecarboxylate

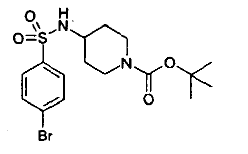

To a stirred solution of 1,1-dimethylethyl 4-amino-1-piperidinecarboxylate (6.0g, 30mmol) in chloroform (100mL) was added 4-bromobenzenesulfonyl chloride (7.0g, 29mmol) portion wise over 5 mins. The reaction was stirred at ambient temperature for 1h. The reaction was washed sequentially with water, saturation aqueous sodium bicarbonate, saturated aqueous citric acid, dried (hydrophobic frit) and concentrated *in vacuo* to a yellow oil. Azeotroping with Ether revealed the title compound as a white solid (10.87g, 87%).
LCMS rt = 1.25mins, MH- = 419

### Intermediate 94

### 1,1-Dimethylethyl 4-({[4-(4,4,5,5-tetramethyl-1,3,2-dioxaborolan-2-yl)phenyl]-sulfonyl}amino)-1-piperidinecarboxylate

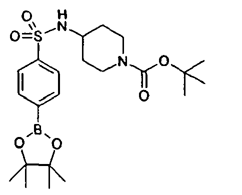

To a stirred suspension of 1,1-dimethylethyl 4-{[(4-bromophenyl)sulfonyl]amino}-1-piperidinecarboxylate (2.0g, 4.77mmol) and potassium acetate (1.4g, 14.31mmol) in dimethylformamide (60mL) at 60°C was added bis(pinnacolato)diboron (2.91g, 11.45mmol) as a solution in dimethylformamide (20mL), heating at 60°C continued for 1.5 h. Filtration and concentration *in vacuo* yielded a grey solid. The solid was taken up in water and extracted with ethyl acetate. The combined extracts were dried (hydrophobic frit) and concentrated *in vacuo* to a yellow oil. Purification by chromatography (100g silica, 0-100% ethylacetate in cyclohexane 40mins) gave the title compound as a colourless crystalline solid (1.69g, 76%).
LCMS rt=1.37mins, MH- = 465

### Intermediate 95

### 4-Bromo-2-(1,1-dimethylethyl)-1H-pyrrolo[2,3-b]pyridine

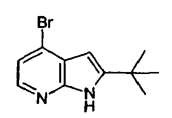

2-(1,1-Dimethylethyl)-1*H*-pyrrolo[2,3-*b*]pyridine 7-oxide (11g, 58mmol) and tetramethylammonium bromide (13.49g, 87mmol) were placed in DMF (78ml). The mixture was cooled to 0°C and treated with a portionwise additions of methane sulfonic anhydride (20.2g, 116mmol). The reaction was stirred at 5°C for 1 hour and then allowed to warm to room temperature. The mixture was stirred for a further four hours and the solid collected. (3.4g).
LCMS rt = 1.27 min, MH+ 255

### Intermediate 96

### 4-Chloro-2-(1-methylethyl)-1H-pyrrolo[2,3-b]pyridine

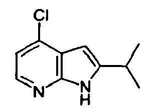

2-(1-Methylethyl)-1*H*-pyrrolo[2,3-*b*]pyridine 7-oxide (1.23g, 7mmol) was suspended in DMF(8ml) and heated to 50°C. Methanesulfonyl chloride (2.6ml) was added dropwise to the mixture over 15 minutes. The reaction was heated at 50°C for an hour then treated with a further addition of methanesulfonyl chloride (1 ml). The reaction was heated at 70°C for a further hour. The reaction was poured into water (50ml) and neutralised with 2M-sodium hydroxide. The mixture was extracted with DCM (3x30ml). The organic phases were evaporated and the residue was purified on silica SPE using 10% ethyl acetateDCM. The main fraction was evaporated to give an orange foam (0.981 g).
LCMS rt = 1.19min, MH+ 195

### Intermediate 97

### 4-Bromo-2-(difluoromethyl)-1H-pyrrolo[2,3-b]pyridine

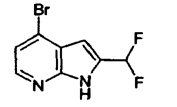

To a solution of 4-bromo-2-(difluoromethyl)-1-(phenylsulfonyl)-1*H*-pyrrolo[2,3-*b*]pyridine (1.6g, 4.13 mmol) in THF at ambient temperature was added TBAF (6ml, 6.0 mmol, 1 M in THF) dropwise. Stirred at ambient temperature for 2h and then allowed to stand over night. The crude reaction mixture was added to a preconditioned (with methanol 100ml) 70g SCX column. Eluted column with methanol (200 ml) followed by 2M methanolic ammonia (200ml). The appropriate fractions were evaporated in two batches to give the title compound (360 mg) and (250mg) as cream solids.
LCMS rt = 1.05 min, MH+ 247

Intermediate 98 was similarly prepared :

### Intermediate 98

### 4-Bromo-2-methyl-1H-pyrrolo[2,3-b]pyridine

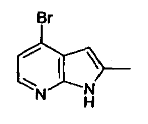

LCMS rt = 2.97 min, MH+ = 211, 213

### Intermediate 99

### 4-Bromo-2-(difluoromethyl)-1-(phenylsulfonyl)-1H-pyrrolo[2,3-b]pyridine

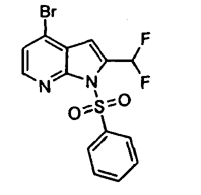

To a solution of 4-bromo-1-(phenylsulfonyl)-1*H*-pyrrolo[2,3-*b*]pyridine-2-carbaldehyde (0.420g, 1.15mM) in DCM (10ml) at room temperature was added Deoxyfluor^{™} (0.64ml, 3.48mM). The reaction was stirred for 2 hrs and left standing overnight. Reaction mixture was poured carefully into aqeous sodium bicarbonate solution. The organic layer was separated and washed with brine (20ml) and aq. saturated ammonium chloride solution, dried with a hydrophobic frit and solvent was removed in vacuo leaving a yellow solid. Solid was dissolved in DCM (20ml), washed with HCl (1 M, 20ml x 2) and water (20ml x 2), filtered through a phase separator and evaporated to dryness to furnish 4-bromo-2-(difluoromethyl)-1-(phenylsulfonyl)-1*H*-pyrrolo[2,3-*b*]pyridine as a yellow solid (0.380g).
LCMS rt=3.55min, MH+ = 387, 389

### Intermediate 100

### 4-Bromo-N-(2-hydroxy-1,1-dimethylethyl)benzenesulfonamide

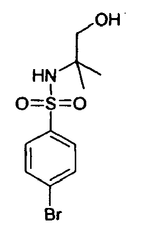

To a solution of 4-bromobenzenesulfonyl chloride (2.5g, 0.01mole) in anhydrous dichloromethane (20ml) was added triethylamine (2.02g, 2.8ml, 0.022mole), and 2-amino-2-methyl-1-propanol (980mg, 1.1ml, 0.01mole) and the resulting mixture stirred at 20 °C for 3hr. The reaction mixture was diluted with dichloromethane (200ml), washed with saturated sodium bicarbonate (50ml), 2N hydrochloric acid (50ml), water (50ml), and dried (phase separator), and evaporated to dryness. The residual solid was triturated under ether, with stirring (2hr), filtered, washed well with ether and air dried to yield the title compound as a colourless solid (1.84g, 60%).
LCMS rt = 0.97 minutes, MH+ = 308

### Intermediate 101

### 4-Bromo-N-(2-hydroxy-2-methylpropyl)benzenesulfonamide

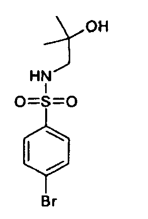

To a solution of 4-bromobenzenesulfonyl chloride (2.5g, 0.01mole) in anhydrous dichloromethane (20ml) was added triethylamine (2.02g, 2.8ml, 0.022mole) and 1-amino-2-methyl-2-propanol (980mg, 1.1ml, 0.011mole), and the resulting mixture stirred at 20°C for 3hr. The reaction mixture was diluted with dichloromethane (200ml), washed with saturated sodium bicarbonate (50ml), water (50ml), 2N hydrochloric acid (50ml), and water (50ml) and dried (phase separator), and evaporated to dryness. The residual solid was triturated under ether, with stirring (2hr), filtered, washed well with ether and air dried to yield the title compound as a colourless solid (2.34g, 76%).
LCMS rt = 0.95 minutes, MH+ = 309

### Intermediate 102

### 4-Bromo-N-[(2S)-2-hydroxypropyl]benzenesulfonamide

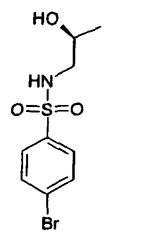

To a stirred, cooled (0°C), solution of 4-bromobenzenesulfonyl chloride (2,5g, 0.01mole) in anhydrous dichloromethane (20ml) was added triethylamine (2.02g, 2.8ml, 0.02mole), and (2*R*)-1-amino-2-propanol (1.13g, 1.16ml, 0.015mole) and the resulting solution stirred at 0 to 20°C for 18hr. The reaction mixture was diluted with dichloromethane (200ml), washed with saturated sodium bicarbonate (50ml), 2N hydrochloric acid (50ml), water (50ml), and dried (phase separator), and evaporated to dryness. The residual solid was triturated under ether, with stirring (2hr), filtered, washed well with ether and air dried to yield the title compound as a colourless solid (2.04g, 66%).
LCMS rt = 0.90 minutes, MH+ = 296

### Intermediate 103

### 4-Bromo-N-[(2R)-2-hydroxypropyl]benzenesulfonamide

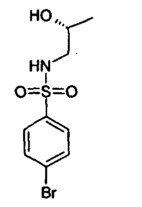

To a stirred, cooled (0°C), solution of 4-bromobenzenesulfonyl chloride (2,5g, 0.01mole) in dichloromethane (25ml) was added triethylamine (2.02g, 2.8ml, 0.02mole), and (2*S*)-1-amino-2-propanol (1.13g, 1.16ml, 0.015mole) and the resulting solution stirred at 0 to 20°C for 18hr. The reaction mixture was diluted with dichloromethane (150ml), washed with saturated sodium bicarbonate (50ml), 2N hydrochloric acid (50ml), water (50ml), and dried (phase separator), and evaporated to dryness. The residual solid was triturated under ether/petroleum ether 2:1, filtered, washed well with ether and air dried to yield the title compound as a colourless solid (2.46g, 84%).
LCMS rt = 0.90 minutes, MH+ = 296

### Intermediate 104

### 4-Bromo-N-[(1S)-2-hydroxy-1-methylethyl]benzenesulfonamide

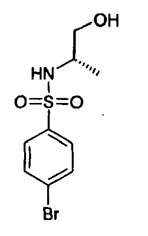

To a stirred, cooled (0°C), solution of 4-bromobenzenesulfonyl chloride (2,5g, 0.01mole) in dichloromethane (30ml) was added triethylamine (2.02g, 2.8ml, 0.02mole), and (2R)-2-amino-1-propanol (1.13g, 0.015mole) and the resulting mixture stirred at 5 to 20°C over 18hr. The reaction mixture was diluted with dichloromethane (150ml), washed with saturated sodium bicarbonate (50ml), 2N hydrochloric acid (50ml), water (50ml), and dried (phase separator), and evaporated to dryness. The residual solid was triturated under ether, filtered, and air dried to yield the title compound as a colourless solid (2.16g, 73%).
LCMS rt = 0.90 minutes, MH+ = 295

### Intermediate 105

### 4-Bromo-N-[(1R)-2-hydroxy-1-methylethyl]benzenesulfonamide

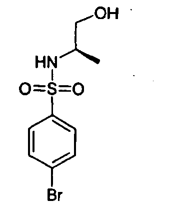

To a stirred, cooled (0°C), solution of 4-bromobenzenesulfonyl chloride (2.5g, 0.01 mole) in dichloromethane (30ml) was added triethylamine (2.02g, 2.8ml, 0.02mole), and (2S)-2-amino-1-propanol (1.13g, 0.015mole) and the resulting mixture stirred at 0 to 20°C over 18hr. The reaction mixture was diluted with dichloromethane (100ml), washed with saturated sodium bicarbonate (50ml), 2N hydrochloric acid (50ml), water (50ml), and dried (phase separator), and evaporated to dryness. The residual solid was triturated under ether, filtered, and air dried to yield the title compound as a colourless solid (2.27g, 77%).
LCMS rt = 0.90 minutes, MH+ = 296

### Intermediate 106

### 4-Bromo-N-[(1S,2R)-2-hydroxy-1-methylpropyl]benzenesulfonamide

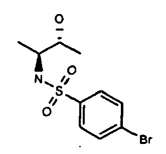

A mixture of (2*R*,3*S*)-3-amino-2-butanol (105.8 mg, 1.187 mmol), 4-bromobenzenesulfonyl chloride (303 mg, 1.187 mmol), triethylamine (0.331 mL, 2.374 mmol) in anhydrous dichloromethane (5 mL) was stirred at room temperature under nitrogen overnight, diluted with DCM (10ml), washed with water (10ml) and evaporated in *vacuo* to give a yellow oil (289mg) which was dissolved in DCM (20ml), washed with 2M HCl (15ml). The DCM extract was evaporated in vacuo to give the title compound as a yellow oil (182mg).
LCMS rt = 2.66 mins, MH+= 310

### Intermediate 107

### 4-Bromo-N-[(1S,2S)-2-hydroxycyclopentyl]benzenesulfonamide

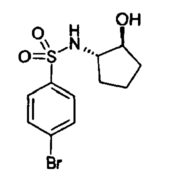

To a suspension of (1*S*,2*S*)-2-aminocyclopentanol hydrochloride (78 mg, 0.567 mmol) in dichloromethane (DCM) (4 mL) stirred under nitrogen at room temperature was added solid 4-bromobenzenesulfonyl chloride (145 mg, 0.567 mmol) in one charge. The reaction mixture was stirred at 20°C for 72 hr. The reaction was diluted with dichloromethane (6 mL) washed with saturated sodium bicarbonate solution (10 mL), water (10 mL) and 10% citric acid solution (10 mL), dried using a hydrophobic frit and evaporated *in vacuo* to give the title compound as an oil (100 mg).
LCMS: rt = 2.67 mins, MH+ = 320, 322

### Intermediate 108

### 4-Bromo-N-(2-hydroxy-1,2-dimethylpropyl)benzenesulfonamide

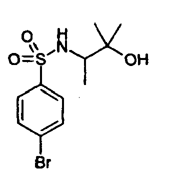

To a solution of 3-amino-2-methyl-2-butanol (296mg, 2.87 mmol) and triethylamine (0.420 mL, 3.01 mmol) in dichloromethane (DCM) (17.5 mL) stirred under nitrogen at 20°C was added solid 4-bromobenzenesulfonyl chloride (700 mg, 2.74 mmol) in one charge. The reaction mixture was stirred at 20 °C for 2 hr. The reaction was washed with saturated sodium bicarbonate solution 25 ml, water 25 ml and 10% citric acid solution 25 ml, dried using a hydrophobic frit and evaporated *in vacuo* to give the title compound as a white solid (649 mg).
LCMS: rt = 2.71 mins, MH+ = 322, 324

### Intermediate 109

### 4-Bromo-N-(2-hydroxy-1,1-dimethylpropyl)benzenesulfonamide

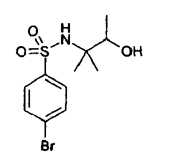

To a solution of 3-amino-3-methyl-2-butanol hydrochloride (513 mg, 3.67 mmol) and triethylamine (1.08 mL, 7.75 mmol) in dichloromethane (DCM) (22.5 mL) stirred under nitrogen at 20°C was added solid 4-bromobenzenesulfonyl chloride (900 mg, 3.52 mmol) in in one charge. The reaction mixture was stirred at 20 °C for 16 h. The reaction was washed with saturated sodium bicarbonate solution (30 ml), water (30 ml) and 10% citric acid solution (30 ml), dried using a hydrophobic frit and evaporated *in vacuo* to give the title compound as a white solid (680 mg).
LCMS: rt = 2.82 mins, MH+ = 322, 325

### Intermediate 110

### [(4-Bromo-1H-pyrrolo[2,3-b]pyridin-2-yl)methyl]dimethylamine

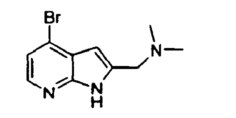

4-Bromo-1-(phenylsulfonyl)-1*H*-pyrrolo[2,3-*b*]pyridine-2-carbaldehyde (400mg) and 2M dimethylamine (0.8ml) were dissolved in THF (15ml) and stirred at room temperature under nitrogen for approximately 3hrs. Sodium triacetoxyborohydride (700mg) and acetic acid (0.007ml) was added and the reaction stirred at room temperature for 2hrs then overnight. The reaction mixture was treated with sodium bicarbonate (saturated, 30ml), extracted with DCM (2x30ml), dried using a phase separator and concentrated *in vacuo* to afford a cream solid. The solid was purified by flashmaster (silica, 50g, 0-50% ethylacetate: cyclohexane over 40 mins). The relevant fractions were collected and concentrated *in vacuo* to afford a white solid (220mg). The white solid was dissolved in dioxane (22ml) and 2M NaOH (5.5ml) was added and the reaction mixture stirred at 70°C overnight. The reaction mixture was concentrated *in vacuo.* The reaction mixture was concentrated *in vacuo* to afford a yellow solid. The solid was then dissolved in water (30ml) and extracted with DCM (2x30ml) at pH7, dried using a phase separator and concentrated *in vacuo* to afford the title compound as a yellow solid (73mg).
LCMS rt = 2.06 mins, MH+ = 254/256

### Intermediate 111

### 1,1-Dimethylethyl 4-[(4-bromo-1H-pyrrolo[2,3-b]pyridin-2-yl)methyl]-1-piperazinecarboxylate

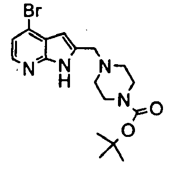

4-Bromo-1-(phenylsulfonyl)-1*H*-pyrrolo[2,3-*b*]pyridine-2-carbaldehyde (500mg) and 1,1-dimethylethyl 1-piperazinecarboxylate (385mg) were dissolved in THF (15ml). Acetic acid (1 equivalent) was added and the reaction stirred at 0°C for 5 mins. Sodium triacetoxyborohydride (870mg) was added and the reaction stirred under nitrogen for approximately 3hrs. The reaction mixture was treated with sodium bicarbonate (saturated, 30ml) and extracted with DCM (2x30ml), dried using a phase separator and concentrated *in vacuo* to afford a yellow oil. The oil was purified by flashmaster (silica, 50g, 0-50% ethylacetate : cyclohexane over 40mins). The relevant fractions were collected and concentrated *in vacuo* to afford a white solid (678mg). The white solid was dissolved in dioxane (68ml) and 2M NaOH (6.85ml) was added and reaction mixture stirred at 65°C overnight. The reaction mixture was concentrated *in vacuo* to afford the title compound as yellow solid (457mg).
LCMS rt = 2.81 mins, MH+ = 395/397

### Intermediate 112

### 1-Methylcyclopropanecarbonyl chloride

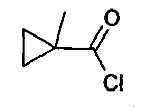

To a stirred, cooled (0 °C), solution of 1-methylcyclopropanecarboxylic acid (10.1g, 0.1mole) in anhydrous dichloromethane (50ml) was added dropwise, under an atmosphere of nitrogen, oxalyl chloride (10ml, 14g, 0.11mole) in anhydrous dichloromethane (10ml) over 30 minutes keeping the temperature below 0 °C (ice/salt bath). After the addition was complete the reaction mixture was stirred at 0 °C for 2hr and then allowed to warm to 20 °C and left overnight at 20 °C.
Used crude in next reaction.

### Intermediate 113

### N,1-Dimethyl-N-(methyloxy)cyclopropanecarboxamide

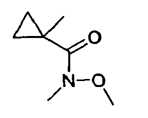

To a cooled (0 °C), stirred solution of N,O-dimethylhydroxylamine HCl (9.78g; 0.1mole) in anhydrous dichloromethane (100ml) was added triethylamine (30ml) and to this stirred mixture was added, under an atmosphere of nitrogen, a solution of 1-methylcyclopropanecarbonyl chloride (reaction mixture from intermediate 112) drop wise keeping the temperature below) 0 °C by means of an ice/water bath. The resulting suspension was stirred at 0 °C for 3hr and then allowed to warm to 20 °C and left at 20 °C overnight. The reaction mixture was poured into dichloromethane (200ml) and water (100ml) and stirred at 20 °C for 30 minutes. The organic layer was separated and washed with saturated sodium bicarbonate (100ml), water (100ml), and dried (phase separator) and evaporated to dryness. The residual brown liquid was purified by chromatography (50g bond elut cartridge), eluting with cyclohexane: ethyl acetate (gradient 20:1, 10:1, 5:1). The appropriate fractions were combined and evaporated to dryness to give the title compound as a straw coloured liquid (10.7g; used crude).

### Intermediate 114

### 2-(1-Methylcyclopropyl)-1H-pyrrolo [2, 3-b] pyridine

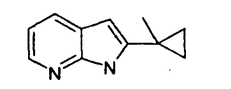

To a stirred, cooled (-5 °C, ice/salt bath) mixture of 1,1-dimethylethyl (3-methyl-2-pyridinyl)carbamate (10.4g; 0.05mole) in anhydrous tetrahydrofuran (70ml) was added, under an atmosphere of nitrogen, n-butyllithium (2.0M solution in cyclohexane, 50ml, 0.1 mole) dropwise over 45 minutes keeping the temperature between -5 and 0 °C. After the addition was complete red suspension was stirred at below 0 °C for 1 hr. and then a solution of *N*,1-dimethyl-*N*-(methyloxy)cyclopropanecarboxamide (7.87g; 0.055mole) in anhydrous tetrahydrofuran (30ml) in a single portion. The temperature rose to 18 °C after the addition and then the suspension stirred at 0 °C for 2hr. and then allowed to warm to 10 °C. The mixture was then poured into 5N hydrochloric acid (100ml) and the resulting mixture heated to 65 °C for 2hr. Left overnight at 20 °C. The aqueous layer was separated and 10M sodium hydroxide added until the solution was between pH10-12, with ice/water cooling. The suspension was filtered, the solid washed well with water and air dried to furnish the title compound as a yellow solid (6.46g : 80%).
LCMS rt = 0.94 minutes, MH+ = 173

### Intermediate 115

### 2-(1-Methylcyclopropyl)-1H-pyrrolo[2,3-b]pyridine 7-oxide

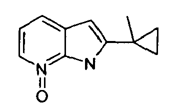

To a stirred, cooled (15 °C) solution of 2-(1-methylcyclopropyl)-1*H*-pyrrolo [2, 3-*b*] pyridine (2.9g; 0.017mole) in ethyleneglycol dimethyl ether (50ml) was added, under an atmosphere of nitrogen, a solution of m-chloroperoxybenzoic acid (5.9g, 1.22equiv. of 60%) in ethyleneglycol dimethyl ether (10ml) keeping the temperature between 15 and 25 °C. After the addition was complete the mixture was stirred at 20 °C for 30 minutes, and then sodium metabisuphite 5% solution (50ml) added along with dichloromethane (100ml). The organic was separated and concentrated to ∼50ml. Dichloromethane (100ml) added and the solution dried (phase separator) and evaporated to dryness to leave the title compound as an orange solid (8.7g, 100%+). Used crude in the next reaction.
LCMS rt = 2.38 minutes, MH+ 189

### Intermediate 116

### 4-Chloro-2-(1-methylcyclopropyl)-1H-pyrrolo[2,3-b]pyridine

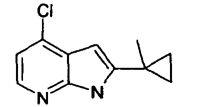

To a solution of 2-(1-methylcyclopropyl)-1*H*-pyrrolo[2,3-*b*]pyridine 7-oxide (8g crude,-3.2g, 0.017mole) in N, N,dimethylformamide (20ml) was added methanesulphonyl chloride (5ml, excess) at 50 °C and after the addition was complete the mixture was stirred at 75 °C for 3hr. The cooled reaction mixture was poured into water (250ml) the mixture cooled (ice/water) and basified with the addition of 10M sodium hydroxide until ph8 to 9 was obtained. The grey suspension was extracted with dichloromethane (3x100ml), the combined organic extract was washed with water (3x75ml) and dried (hydrophobic frit) and evaporated to dryness to leave a black/red oil. Purification by chromatography (70g, bond elut) eluting with cyclohexane : ethyl acetate = 20:1, 10:1, and 5:1. The appropriate fractions were combined and evaporated to dryness to give the title compound as an orange solid. (690mg). Crude product ∼70% pure. Used without further purification.
LCMS rt = 1.21 minutes, MH+ 207

### Intermediate 117

### 4-Bromo-N-[(3S)-1,1-dioxidotetrahydro-3-thienyl]benzenesulfonamide

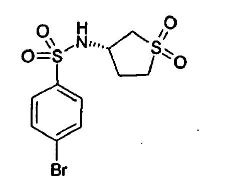

To a suspension of [(3*S*)-1,1-dioxidotetrahydro-3-thienyl]amine (1.0 g, 7.4 mmol) (which may be prepared, for example, by the method described in WO 2006/094063), and 4-bromobenzenesulfonyl chloride (1.8 g, 7.04 mmol) in dichloromethane (25 mL) was added triethylamine (1.08 mL, 7.74 mmol) [Any exotherm controlled with a water bath]. After 1.5 h the reaction was treated with water (50 mL) and diluted further with dichloromethane (100 mL). The organic layer was washed with aqueous sodium carbonate (50 mL) and saturated aqueous citric acid (50 mL) before drying and concentration *in vacuo* to give the require product as an orange solid (1.62 g, 65%).
LCMS rt = 2.59 mins MH⁻ = 352, 354

Intermediate 118 was similarly prepared.

### Intermediate 118

### 4-Bromo-N-[(3R)-1,1-dioxidotetrahydro-3-thienyl]benzenesulfonamide

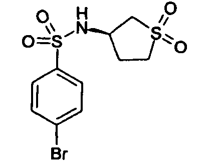

LCMS rt = 2.58 mins, MH⁻ = 352, 354

### Intermediate 119

### N-[(3R)-1,1-Dioxidotetrahydro-3thienyl]-4-(4,4,5,5-tetramethyl-1,3,2-dioxaborolan-2-yl)benzenesulfonamide

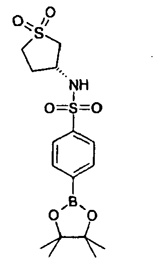

4-Bromo-*N*-[(3*R*)-1,1-dioxidotetrahydro-3-thienyl]benzenesulfonamide (610 mg, 1.72 mmol), bispinacolatodiboron (1.47 g, 5.78 mmol), potassium acetate (680 mg, 6.93 mmol) and palladium (II) acetate (30 mg, 0.12 mmol) were dissolved in anhydrous DMF and degassed for 10 mins. The reaction mixture was stirred at 65 °C under nitrogen atmosphere for approximately 2 hours. The reaction mixture was decanted and concentrated *in vacuo* to afford a brown solid. The solid was diluted with water (50 ml) and extracted with DCM (2 x 50 ml), dried using a phase separator and concentrated *in vacuo* to afford a yellow solid. The solid was triturated with ether and filtered affording the title compound as a cream solid (170 mg, 25%).
LCMS rt = 2.97 mins, MH⁻ = 400

### Intermediate 120

### N-[(3S)-1,1-Dioxidotetrahydro-3-thienyl]-4-(4,4,5,5-tetramethyl-1,3,2-dioxaborolan-2-yl)benzenesulfonamide

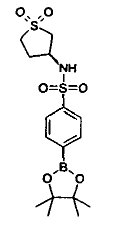

4-Bromo-*N*-[(3*S*)-1,1-dioxidotetrahydro-3-thienyl]benzenesulfonamide (1 g, 2.82 mmol), bispinacolatodiboron (1.8 g, 7.05 mmol), potassium acetate (830 mg, 8.46 mmol) and palladium (II) acetate (31mg, 014 mmol) were dissolved in anhydrous DMF and degassed for 10 mins. The reaction mixture was stirred at 65 °C under nitrogen atmosphere for approximately 2 hours, then overnight. The reaction mixture was cooled, decanted and concentrated *in vacuo* to afford a dark brown solid. The solid was treated with water (50 ml) then extracted with DCM (2 x 50 ml), dried using a phase separator and concentrated *in vacuo* to afford a yellow/brown solid. The solid was triturated with ether and filtered to afford the title compound as a cream solid (420 mg, 35%).
LCMS rt = 2.98 mins, MH⁻ = 400

### Intermediate 121

### (2-Methyl-1H-pyrrolo[2,3-b]pyridin-4-yl)boronic acid

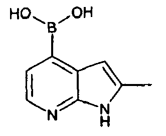

4-Bromo-2-methyl-1*H*-pyrrolo[2,3-*b*]pyridine (1.6 g, 7.58 mmol) in THF (15 vols, 24 ml) was added to a stirring suspension of sodium hydride (380 mg, 9.48 mmol) in THF (5 vols, 8ml) at 0 °C under nitrogen atmosphere. The reaction mixture was degassed upon full addition then cooled to -78 °C. n-BuLi (2.5M in hexanes) (6 ml, 14.96 mmol) was added dropwise and the reaction allowed to proceed at -78 °C for approximately 30 mins. Triisopropylborate (5.7 ml, 22.74 mmol) was added dropwise and the reaction mixture stirred at -78 °C for 1 hour. The reaction mixture was allowed to warm to 0 °C and was quenched with water (50 ml). The layers were separated and the aqueous layer extracted with ethyl acetate (2 x 40 ml). The combined organics were washed with 2m sodium hydroxide (50 ml) then the combined aqueous layers treated with 2M hydrochloric acid to pH 7 at 0 °C. The aqueous layer was then extracted with ethyl acetate (2 x 50 ml) and the organic layers combined and concentrated *in vacuo* to afford the title compound as a white/cream solid (550 mg, 41%).
LCMS rt = 1.73 mins, MH+ = 177

### Intermediate 122

### 1,1-Dimethylethyl [2-({[4-(2-formyl-1H-pyrrolo[2,3-b]pyridin-4-yl)phenyl]sulfonyl}-amino)ethyl]carbamate

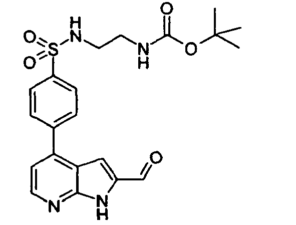

1,1-Dimethylethyl [2-({[4-(4,4,5,5-tetramethyl-1,3,2-dioxaborolan-2-yl)phenyl]sulfonyl}-amino)ethyl]carbamate (1.4 g, 3.29 mmol), 4-bromo-1-(phenylsulfonyl)-1*H*-pyrrolo[2,3-*b*]pyridine-2-carbaldehyde) (1 g, 2.74 mmol), chloro(di-2-norbornylphosphino)(2'-dimethylamino-1,1'-biphenyl-2-yl) palladium(II) (150 mg, 0.27 mmol) and potassium phosphate (585 mg, 2.74 mmol) were dissolved in dioxane:water (5:1) (20 ml) and degassed. The reaction mixture was then refluxed at 105 °C under nitrogen atmosphere for 4 hours. chloro(di-2-norbornylphosphino)(2'-dimethylamino-1,1'-biphenyl-2-yl) palladium(II) (75 mg, 0.14 mmol) was added and the reaction mixture refluxed at 105 °C under nitrogen atmosphere overnight. 10 M sodium hydroxide (1 ml) was added and the reaction mixture refluxed at 105 °C for 1 hour. The mixture was treated with 1 M sodium bicarbonate (50 ml) and extracted with DCM (3 x 50 ml), dried using a phase separator and concentrated *in vacuo* to afford a brown oil. The oil was dissolved in DCM and pre-absorbed onto Florisil before purification by flash column chromatography (silica, 50g, DCM:ethyl acetate 0-100%). The relevant fractions were combined and concentrated *in vacuo* to afford the title compound as a brown solid (560 mg, 39%).
LCMS rt = 2.96 mins, MH+ = 445

### Intermediate 123

### 4-Bromo-N-cyclohexylbenzenesulfonamide

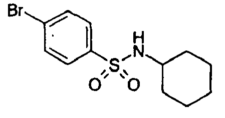

4-Bromobenzenesulfonyl chloride (5 g, 19.6 mmol), cyclohexylamine (3.4 ml, 29.4 mmol) and triethylamine (3 ml, 21.6 mmol) were dissolved in chloroform (60 ml) and stirred at room temperature under nitrogen atmosphere for 1 hour. The reaction mixture was washed with water (50 ml) followed by sodium bicarbonate solution (50 ml) and citric acid (50 ml) before being dried using a phase separator and concentrating *in vacuo* to afford the title compound as a peach oil (5.77 g, 92%).
LCMS rt = 3.48 mins, MH+ = 318, 320

### Intermediate 124

### N-Cyclohexyl-4-(4,4,5,5-tetramethyl-1,3,2-dioxaborolan-2-yl)benzenesulfonamide

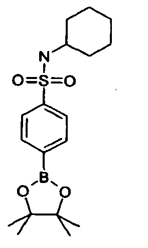

4-Bromo-*N*-cyclohexylbenzenesulfonamide (2 g, 6.28 mmol), bispinacolatodiboron (3.99 g, 15.7 mmol), potassium acetate (1.85 g, 18.84 mmol) and palladium (II) acetate (70 mg, 0.31 mmol) were dissolved in DMF (50 ml) and degassed. The reaction mixture was heated at 650C under nitrogen atmosphere for 3 hours. The reaction mixture was decanted and concentrated *in vacuo* to afford a grey solid. The solid was dissolved in water (50 ml), extracted with DCM (2 x 50 ml), dried using a phase separator and concentrated *in vacuo* to afford a white solid. The solid was dissolved in ethyl acetate and washed with water (2 x 40 ml) and concentrated *in vacuo* to afford a cream solid. The solid was triturated with cyclohexane and filtered to afford the title compound as a white solid (1.16 g, 51%).
LCMS rt = 3.64 mins, MH+ = 366

### Intermediate 125

### (2-Cyclopropyl-1H-pyrrolo[2,3-b]pyridin-4-yl)boronic acid

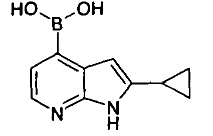

To a solution of 4-bromo-2-cyclopropyl-1H-pyrrolo[2,3-b]pyridine (3.623 g, 15.28 mmol), in anhydrous tetrahydrofuran (THF) (90 ml) stirred under nitrogen at 10°C was added solid sodium hydride 60% wt on mineral oil (0.764 g, 19.10 mmol) in one charge. The reaction mixture was allowed to warm to 20 °C and stirred for 45 mins. The reaction was cooled to -78 °C and n-butyl-lithium (14 mL, 35.0 mmol) was added dropwise during 15 min. The reaction was stirred at -78 °C for 45 mins. Triisopropylborate (10.6 mL, 45.7 mmol) was added dropwise during 15 min. The reaction mixture was allowed to warm to 20 °C over 1.5 h. The reaction mixture was quenched with water, (70 mL) and the reaction mixture was diluted with ethylacetate (70 mL) The organic was separated and the aqueous layer was adjusted to pH7 with 2N aqueous hydrochloric acid (ca. 15 mL). The suspension was extracted with ethyl acetate (2 x 200 mL). The combined organics were dried using a hydrophobic frit and concentrated *in vacuo* to give the title compound as an orange solid (1.33 g, 43.1%).
LCMS: rt = 1.85 mins, MH+ = 203

### Intermediate 126

### 4-Bromo-2-cyclopropyl-1H-pyrrolo[2,3-b]pyridine

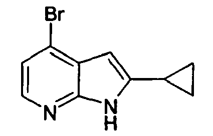

To a solution of 2-cyclopropyl-1H-pyrrolo[2,3-b]pyridine 7-oxide (4.10 g, 23.54 mmol), and methane sulfonic anhydride (8.20 g, 47.1 mmol) in N,N-dimethylformamide (DMF) (105 ml) stirred under nitrogen at 20°C was added tetrametylammonium bromide (5.44 g, 35.3 mmol) in one charge. The reaction mixture was stirred at 20 °C for 18 h. The reaction was poured onto water (400 mL) and stirred for 1 h. The aqueous was adjusted to pH>11 with 10N aqueous sodium hydroxide (ca. 10 mL). The suspension was stirred for 1 h. The precipitate was collected by filtration. The solid was dried in air to give the title compound as an orange solid (3.628 g).
LCMS: rt = 3.24 mins, [MH+] = 237, 239.

### Intermediate 127

### N-Methyl-N-(methyloxy)propanamide

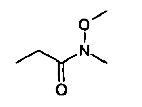

To a stirred solution of *N*,*O*-dimethylhydroxylamine hydrochoride (20.14g, 0.206mol) in water (40mL) at 0°C was added an aqueous solution of potassium carbonate (42.9g, 0.310mol in 70mL water) over 9 minutes at 0 to 5°C. Dichloromethane (60mL) was added and the mixture allowed to re-cool to 0°C. Propionyl chloride (18.8mL, 0.216mol) in dichloromethane (10mL) was then added dropwise over 37 mins at 0 to 7°C. The reaction was stirred at 1 ± 1 °C for 35 minutes then allowed to warm to 20°C over 15 minutes and stirred at 20°C under nitrogen overnight (16 hours). The phases were allowed to separate. Additional water (10mL) was added and the mixture stirred for 30 mins. The phases were then separated and the aqueous layer extracted with dichloromethane (2 x 40mL). The organic layers were combined and dried over magnesium sulfate (10g). The mixture was filtered and evaporated. Toluene (40mL) was added and the mixture evaporated to remove about half of the toluene. Further toluene was added and the mixture was filtered. The mixture was then concentrated *in vacuo* to yield the title compound (75%).

### Example 1

### 2-[4-(4-{[(2-Aminoethyl)amino]sulfonyl}phenyl)-1H-pyrrolo[2,3-b]pyridin-2-yl]acetamide bis(trifluoroacetate)

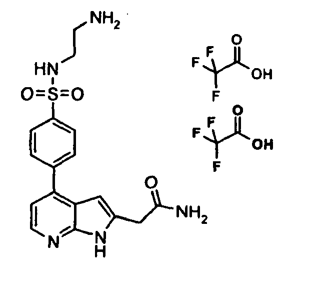

1,1-Dimethylethyl{2-[({4-[2-(2-amino-2-oxoethyl)-1*H*-pyrrolo[2,3-*b*]pyridin-4-yl]phenyl}sulfonyl)amino]ethyl}carbamate was dissolved in DCM : TFA (1:1, 4ml) and stirred at room temperature for 30 mins. The reaction was evaporated to give the title compound as a pale yellow gum (0.064g).
MH+374, rt= 1.00 mins

### Example 2

### 2-[4-(4-{[(3-Aminopropyl)amino]sulfonyl}phenyl)-1H-pyrrolo[2,3-b]pyridin-2-yl]acetamide bis(trifluoroacetate)

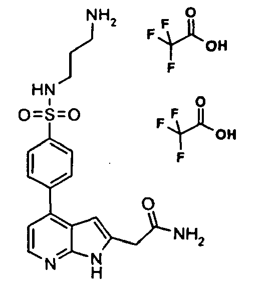

1,1-Dimethylethyl{3-[({4-[2-(2-amino-2-oxoethyl)-1*H*-pyrrolo[2,3-*b*]pyridin-4-yl]phenyl}sulfonyl)amino]propyl}carbamate was dissolved in DCM : TFA (1:1, 4ml) and stirred at room temperature for 30 mins. The reaction was evaporated to give the title compound as a pale yellow gum (0.050g).
MH+388, rt= 1.91 mins

### Example 3

### 1,1-Dimethylethyl {3-[({4-[2-(2-amino-2-oxoethyl)-1H-pyrrolo[2,3-b]pyridin-4-yl]phenyl}sulfonyl)amino]propyl}carbamate

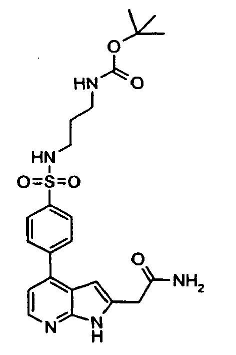

1,1-Dimethylethyl (3-aminopropyl)carbamate (0.4mmol) and pentafluorophenyl 4-(4,4,5,5-tetramethyl-1,3,2-dioxaborolan-2-yl)benzenesulfonate (0.4mmol) were treated with TEA (0.200ml) and heated in the Biotage Initiator mw at 120°C for 10 mins. The mixture was treated with bis(diphenylphosphino)ferrocene palladium (II) chloride (0.008g), sodium carbonate (0.034g) and 2-(4-bromo-1*H*-pyrrolo[2,3-*b*]pyridin-2-yl)acetamide (0.050g) in dioxan/water (5:1, 1ml). The mixture was heated in the Biotage Initiator mw at 150 °C for 30 mins. The reaction was filtered, washing with methanol and then purified by MDAP.
MH+488, rt= 3.45 mins

### Example 4

### 1,1-Dimethylethyl {2-[({4-[2-(2-amino-2-oxoethyl)-1H-pyrrolo[2,3-b]pyridin-4-yl]phenyl}sulfonyl)amino]ethyl}carbamate

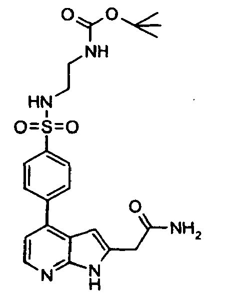

1,1-Dimethylethyl (2-aminoethyl)carbamate (0.4mmol) and pentafluorophenyl 4-(4,4,5,5-tetramethyl-1,3,2-dioxaborolan-2-yl)benzenesulfonate (0.4mmol) were treated with TEA (0.200ml) and heated in the Biotage Initiator mw at 120°C for 10 mins. The mixture was treated with bis(diphenylphosphino)ferrocene palladium (II) chloride (0.008g), sodium carbonate (0.034g) and 2-(4-bromo-1*H*-pyrrolo[2,3-*b*]pyridin-2-yl)acetamide (0.050g) in dioxan/water (5:1, 1ml). The mixture was heated in the Biotage Initiator mw at 150 °C for 30 mins. The reaction was filtered, washing with methanol and then purified by MDAP.
MH+474, rt= 3.27 mins

### Example 5

### Formic acid - N-(2-aminoethyl)-4-[2-(hydroxymethyl)-1H-pyrrolo[2,3-b]pyridin-4-yl]benzenesulfonamide (1:1)

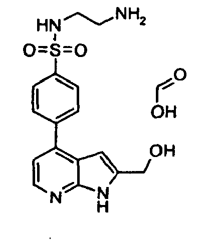

1,1-Dimethylethyl(2-{[(4-{2-(hydroxymethyl)-1-[(4-methylphenyl)sulfonyl]-1*H*-pyrrolo[2,3-*b*]pyridin-4-yl}phenyl)sulfonyl]amino}ethyl)carbamate (0.210g, 0.35mmol) in chloroform (2ml) was treated with p-toluenesulphonic acid (0.120g, 0.63mmol) and heated in a sealed mw vessel at 120°C for 10 mins (150W). The suspension was evaporated and the gummy residue was dissolved in 5% potassium hydroxide in methanol (4ml) and heated in the mw at 120°C for 5 mins (Max power 50W). The resulting suspension was evaporated, treated with water (5ml), neutralized with 2N HCl and extracted with EtOAc (3x5ml). Only 0.025g extracted into the organic phase so the aq. was evaporated and the residue purified by MDAP to give the title compound as a white solid (0.0125g, 9%).
MH+347, rt= 1.91 mins

### Example 6

### N-(2-Aminoethyl)-4-[2-(3-hydroxypropyl)-1H-pyrrolo[2,3-b]pyridin-4-yl]benzenesulfonamide

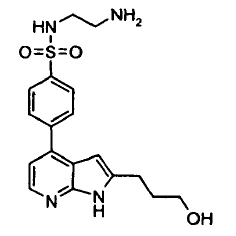

A solution of formic acid - *N*-(2-aminoethyl)-4-[2-(3-hydroxy-1-propyn-1-yl)-1*H*-pyrrolo[2,3-*b*]pyridin-4-yl]benzenesulfonamide (1:1) (0.012g, 0.032mmol) in methanol (1ml) was hydrogenated over 5% palladium on carbon for 4 hrs. The suspension was filtered through an aminopropyl cartridge (500mg) and evaporated to give the title compound as a white solid (0.0021 g, 17%).
MH+375, rt= 2.04 mins
An impure sample (0.0045g) was recovered by washing cartridge with MeOH.NH₃ and dimethylformamide.

### Example 7

### 1,1-Dimethylethyl [4-(4-{[(1,1-dioxidotetrahydro-3-thienyl)amino]sulfonyl}phenyl)-1H-pyrrolo[2,3-b]pyridin-2-yl]acetate

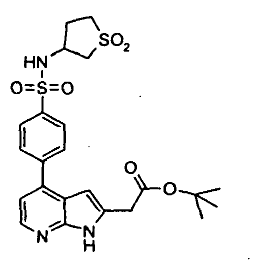

Tetrahydro-3-thiophenamine 1,1-dioxide (0.8mmol) and pentafluorophenyl 4-(4,4,5,5-tetramethyl-1,3,2-dioxaborolan-2-yl)benzenesulfonate (0.360g, 0.8mmol) were treated with TEA (0.400ml) and heated in the Biotage Initiator mw at 120°C for 20 mins. The mixture was treated with bis(diphenylphosphino)ferrocene palladium (II) chloride (0.020g), sodium carbonate (0.068g) and 1,1-dimethylethyl (4-bromo-1*H*-pyrrolo[2,3-*b*]pyridin-2-yl)acetate (0.250g,0.8mmol) in dioxan/water (5:1, 2ml). The mixture was heated in the Biotage Initiator mw at 150 °C for 30 mins. The reaction was filtered, evaporated and purified by MDAP (using a water / acetonitrile gradient containing 0.1% formic acid) to give the title compound (0.183g).
MH+506, rt= 3.00 mins

### Example 8

### 1,1-Dimethylethyl [4-(4-{[(1,1-dioxidotetrahydro-2H-thiopyran-4-yl)amino]sulfonyl}phenyl)-1H-pyrrolo[2,3-b]pyridin-2-yl]acetate

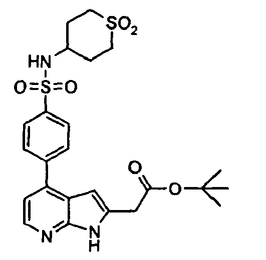

(1,1-Dioxidotetrahydro-2H-thiopyran-4-yl)amine hydrochloride (0.8mmol) and pentafluorophenyl4-(4,4,5,5-tetramethyl-1,3,2-dioxaborolan-2-yl)benzenesulfonate (0.360g,0.8mmol) were treated with TEA (0.400ml) and heated in the Biotage Initiator mw at 120°C for 20 mins. The mixture was treated with bis(diphenylphosphino)ferrocene palladium (II) chloride (0.020g), sodium carbonate (0.068g) and 1,1-dimethylethyl (4-bromo-1*H*-pyrrolo[2,3-*b*]pyridin-2-yl)acetate (0.250g,0.8mmol) in dioxan/water (5:1, 2ml). The mixture was heated in the Biotage Initiator mw at 150 °C for 30 mins. The reaction was filtered, evaporated and purified by MDAP (using a water / acetonitrile gradient containing 0.1% formic acid) to give the title compound (0.111g).
MH+520, rt= 2.90 mins

### Example 9

### 2-(Phenylmethyl)-4-[4-(1-pyrrolidinylsulfonyl)phenyl]-1H-pyrrolo[2,3-b]pyridine

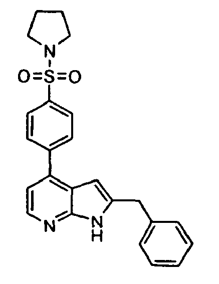

A solution of 1-(phenylsulfonyl)-4-[4-(1-pyrrolidinylsulfonyl)phenyl]-1*H*-pyrrolo[2,3-*b*]pyridine (0.100g, 0.214mmol) in THF (6ml) was stirred at -78°C under nitrogen and was treated with 2M LDA in heptane/THF/ethylbenzene (0.118ml, 0.235mmol) dropwise. The reaction was stirred for 1 hour at -78°C. The reaction was warmed to 0°C using an ice-bath and this temperature was maintained for 5 mins. The reaction mixture was returned to -78°C and benzyl bromide (0.032ml, 0.267mmol) in THF (0.5ml) was added dropwise. The reaction was maintained at -78°C for 30 mins then allowed to warm to room temperature overnight (18 hrs). The reaction was quenched with aq. ammonium chloride (20ml) and extracted with EtOAc (3x15ml). The organic layers were combined and reduced under *vacuum* to afford crude 2-(phenylmethyl)-1-(phenylsulfonyl)-4-[4-(1-pyrrolidinylsulfonyl)phenyl]-1*H*-pyrrolo[2,3-*b*]pyridine. A solution of 1-(phenylsulfonyl)-4-[4-(1-pyrrolidinylsulfonyl)phenyl]-1*H*-pyrrolo[2,3-*b*]pyridine (0.100g, 0.214mmol) in THF (6ml) was stirred at -78°C under nitrogen and was treated with 2M LDA in heptane/THF/ethylbenzene (0.214ml, 0.428mmol) dropwise. The reaction was stirred for 2 hrs at -78°C. Benzyl bromide (0.056ml, 0.47mmol) in THF (0.5ml) was added dropwise. The reaction was maintained at -78°C for 1 hour then allowed to warm to room temperature overnight (18 hrs). The reaction was quenched with aq. ammonium chloride (20ml) and extracted with EtOAc (3x15ml). The organic layers were combined and reduced under *vacuum* to afford crude 2-(phenylmethyl)-1-(phenylsulfonyl)-4-[4-(1-pyrrolidinylsulfonyl)phenyl]-1*H*-pyrrolo[2,3-*b*]pyridine. The reaction above was repeated on a further batch of 1-(phenylsulfonyl)-4-[4-(1-pyrrolidinylsulfonyl)phenyl]-1*H*-pyrrolo[2,3-*b*]pyridine (0.100g) to afford a third batch of crude 2-(phenylmethyl)-1-(phenylsulfonyl)-4-[4-(1-pyrrolidinylsulfonyl)phenyl]-1*H*-pyrrolo[2,3-*b*]pyridine. The three batches of crude 2-(phenylmethyl)-1-(pheny/sulfonyl)-4-[4-(1-pyrrolidinylsu/fonyl)phenyl]-1*H*-pyrrolo[2,3-*b*]pyridine were combined and purified using 20g silica FlashMaster II eluting with EtOAc to cyclohexane, 0 to 100%, over 20 mins affording still impure 2-(phenylmethyl)-1-(phenylsulfonyl)-4-[4-(1-pyrrolidinylsulfonyl)phenyl]-1*H*-pyrrolo[2,3-*b*]pyridine. This was dissolved in dioxan (1ml) and water (0.2ml) then treated with sodium hydroxide (30mg). The reaction was heated in the Biotage Initiator mw for 30 mins at 140 °C. The reaction was treated with more sodium hydroxide (30mg) and heated for a further 1 hour at 150 °C. The reaction was reduced under *vacuum,* acidified to pH8 with HCl, diluted with water (20ml) and extracted with DCM (3x15ml). The organic layers were combined and reduced under *vacuum* to afford the crude product (0.040g) which was purified by MDAP to afford the title compound (0.0108g).
MH+418, rt= 3.46 mins

### Example 10

### Example 10a

### 2-Methyl-4-[4-(1-pyrrolidinylsulfonyl)phenyl]-1H-pyrrolo[2,3-b]pyridine

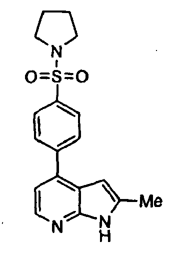

A solution of 1-(phenylsulfonyl)4-[4-(1-pyrrolidinylsulfonyl)phenyl]-1*H*-pyrrolo[2,3-*b*]pyridine (0.100g, 0.214mmol) in THF (3ml) was stirred at -30°C under nitrogen and was treated with 2M LDA in heptane/THF/ethylbenzene (0.214ml, 0.428mmol) dropwise. The reaction was stirred for 40 mins at -30°C and methyl iodide (0.080ml, 1.28mmol) was added. The reaction was allowed to warm to room temperature. The reaction was stirred at room temperature for 2 hrs. The reaction was quenched with aq. ammonium chloride (20ml) and extracted with EtOAc (3x15ml). The organic layers were combined then reduced under *vacuum* to afford crude material which was purified using 20g silica FlashMaster II eluting with 0 to 100%, EtOAc to cyclohexane over 20 mins to afford 2-methyl-1-(phenylsulfonyl)4-[4-(1-pyrrolidinylsulfonyl)phenyl]-1*H*-pyrrolo[2,3-*b*]pyridine (0.087g, 70% desired product). This was dissolved in dioxan (1.5ml) and water (0.3ml) then treated with sodium hydroxide (50mg). The reaction was heated in the Biotage Initiator mw at 150 °C for 40 mins. The reaction was reduced under *vacuum,* diluted with water (20ml), acidified to pH9 with HCl and extracted with DCM (20mlx2). The organic layers were combined and reduced under *vacuum* to give the crude material (0.065g) which was purified by MDAP to afford the title compound (0.0089g).
MH+342, rt= 3.05 mins

### Example 10b

### 2-Methyl-4-[4-(1-pyrrolidinylsulfonyl)phenyl]-1H-pyrrolo[2,3-b]pyridine trifluoroacetate

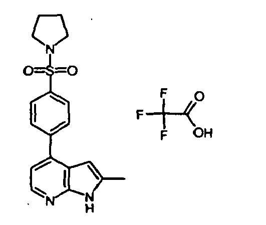

Example 10b was prepared similarly to Example 62.
LCMS rt = 3.07 min, m/z = 342

### Example 11

### {4-[4-(1-Pyrrolidinylsulfonyl)phenyl]-1H-pyrrolo[2,3-b]pyridin-2-yl}methanol

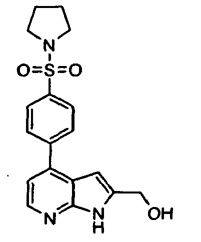

To a solution of 1-(phenylsulfonyl)-4-[4-(1-pyrrolidinylsulfonyl)phenyl]-1*H*-pyrrolo[2,3-*b*]pyridine (0.467g, 1.0mmol) in dry THF (10ml) at -40°C under nitrogen and was added 2M LDA in heptane/THF/ethylbenzene (1.0ml, 2.0mmol) dropwise. The reaction was stirred for 30 mins at -40°C and cooled to -60°C. Paraformaldehyde(0.045g, 1.12mmol) was added in one portion. The reaction was allowed to warm to ambient temperature overnight. Saturated ammonium chloride was added and extracted with DCM (2x20ml). The organic layers were combined, washed with aq. ammonium chloride solution and reduced under *vacuum* to afford an orange solid (0.450g). This was purified by silica SPE cartridge (10g) eluting with DCM to 10% EtOAc in DCM. {1-(Phenylsulfonyl)-4-[4-(1-pyrrolidinylsulfonyl)phenyl]-1*H*-pyrrolo[2,3-*b*]pyridin-2-yl}methanol was obtained as a yellow oil (0.040g). This material in dioxan (2ml) and methanol (2ml) was treated with sodium hydroxide (50% w/w, 0.5ml, 6.2mmol) and left to stand at ambient temperature overnight. The reaction was neutralized with 2M HCl and almost evaporated to dryness. The residue was dissolved in DCM (20ml) and washed with saturated sodium bicarbonate solution, brine and evaporated to give a yellow solid (0.140g). A portion of this material (0.040g) was purified by MDAP to afford the title compound as a cream solid (0.03g, 10%).
MH+356, rt= 2.71 mins

### Example 12

### 1,1-Dimethylethyl (4-{4-[(methylsulfonyl)amino]phenyl}-1H-pyrrolo[2,3-b]pyridin-2-yl)acetate

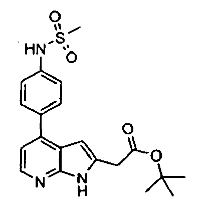

1,1-Dimethylethyl (4-bromo-1*H*-pyrrolo[2,3-*b*]pyridin-2-yl)acetate (0.311g, 1mmol), {4-[(methylsulfonyl)amino]phenyl}boronic acid (0.320g, 1.5mmol), sodium carbonate (0.090g) and bis(diphenylphosphino)ferrocene palladium (II) chloride (0.026g) were mixed in dioxan/water (5:1, 3ml). The mixture was heated in the Biotage Initiator mw at 180°C for 3 mins. The mixture was separated between DCM (50ml) and water (10ml). The organic phase was evaporated and purified by silica SPE cartridge eluting with EtOAc/DCM (0-50%) over an hour. The main fraction was evaporated to give the title compound as a mustard coloured crystalline solid (0.301 g).
MH+402, rt= 3.0 mins

### Example 13

### 1,1-Dimethylethyl {4-[4-(aminosulfonyl)phenyl]-1H-pyrrolo[2,3-b]pyridin-2-yl}acetate trifluoroacetate

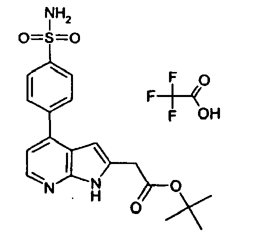

A solution of 1,1-dimethylethyl (4-bromo-1*H*-pyrrolo[2,3-*b*]pyridin-2-yl)acetate (31mg, 0.1mmol) in dioxan:water, 5:1 (1mL) was added to a mw vessel containing potassium phosphate (64mg, 0.3mmol) and 2'-(dimethylamino)-2-biphenylyl-palladium (II) chloride dinorbornyl-phosphine complex (0.3mg, 0.5 mol%) and [4-(aminosulfonyl)phenyl]boronic acid (20mg, 0.1mmol). The reaction mixture was heated at 130°C in the mw for 30 mins. The reaction mixture was applied directly to a C18 cartridge (500mg) and eluted with 0.1 % TFA in acetonitrile (3 x 1mL). Concentration by blow down and purification by mass directed HPLC gave the title compound as a yellow gum.
LCMS RT= 3.08min ES+ve 401 m/z (MH)⁺

Example 14 was similarly prepared:

| **Example** | Compound | LCMS rt, | m/z |
|---|---|---|---|
| | | min | MH+ |
| **14** | 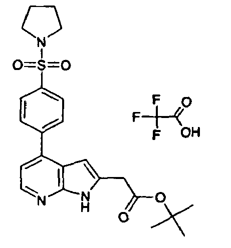 | 3.42 | 442 |
| | 1,1-dimethylethyl {4-[4-(1-pyrrolidinylsulfonyl)phenyl]-1H-pyrrolo[2,3-b]pyridin-2-yl}acetate trifluoroacetate | | |

### Example 15

### 2-(Difluoromethyl)-4-[4-(1-pyrrolidinylsulfonyl)phenyl]-1H-pyrrolo[2,3-b]pyridine

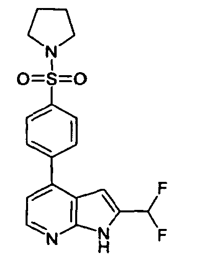

To a solution of 1-(phenylsulfonyl)-4-[4-(1-pyrrolidinylsulfonyl)phenyl]-1*H*-pyrrolo[2,3-*b*]pyridine-2-carbaldehyde (0.100g, 0.20mmol) in dry DCM (2ml) at room temperature under nitrogen was added DeoxyFluor^{™} (0.075ml, 0.4mmol) dropwise. The reaction was stirred at room temperature for 6 hrs. The reaction was poured carefully into aq. saturated sodium carbonate and extracted with DCM (2x20ml). The combined organic extracts were dried (MgSO₄) and evaporated to give crude 2-(difluoromethyl)-1-(phenylsulfonyl)-4-[4-(1-pyrrolidinylsulfonyl)phenyl]-1*H*-pyrrolo[2,3-*b*]pyridine (0.120g) as an orange gum. To a solution of the above material (0.120g, 0.1mmol) in THF (3ml) was added a solution of TBAF in THF (1M, 0.5ml, 0.55mmol). The reaction was stirred for 3 hrs at room temperature. Water was added and the mixture was extracted with DCM (2x20ml). The combined organic extracts were dried with brine and evaporated to give a brown solid (0.100g) which was purified by MDAP. The appropriate fractions were evaporated to give the title compound as a cream fluffy solid (0.008g).
MH+378, rt= 3.14 mins

### Example 16

### Example 16a

### N-Methyl-2-(4-{4-[(methylsulfonyl)amino]phenyl}-1H-pyrrolo[2,3-b]pyridin-2-yl)acetamide

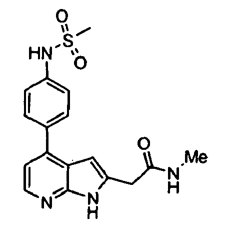

2-(4-Bromo-1*H*-pyrrolo[2,3-*b*]pyridin-2-yl)-*N*-methylacetamide (0.14mmol), {4-[(methylsulfonyl)amino]phenyl}boronic acid (0.045g, 0.21 mmol), sodium carbonate (0.014g) and bis(diphenylphosphino)ferrocene palladium (II) chloride (0.004g) were treated with dioxan/water (5:1, 1ml). The mixture was heated in the Biotage Initiator mw at 180 °C for 3 mins. The reaction was diluted with DCM (15ml) and washed with water (5ml). The organic phase was evaporated and purified by MDAP. The main peak was evaporated to give the title compound as a cream solid (0.005g).
MH+359, rt= 2.26 mins

### Example 16b

### N-Methyl-2-(4-{4-[(methylsulfonyl)amino]phenyl}-1H-pyrrolo[2,3-b]pyridin-2-yl)acetamide trifluoroacetate

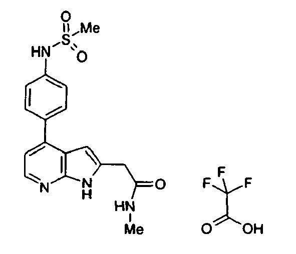

(4-{4-[(Methylsulfonyl)amino]phenyl}-1*H*-pyrrolo[2,3-*b*]pyridin-2-yl)acetic acid (0.05mmol, 17.3mg), HATU (0.05mmol 19.0mg) and DIPEA(0.15mmol, 30µl) in DMF (300µl) were combined and left for 5mins. Methylamine (0.1mmol, 3.1mg) in DMF (100µl) was then added and reaction mixtures left for 20hrs. Purification by mass directed HPLC gave the title compound.
LCMS RT= 2.39 min ES+ve 359 (MH⁺)

Examples 17 to 24 were similarly prepared:

| **Example** | Compound | LCMS rt, | m/z |
|---|---|---|---|
| | | min | MH+ |
| **17** | 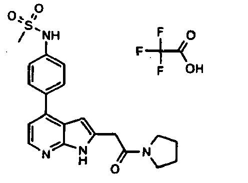 | 2.53 | 399 |
| | N-(4-{2-[2-oxo-2-(1-pyrrolidinyl)ethyl]-1H-pyrrolo[2,3-b]pyridin-4-yl}phenyl)methanesulfonamide trifluoroacetate | | |
| **18** | 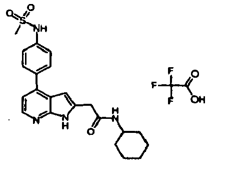 | 2.86 | 427 |
| | N-cyclohexyl-2-(4-{4-[(methylsulfonyl)amino]phenyl}-1H-pyrrolo[2,3-b]pyridin-2-yl)acetamide trifluoroacetate | | |
| **19** | 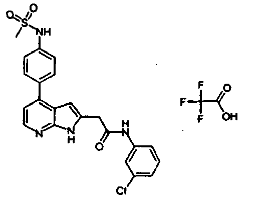 | 3.17 | 455 |
| | N-(3-chlorophenyl)-2-(4-{4-[(methylsulfonyl)amino]phenyl}-1H-pyrrolo[2,3-b]pyridin-2-yl)acetamide trifluoroacetate | | |
| **20** | 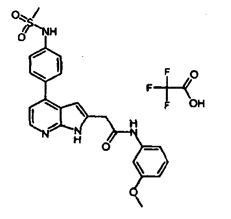 | 2.93 | 451 |
| | N-[3-(methyloxy)phenyl]-2-(4-{4-[(methylsulfonyl)amino]phenyl}-1H-pyrrolo[2,3-b]pyridin-2-yl)acetamide trifluoroacetate | | |
| **21** | 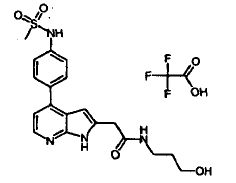 | 2.39 | 403 |
| | N-(3-hydroxypropyl)-2-(4-{4-[(methylsulfonyl)amino]phenyl}-1H-pyrrolo[2,3-b]pyridin-2-yl)acetamide trifluoroacetate (salt) | | |
| **22** | 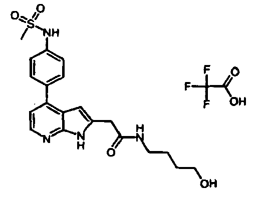 | 2.35 | 417 |
| | N-(4-hydroxybutyl)-2-(4-{4-[(methylsulfonyl)amino]phenyl}-1H-pyrrolo[2,3-b]pyridin-2-yl)acetamide trifluoroacetate (salt) | | |
| **23** | 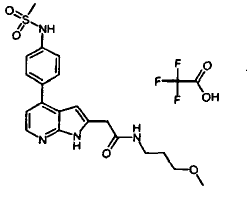 | 2.4 | 417 |
| | *N*-[3-(methyloxy)propyl]-2-(4-{4-[(methylsulfonyl)amino]phenyl}-1*H*-pyrrolo[2,3-*b*]pyridin-2-yl)acetamide trifluoroacetate | | |
| **24** | 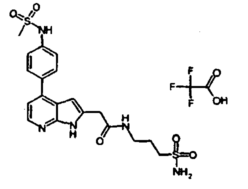 | 2.35 | 466 |
| | N-[3-(aminosulfonyl)propyl]-2-(4-{4-[(methylsulfonyl)amino]phenyl}-1H-pyrrolo[2,3-b]pyridin-2-yl)acetamide trifluoroacetate | | |

### Example 25

### Example 25a

### N,N-Dimethyl-2-(4-{4-[(methylsulfonyl)amino]phenyl}-1H-pyrrolo[2,3-b]pyridin-2-yl)acetamide

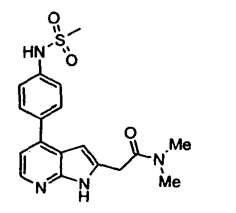

2-(4-Bromo-1*H*-pyrrolo[2,3-*b*]pyridin-2-yl)-*N*,*N*-dimethylacetamide (0.14mmol), {4-[(methylsulfonyl)amino]phenyl}boronic acid (0.045g, 0.21mmol), sodium carbonate (0.014g) and bis(diphenylphosphino)ferrocene palladium (II) chloride (0.004g) were treated with dioxan/water (5:1, 1ml). The mixture was heated in the Biotage Initiator mw at 180 °C for 3 mins. The reaction was diluted with DCM (15ml) and washed with water (5ml). The organic phase was evaporated and purified by MDAP. The main peak was evaporated to give the title compound as a brown gum (0.014g).
MH+373, rt= 2.33 mins

### Example 25b

### N,N-Dimethyl-2-(4-{4-[(methylsulfonyl)amino]phenyl}-1H-pyrrolo[2,3-b]pyridin-2-yl)acetamide trifluoroacetate

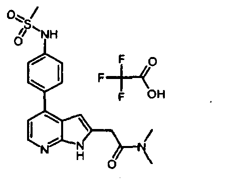

Example 25b was prepared similarly to Example 16b.
LCMS rt = 2.38 min, m/z MH⁺ = 373

### Example 26

### N-[(4-{4-[(Methylsulfonyl)amino]phenyl}-1H-pyrrolo[2,3-b]pyridin-2-yl)acetyl]glycine trifluoroacetate

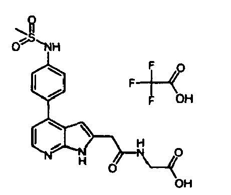

(4-{4-[(Methylsulfonyl)amino]phenyl}-1*H*-pyrrolo[2,3-*b*]pyridin-2-yl)acetic acid (0.05mmol, 17.3mg), HATU (0.05mmol 19.0mg) and DIPEA (0.15mmol, 30µl) were combined in DMF (300µl) and left to stand for 5mins. Tert-Buglycine ester (0.1mmol, 13.0mg) as a solution in DMF (100µl) was then added and the reaction mixture left overnight. Purification by mass directed HPLC gave 1,1-dimethylethyl *N*-[(4-{4-[(methylsulfonyl)amino]phenyl}-1*H-*pyrrolo[2,3-*b*]pyridin-2-yl)acetyl]glycinate. To this was added CHCl₃ (200µl) and TFA (200µl) and solution left to stand for 2hrs. Concentration by blow down gave the title compound (0.001g).
RT= 2.32min m/z = 403 ES+ (MH⁺)

Example 27 was similarly prepared from (4-{4-[(methylsulfonyl)amino]phenyl}-1*H-*pyrrolo[2,3-*b*]pyridin-2-yl)acetic acid:

| **Example** | Compound | LCMS rt, | m/z |
|---|---|---|---|
| | | min | MH+ |
| **27** | 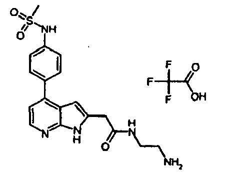 | 2.05 | 388 |
| | N-(2-aminoethyl)-2-(4-{4-[(methylsulfonyl)amino]phenyl}-1H-pyrrolo[2,3-b]pyridin-2-yl)acetamide trifluoroacetate | | |

### Example 28

### N,N-Dimethyl-N'-({4-[4-(1-pyrrolidinylsulfonyl)phenyl]-1H-pyrrolo[2,3-b]pyridin-2-yl}methyl)-1,2-ethanediamine trifluoroacetate

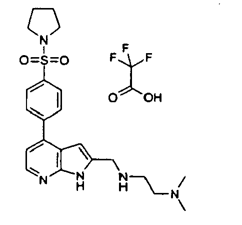

1-(Phenylsulfonyl)-4-[4-(1-pyrrolidinylsulfonyl)phenyl]-1*H*-pyrrolo[2,3-*b*]pyridine-2-carbaldehyde (0.1mmol, 42.12mg) as a solution in THF (200µl) was added to *N,N-*dimethyl-1,2-ethanediamine (0.1mmol, 9mg) in THF (300µl) the reaction mixture was left for 1 hr. Sodium triacetoxyborohydride (0.425mmol, 89.8mg) as a solution in DCM (400µl) was added and left for 60hrs. Sample concentrated by blow down. To this MeOH (1ml) and NaOH (6M, 100µl) were added and left under reflux for 2hrs. This was neutralised with HCl (5M, 200µl) followed by purification on an SCX cartridge (1g) eluting firstly with MeOH and then NH₃/MeOH solution. Concentration by blow down and further purification by mass directed HPLC gave title compound.
LCMS RT= 2.12 min, ES+ve 428 (MH⁺)

Examples 29 to 37 were similarly prepared from 4-bromo-1-(phenylsulfonyl)-1*H-*pyrrolo[2,3-*b*]pyridine-2-carbaldehyde:

| **Example** | Compound | LEMS rt, min | m/z MH+ |
|---|---|---|---|
| **29** | 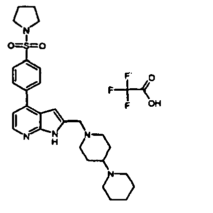 | 2.06 | 508 |
| | 1'-({4-[4-(1-pyrrolidinylsulfonyl)phenyl]-1H-pyrrolo[2,3-b]pyridin-2-yl}methyl)-1,4'-bipiperidine trifluoroacetate | | |
| **30** | 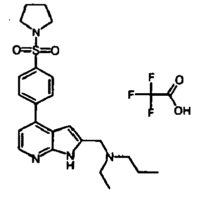 | 2.33 | 427 |
| | N-ethyl-N-({4-[4-(1-pyrrolidinylsulfonyl)phenyl]-1H-pyrrolo[2,3-b]pyridin-2-yl}methyl)-1-propanamine trifluoroacetate | | |
| **31** | 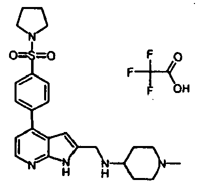 | 1.98 | 454 |
| | 1-methyl-N-({4-[4-(1-pyrrolidinylsulfonyl)phenyl]-1H-pyrrolo[2,3-b]pyridin-2-yl}methyl)-4-piperidinamine trifluoroacetate | | |
| **32** | 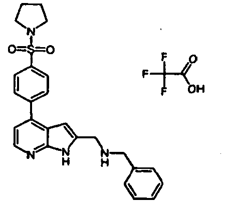 | 2.51 | 447 |
| | (phenylmethyl)({4-[4-(1-pyrrolidinylsulfonyl)phenyl]-1H-pyrrolo[2,3-b]pyridin-2-yl}methyl)amine trifluoroacetate | | |
| **33** | 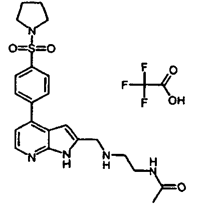 | 2.2 | 442 |
| | N-{2-[({4-[4-(1-pyrrolidinylsulfonyl)phenyl]-1H-pyrrolo[2,3-b]pyridin-2-yl}methyl)amino]ethyl}acetamide trifluoroacetate | | |
| **34** | 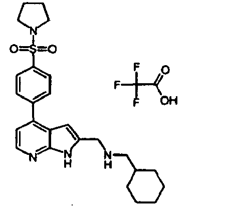 | 2.55 | 453 |
| | (cyclohexylmethyl)({4-[4-(1-pyrrolidinylsulfonyl)phenyl]-1H-pyrrolo[2,3-b]pyridin-2-yl}methyl)amine trifluoroacetate | | |
| **35** | 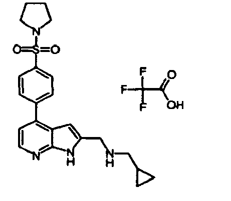 | 2.31 | 411 |
| | (cyclopropylmethyl)({4-[4-(1-pyrrolidinylsulfonyl)phenyl]-1H-pyrrolo[2,3-b]pyridin-2-yl}methyl)amine trifluoroacetate | | |
| **36** | 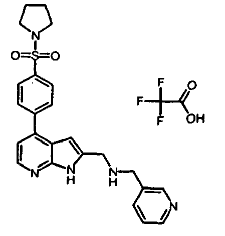 | 2.26 | 448 |
| | (3-pyridinylmethyl)({4-[4-(1-pyrrolidinylsulfonyl)phenyl]-1H-pyrrolo[2,3-b]pyridin-2-yl}methyl)amine trifluoroacetate | | |
| **37** | 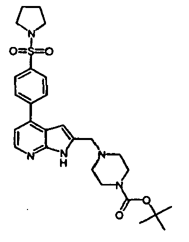 | 2.72 | 526 |
| | 1,1-dimethylethyl 4-({4-[4-(1-pyrrolidinylsulfonyl)phenyl]-1H-pyrrolo[2,3-b]pyridin-2-yl}methyl)-1-piperazinecarboxylate | | |

### Example 38

### {3-[({4-[4-(1-Pyrrolidinylsulfonyl)phenyl]-1H-pyrrolo[2,3-b]pyridin-2-yl}methyl)oxy]phenyl}acetic acid trifluoroacetate

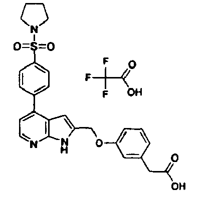

A solution of (3-hydroxyphenyl)acetic acid (30mg, 0.2mmol) in dry THF (300µL) was treated with a solution of potassium t-butoxide (1M in THF, 200µL). The reaction mixture was allowed to stand at 20°C for 5 min. prior to the addition of a solution of {1-(phenylsulfonyl)-4-[4-(1-pyrrolidinylsulfonyl)phenyl]-1*H*-pyrrolo[2,3-*b*]pyridin-2-yl}methyl methanesulfonate (38mg, 0.066mmol). The reaction mixture was stirred at 20°C for 2 h. prior to heating at 100°C for 5 min. in the microwave. The product was applied directly to a C18 cartridge (500mg) and eluted with 1% TFA in acetonitrile. Concentration by blow down and purification by mass directed HPLC gave the title compound (0.0023g).
LCMS RT= 3.27min ES+ve 491 m/z (MH)⁺

Examples 39 to 61 were similarly prepared:

| **Example** | Compound | LCMS | m/z |
|---|---|---|---|
| | | RT, min | |
| **39** | 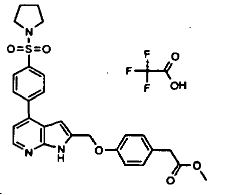 | 3.38 | 506 |
| | methyl {4-[({4-[4-(1-pyrrolidinylsulfonyl)phenyl]-1*H*-pyrrolo[2,3-*b*]pyridin-2-yl}methyl)oxy]phenyl}acetate trifluoroacetate | | |
| **40** | 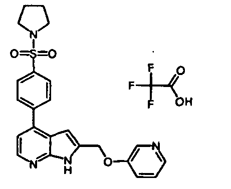 | 2.94 | 435 |
| | 2-[(3-pyridinyloxy)methyl]-4-[4-(1-pyrrolidinylsulfonyl)phenyl]-1H-pyrrolo[2,3-b]pyridine trifluoroacetate | | |
| **41** | 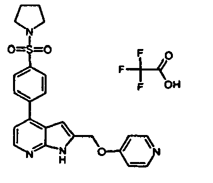 | 2.99 | 435 |
| | 2-[(4-pyridinyloxy)methyl]-4-[4-(1-pyrrolidinylsulfonyl)phenyl]-1H-pyrrolo[2,3-b]pyridine trifluoroacetate | | |
| **42** | 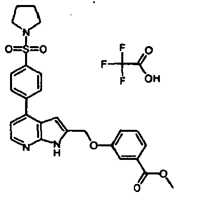 | 3.48 | 492 |
| | methyl 3-[({4-[4-(1-pyrrolidinylsulfonyl)phenyl]-1H-pyrrolo[2,3-b]pyridin-2-yl}methyl)oxy]benzoate trifluoroacetate | | |
| **43** | 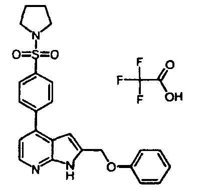 | 3.49 | 434 |
| | 2-[(phenyloxy)methyl]-4-[4-(1-pyrrolidinylsulfonyl)phenyl]-1H-pyrrolo[2,3-b]pyridine trifluoroacetate | | |
| **44** | 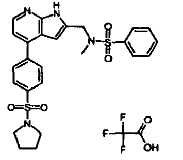 | 3.34 | 511 |
| | N-methyl-N-({4-[4-(1-pyrrolidinylsulfonyl)phenyl]-1H-pyrrolo[2,3-b]pyridin-2-yl}methyl)benzenesulfonamide trifluoroacetate | | |
| **45** | 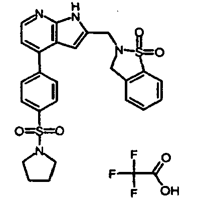 | 3.21 | 509 |
| | 2-({4-[4-(1-pyrrolidinylsulfonyl)phenyl]-1H-pyrrolo[2,3-b]pyridin-2-yl}methyl)-2,3-dihydro-1,2-benzisothiazole 1,1-dioxide trifluoroacetate | | |
| **46** | 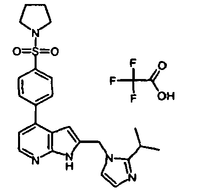 | 2.39 | 449 |
| | 2-{[2-(1-methylethyl)-1H-imidazol-1-yl]methyl}-4-[4-(1-pyrrolidinylsulfonyl)phenyl]-1H-pyrrolo[2,3-b]pyridine trifluoroacetate | | |
| **47** | 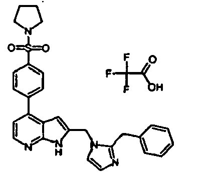 | 2.51 | 497 |
| | 2-{[2-(phenylmethyl)-1H-imidazol-1-yl]methyl}-4-[4-(1-pyrrolidinylsulfonyl)phenyl]-1H-pyrrolo[2,3-b]pyridine trifluoroacetate | | |
| **48** | 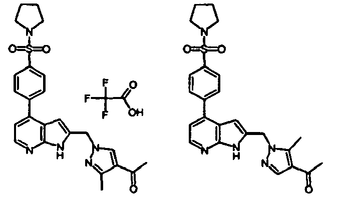 | 3.06 | 421 |
| | 1-[5-methyl-1-({4-[4-(1-pyrrolidinylsulfonyl)phenyl]-1H-pyrrolo[2,3-b]pyridin-2-yl}methyl)-1H-pyrazol-4-yl]ethanone trifluoroacetate : 1-[3-methyl-1-({4-[4-(1-pyrrolidinylsulfonyl)phenyl]-1H-pyrrolo[2,3-b]pyridin-2-yl}methyl)-1H-pyrazol-4-yl]ethanone trifluoroacetate 40 : 60 | | |
| **49** | 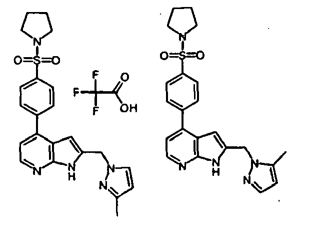 | 2.98 | 463 |
| | 2-[(3-methyl-1H-pyrazol-1-yl)methyl]-4-[4-(1-pyrrolidinylsulfonyl)phenyl]-1H-pyrrolo[2,3-b]pyridine trifluoroacetate : 2-[(5-methyl-1H-pyrazol-1-yl)methyl]-4-[4-(1-pyrrolidinylsulfonyl)phenyl]-1H-pyrrolo[2,3-b]pyridine trifluoroacetate 60 : 40 | | |
| **50** | 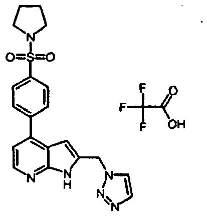 | 2.81 | 408 |
| | 4-[4-(1-pyrrolidinylsulfonyl)phenyl]-2-(1H-1,2,3-triazol-1-ylmethyl)-1H-pyrrolo[2,3-b]pyridine trifluoroacetate | | |
| **51** | 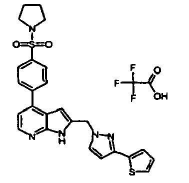 | 3.37 | 489 |
| | 4-[4-(1-pyrrolidinylsulfonyl)phenyl]-2-{[3-(2-thienyl)-1H-pyrazol-1-yl]methyl}-1H-pyrrolo[2,3-b]pyridine trifluoroacetate | | |
| **52** | 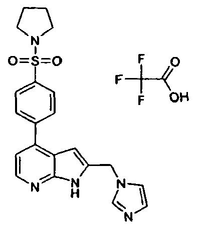 | 2.34 | 408 |
| | 2-(1H-imidazol-1-ylmethyl)-4-[4-(1-pyrrolidinylsulfonyl)phenyl]-1H-pyrrolo[2,3-b]pyridine trifluoroacetate | | |
| **53** | 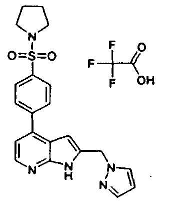 | 2.97 | 408 |
| | 2-(1H-pyrazol-1-ylmethyl)-4-[4-(1-pyrrolidinylsulfonyl)phenyl]-1H-pyrrolo[2,3-b]pyridine trifluoroacetate | | |
| **54** | 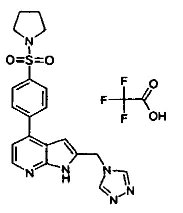 | 2.75 | 409 |
| | 4-[4-(1-pyrrolidinylsulfonyl)phenyl]-2-(4H-1,2,4-triazol-4-ylmethyl)-1H-pyrrolo[2,3-b]pyridine trifluoroacetate | | |
| **55** | 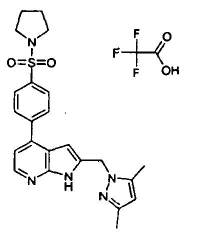 | 3.12 | 436 |
| | 2-[(3,5-dimethyl-1H-pyrazol-1-yl)methyl]-4-[4-(1-pyrrolidinylsulfonyl)phenyl]-1H-pyrrolo[2,3-b]pyridine trifluoroacetate | | |
| **56** | 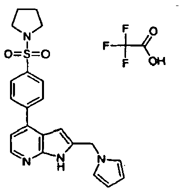 | 3.27 | 407 |
| | 4-[4-(1-pyrrolidinylsulfonyl)phenyl]-2-(1H-pyrrol-1-ylmethyl)-1H-pyrrolo[2,3-b]pyridine trifluoroacetate | | |
| **57** | 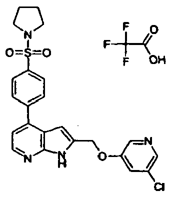 | 3.27 | 469 |
| | 2-{[(5-chloro-3-pyridinyl)oxy]methyl}-4-[4-(1-pyrrolidinylsulfonyl)phenyl]-1H-pyrrolo[2,3-b]pyridine trifluoroacetate | | |
| **58** | 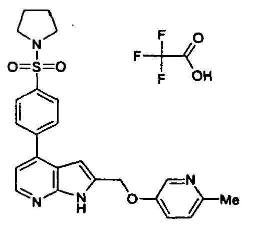 | 2.72 | 449 |
| | 2-{[(6-methyl-3-pyridinyl)oxy]methyl}-4-[4-(1-pyrrolidinylsulfonyl)phenyl]-1H-pyrrolo[2,3-b]pyridine trifluoroacetate | | |
| **59** | 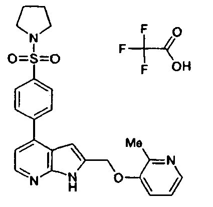 | 2.64 | 449 |
| | 2-{[(2-methyl-3-pyridinyl)oxy]methyl}-4-[4-(1-pyrrolidinylsulfonyl)phenyl]-1H-pyrrolo[2,3-b]pyridine trifluoroacetate | | |
| **60** | 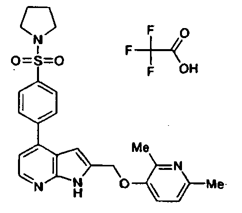 | 2.49 | 463 |
| | 2-{[(2,6-dimethyl-3-pyridinyl)oxy]methyl}-4-[4-(1-pyrrolidinylsulfonyl)phenyl]-1H-pyrrolo[2,3-b]pyridine trifluoroacetate | | |
| **61** | 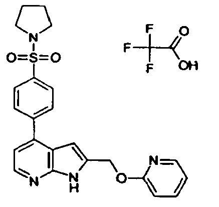 | 2.81 | 435 |
| | 2-[(2-pyridinyloxy)methyl]-4-[4-(1-pyrrolidinylsulfonyl)phenyl]-1H-pyrrolo[2,3-b]pyridine trifluoroacetate | | |

### Example 62

### N-(2-Aminoethyl)-4-(2-methyl-1H-pyrrolo[2,3-b]pyridin-4-yl)benzenesulfonamide trifluoroacetate

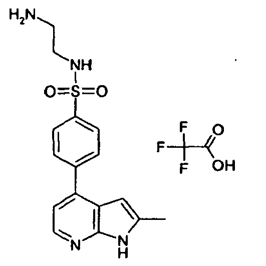

A solution of 4-bromo-2-methyl-1*H*-pyrrolo[2,3-*b*]pyridine (21 mg, 0.1mmol) in dioxan:water, 5:1 (0.5mL) was added to a mw vessel containing potassium phosphate (64mg, 0.3mmol) and 2'-(dimethylamino)-2-biphenyl-palladium (II) chloride dinorbornyl-phosphine complex (0.3mg, 0.5 mol%). A solution of 1,1-dimethylethyl [2-({[4-(4,4,5,5-tetramethyl-1,3,2-dioxaborolan-2-yl)phenyl]sulfonyl}amino)ethyl]carbamate (64mg, 0.15mmol) in dioxan:water, 5:1 (0.5mL) was added and the reaction mixture was heated at 130°C in the mw for 30 mins. The reaction mixture was treated with 40% KF supported on Alumina (220mg, 0.15mmol) and heated at 130°C in the mw for 15 mins. The reaction mixture was applied directly to a C18 cartridge (500mg) and eluted with 0.1% TFA in acetonitrile (3 x 1mL). Concentration by blow down and purification by mass directed HPLC followed by re-evaporation from CHCl₃ : TFA 1:1 (1ml) gave the title compound (0.0148g).
LCMS RT= 1.99min ES+ve 330 m/z (MH)⁺

Example 63 was similarly prepared:

| **Example** | Compound | LCMS rt, | m/z |
|---|---|---|---|
| | | min | |
| **63** | 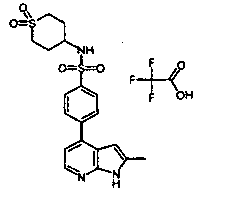 | 2.59 | 420 |
| | N-(1,1-dioxidotetrahydro-2H-thiopyran-4-yl)-4-(2-methyl-1H-pyrrolo[2,3-b]pyridin-4-yl)benzenesulfonamide trifluoroacetate | | |

### Example 64

### 4-[2-(Difluoromethyl)-1H-pyrrolo[2,3-b]pyridin-4-yl]-N-(1,1-dioxidotetrahydro-3-thienyl)benzenesulfonamide

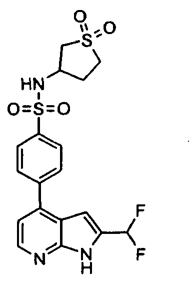

To a solution of 4-bromo-1-(phenylsulfonyl)-1*H*-pyrrolo[2,3-*b*]pyridine-2-carbaldehyde (0.420g, 1.15mmol) in dry DCM (10ml) at room temperature under nitrogen was added DeoxyFluor^{™} (0.64ml, 3.48mmol) dropwise. The reaction was stirred at room temperature for 2 hrs and left standing overnight. The reaction was poured carefully into aq. saturated sodium bicarbonate. The organic phase was separated off, washed with brine and aq. ammonium chloride, dried using a phase separator and evaporated to give a yellow solid which contained a lot of ammonium chloride. This was dissolved in DCM (20ml) and washed well with 1 M HCl (2x20ml) followed by water (2x20ml). The combined organic extracts were filtered through a phase separator and evaporated to dryness to give 4-bromo-2-(difluoromethyl)-1-(phenylsulfonyl)-1*H*-pyrrolo[2,3-*b*]pyridine (0.380g, 85%) as a yellow solid.

4-Bromo-2-(difluoromethyl)-1-(phenylsulfonyl)-1*H*-pyrrolo[2,3-*b*]pyridine (0.077g, 0.2mmol), *N*-(1,1-dioxidotetrahydro-3-thienyl)-4-(4,4,5,5-tetramethyl-1,3,2-dioxaborolan-2-yl)benzenesulfonamide (0.100g, 0.25mmol), bis(diphenylphosphino)ferrocene palladium II chloride (0.010g) and sodium carbonate (0.042g, 0.4mmol) in 1,4-dioxane (2ml) and water (0.7ml) were stirred at 120°C in the Biotage Initiator mw for 40 mins. DCM (50ml) and brine (20ml) were added. The DCM layer was evaporated to dryness and the product purified by chromatography (5g silica SPE cartridge) using DCM to 10% EtOAc in DCM to 20% EtOAc in DCM . The appropriate fractions were combined and evaporated to give 4-[2-(difluoromethyl)-1-(phenylsulfonyl)-1*H*-pyrrolo[2,3-*b*]pyridin-4-yl]-*N*-(1,1-dioxidotetrahydro-3-thienyl)benzenesulfonamide as a yellow foam (0.065g, 56%).

To a solution of 4-[2-(difluoromethyl)-1-(phenylsulfonyl)-1*H*-pyrrolo[2,3-*b*]pyridin-4-yl]-*N-*(1,1-dioxidotetrahydro-3-thienyl)benzenesulfonamide (0.070g, 0.12mmol) in dry THF (3ml) was added a solution of TBAF in THF (1M, 0.2ml, 0.2mmol). The reaction was stirred for 1 hour at room temperature and left to stand overnight. DCM (20ml) and saturated aq. ammonium chloride were added. The organic phase was separated. The aq. phase was extracted with DCM (20ml). The combined organic phases were dried (phase separator) and evaporated to give an orange solid (0.025g) which was purified by MDAP (using a water / acetonitrile gradient containing 0.1% formic acid) to give the title compound as a white solid (0.004g, 8%).
MH+442 rt= 0.86 mins

### Example 64 - Enantiomer 1

### 4-[2-(Difluoromethyl)-1H-pyrrolo[2,3-b]pyridin-4-yl]-N-(1,1-dioxidotetrahydro-3-thienyl)benzenesulfonamide - Enantiomer 1

4-[2-(Difluoromethyl)-1*H*-pyrrolo[2,3-*b*]pyridin-4-yl]-*N*-(1,1-dioxidotetrahydro-3-thienyl)benzenesulfonamide was separated into the individual enantiomers using the following method:
Prep method : MeOH (+0.1%TEA,+0.1%HOAc), flow rate = 20.0ml/min, Column 22.1mmid x 25cm Chirobiotic "TAG", rt (mins) Isomer 1, 8mins, total run time 20mins. Analytical method : MeOH (+0.1%TEA,+0.1%HOAc), flow rate = 1.0ml/min, Column 4.6mmid x 25cm Chirobiotic "TAG". Rt (mins) 4.88. LCMS = rt = 0.91 mins, MH+ = 442

### Example 64 - Enantiomer 2

### 4-[2-(Difluoromethyl)-1H-pyrrolo[2,3-b]pyridin-4-yl]-N-(1,1-dioxidotetrahydro-3-thienyl)benzenesulfonamide - Enantiomer 2

4-[2-(Difluoromethyl)-1*H*-pyrrolo[2,3-*b*]pyridin-4-yl]-*N*-(1,1-dioxidotetrahydro-3-thienyl)benzenesulfonamide was separated into the individual enantiomers using the following method:
Prep method : MeOH (+0.1%TEA,+0.1%HOAc), flow rate = 20.0ml/min, Column 22.1mmid x 25cm Chirobiotic "TAG", rt (mins) Isomer 2,15mins, total run time 20mins. Analytical method : MeOH (+0.1%TEA,+0.1%HOAc), flow rate = 1.0ml/min, Column 4.6mmid x 25cm Chirobiotic "TAG". Rt (mins) 8.39.
LCMS = rt = 0.91 mins, MH+ = 442

### Example 65

### 4-[2-(Difluoromethyl)-1H-pyrrolo[2,3-b]pyridin-4-yl]-N-(1,1-dioxidotetrahydro-2H-thiopyran-4-yl)benzenesulfonamide

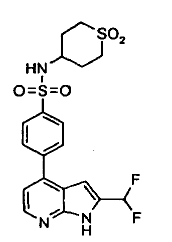

To a solution of 4-[2-(difluoromethyl)-1-(phenylsulfonyl)-1*H*-pyrrolo[2,3-*b*]pyridin-4-yl]-*N-*(1,1-dioxidotetrahydro-2*H*-thiopyran-4-yl)benzenesulfonamide (0.090g, 0.15mmol) in dry THF (3ml) was added a solution of TBAF in THF (1M, 0.2ml, 0.2mmol). The reaction was stirred for 1 hour at room temperature and left to stand overnight. DCM (20ml) and saturated aq. ammonium chloride were added. The organic phase was separated. The aq. phase was extracted with DCM (20ml). The combined organic phases were dried (phase separator) and evaporated to give a gum (0.050g) which was purified by MDAP to give the title compound as a white solid (0.0046g, 7%).
MH+456, rt= 0.86 mins

### Example 66

### 2-(1-Methylethyl)-4-[4-(1-pyrrolidinylsulfonyl)phenyl]-1H-pyrrolo[2,3-b]pyridine

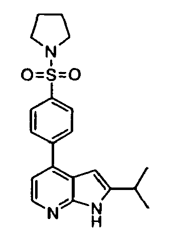

2-(1-Methylethenyl)-1-(phenylsulfonyl)-4-[4-(1-pyrrolidinylsulfonyl)phenyl]-1*H*-pyrrolo[2,3-*b*]pyridine (0.037g, 0.073mmol) was dissolved in methanol (1.5ml) and dimethylformamide (0.4ml). The material was reduced using the H-cube (flow rate set at 1ml/min, H₂ full, no heating). The first fraction collected was reduced to give 2-(1-methylethyl)-1-(phenylsulfonyl)-4-[4-(1-pyrrolidinylsulfonyl)phenyl]-1*H*-pyrrolo[2,3-*b*]pyridine (0.015g). Due to poor solubility the product was washed through using 10% dimethylformamide in methanol and some more product was eluted (0.010g).

2-(1-Methylethyl)-1-(phenylsulfonyl)-4-[4-(1-pyrrolidinylsulfonyl)phenyl]-1*H*-pyrrolo[2,3-*b*]pyridine (0.015g) was dissolved in THF (2ml) and treated with powdered potassium hydroxide (0.010g). The reaction was stirred for 2 hrs. The reaction was treated with 5M sodium hydroxide (0.150ml) and methanol (1ml) and solubility improved. The reaction was stirred for a further 1 hour (some deprotected product) and overnight (18 hrs). The reaction was poured into water (20ml) and extracted with DCM (2x20ml). The organic layers were combined and reduced under *vacuum* to give the crude material. 2-(1-Methylethyl)-1-(phenylsulfonyl)-4-[4-(1-pyrrolidinylsulfonyl)phenyl]-1*H*-pyrrolo[2,3-*b*]pyridine (0.010g) was dissolved in THF (2ml) and treated with powdered potassium hydroxide (0.010g). The reaction was stirred for 2 hrs. The reaction was treated with 5M sodium hydroxide (0.150ml) and methanol (1ml) and solubility improved. The reaction was stirred for a further 1 hour (mostly deprotected product) and overnight (18 hrs). The reaction was poured into water (20ml) and extracted with DCM (2x20ml). The organic layers were combined and reduced under *vacuum* to give the crude material. Both batches of crude material were which combined and purified by silica preparative plate eluting with EtOAc to afford the title compound (0.0055g).
MH+370, rt= 3.32 mins

### Example 67

### N-(1,1-Dioxidotetrahydro-2H-thiopyran-4-yl)-4-[2-(1-methylethyl)-1H-pyrrolo[2,3-b]pyridin-4-yl]benzenesulfonamide

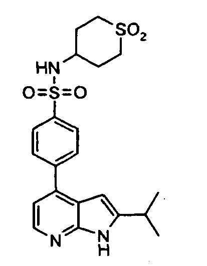

*N*-(1,1-Dioxidotetrahydro-2*H*-thiopyran-4-yl)-4-[2-(1-methylethenyl)-1-(phenylsulfonyl)-1*H-*pyrrolo[2,3-*b*]pyridin-4-yl]benzenesulfonamide (0.066g, 0.112mmol) in methanol (1.75ml) and dimethylformamide (0.5ml) was reduced using the H-cube (flow rate set at 1ml/min, H₂ full, no heating). 10% Dimethylformamide in methanol and 70% dimethylformamide in methanol were used as solvent to wash through the products. The first fraction was reduced under vacuum to yield *N*-(1,1-dioxidotetrahydro-2*H*-thiopyran-4-yl)-4-[2-(1-methylethyl)-1-(phenylsulfonyl)-1*H*-pyrrolo[2,3-*b*]pyridin-4-yl]bnzenesulfonamide (0.048g). This material was dissolved in THF (2ml) and treated with powdered potassium hydroxide (0.022g). The reaction was stirred for 2 hrs. The reaction was treated with 5M sodium hydroxide (0.200ml) and methanol (1ml) and solubility improved. The reaction was stirred for a further 1 hour and left overnight (18 hrs). The reaction was poured into water (20ml), acidified to pH8 with HCl and extracted with DCM (2x20ml). The organic layers were combined and reduced under *vacuum* to give the crude material (0.043g) which was purified by MDAP (using a water / acetonitrile gradient containing 0.1% formic acid). Fractions containing the product were reduced under *vacuum* to give material (0.011g) which was triturated with methanol to afford the title compound as a white solid (0.0035g). MH+448, rt= 2.86 mins

### Example 68

### 2-Ethyl-4-[4-(1-pyrrolidinylsulfonyl)phenyl]-1H-pyrrolo[2,3-b]pyridine

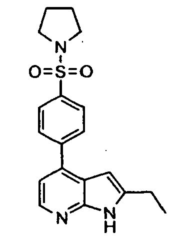

2-Ethynyl-4-[4-(1-pyrrolidinylsulfonyl)phenyl]-1*H*-pyrrolo[2,3-*b*]pyridine (0.017g, 0.048mmol) was dissolved in methanol (1ml) and dimethylformamide (0.2ml). The material was reduced using the H-cube, the solvent used to wash through the product was 10% dimethylformamide in methanol. The second fraction collected was reduced to afford (0.006g). The third fraction collected was reduced to afford (0.0025g). The 2 batches of material were combined and purified by preparative TLC eluting with EtOAc twice. The product was removed from the plate and extracted with EtOAc to afford the title compound (0.0044g).
MH+356, rt= 3.20 mins

### Example 69

### N-(1,1-Dioxidotetrahydro-2H-thiopyran-4-yl)-4-[2-(trifluoromethyl)-1H-pyrrolo[2,3-b]pyridin-4-yl]benzenesulfonamide

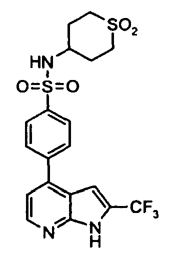

4-Chloro-2-(trifluoromethyl)-1*H*-pyrrolo[2,3-*b*]pyridine (0.070g, 0.3mmol), *N*-(1,1-dioxidotetrahydro-2*H*-thiopyran-4-yl)-4-(4,4,5,5-tetramethyl-1,3,2-dioxaborolan-2-yl)benzenesulfonamide (0.100g, 0.24mmol), bis(diphenylphosphino)ferrocene palladium II chloride (0.010g, 0.014mmol) and sodium carbonate (0.028g, 0.23mmol) in dioxane-water (2ml) were stirred at 190°C in the Biotage Initiator mw for 10 mins. The reaction was filtered, evaporated then dissolved in 9:1 DCM:methanol (20ml) and washed with water (10ml). The organic phase was separated, silica added and then evaporated. The dried silica was put onto a silica SPE cartridge (20g) and eluted with 7:3 DCM:EtOAc followed by 9:1 DCM:methanol. The main fraction was evaporated, triturated with hot methanol and the title compound obtained as a buff solid on filtration (0.030g, 26%).
MH+474, rt= 2.9 mins

### Example 70

### 4-[4-(1-Pyrrolidinylsulfonyl)phenyl]-2-(trifluoromethyl)-1H-pyrrolo[2,3-b]pyridine

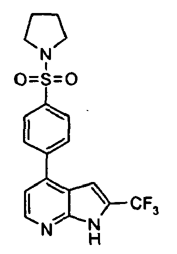

4-Chloro-2-(trifluoromethyl)-1*H*-pyrrolo[2,3-*b*]pyridine (0.070g, 0.3mmol), [4-(1-pyrrolidinylsulfonyl)phenyl]boronic acid (0.120g, 0.47mmol), bis(diphenylphosphino)ferrocene palladium II chloride (0.010g, 0.014mmol) and sodium carbonate (0.028g, 0.23mmol) in dioxane-water (2ml) were stirred at 190°C in the Biotage Initiator mw for 5 mins. The reaction was filtered, and the filter paper washed with 9:1 DCM:methanol (10ml). The filtrate was diluted with DCM (15ml) and washed with water (10ml). The organic phase was treated with Fluorosil and evaporated. The material was chromatographed on a silica SPE cartridge and eluted with EtOAc-DCM (0-25% over 40 mins). The main fraction was evaporated to give the title compound as a white solid which was washed with methanol (0.030g, 25%).
MH+396, rt= 3.3 mins

### Example 71

### N-(1,1-Dioxidotetrahydro-3-thienyl)-4-[2-(trifluoromethyl)-1H-pyrrolo[2,3-b]pyridin-4-yl]benzenesulfonamide

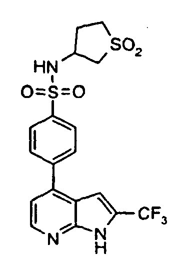

4-Chloro-2-(trifluoromethyl)-1*H*-pyrrolo[2,3-*b*]pyridine (0.070g, 0.3mmol), *N*-(1,1-dioxidotetrahydro-3-thienyl)-4-(4,4,5,5-tetramethyl-1,3,2-dioxaborolan-2-yl)benzenesulfonamide (0.200g, 0.50mmol), bis(diphenylphosphino)ferrocene palladium II chloride (0.010g, 0.014mmol) and sodium carbonate (0.028g, 0.23mmol) in dioxane-water (2ml) were stirred at 190°C in the Biotage Initiator mw for 10 mins. The reaction was filtered, and the filter paper washed with 9:1 DCM:methanol (10ml). The filtrate was diluted with DCM (15ml) and washed with water (10ml). The organic phase was treated with Fluorosil and evaporated. The material was chromatographed on a silica SPE cartridge and eluted with EtOAc-DCM (0-30%) then 9:1 DCM:methanol. The main fraction was evaporated and triturated with hot methanol to give the title compound as a buff solid (0.013g, 9%).
MH-458, rt= 0.94 mins

### Example 72

### 2-[4-(4-{[(1,1-Dioxidotetrahydro-2H-thiopyran-4-yl)amino]sulfonyl}phenyl)-1H-pyrrolo[2,3-b]pyridin-2-yl]-N-[3-(methyloxy)phenyl]acetamide

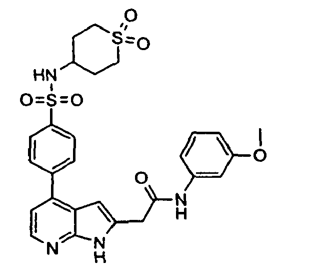

2-(4-Bromo-1*H*-pyrrolo[2,3-*b*]pyridin-2-yl)-*N*-[3-(methyloxy)phenyl]acetamide (0.050g, 0.14mmol), *N*-(1,1-dioxidotetrahydro-2*H*-thiopyran-4-yl)-4-(4,4,5,5-tetramethyl-1,3,2-dioxaborolan-2-yl)benzenesulfonamide (0.070g, 0.168mmol), bis(diphenylphosphino)ferrocene palladium (II) chloride (0.004g) and sodium carbonate (0.014g, 0.132mmol) were treated with 5:1 dioxan : water (1ml) and heated in the Biotage Initiator mw at 180°C for 10 min. The reaction was incomplete and was heated at 180°C for 3 min and again for 3 min at 180°C. The solution was evaporated and the solid was triturated with water. The solid was dried *in vacuo* and purified by silica SPE eluting with DCM - EtOAc (0%-40%) and DCM - MeOH (5%-10%). The main fraction was collected and evaporated to give a brown gum which was crystallised from MeOH to give a buff coloured solid (0.020g, 25%).
MH+569, rt= 0.91 min

### Example 73

### 2-[4-(4-{[(1,1-Dioxidotetrahydro-3-thienyl)amino]sulfonyl}phenyl)-1H-pyrrolo[2,3-b]pyridin-2-yl]-N-[3-(methyloxy)phenyl]acetamide

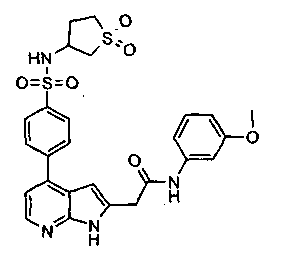

2-(4-Bromo-1*H*-pyrrolo[2,3-*b*]pyridin-2-yl)-*N*-[3-(methyloxy)phenyl]acetamide (0.050g, 0.14mmol), *N*-(1,1-dioxidotetrahydro-3-thienyl)-4-(4,4,5,5-tetramethyl-1,3,2-dioxaborolan-2-yl)benzenesulfonamide (0.070g, 0.174mmol), bis(diphenylphosphino)ferrocene palladium (II) chloride (0.004g) and sodium carbonate (0.014g, 0.132mmol) were treated with 5:1 dioxan : water (1ml) and heated in the Biotage Initiator mw at 180°C for 10 min. The reaction was incomplete and was heated at 180°C for 10 min and again for 30 min at 180°C. The reaction was pre-absorbed onto silica and purified by silica SPE eluting with DCM - MeOH (3%-10%). The main fraction was evaporated to give a brown gum (0.018g). This was purified by MDAP and the main peak evaporated to give the title compound as a white solid (0.006g, 8%).
MH+555, rt= 0.91 min

### Example 74

### N-(1,1-Dioxldotetrahydro-3-thienyl)-4-{2-[2-oxo-2-(1-pyrrolidinyl)ethyl]-1H-pyrrolo[2,3-b]pyridin-4-yl}benzenesulfonamide

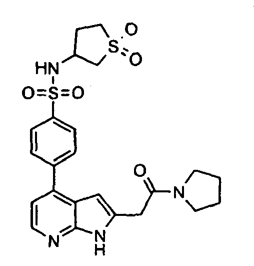

4-Bromo-2-[2-oxo-2-(1-pyrrolidinyl)ethyl]-1*H*-pyrrolo[2,3-*b*]pyridine (0.100g, 0.31mmol), *N-*(1,1-dioxidotetrahydro-3-thienyl)-4-(4,4,5,5-tetramethyl-1,3,2-dioxaborolan-2-yl)benzenesulfonamide (0.200g, 0.5mmol), bis(diphenylphosphino)ferrocene palladium (II) chloride (0.010g) and sodium carbonate (0.028g, 0.264mmol) were treated with dioxan : water (5:1, 2ml) and heated in the Biotage Initiator mw at 190°C for 10 min. The reaction was filtered, evaporated and purified twice by MDAP. The main fraction was evaporated to give the title compound as a pale yellow solid (0.018g, 11%).
MH+503, rt= 2.53 min

### Example 75

### Example 75a

### N-(1,1-Dioxidotetrahydro-2H-thiopyran-4-yl)-4-{2-[2-oxo-2-(1-pyrrolidinyl)ethyl]-1H-pyrrolo[2,3-b]pyridin-4-yl}benzenesulfonamide

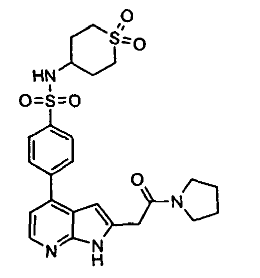

4-Bromo-2-[2-oxo-2-(1-pyrrolidinyl)ethyl]-1*H*-pyrrolo[2,3-*b*]pyridine (0.100g, 0.31mmol), ), *N*-(1,1-dioxidotetrahydro-2*H*-thiopyran-4-yl)-4-(4,4,5,5-tetramethyl-1,3,2-dioxaborolan-2-yl)benzenesulfonamide (0.200g, 0.48mmol), bis(diphenylphosphino)ferrocene palladium (II) chloride (0.010g, 0.012mmol) and sodium carbonate (0.028g, 0.264mmol) were treated with dioxan : water (5:1, 2ml) and heated in the Biotage Initiator mw at 190°C for 10 min. The reaction was filtered, the filtrate evaporated and purified by MDAP. The main fraction was collected and evaporated to give a dark orange solid (0.083g). This solid was re-purified by MDAP and the main fractions were evaporated, triturated in MeOH and the buff solid dried to give the title compound (0.018g, 11%).
MH+517, rt=0.81 min

### Example 75b

### N-(1,1-Dioxidotetrahydro-2H-thiopyran-4-yl)-4-{2-[2-oxo-2-(1-pyrrolidinyl)ethyl]-1H-pyrrolo[2,3-b]pyridin-4-yl}benzenesulfonamide trifluoroacetate

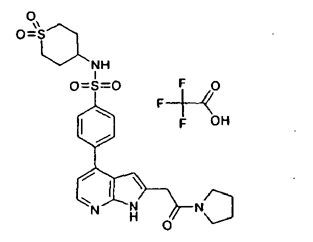

Example 75b was prepared similarly to Example 91.
LCMS rt = 2.49 min, m/z MH⁺ = 517

### Example 76

### 4-[4-(1-Pyrrolidinylsulfonyl)phenyl]-2-(2H-1,2,3-triazol-2-ylmethyl)-1H-pyrrolo[2,3-b]pyridine

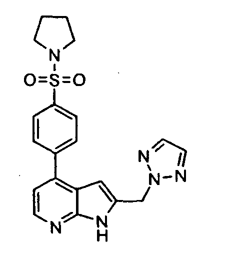

To a slurry of sodium hydride (60% dispersion in oil; 0.005g, 0.12mmol) in dry DMF (1ml) under nitrogen at room temperature was added triazole (0.01ml, 0.17mmol) in one portion. The reaction was stirred at room temperature for 15 min. {1-(Phenylsulfonyl)-4-[4-(1-pyrrolidinylsulfonyl)phenyl]-1*H*-pyrrolo[2,3-*b*]pyridin-2-yl}methyl methanesulfonate (0.050g, 0.09mmol) was added in one portion, the reaction stirred at room temperature for 3 hrs and allowed to stand overnight. Saturated ammonium chloride was added and it was extracted with DCM (2x30ml). The combined organic extracts were evaporated to give a crude mixture of 1-(phenylsulfonyl)-4-[4-(1-pyrrolidinylsulfonyl)phenyl]-2-(1*H*-1,2,3-triazol-1-ylmethyl)-1*H*-pyrrolo[2,3-*b*]pyridine and 1-(phenylsulfonyl)-4-[4-(1-pyrrolidinylsulfonyl)phenyl]-2-(2*H*-1,2,3-triazol-2-ylmethyl)-1*H*-pyrrolo[2,3-*b*]pyridine (0.060g). The crude mixture of 1-(phenylsulfonyl)-4-[4-(1-pyrrolidinylsulfonyl)phenyl]-2-(1*H*-1,2,3-triazol-1-ylmethyl)-1*H*-pyrrolo[2,3-*b*]pyridine and 1-(phenylsulfonyl)-4-[4-(1-pyrrolidinylsulfonyl)phenyl]-2-(2*H*-1,2,3-triazol-2-ylmethyl)-1*H*-pyrrolo[2,3-*b*]pyridine (0.060g) in MeOH (2ml) was treated with 2M sodium hydroxide (2ml) and heated in the Biotage Initiator mw at 80°C for 15 min. The reaction was evaporated to dryness, DMSO (1ml) added and purification by MDAP afforded the title compound as a white solid (0.0018g).
MH+409, rt= 2.95 min

### Example 77

### 4-[4-(1-Pyrrolidinylsulfonyl)phenyl]-2-(1H-tetrazol-1-ylmethyl)-1H-pyrrolo[2,3-b]pyridine

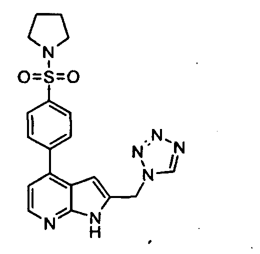

### Example 78

### 4-[4-(1-Pyrrolidinylsulfonyl)phenyl]-2-(2H-tetrazol-2-ylmethyl)-1H-pyrrolo[2,3-b]pyridine

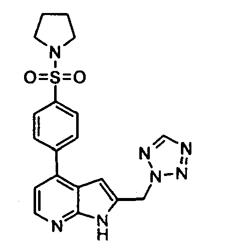

To sodium hydride (60% dispersion in oil; 0.015g, 0.38mmol) in dry DMF (2ml) was added tetrazole (0.030g, 0.42mmol) in one portion at room temperature under nitrogen. After 10 mins [4-Bromo-1-(phenylsulfonyl)-1*H*-pyrrolo[2,3-*b*]pyridin-2-yl]methyl methanesulfonate (0.1g, 0.22mmol) was added and the reaction was stirred at room temperature for 4 hrs. DCM (20ml) and water (5ml) were added. The aqueous phase was extracted with DCM (20ml). The combined organic extracts were washed with brine (20ml) and evaporated to give a crude mixture of 4-bromo-1-(phenylsulfonyl)-2-(1*H*-tetrazol-1-ylmethyl)-1*H-*pyrrolo[2,3-*b*]pyridine and 4-bromo-1-(phenylsulfonyl)-2-(2*H*-tetrazol-2-ylmethyl)-1*H-*pyrrolo[2,3-*b*]pyridine as a brown gum (0.100g). 4-Bromo-1-(phenylsulfonyl)-2-(1*H-*tetrazol-1-ylmethyl)-1*H*-pyrrolo[2,3-*b*]pyridine and 4-bromo-1-(phenylsulfonyl)-2-(2*H-*tetrazol-2-ylmethyl)-1*H*-pyrrolo[2,3-*b*]pyridine (0.100g, 0.24mmol), [4-(1-pyrrolidinylsulfonyl)phenyl]boronic acid (0.067g, 0.26mmol), bis(diphenylphosphino)ferrocene palladium (II) chloride (0.010g, 0.012mmol) and sodium carbonate (0.050g, 0.48mmol) in dioxan (3ml) and water (1ml) were heated in the Biotage Initiator mw at 120°C for 1 hour. DCM (50ml) and water (10ml) were added. The aqueous phase was extracted with DCM (20ml). The combined organic phases were evaporated to give a crude mixture of 1-(phenylsulfonyl)-4-[4-(1-pyrrolidinylsulfonyl)phenyl]-2-(1*H-*tetrazol-1-ylmethyl)-1*H*-pyrrolo[2,3-*b*]pyridine and 1-(phenylsulfonyl)-4-[4-(1-pyrrolidinylsulfonyl)phenyl]-2-(2*H*-tetrazol-2-ylmethyl)-1*H*-pyrrolo[2,3-*b*]pyridine as a dark brown gum (0.100g, 81%). To a solution of the mixture of 1-(phenylsulfonyl)-4-[4-(1-pyrrolidinylsulfonyl)phenyl]-2-(1*H*-tetrazol-1-ylmethyl)-1*H*-pyrrolo[2,3-*b*]pyridine and 1-(phenylsulfonyl)-4-[4-(1-pyrrolidinylsulfonyl)phenyl]-2-(2H-tetrazol-2-ylmethyl)-1*H-*pyrrolo[2,3-b]pyridine (0.100g, 0.18mmol) in THF (1ml) was added 3M sodium hydroxide (0.1 ml) in MeOH and the reaction stirred at room temperature for 1 hour. 2M hydrochloric acid (3ml) was added and the mixture was evaporated to dryness. The solid was triturated with DMSO and the resulting DMSO solution was purified by MDAP.

4-[4-(1-Pyrrolidinylsulfonyl)phenyl]-2-(1*H*-tetrazol-1-ylmethyl)-1*H*-pyrrolo[2,3-*b*]pyridine was obtained as a brown solid (0.0058g, 8%).
MH+410, rt= 0.92 min
4-[4-(1-Pyrrolidinylsulfonyl)phenyl]-2-(2*H*-tetrazol-2-ylmethyl)-1*H*-pyrrolo[2,3-*b*]pyridine was obtained as a white solid (0.0068g, 9%).
MH+410, rt=0.96 min

### Example 79

### 4-{4-[4-(1-Pyrrolidinylsulfonyl)phenyl]-1H-pyrrolo[2,3-b]pyridin-2-yl}-1-butanol trifluoroacetate

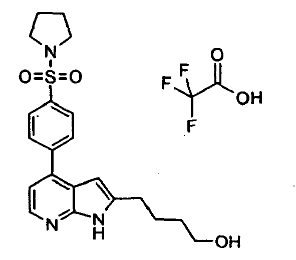

A solution of 4-{4-[4-(1-pyrrolidinytsulfonyl)phenyl]-1H-pyrrolo[2,3-b]pyridin-2-yl}-3-butyn-1-ol (15mg, 0.04mmol) in methanol:EtOAc 1:1 was placed under an atmosphere of nitrogen prior to the addition of 10% Palladium on carbon (10mg). The reaction mixture was stirred under an atmosphere of hydrogen at 22°C for 1.5h. The reaction mixture was applied to an aminopropyl cartridge (1g), preconditioned with chloroform (2mL). The crude title compound was eluted with chloroform:methanol (2 x 2mL) and concentrated by blow down. Purification by mass directed HPLC gave the title compound.
LCMS RT= 2.88min ES+ve 398 m/z (MH)⁺

Example 80 similarly prepared:

| **Example** | Compound | LCMS rt, | m/z |
|---|---|---|---|
| | | min | MH+ |
| **80** | 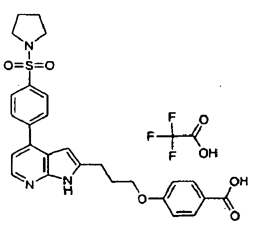 | 3.41 | 506 |
| | 4-[(3-{4-[4-(1-pyrrolidinylsulfonyl)phenyl]-1H-pyrrolo[2,3-b]pyridin-2-yl}-2-propyn-1-yl)oxy]benzoic acid | | |

### Example 81

### Example 81a

### N-[(2S)-2-Hydroxypropyl]-4-(2-methyl-1H-pyrrolo[2,3-b]pyridin-4-yl)benzenesulfonamide

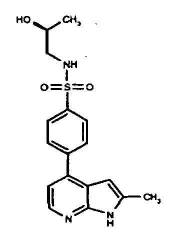

Example 81 a was prepared similarly to Example 195.
LCMS rt = 2.53 mins, MH+ = 346

### Example 81b

### N-[(2S)-2-Hydroxypropyl]-4-(2-methyl-1H-pyrrolo[2,3-b]pyridin-4-yl)benzenesulfonamide trifluoroacetate

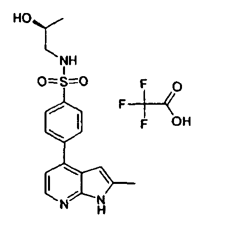

(2*S*)-1-Amino-2-propanol (33.75mg, 0.45mmol), pentafluorophenyl 4-(4,4,5,5-tetramethyl-1,3,2-dioxaborolan-2-yl)benzenesulfonate (191mg, 0.425mmol) and TEA (200µL) were heated in a mw at 120°C for 10 mins. The product (LCMS RT= 2.49min ES+ve 346 m/z (MH) ⁺) was used crude. A solution of 4-bromo-2-methyl-1-(phenylsulfonyl)-1*H-*pyrrolo[2,3-b]pyridine (35mg, 0.1mmol) in dioxan:water, 5:1 (0.5mL) was added to a mw vessel containing potassium phosphate (64mg, 0.3mmol) and 2'(dimethylamino)-2-biphenyl-palladium II chloride dinorbornylphosphine complex (0.3mg, 0.5 mol%). A solution of crude [4-({[(2S)-2-hydroxypropyl]amino}sulfonyl)phenyl]boronic acid (-0.25mmol) in dioxan:water, 5:1 (0.5mL) was added. The reaction mixture was heated at 120°C in the mw for 30 mins. The reaction mixture was treated with 40% KF supported on Alumina (220mg, 0.15mmol) and heated at 120°C in the mw for 10 mins. The reaction mixture was applied directly to a C18 cartridge (500mg) and eluted with 0.1% TFA in acetonitrile (3 x 1 mL). Concentration by blow down followed by treatment with a solution of DCM:TFA, 1:1 and re concentration by blow down afforded the crude product. Purification by mass directed HPLC gave the title compound.
LCMS RT= 2.58min ES+ve 346 m/z (MH)⁺

Examples 82 to 89 were similarly prepared:

| **Example** | Compound | LCMS | m/z |
|---|---|---|---|
| | | rt, min | MH+ |
| **82** | 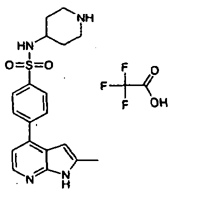 | 2.17 | 371 |
| | 4-(2-methyl-1*H*-pyrrolo[2,3-*b*]pyridin-4-yl)-N-4-piperidinylbenzenesulfonamide trifluoroacetate | | |
| **83** | 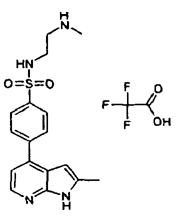 | 2.50 | 346 |
| | *N*-[2-(methylamino)ethyl]-4-(2-methyl-1*H*-pyrrolo[2,3-*b*]pyridin-4-yl)benzenesulfonamide trifluoroacetate | | |
| **84** | 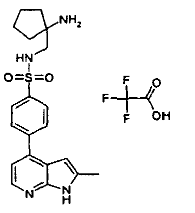 | 2.21 | 385 |
| | *N*-[(1-aminocyclopentyl)methyl]-4-(2-methyl-1*H*-pyrrolo[2,3-*b*]pyridin-4-yl)benzenesulfonamide trifluoroacetate | | |
| **85** | 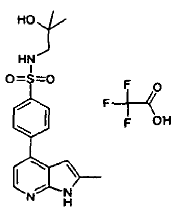 | 2.65 | 360 |
| | *N*-(2-hydroxy-2-methylpropyl)-4-(2-methyl-1*H*-pyrrolo[2,3-*b*]pyridin-4-yl)benzenesulfonamide trifluoroacetate | | |
| **86** | 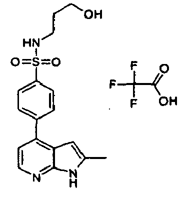 | 2.54 | 346 |
| | *N*-(3-hydroxypropyl)-4-(2-methyl-1*H*-pyrrolo[2,3-*b*]pyridin-4-yl)benzenesulfonamide trifluoroacetate | | |
| **87** | 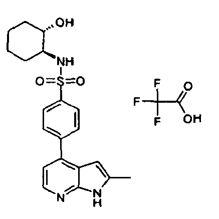 | 2.83 | 386 |
| | *N*-[(1*S*,2*S*)-2-hydroxycyclohexyl]-4-(2-methyl-1*H*-pyrrolo[2,3-*b*]pyridin-4-yl)benzenesulfonamide trifluoroacetate | | |
| **88** | 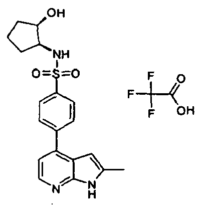 | 2.70 | 372 |
| | *N*-[(1*S*,2*R*)-2-hydroxycyclopentyl]-4-(2-methyl-1*H*-pyrrolo[2,3-*b*]pyridin-4-yl)benzenesulfonamide trifluoroacetate | | |
| **89** | 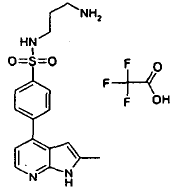 | 2.13 | 345 |
| | *N*-(3-aminopropyl)-4-(2-methyl-1*H*-pyrrolo[2,3-*b*]pyridin-4-yl)benzenesulfonamide trifluoroacetate | | |

### Example 90

### Example 90a

### N-[(2R)-2-Hydroxypropyl]-4-(2-methyl-1H-pyrrolo[2,3-b]pyridin-4-yl)benzenesulfonamide trifluoroacetate

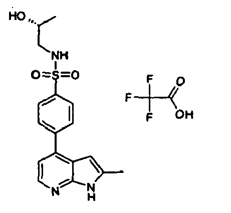

Example 90a was prepared similarly to Example 81b.
LCMS rt = 2.56 min, m/z MH+ = 346

### Example 90b

### N-[(2R)-2-Hydroxypropyl]-4-(2-methyl-1H-pyrrolo[2,3-b]pyridin-4-yl)benzenesulfonamide

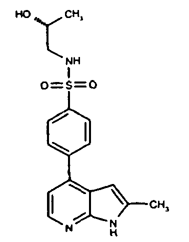

Example 90b was prepared similarly to Example 195.
LCMS rt = 2.53 mins, MH+ = 346

### Example 91

### N-(1,1-Dioxidotetrahydro-2H-thiopyran-4-yl)-4-[2-(1-hydroxyethyl)-1H-pyrrolo[2,3-b]pyridin-4-yl]benzenesulfonamide trifluoroacetate

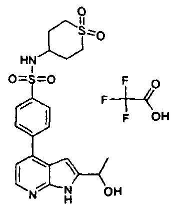

A solution of 1-[4-bromo-1-(phenylsulfonyl)-1*H*-pyrrolo[2,3-*b*]pyridin-2-yl]ethanol (38mg, 0.1mmol) in dioxan:water, 5:1 (0.5mL) was added to a mw vessel containing potassium phosphate (64mg, 0.3mmol) and 2'(dimethylamino)-2-biphenyl-palladium II chloride dinorbornylphosphine complex (0.3mg, 0.5 mol%). A solution of 4-({[4-(4,4,5,5-tetramethyl-1,3,2-dioxaborolan-2-yl)phenyl]sulfonyl}methyl)thiomorpholine 1,1-dioxide (62mg, 0.15mmol) in dioxan:water, 5:1 (0.5mL) was added. The reaction mixture was heated at 130°C in the mw for 30 mins. The reaction mixture was applied directly to a C18 cartridge (500mg) and eluted with 0.1% TFA in acetonitrile (3 x 1mL). Concentration by blow down followed by treatment with a solution of DCM:TFA, 1:1 and re concentration by blow down afforded the crude product. Purification by mass directed HPLC gave the title compound.
LCMS rt= 2.40min ES+ve 450 m/z (MH)⁺

Examples 92 to 99 were similarly prepared:

| **Example** | Compound | LCMS | m/z |
|---|---|---|---|
| | | rt, min | MH+ |
| **92** | 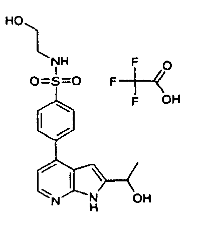 | 2.26 | 362 |
| | *N*-(2-hydroxyethyl)-4-[2-(1-hydroxyethyl)-1*H*-pyrrolo[2,3-*b*]pyridin-4-yl]benzenesulfonamide trifluoroacetate | | |
| **93** | 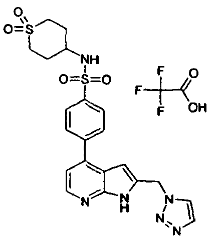 | 2.45 | 487 |
| | N-(1,1-dioxidotetrahydro-2H-thiopyran-4-yl)-4-[2-(1H-1,2,3-triazol-1-ylmethyl)-1H-pyrrolo[2,3-b]pyridin-4-yl]benzenesulfonamide trifluoroacetate | | |
| **94** | 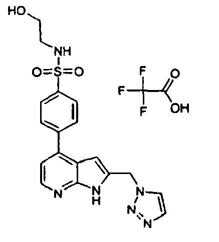 | 2.32 | 399 |
| | N-(2-hydroxyethyl)-4-[2-(1H-1,2,3-triazol-1-ylmethyl)-1H-pyrrolo[2,3-b]pyridin-4-yl]benzenesulfonamide trifluoroacetate | | |
| **95** | 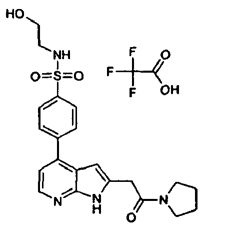 | 2.37 | 429 |
| | N-(2-hydroxyethyl)-4-{2-[2-oxo-2-(1-pyrrolidinyl)ethyl]-1H-pyrrolo[2,3-b]pyridin-4-yl}benzenesulfonamide trifluoroacetate | | |
| **96** | 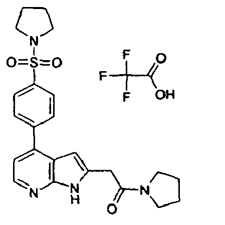 | 2.85 | 439 |
| | 2-[2-oxo-2-(1-pyrrolidinyl)ethyl]-4-[4-(1-pyrrolidinylsulfonyl)phenyl]-1H-pyrrolo[2,3-b]pyridine trifluoroacetate | | |
| **97** | 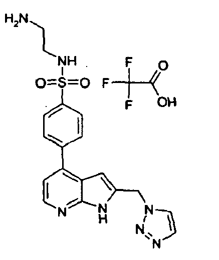 | 1.92 | 398 |
| | N-(2-aminoethyl)-4-[2-(1H-1,2,3-triazol-1-ylmethyl)-1H-pyrrolo[2,3-b]pyridin-4-yl]benzenesulfonamide trifluoroacetate | | |
| **98** | 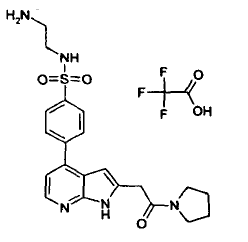 | 1.98 | 428 |
| | N-(2-aminoethyl)-4-{2-[2-oxo-2-(1-pyrrolidinyl)ethyl]-1H-pyrrolo[2,3-b]pyridin-4-yl}benzenesulfonamide trifluoroacetate | | |
| **99** | 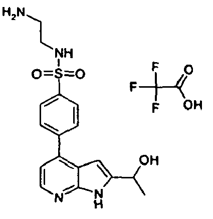 | 1.88 | 362 |
| | *N*-(2-aminoethyl)-4-[2-(1-hydroxyethyl)-1*H*-pyrrolo[2,3-*b*]pyridin-4-yl]benzenesulfonamide trifluoroacetate | | |

### Example 100

### Example 100a

### 1-{4-[4-(1-Pyrrolidinylsulfonyl)phenyl]-1H-pyrrolo[2,3-b]pyridin-2-yl}ethanol

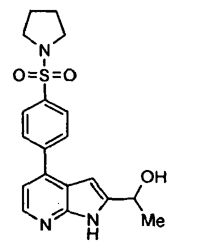

1-{4-[4-(1-Pyrrolidinylsulfonyl)phenyl]-1*H*-pyrrolo[2,3-*b*]pyridin-2-yl}ethanone (0.020g, 0.05mmol) in THF (1ml) and water (0.4ml) (formed suspension) was treated with sodium borohydride (0.0061g, 0.16mmol) and stirred for 1 hour under nitrogen. After 20 mins the reaction had become a solution. The reaction was diluted with water (15ml) and extracted with DCM (3x10ml). The organic layers were combined, washed with water (10ml), dried (phase separator) and reduced under *vacuum* to afford the crude material (0.010g). This was purified using a silica prep plate eluting with EtOAc containing 10% methanol to afford the title compound (0.0055g).
MH+372, rt= 2.80 mins

### Example 100b

### 1-{4-[4-(1-Pyrrolidinylsulfonyl)phenyl]-1H-pyrrolo[2,3-b]pyridin-2-yl}ethanol trifluoroacetate

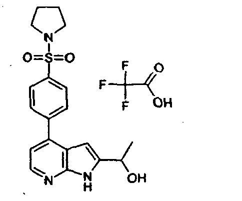

Example 100b was prepared similarly to Example 91.
LCMS rt = 2.78 min, m/z MH+ = 372

### Example 101

### N-[4-(2-Methyl-1H-pyrrolo[2,3-b]pyridin-4-yl)phenyl]methanesulfonamide

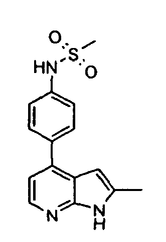

*N*-{4-[2-Methyl-1-(phenylsulfonyl)-1*H*-pyrrolo[2,3-*b*]pyridin-4-yl]phenyl}methanesulfonamide (0.091g, 0.204mmol) was dissolved in dioxan (5ml) and water (1ml). 10M Sodium hydroxide (1ml) was added and the reaction mixture stirred at room temperature overnight. The mixture was heated in the Biotage Initiator mw at 150°C for 15 min. The reaction mixture was partitioned between DCM : EtOAc (1:1, 25ml) and water (10ml). Saturated ammonium chloride was added to pH6 to the aqueous layer. The aqueous phase was extracted with EtOAc (20ml). The combined organic layers were dried (hydrophobic frit), concentrated *in vacuo* to give a yellow foam which was dissolved in MeOH (2ml) and eluted through an NH₂ cartridge to remove the TFA. The column was washed with 2 volumes of MeOH. The filtrate was concentrated to give the title compound as a white solid (0.044g, 72%).
MH+302, rt= 0.75 min

### Example 102

### Example 102a

### N-Methyl-N-[4-(2-methyl-1H-pyrrolo[2,3-b]pyridin-4-yl)phenyl]methanesulfonamide

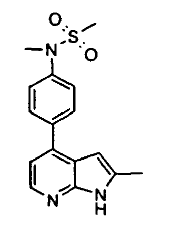

*N*-Methyl-*N*-{4-[2-methyl-1-(phenylsulfonyl)-1*H*-pyrrolo[2,3-*b*]pyridin-4-yl]phenyl}methanesulfonamide (0.090g, 0.198mmol) was dissolved in dioxin (3.5ml) and heated at 150°C for 15 min in the Biotage Initiator mw with 10M sodium hydroxide (0.5ml) and water (0.5ml). The reaction mixture was partitioned between EtOAc (25ml) and water (10ml) and saturated citric acid was added to pH6. The aqueous phase was extracted with EtOAc (20ml). The combined organic layers were dried (hydrophobic frit), concentrated *in vacuo* and the residue purified by MDAP. The desired fraction was concentrated to give a yellow glass. This was dissolved in MeOH (2ml) and eluted through an NH₂ cartridge to remove the TFA. The column was washed with 2 volumes of MeOH and the solvent removed *in vacuo* to give the title compound as a white solid (0.046g, 74%).
MH+316, rt= 0.86 min

### Example 102b

### N-Methyl-N-[4-(2-methyl-1H-pyrrolo[2,3-b]pyridin-4-yl)phenyl]methanesulfonamide trifluoroacetate

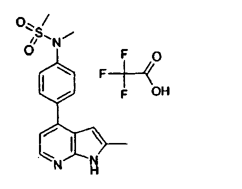

A solution of methanesulfonyl chloride (22mg, 0.2mmol) in dry DCM (0.5mL), was added to a solution of methyl{4-[2-methyl-1-(phenylsulfonyl)-1*H*-pyrrolo[2,3-*b*]pyridin-4-yl]phenyl}amine (38mg, 0.1 mmol) in dry DMF (0.5mL) and the resultant reaction mixture was treated with DIPEA (64uL). The reaction mixture was allowed to stand for 16 h prior to addition to an aminopropyl cartridge (1g), preconditioned with DCM (2mL). The crude title compound was eluted with DCM (2 x 2mL) and concentrated by blow down. Purification by mass directed HPLC gave the title compound.
LCMS RT= 2.82min, ES+ve 316 m/z (MH)⁺

### Example 103

### N-(1,1-Dioxidotetrahydro-2H-thiopyran-4-yl)-N-methyl-4-(2-methyl-1H-pyrrolo[2,3-b]pyridin-4-yl)benzenesulfonamide

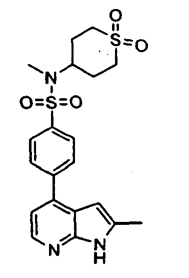

A mixture of *N*-(1,1-dioxidotetrahydro-2*H*-thiopyran-4-yl)-*N*-methyl-4-[2-methyl-1-(phenylsulfonyl)-1*H*-pyrrolo[2,3-*b*]pyridin-4-yl]benzenesulfonamide (0.053g, 0.09mmol) in 5M sodium hydroxide (1ml) and dioxan (4ml) was heated in the Biotage Initiator mw at 150°C for 15 min. The mixture was partitioned between DCM (10ml) and water (10ml). The aqueous phase was acidified to pH5 with citric acid. The organic phase was dried (hydrophobic frit), concentrated *in vacuo* and the residue purified by MDAP. The desired fractions were combined and concentrated *in vacuo* to give the title compound (0.022g, 56%).
MH+434, rt= 2.75 min

### Example 104

### ({4-[4-(1-Pyrrolidinylsulfonyl)phenyl]-1H-pyrrolo[2,3-b]pyridin-2-yl}methyl)amine or 1-{4-[4-(1-Pyrrolidinylsulfonyl)phenyl]-1H-pyrrolo[2,3-b]pyridin-2-yl}methanamine

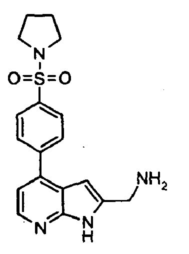

A solution of ({1-(phenylsulfonyl)-4-[4-(1-pyrrolidinylsulfonyl)phenyl]-1*H*-pyrrolo[2,3-*b*]pyridin-2-yl}methyl)amine (0.055g, 0.11mmol) in dioxan (1ml) was treated with 5M sodium hydroxide (0.250ml) and heated in the Biotage Initiator mw at 140°C for 40 min. The reaction was poured into water (20ml), neutralised to pH8 using hydrochloric acid and extracted with DCM (2x20ml). The combined organic layers were reduced *in vacuo* to give (0.023g) which was purified by MDAP to afford the title compound (0.009g, 23%).
MH+357, rt= 2.27 min

### Example 105

### N-(1,1-Dioxidotetrahydro-3-thienyl)-4-(2-methyl-1H-pyrrolo[2,3-b]pyridin-4-yl)benzenesulfonamide

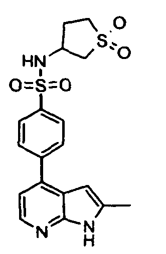

4-Bromo-2-methyl-1-(phenylsulfonyl)-1*H*-pyrrolo[2,3-*b*]pyridine (0.120g, 0.342mmol), *N*-(1,1-dioxidotetrahydro-3-thienyl)-4-(4,4,5,5-tetramethyl-1,3,2-dioxaborolan-2-yl)benzenesulfonamide (0.274g, 0.683mmol), potassium phosphate tribasic (0.216g, 1.02mmol) and 2-(dimethylamino)-2-biphenyl palladium chloride dinorbornyl phosphine complex (0.010g, 0.02mmol) in dioxan : water (5:1, 5ml) were heated at 150°C for 45 min in the Biotage Initiator mw. Sodium hydroxide (0.050g, 1.7mmol) was added and the mixture heated at 120°C for 1.5 hrs in the Biotage Initiator mw. Sodium hydroxide (0.050g, 1.7mmol) was added and heating at 120°C was continued for a further 2 hrs. The mixture was reduced *in vacuo*, diluted with water (35ml) and extracted with DCM (3x25ml). The combined organic extracts were dried (phase separator), reduced *in vacuo* and purified in 2 batches by MDAP to give the title compound as a dark gum (0.022g, 16%).
MH+406, rt= 2.57 min

### Example 106

### N-(2-Hydroxyethyl)-4-(2-methyl-1H-pyrrolo[2,3-b]pyridin-4-yl)benzenesulfonamide

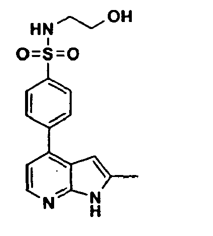

2-Methyl-1-(phenylsulfonyl)-4-(4,4,5,5-tetramethyl-1,3,2-dioxaborolan-2-yl)-1*H*-pyrrolo[2,3-b]pyridine (0.200g, 0.5mmol), 2-(dimethylamino)-2-biphenyl palladium (II) chloride dinorbornyl phosphine (0.014g, 0.025mmol), potassium phosphate tribasic (0.320g, 1.5mmol) and 4-bromo-*N*-(2-hydroxyethyl)benzenesulfonamide (0.280g, 1mmol) in dioxan/water (5:1, 5ml) were heated in the Biotage Initiator mw at 150°C for 30 min. Sodium hydroxide (0.085g, 10eq) was introduced to the mw vial and the mixture heated in the Biotage Initiator mw at 150°C for 1 hour. The mixture was reduced *in vacuo,* diluted with water (30ml) and brine (10ml) and extracted with DCM (3x30ml). The combined organic extracts were dried (phase separator) and reduced *in vacuo* and purified by MDAP to afford the title compound as a yellow solid (0.0295g, 18%).
MH+332, rt= 2.39 min

### Example 107

### 4-[2-(1,1-Dimethylethyl)-1H-pyrrolo[2,3-b]pyridin-4-yl]-N-(1,1-dioxidotetrahydro-3-thienyl)benzenesulfonamide trifluoroacetate

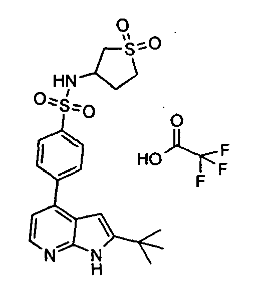

A solution of 4-chloro-2-(1,1-dimethylethyl)-1*H*-pyrrolo[2,3-*b*]pyridine (21 mg, 0.1mmol) in dioxan (0.5mL) was added to a microwave vessel containing 2'(dimethylamino)-2-biphenyl-palladium II chloride dinorbornylphosphine complex (0.25mg, 0.5 mol%). A solution of *N*-(1,1-dioxidotetrahydro-3-thienyl)-4-(4,4,5,5-tetramethyl-1,3,2-dioxaborolan-2-yl)benzenesulfonamide (60mg, 0.15mmol) in dioxan (0.5mL) was added followed by a solution of potassium phosphate (64mg, 0.3mmol) in water (200µl). The reaction mixture was heated at 130°C in the microwave for 30 minutes. The reaction mixture was applied directly to a C18 cartridge (500mg) and eluted with 0.1% TFA in acetonitrile (3 x 1mL). Purification by mass directed HPLC gave the title compound.
LCMS RT= 3.07 min ES+ve 448 m/z (MH)⁺

Examples 108 to 110 were similarly prepared:

| **Example** | Compound | LCMS | m/z |
|---|---|---|---|
| | | rt, min | MH+ |
| **108** | 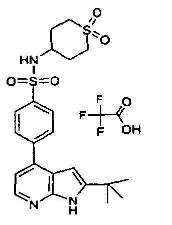 | 3.06 | 462 |
| | 4-[2-(1,1-dimethylethyl)-1*H*-pyrrolo[2,3-*b*]pyridin-4-yl]-*N*-(1,1-dioxidotetrahydro-2*H*-thiopyran-4-yl)benzenesulfonamide trifluoroacetate | | |
| **109** | 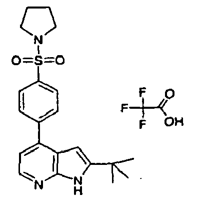 | 3.51 | 384 |
| | 2-(1,1-dimethylethyl)-4-[4-(1-pyrrolidinylsulfonyl)phenyl]-1*H*-pyrrolo[2,3-*b*]pyridine trifluoroacetate | | |
| **110** | 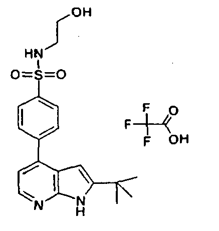 | 2.92 | 373 |
| | 4-[2-(1,1-dimethylethyl)-1*H*-pyrrolo[2,3-*b*]pyridin-4-yl]-*N*-(2-hydroxyethyl)benzenesulfonamide trifluoroacetate | | |

Similarly prepared were the compounds in the table below at the temperatures* indicated:

| **Example** | Compound | Compound Name | LCMS | MH+ | Temp* |
|---|---|---|---|---|---|
| | Structure | | rt, mins | | °C |
| **111** | 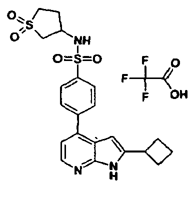 | 4-(2-cyclobutyl-1H-pyrrolo[2,3-b]pyridin-4-yl)-N-(1,1-dioxidotetrahydro-3-thienyl)benzenesulfonamide trifluoroacetate | 3.06 | 446 | 130 |
| **112** | 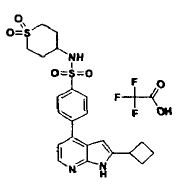 | 4-(2-cyclobutyl-1H-pyrrolo[2,3-b]pyridin-4-yl)-N-(1,1-dioxidotetrahydro-2H-thiopyran-4-yl)benzenesulfonamide trifluoroacetate | 3.03 | 460 | 130 |
| **113** | 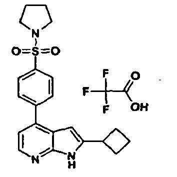 | 2-cyclobutyl-4-[4-(1-pyrrolidinylsulfonyl)phenyl]-1H-pyrrolo[2,3-b]pyridine trifluoroacetate | 3.52 | 382 | 130 |
| **114** | 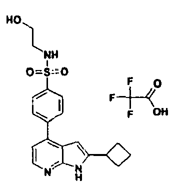 | 4-(2-cyclobutyl-1H-pyrrolo[2,3-b]pyridin-4-yl)-N-(2-hydroxyethyl)benzenesulfona mide trifluoroacetate | 2.9 | 372 | 130 |
| **115** | 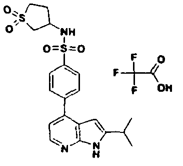 | N-(1,1-dioxidotetrahydro-3-thienyl)-4-[2-(1-methylethyl)-1H-pyrrolo[2,3-b]pyridin-4-yl]benzenesulfonamide trifluoroacetate | 2.97 | 434 | 130 |
| **116** | 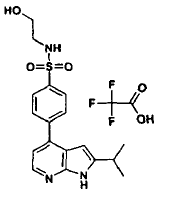 | N-(2-hydroxyethyl)-4-[2-(1-methylethyl)-1H-pyrrolo[2,3-b]pyridin-4-yl]benzenesulfonamide trifluoroacetate | 2.8 | 360 | 130 |
| **117** | 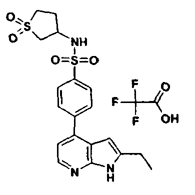 | N-(1,1-dioxidotetrahydro-3-thienyl)-4-(2-ethyl-1H-pyrrolo[2,3-b]pyridin-4-yl)benzenesulfonamide trifluoroacetate | 2.83 | 420 | 130 |
| **118** | 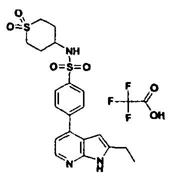 | N-(1,1-dioxidotetrahydro-2H-thiopyran-4-yl)-4-(2-ethyl-1H-pyrrolo[2,3-b]pyridin-4-yl)benzenesulfonamide trifluoroacetate | 2.8 | 434 | 130 |
| **119** | 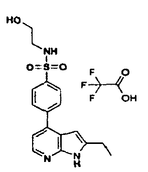 | 4-(2-ethyl-1H-pyrrolo[2,3-b]pyridin-4-yl)-N-(2-hydroxyethyl)benzenesulfona mide trifluoroacetate | 2.64 | 346 | 130 |
| **120** | 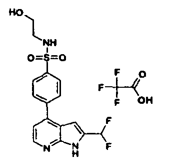 | 4-[2-(difluoromethyl)-1H-pyrrolo[2,3-b]pyridin-4-yl]-N-(2-hydroxyethyl)benzenesulfona mide trifluoroacetate | 2.63 | 368 | 130 |
| **121** | 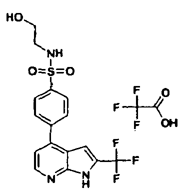 | N-(2-hydroxyethyl)-4-[2-(trifluoromethyl)-1H-pyrrolo[2,3-b]pyridin-4-yl]benzenesulfonamide trifluoroacetate | 2.86 | 386 | 120 |
| **122** | 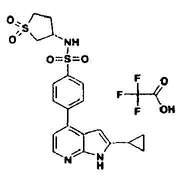 | 4-(2-cyclopropyl-1H-pyrrolo[2,3-b]pyridin-4-yl)-N-(1,1-dioxidotetrahydro-3-thienyl)benzenesulfonamide trifluoroacetate | 2.84 | 432 | 120 |
| **123** | 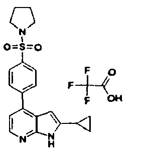 | 2-cyclopropyl-4-[4-(1-pyrrolidinylsulfonyl)phenyl]-1H-pyrrolo[2,3-b]pyridine trifluoroacetate | 3.28 | 368 | 120 |
| **124** | 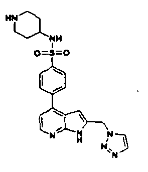 | N-4-piperidinyl-4-[2-(1H-1,2,3-triazol-1-ylmethyl)-1H-pyrrolo[2,3-b]pyridin-4-yl]benzenesulfonamide | 2.09 | 438 | 120 |
| **125** | 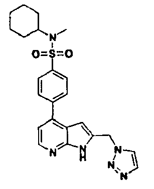 | N-cyclohexyl-N-methyl-4-[2-(1H-1,2,3-triazol-1-ylmethyl)-1H-pyrrolo[2,3-b]pyridin-4-yl]benzenesulfonamide | 3.25 | 451 | 120 |
| **126** | 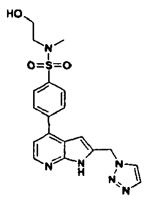 | N-(2-hydroxyethyl)-N-methyl-4-[2-(1H-1,2,3-triazol-1-ylmethyl)-1H-pyrrolo[2,3-b]pyridin-4-yl]benzenesulfonamide | 2.52 | 413 | 120 |
| **127** | 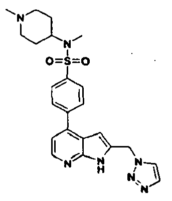 | N-methyl-N-(1-methyl-4-piperidinyl)-4-[2-(1H-1,2,3-triazol-1-ylmethyl)-1H-pyrrolo[2,3-b]pyridin-4-yl]benzenesulfonamide | 2.18 | 466 | 120 |
| **128** | 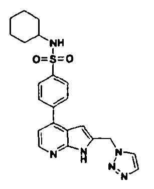 | N-cyclohexyl-4-[2-(1H-1,2,3-triazol-1-ylmethyl)-1H-pyrrolo[2,3-b]pyridin-4-yl]benzenesulfonamide | 3.08 | 437 | 120 |
| **129** | 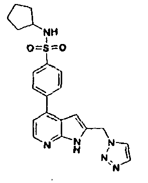 | N-cyclopentyl-4-[2-(1H-1,2,3-triazol-1-ylmethyl)-1H-pyrrolo[2,3-b]pyridin-4-yl]benzenesulfonamide | 2.96 | 423 | 120 |
| **130** | 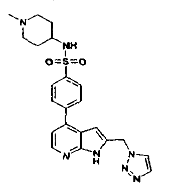 | N-(1-methyl-4-piperidinyl)-4-[2-(1H-1,2,3-triazol-1-ylmethyl)-1H-pyrrolo[2,3-b]pyridin-4-yl]benzenesulfonamide | 2.08 | 452 | 120 |
| **131** | 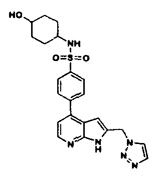 | N-(4-hydroxycyclohexyl)-4-[2-(1H-1,2,3-triazol-1-ylmethyl)-1H-pyrrolo[2,3-b]pyridin-4-yl]benzenesulfonamide | 2.51 | 453 | 120 |
| **132** | 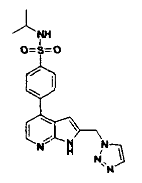 | N-(1-methylethyl)-4-[2-(1H-1,2,3-triazol-1-ylmethyl)-1H-pyrrolo[2,3-b]pyridin-4-yl]benzenesulfonamide | 2.78 | 397 | 120 |
| **133** | 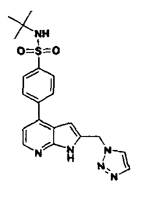 | N-(1,1-dimethylethyl)-4-[2-(1H-1,2,3-triazol-1-ylmethyl)-1H-pyrrolo[2,3-b]pyrid in-4-yl]benzenesulfonamide | 2.88 | 411 | 120 |
| **134** | 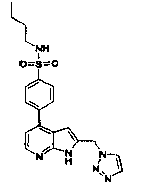 | N-butyl-4-[2-(1H-1,2,3-triazol-1-ylmethyl)-1H-pyrrolo[2,3-b]pyridin-4-yl]benzenesulfonamide | 2.97 | 411 | 120 |
| **135** | 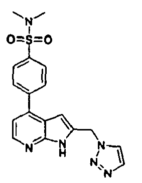 | N,N-dimethyl-4-[2-(1H-1,2,3-triazol-1-ylmethyl)-1H-pyrrolo[2,3-b]pyridin-4-yl]benzenesulfonamide | 2.76 | 383 | 120 |

### Example 136

### Example 136a

### N-(2-Aminoethyl)-4-[2-(difluoromethyl)-1H-pyrrolo[2,3-b]pyridin-4-yl]benzenesulfonamide

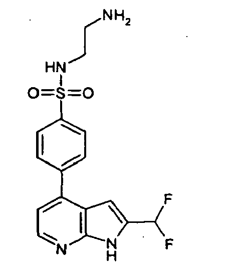

### Method A

N-(2-Aminoethyl)-4-[2-(difluoromethyl)-1-(phenylsulfonyl)-1H-pyrrolo[2,3-b]pyridin-4-yl]benzenesulfonamide (350mg, 0.691mmol) in tetrahydrofuran (10mL) was treated with 1 M tetrabutylammonium fluoride in tetrahydrofuran (2mL, 2.07mmol). The reaction mixture was left in solution for 16h and then eluted through an ion exchange column (20g, type SCX). The column was washed with MeOH (150mL) and the desired product released by 2M ammonia in MeOH elution (200mL). The solvent was removed under vacuum and the solid purified by autopreparative HPLC. The desired fractions were combined and concentrated under vacuum. The colourless residue was dissolved in MeOH (3mL) and eluted through an ion exchange column (2g, type NH2). The column was washed with MeOH (20mL) and the fraction concentrated to afford the desired compound as a white solid (103mg).
LCMS rt = 2.43mins, MH+=367

### Method B

A mixture of 1,1-dimethylethyl {2-[({4-[2-(difluoromethyl)-1H-pyrrolo[2,3-b]pyridin-4-yl]phenyl}sulfonyl)amino]ethyl}carbamate (342 mg, 0.735 mmol) and trifluoroacetic acid (283 µL, 3.674 mmol) was stirred at room temperature under nitrogen atmosphere for 10 hours. Trifluoroacetic acid (283 µL, 3.674 mmol) was added and the mixture was stirred at room temperature under nitrogen atmosphere for further 10 hours. Trifluoroacetic acid (283 µL, 3.674 mmol) was added and the mixture was stirred at room temperature under nitrogen atmosphere for further 6 hours. The reaction mixture was partitioned between a saturated solution of sodium carbonate (30 mL) and dichloromethane (300 mL), controlling the pH of the aqueous phase to 11. The organic layer was dried (hydrophobic frit) and concentrated under vacuum. The dry residue was purified by chromatography on silica (FlashMaster) using a gradient of methanol/dichloromethane/1% triethylamine (0-15%). After concentration of the desired fractions under vacuum, the residue was further purified by to afford, after evaporation of the solvents a semi pure compound, further purified by autopreparative HPLC to afford, after concentration of the solvent, the title compound (8.2 mg).
LCMS rt = 2.47 mins, MH+ = 367

### Example 136b

### N-(2-Aminoethyl)-4-[2-(difluoromethyl)-1H-pyrrolo[2,3-b]pyridin-4-yl]benzenesulfonamide trifluoroacetate

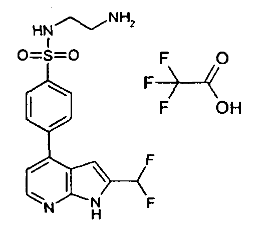

### Method A

To a solution of 1,1-dimethylethyl {2-[({4-[2-(difluoromethyl)-1*H*-pyrrolo[2,3-*b*]pyridin-4-yl]phenyl}sulfonyl)amino]ethyl}carbamate (160mg, 0.34 mmol) in DCM (3 ml) was added TFA (3 ml) and the solution allowed to stand for 2h. The reaction mixture was evaporated to dryness and triturated with ether (2 x 50 mL), dried *in vacuo* at 50°C for 4h to give the title compound as a cream solid (110 mg).
LCMS rt=2.36 mins, MH+=367

### Method B

Example 136b was prepared similarly to Example 147 Method B at a temperature* of 130°C.
LCMS rt=2.14mins, MH+ = 367

### Example 136c

### Formic acid - N-(2-Aminoethyl)-4-[2-(difluoromethyl)-1H-pyrrolo[2,3-b]pyridin-4-yl]benzenesulfonamide (1:1)

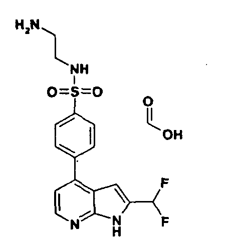

Example 136c was prepared similarly to Example 154 but prior to MDAP the sample was suspended in 1:1 TFA:DCM (1 mL) to afford the deprotected material.
LCMS rt=2.19 mins, MH+ 367

### Example 137

### Example 137a

### N-(2-Aminoethyl)-4-[2-(trifluoromethyl)-1H-pyrrolo[2,3-b]pyridin-4-yl]benzenesulfonamide

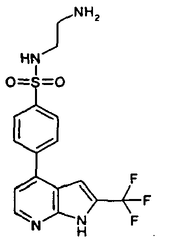

[2-(Trifluoromethyl)-1H-pyrrolo[2,3-b]pyridin-4-yl]boronic acid (500mg, 2.183mmol) and 1,1-dimethylethyl (2-{[(4-bromophenyl)sulfonyl]amino}ethyl)carbamate (993mg, 2.620mmol), bis(diphenylphosphino)ferrocene palladium (II) chloride (178mg, 0.218mmol) and a solution of 1 M sodium hydrogenocarbonate (6.56mL, 6.54mmol) in isopropanol (13mL) were heated in the Biotage Initiator mw at 120°C for 30 mins in a sealed vial. The reaction mixture was partitioned between DCM (30ml) and water (30ml). The suspension which had appeared was filtered off under vacuum and the white solid washed with water (2x5mL). The solid was dried and then suspended in DCM (10mL) and treated with trifluoroacetic acid (1 mL). The reaction mixture was left into solution for 16h. The reaction mixture was partitioned between DCM (30ml) and a saturated solution of sodium carbonate to reach pH=7 in the aqueous layer. The suspension was filtered under vacuum and the solid washed with water (5mL) and dried under vacuum. The compound was dissolved in DCM:MeOH (1:1, 10mL) and passed through an ion exchange column (5g, type NH2). The column was washed with 2 volumes of MeOH:DCM (1:1) and the fraction concentrated under vacuum to afford the title compound as a white solid (408mg).
LCMS rt = 2.53mins, MH+=385

### Example 137b

### N-(2-Aminoethyl)-4-[2-(trifluoromethyl)-1H-pyrrolo[2,3-b]pyridin-4-yl]benzenesulfonamide trifluoroacetate

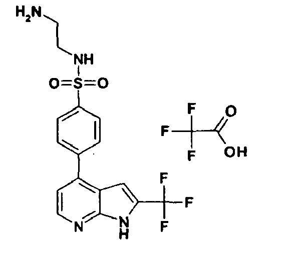

Example 137b was prepared similarly to Example 147 Method B at a temperature* of 120°C.
LCMS RT=2.29, MH+ 385

### Example 138

### N-(1-Methylethyl)-2-(4-{4-[(methylsulfonyl)amino]phenyl}-1H-pyrrolo[2,3-b]pyridin-2-yl)acetamide trifluoroacetate

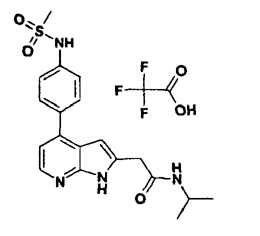

Example 138 was prepared similarly to Example 16b.
LCMS rt=2.46 mins, MH+=387

### Example 139

### N-({4-[4-(1-Pyrrolidinylsulfonyl)phenyl]-1H-pyrrolo[2,3-b]pyridin-2-yl}methyl)-3-pyridinamine trifluoroacetate

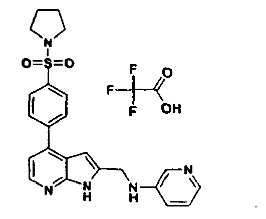

Example 139 was prepared similarly to Example 28.
LCMS rt = 2.38 mins, MH+=434

### Example 140

### Example 140a

### N-4-Piperidinyl-4-[2-(trifluoromethyl)-1H-pyrrolo[2,3-b]pyridin-4-yl]benzenesulfonamide dihydrochloride

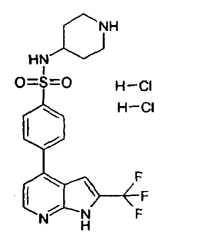

1,1-Dimethylethyl 4-[({4-[2-(trifluoromethyl)-1*H*-pyrrolo[2,3-*b*]pyridin-4-yl]phenyl}-sulfonyl)amino]-1-piperidinecarboxylate (160mg, 0.0003mole) was dissolved in concentrated hydrochloric acid (3ml) and stirred for 30 minutes at room temperature. The reaction mixture was evaporated to dryness, toluene (40ml) added and the mixture evaporated to dryness. This procedure was repeated with ethyl acetate (2x40ml) and the residual gum taken up in methanol (20ml) and evaporated to dryness. The residual foam/gum was triturated under anhydrous ether (20ml) for 2hrs. The solid was filtered off washed with ether and dried *in vacuo* to furnish the title compound (bis hydrochloride salt) as an off white solid (143mg, 96%). LCMS rt = 2.30 mins, MH+ = 425. Purity 94/95%.

### Example 140b

### N-4-Piperidinyl-4-[2-(trifluoromethyl)-1H-pyrrolo[2,3-b]pyridin-4-yl]benzenesulfonamide hydrochloride

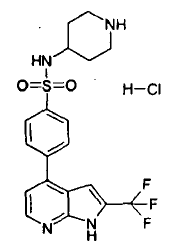

A sample of *N*-4-piperidinyl-4-[2-(trifluoromethyl)-1*H*-pyrrolo[2,3-*b*]pyridin-4-yl]benzenesulfonamide dihydrochloride (80mg) was further purified by crystallization from methanol (5ml). The solid was filtered and dried to give title compound (mono hydrochloride salt) as a colourless solid (30mg).
LCMS rt = 2.30min, MH+ = 425

### Example 140c

### Formic acid - N-4-Piperidinyl-4-[2-(trifluoromethyl)-1H-pyrrolo[2,3-b]pyridin-4-yl]benzenesulfonamide

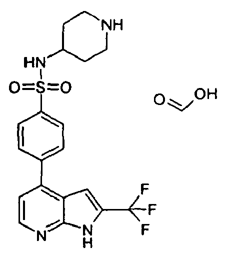

Pentafluorophenyl 4-(4,4,5,5-tetramethyl 1,1,3,2-dioxaboran-2-yl)benzenesulphonate (340mg, 0.75mmol), triethylamine (128ul, 1mmol), boc-1-aminopiperidine (200mg, 1mmol) and dioxan (1ml) were heated in a Biotage Initiator at 120°C for fifteen minutes. The mixture was treated with chloro[2'-(dimethylamino)-2-biphenylyl]palladium - (1*R*,4*S*)-bicyclo[2.2.1]hept-2-yl[(1*S*,4*R*)-bicyclo[2.2.1]hept-2-yl]phosphane (1:1) (38mg, 0.0075mmol), 4-chloro-2-(trifluoromethyl)-1*H*-pyrrolo[2,3-*b*]pyridine (110mg, 0.5mmol), potaaium phosphate (160mg, 0.75mmol) and 4:1 dioxan/water (4ml). The mixture was heated at 120°C for thirty minutes in the microwave. The mixture was blown down under nitrogen. The mixture was treated with 1:1 trifluoroacetic acid and DCM (5ml) for an hour then evaporated. The gum was dissolved in 1:1 methanol/DMSO and purified by MDAP. The main fraction was evaporated to give a cream solid (64mg).
LCMS rt = 0.85 min, MH+ 425

Examples 141 to 143 were similarly prepared:

| **Example** | Compound | Compound Name | LCMS | MH+ |
|---|---|---|---|---|
| | Structure | | rt, | |
| | | | mins | |
| **141** | 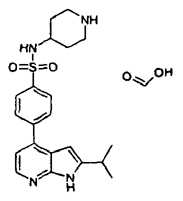 | formic acid-4-[2-(1-methylethyl)-1*H*-pyrrolo[2,3-*b*]pyridin-4-yl]-*N*-4-piperidinylbenzenesulfonamide | 0.88 | 399 |
| **142** | 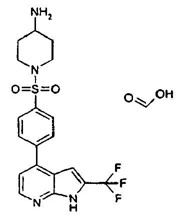 | formic acid-1-({4-[2-(trifluoromethyl)-1*H*-pyrrolo[2,3-*b*]pyridin-4-yl]phenyl}sulfonyl)-4-piperidinamine | 0.87 | 425 |
| **143** | 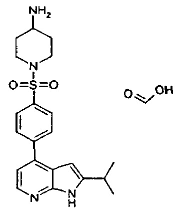 | formic acid-1-({4-[2-(1-methylethyl)-1*H*-pyrrolo[2,3-b]pyridin-4-yl]phenyl}sulfonyl)-4-piperidinamine | 0.89 | 399 |

### Example 144

### Example 144a

### 4-[2-(1,1-Dimethylethyl)-1H-pyrrolo[2,3-b]pyridin-4-yl]-N-4-piperidinylbenzenesulfonamide dihydrochloride

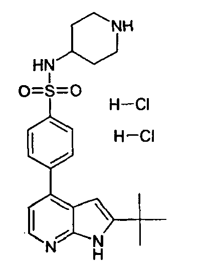

1,1-Dimethylethyl 4-[({4-[2-(1,1-dimethylethyl)-1*H*-pyrrolo[2,3-*b*]pyridin-4-yl]phenyl}sulfonyl)amino]-1-piperidinecarboxylate (292mg, 0.00057mole) was dissolved in concentrated hydrochloric acid (3ml) and stirred for 2h. The reaction mixture was evaporated to dryness, toluene (40ml) added and the mixture evaporated to dryness. This procedure was repeated with ethyl acetate (2x40ml) and the residual gum taken up in methanol (20ml) and evaporated to dryness. The residual foam/gum was triturated under anhydrous ether (20ml) for 2h. The solid was filtered off washed with ether and dried *in vacuo* to furnish the title compound (bis hydrochloride salt) as a yellow solid (270mg, 97%). LCMS rt = 0.90min, MH+ = m/z = 413. Purity 96/97%.

### Example 144b

### Formic acid - 4-[2-(1,1-Dimethylethyl)-1H-pyrrolo[2,3-b]pyridin-4-yl]-N-4-piperidinylbenzenesulfonamide (1:1)

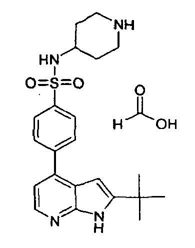

A sample of *N*-4-piperidinyl-4-[2-(trifluoromethyl)-1*H*-pyrrolo[2,3-*b*]pyridin-4-yl]benzenesulfonamide hydrochloride (90mg) was further purified by mass directed autoprep. The appropriate fractions were combined and evaporated to dryness to give title compound (formate salt) as a colourless solid (53mg).
LCMS rt = 0.90min, MH+= 413

### Example 145

### Example 145a

### 4-(2-Cyclopropyl-1H-pyrrolo[2,3-b]pyridin-4-yl)-N-(2-hydroxyethyl)-benzenesulfonamide

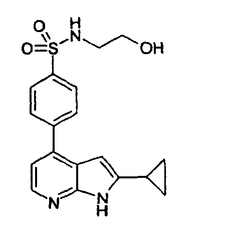

### Method A

A suspension of 4-chloro-2-cyclopropyl-1*H*-pyrrolo[2,3-*b*]pyridine (0.25g, 1.3mmol), potassium phosphate (0.272g, 1.3mmol), *N*-(2-hydroxyethyl)-4-(4,4,5,5-tetramethyl-1,3,2-dioxaborolan-2-yl)benzenesulfonamide (0.509g, 1.56mmol) and 2'-(dimethylamino)-2-biphenyl-palladium(II) chloride dinorbonylphosphine complex (0.068g, ca 10 mol%) in 5:1 dioxane:water (18mL) was degassed for 10 mins before being heated to 120°C in a microwave for 30 mins (Biotage Initiator). The reaction was diluted with water (20mL) and extracted with 1:1 chloroform/ethylacetate (3x50mL). The combined extracts were dried (hydrophobic frit) and concentrated *in vacuo* to a brown oil. Purification by chromatography (preabsorption onto Florosil^{™} (60-100 mesh) followed by silica 100g, 0-100% ethylacetate in cyclohexane - 50% methanol/dichloromethane 60 mins) removed some impurities. Further chromatography (silica 100g, 0-20% methanol in dichloromethane 60 mins) gave a peach coloured solid. Further purification by MDAP gave the title compound (30mg).

LCMS rt = 2.69 mins, MH+ = 358. NMR (400MHz, CD₃OD) δ : 11.7 (1H, s), 8.18 (1H, d), 7.94 (4H, m), 7.71 (1H, bs), 7.18 (1H, d), 6.34 (1H, s), 4.72 (1H, t), 3.41 (2H, q), 2.85 (2H, t), 2.07 (1H, m), 1.00 (2H, m), 0.89 (2H, m).

### Method B

The reaction was carried out in two batches. In each batch, 4-chloro-2-cyclopropyl-1*H-*pyrrolo[2,3-b]pyridine (0.5g, 2.59mmol), potassium phosphate (0.504g, 2.59mmol), *N*-(2-hydroxyethyl)-4-(4,4,5,5-tetramethyl-1,3,2-dioxaborolan-2-yl)benzenesulfonamide (1.0g, 3.125mmol) and 2'-(dimethylamino)-2-biphenyl-palladium(II) chloride dinorbonylphosphine complex (0.136g, 10mol%) in 5:1 dioxane:water (20mL) were heated to 120°C for 30 mins (Biotage Initiator, very high absorbtion setting). The combined batches were diluted with water (80mL) and was extracted with 1:1 chloroform/ethylacetate (2x200mL). The combined extracts were dried (hydrophobic frit) and concentrated *in vacuo* to a brown solid. Preabsorption onto Florosil^{™} (60-100 mesh) followed by chromatography (silica 100g, 0-25% methanol in dichloromethane 60mins) gave a brown solid. Trituration with diethyl ether: dichloromethane (1:1) gave the title compound as a beige solid, collected by filtration and dried *in vacuo* (1.033g, 55.7%).
LCMS rt = 2.69 mins, MH⁺ = 358

4-(2-Cyclopropyl-1*H*-pyrrolo[2,3-*b*]pyridin-4-yl)-*N*-(2-hydroxyethyl)benzenesulfonamide (194 mg) was recrystalised from isopropanol to give the title compound as pale yellow crystals (150 mg, 77% recovery).

### Example 145b

### 4-(2-Cyclopropyl-1H-pyrrolo[2,3-b]pyridin-4-yl)-N-(2-hydroxyethyl)-benzenesulfonamide hydrochloric acid salt

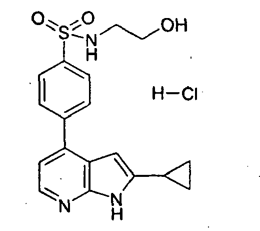

To a solution of 4-(2-cyclopropyl-1*H*-pyrrolo[2,3-*b*]pyridin-4-yl)-*N*-(2-hydroxyethyl)benzenesulfonamide (67mg, 0.187mmol) in methanol (1mL) was added 4M hydrochloric acid in dioxane (0.25mL). The resultant yellow solution was concentrated (nitrogen blowdown) to give the title compound as a yellow solid (75mg, 100%).
LCMS rt = 2.69mins, MH+ = 358.

### Example 145c

### 4-(2-Cyclopropyl-1H-pyrrolo[2,3-b]pyridin-4-yl)-N-(2-hydroxyethyl)-benzenesulfonamide trifluoroacetate

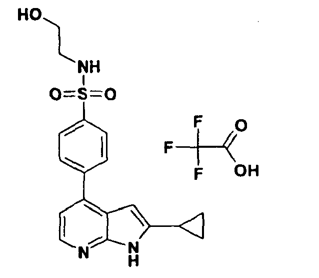

Example 145c was prepared similarly to Example 107 at a temperature of 120°C. LCMS rt=2.66mins, MH+ = 358

### Example 146

### Example 146a

### N-(2-Aminoethyl)-4-(2-ethyl-1H-pyrrolo[2,3-b]pyridin-4-yl)benzene-sulfonamide formate salt

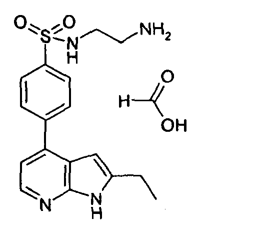

Impure 1,1-dimethylethyl [2-({[4-(2-ethyl-1*H*-pyrrolo[2,3-*b*]pyridin-4-yl)phenyl]sulfonyl}amino)ethyl]carbamate (525mg) was treated with 1:1 DCM/TFA (10ml) and stirred at room temperature for 30 minutes. The brown solution was evaporated and purified by mass directed autoprep. The main fraction was evaporated to give a creamy yellow solid which was dried *in vacuo* (127mg).
LCMS rt = 0.72 min, MH+ 345. NMR (400MHz, δ₆ DMSO) δ : 11.7 (1H, s), 8.28 (1H, s), 8.22 (1H, d), 7.99 (2H, d), 7.95 (2H, d), 7.19 (1H, d), 6.38 (1H, s), 3.70 (brs), 2.97 (2H, t), 2.82 (2H, t), 2.77 (2H, q), 1.29 (3H, t).

### Example 146b

### N-(2-Aminoethyl)-4-(2-ethyl-1H-pyrrolo[2,3-b]pyridin-4-yl)benzenesulfonamide trifluoroacetate

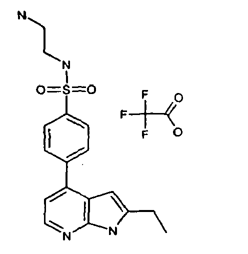

Example 146b was prepared similarly to Example 147 Method B at a temperature of 130°C.
LCMS rt=2.13mins, MH+ = 345

### Example 146c

### N-(2-Aminoethyl)-4-(2-ethyl-1H-pyrrolo[2,3-b]pyridin-4-yl)benzenesulfonamide bis-hydrochloride salt

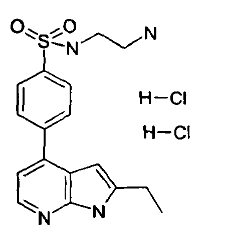

1,1-Dimethylethyl [2-({[4-(2-ethyl-1*H*-pyrrolo[2,3-*b*]pyridin-4-yl)phenyl]sulfonyl}amino)-ethyl]carbamate (7.2g) was dissolved in 1:1 DCM/Dioxan (150ml) and treated with 4M-HCl in dioxan (100ml). The reaction was stirred at room temperature for two hours. The reaction was reduced by evaporation and then diluted with ethanol and the pale yellow solid collected (5.25g).
LCMS rt= 0.71 min, MH+ = 345

### Example 146d

### N-(2-Aminoethyl)-4-(2-ethyl-1H-pyrrolo[2,3-b]pyridin-4-yl)benzenesulfonamide hydrochloride

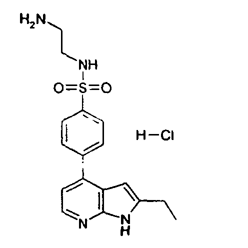

To *N*-(2-aminoethyl)-4-(2-ethyl-1*H*-pyrrolo[2,3-*b*]pyridin-4-yl)benzenesulfonamide (300 mg), IPA (20 ml) was added. The reaction was left stirring at 60°C for ∼3 hours in an attempt to dissolve the freebase. HCl (75 µl, 1.05 eq) was added to the slurry. The reaction was left stirring at 60°C for ∼15 mins before temperature cycling 0-40°C for 2 days. A white solid had formed. The white solid was isolated, washed with IPA (∼1mL) and air dried for - half an hour before drying it *in vacuo* overnight at ambient temperature. Yield = 277.6 mg.

### Example 146e

### N-(2-Aminoethyl)-4-(2-ethyl-1H-pyrrolo[2,3-b]pyridin-4-yl)benzenesulfonamide

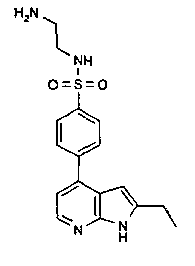

To a solution of 1,1-dimethylethyl [2-({[4-(2-ethyl-1*H*-pyrrolo[2,3-*b*]pyridin-4-yl)phenyl]sulfonyl}amino)ethyl]carbamate (52.73g) in 1,4-dioxane (527ml) and dichloromethane (527ml) was added 4M HCl in 1,4-dioxane (400ml). The resulting suspension was then stirred overnight at room temperature under an N₂ atmosphere. The suspension was then filtered and the solid dried *in vacuo* at 35°C for 2hrs to give a yellow solid (49.23g). The *N*-(2-aminoethyl)-4-(2-ethyl-1*H*-pyrrolo[2,3-*b*]pyridin-4-yl)benzenesulfonamide dihydrochloride (47.84g) was then dissolved in methanol (310ml) and water (96ml) and the solution treated with 2M NaOH (115ml). The resulting suspension was stirred for a further 2hrs. The solid was collected by filtration and was washed well with water. It was then dried *in vacuo* at 35°C overnight to give a cream solid (25.88g). Another crop of solid was isolated from the filtrate. It was collected by filtration and was washed well with water. It was dried *in vacuo* overnight at 35°C to give a cream solid (7.56g). A portion of impure *N*-(2-aminoethyl)-4-(2-ethyl-1*H*-pyrrolo[2,3-*b*]pyridin-4-yl)benzenesulfonamide (25.5g) was then suspended in methanol (250ml) and the resulting mixture heated to reflux and maintained at that temperature for 30mins then treated with a crystalline seed (see below) then allowed to cool to room temperature and stirredd at that temperature overnight. The solid was then collected by filtration and was washed sparingly with ice-cold methanol. It was then dried *in vacuo* at 35°C over the weekend to give a cream solid (19.19g). A portion of this material (14.4g) was dissolved in DMSO (70ml) and then was treated with 3-mercaptopropyl-functionalised silca gel (14g) and the resulting mixture heated to 60°C and maintained at that temperature overnight with overhead agitation. The mixture was then filtered through celite and the silica was washed well with DMSO (50ml). The solution was then treated with cold water (240ml) in small portions. The resulting suspension was stirred for 1 hr with cooling then the solid was collected by filtration and was washed well with water. It was then dried *in vacuo* overnight at 40°C to yield the title compound as a cream coloured solid (13.46g).
LCMS rt = 2.36min, MH+ 345.
NMR (400MHz, δ₄-MeOH) δ : 8.17 (1H, d), 8.00 (2H, d), 7.94 (2H, d), 7.19 (1H, d), 6.39 (1H, d), 2.97 (2H, t), 2.84 (2H, q), 2.70 (2H, t), 1.34 (3H, t).

### Preparation of crystalline N-(2-aminoethyl)-4-(2-ethyl-1H-pyrrolo[2,3-b]pyridin-4-yl)benzenesulfonamide for use as a seed:

Impure *N*-(2-aminoethyl)-4-(2-ethyl-1*H*-pyrrolo[2,3-*b*]pyridin-4-yl)benzenesulfonamide (2g) was suspended in methanol (60ml) and the resulting mixture heated to reflux. The resulting solution was then cooled to room temperature and stirred for 3hrs. The suspension was then filtered and the solid washed sparingly with methanol. It was then dried *in vacuo* at 35°C overnight to yield the pure seed sample of title compound.

### Example 147

### N-(2-Aminoethyl)-4-[2-(1,1-dimethylethyl)-1H-pyrrolo[2,3-b]pyridin-4-yl]benzenesulfonamide trifluoroacetate

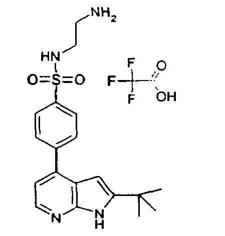

### Method A

Example 147 was prepared similarly to Example 107 but prior to purification by MDAP was treated with a solution of DCM:TFA, 1:1 and re concentrated by blow down to afford the crude product.
LCMS rt = 2.36 min, m/z MH+ = 373

### Method B

A solution of 4-chloro-2-(1,1-dimethylethyl)-1*H*-pyrrolo[2,3-*b*]pyridine (21 mg, 0.1mmol) in dioxan (0.5mL) was added to a microwave vessel containing 2'(dimethylamino)-2-biphenyl-palladium II chloride dinorbornylphosphine complex (0.25mg, 0.5 mol%). A solution of 1,1-dimethylethyl [2-({[4-(4,4,5,5-tetramethyl-1,3,2-dioxaborolan-2-yl)phenyl]sulfonyl}amino)ethyl]carbamate (60mg, 0.15mmol) in dioxan (0.5mL) was added followed by a solution of potassium phosphate (64mg, 0.3mmol) in water (200µl). The reaction mixture was heated at 130°C* in the microwave for 30 minutes. The reaction mixture was applied directly to a C18 cartridge (500mg) and eluted with 0.1% TFA in acetonitrile (3 x 1mL). Concentration by blow down followed by treatment with 1:1 TFA:DCM (1 mL) afforded the deprotected material. Concentration by blow down followed by purification by mass directed HPLC gave the title compound.
LCMS rt= 2.36min, MH+ = 373

Similarly prepared were the compounds in the table below at the temperatures* indicated:

| **Example** | Compund | Compound Name | LCMS rt, | MH+ | Temp* |
|---|---|---|---|---|---|
| | Structure | | mins | | °C |
| **148** | 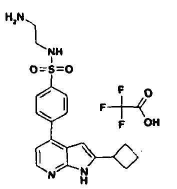 | N-(2-aminoethyl)-4-(2-cyclobutyl-1H-pyrrolo[2,3-b]pyridin-4-yl)benzenesulfonamide trifluoroacetate | 2.34 | 371 | 130 |
| **149** | 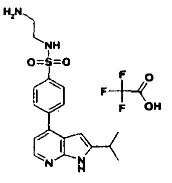 | N-(2-aminoethyl)-4-[2-(1-methylethyl)-1H-pyrrolo[2,3-b]pyridin-4-yl]benzenesulfonamide trifluoroacetate | 2.24 | 359 | 130 |
| **150** | 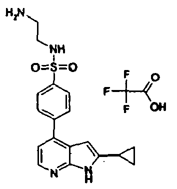 | N-(2-aminoethyl)-4-(2-cyclopropyl-1H-pyrrolo[2,3-b]pyridin-4-yl)benzenesulfonamide trifluoroacetate | 2.17 | 357 | 120 |
| **151** | 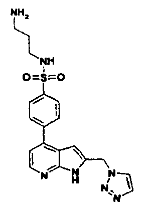 | N-(3-aminopropyl)-4-[2-(1H-1,2,3-triazol-1-ylmethyl)-1H-pyrrolo[2,3-b]pyridin-4-yl]benzenesulfonamide | 2.2 | 412 | 120 |
| **152** | 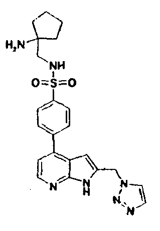 | N-[(1-aminocyclopentyl)methyl]-4-[2-(1H-1,2,3-triazol-1-ylmethyl)-1H-pyrrolo[2,3-b]pyridin-4-yl]benzenesulfonamide | 2.18 | 452 | 120 |
| **153** | 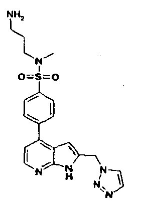 | N-(3-aminopropyl)-N-methyl-4-[2-(1H-1,2,3-triazol-1-ylmethyl)-1H-pyrrolo[2,3-b]pyridin-4-yl]benzenesulfonamide | 2.14 | 426 | 120 |

### Example 154

### N-(2-Hydroxyethyl)-N-methyl-4-(2-methyl-1H-pyrrolo[2,3-b]pyridin-4-yl)benzenesulfonamide trifluoroacetate

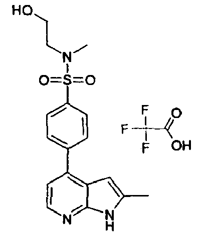

A solution of 2-(methylamino)ethanol (45mg, 0.6mmol) in dry DMF (0.5mL) was treated with triethylamine (200µL) and a solution of 4,4,5,5-tetramethyl-2-(4-{[(pentafluorophenyl)methyl]sulfonyl}phenyl)-1,3,2-dioxaborolane (67.5mg, 0.15mmol) in dry DMF (0.5mL). The reaction mixture was heated at 120°C in the microwave for 10 minutes prior to concentration *in vacuo.* The reaction mixture was resuspended in 1:1 CH₃Cl:MeOH (1mL) and applied to an SCX cartridge (1g, pre-equilibrated with 1:1 CH₃CI:MeOH) and the *N*-(2-hydroxyethyl)-*N*-methyl-4-(4,4,5,5-tetramethyl-1,3,2-dioxaborolan-2-yl)benzenesulfonamide compound eluted with 1:1 CH₃CI:MeOH. The resultant material was suspended in 5:1 dioxan:water (2mL). 1 mL of this solution was dispensed into a microwave vessel and was treated with a solution of potassium phosphate (21mg, 0.1mmol) in water (100µl), 2'(dimethylamino)-2-biphenyl-palladium II chloride dinorbornylphosphine complex (0.25mg, 0.5 mol%) and a solution of 4-bromo-2-methyl-1*H*-pyrrolo[2,3-*b*]pyridine (19mg, 0.09mmol) in dioxan (0.5mL). The reaction mixture was heated at 130°C in the microwave for 30 minutes. The reaction mixture was applied directly to a C18 cartridge (500mg) and eluted with 0.1% TFA in acetonitrile (3 x 1mL). Concentration by blow down followed by purification by mass directed HPLC gave the title compound.
LCMS rt = 2.63min, MH+ = 346

Examples 155 to 177 were similarly prepared:

| **Example** | Compound | Compound Name | LCMS | MH+ |
|---|---|---|---|---|
| | Structure | | rt, | |
| | | | mins | |
| **155** | 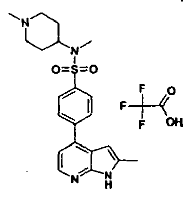 | N-methyl-N-(1-methyl-4-piperidinyl)-4-(2-methyl-1H-pyrrolo[2,3-b]pyridin-4-yl)benzenesulfonamide trifluoroacetate | 2.39 | 399 |
| **156** | 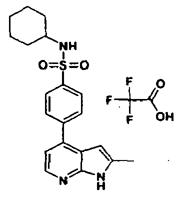 | N-cyclohexyl-4-(2-methyl-1H-pyrrolo[2,3-b]pyridin-4-yl)benzenesulfonamide trifluoroacetate | 3.33 | 370 |
| **157** | 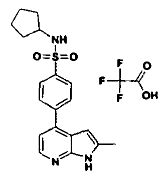 | N-cyclopentyl-4-(2-methyl-1H-pyrrolo[2,3-b]pyridin-4-yl)benzenesulfonamide trifluoroacetate | 3.21 | 356 |
| **158** | 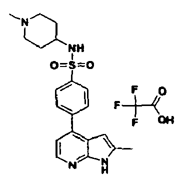 | N-(1-methyl-4-piperidinyl)-4-(2-methyl-1H-pyrrolo[2,3-b]pyridin-4-yl)benzenesulfonamide trifluoroacetate | 2.28 | 385 |
| **159** | 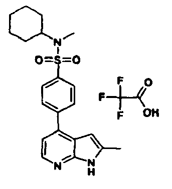 | N-cyclohexyl-N-methyl-4-(2-methyl-1H-pyrrolo[2,3-b]pyridin-4-yl)benzenesulfonamide trifluoroacetate | 3.57 | 384 |
| **160** | 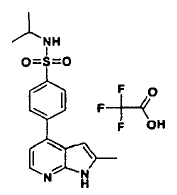 | N-(1-methylethyl)-4-(2-methyl-1H-pyrrolo[2,3-b]pyridin-4-yl)benzenesulfonamide trifluoroacetate | 1.31 | 329 |
| **161** | 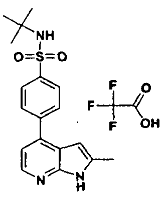 | N-(1,1-dimethylethyl)-4-(2-methyl-1H-pyrrolo[2,3-b]pyridin-4-yl)benzenesulfonamide trifluoroacetate | 3.11 | 344 |
| **162** | 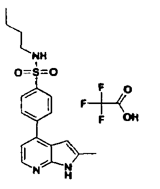 | N-butyl-4-(2-methyl-1H-pyrrolo[2,3-b]pyridin-4-yl)benzenesulfonamide trifluoroacetate | 3.22 | 344 |
| **163** | 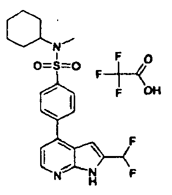 | N-cyclohexyl-4-[2-(difluoromethyl)-1H-pyrrolo[2,3-b]pyridin-4-yl]-N-methylbenzenesulfonamide trifluoroacetate | 3.57 | 420 |
| **164** | 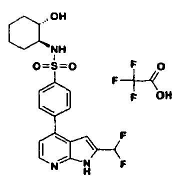 | 4-[2-(difluoromethyl)-1H-pyrrolo[2,3-b]pyridin-4-yl]-N-[(1S,2S)-2-hydroxycyclohexyl]benzenes ulfonamide trifluoroacetate | 2.74 | 422 |
| **165** | 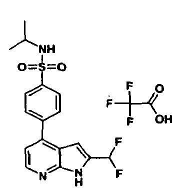 | 4-[2-(difluoromethyl)-1H-pyrrolo[2,3-b]pyridin-4-yl]-N-(1-methylethyl)benzenesulfona mide trifluoroacetate | 3.09 | 366 |
| **166** | 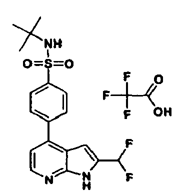 | 4-[2-(difluoromethyl)-1H-pyrrolo[2,3-b]pyridin-4-yl]-N-(1,1-dimethylethyl)benzenesulfon amide trifluoroacetate | 3.2 | 380 |
| **167** | 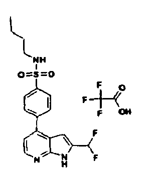 | N-butyl-4-[2-(difluoromethyl)-1H-pyrrolo[2,3-b]pyridin-4-yl]benzenesulfonamide trifluoroacetate | 3.52 | 380 |
| **168** | 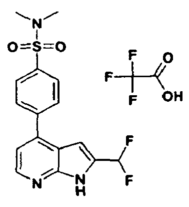 | 4-[2-(difluoromethyl)-1H-pyrrolo[2,3-b]pyridin-4-yl]-N,N-dimethylbenzenesulfonamid e trifluoroacetate | 4.43 | 352 |
| **169** | 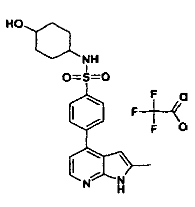 | N-(4-hydroxycyclohexyl)-4-(2-methyl-1H-pyrrolo[2,3-b]pyridin-4-yl)benzenesulfonamide trifluoroacetate | 2.63 | 386 |
| **170** | 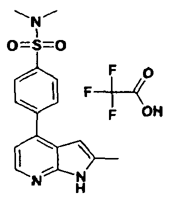 | N,N-dimethyl-4-(2-methyl-1H-pyrrolo[2,3-b]pyridin-4-yl)benzenesulfonamide trifluoroacetate | 3 | 315 |
| **171** | 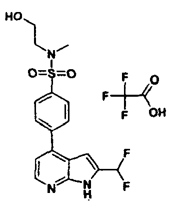 | 4-[2-(difluoromethyl)-1H-pyrrolo[2,3-b]pyridin-4-yl]-N-(2-hydroxyethyl)-N-methylbenzenesulfonamide trifluoroacetate | 2.82 | 381 |
| **172** | 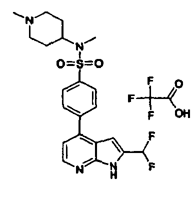 | 4-[2-(difluoromethyl)-1H-pyrrolo[2,3-b]pyridin-4-yl]-N-methyl-N-(1-methyl-4-piperidinyl)benzenesulfonam ide trifluoroacetate | 2.39 | 435 |
| **173** | 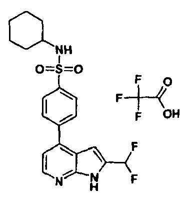 | N-cyclohexyl-4-[2-(difluoromethyl)-1H-pyrrolo[2,3-b]pyridin-4-yl]benzenesulfonamide trifluoroacetate | 3.41 | 406 |
| **174** | 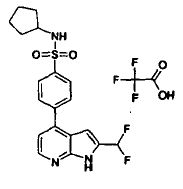 | N-cyclopentyl-4-[2-(difluoromethyl)-1H-pyrrolo[2,3-b]pyridin-4-yl]benzenesulfonamide trifluoroacetate | 3.31 | 392 |
| **175** | 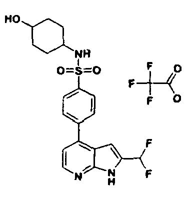 | 4-[2-(difluoromethyl)-1H-pyrrolo[2,3-b]pyridin-4-yl]-N-(4-hydroxycyclohexyl)benzenes ulfonamide trifluoroacetate | 2.79 | 422 |
| **176** | 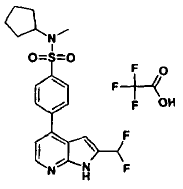 | N-cyclopentyl-4-[2-(difluoromethyl)-1H-pyrrolo[2,3-b]pyridin-4-yl]-N-methylbenzenesulfonamide trifluoroacetate | 3.49 | 405 |
| **177** | 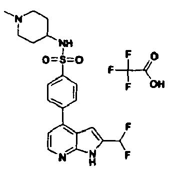 | 4-[2-(difluoromethyl)-1H-pyrrolo[2, 3-b]pyridin-4-yl]-N-(1-methyl-4-piperidinyl)benzenesulfonam ide trifluoroacetate | 2.25 | 421 |

The following compounds were prepared as above but prior to MDAP the samples were suspended in 1:1 TFA:DCM (1mL) to afford the deprotected material:

| **Example** | Compound Structure | Compound Name | LCMS rt, mins | MH+ |
|---|---|---|---|---|
| **178** | 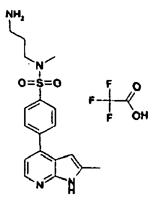 | N-(3-aminopropyl)-N-methyl-4-(2-methyl-1H-pyrrolo[2,3-b]pyridin-4-yl)benzenesulfonamide trifluoroacetate | 3.3 | 359 |
| **179** | 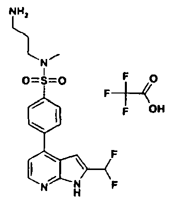 | N-(3-aminopropyl)-4-[2-(difluoromethyl)-1H-pyrrolo[2,3-b]pyridin-4-yl]-N-methylbenzenesulfonamide trifluoroacetate | 3.35 | 394 |
| **180** | 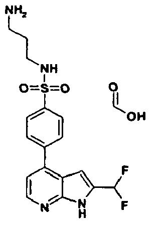 | formic acid-N-(3-aminopropyl)-4-[2-(difluoromethyl)-1H-pyrrolo[2,3-b]pyridin-4-yl]benzenesulfonamide (1:1) | 2.24 | 381 |
| **181** | 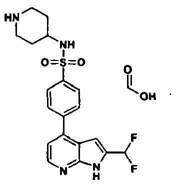 | formic acid - 4-[2-(difluoromethyl)-1H-pyrrolo[2,3-b]pyridin-4-yl]-N-4-piperidinylbenzenesulfonamide (1:1) | 2.26 | 407 |
| **182** | 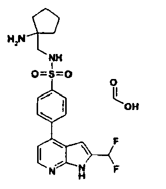 | formic acid - N-[(1-aminocyclopentyl)methyl]-4-[2-(difluoromethyl)-1H-pyrrolo[2,3-b]pyridin-4-yl]benzenesulfonamide (1:1) | 2.32 | 421 |

### Example 183

### 4-[2-(Difluoromethyl)-1H-pyrrolo[2,3-b]pyridin-4-yl]-N-(2-hydroxyethyl)benzenesulfonamide trifluoroacetate

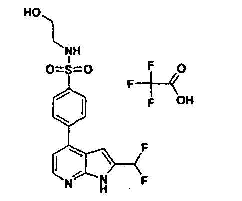

### Method A

Example 183 was prepared similarly to Example 107 at a temperature of 130°C.
LCMS rt = 2.7 mins, MH+ = 368

**Method B**

Example 183 was prepared similarly to Example 154.
LCMS rt = 2.67 mins, MH+ = 367

### Example 184

### 2-{[(5-Methyl-3-pyridinyl)oxy]methyl}-4-[4-(1-pyrrolidinylsulfonyl)phenyl]-1H-pyrrolo[2,3-b]pyridine trifluoroacetate

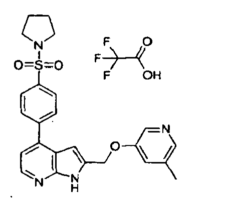

A solution of 5-methyl-3-pyridinol (22mg, 0.2mmol) in dry THF (300µL) was treated with a solution of potassium t-butoxide (1M in THF, 200µL). The reaction mixture was allowed to stand at 20°C for 5 mins prior to the addition of a solution of {4-[4-(1-pyrrolidinylsulfonyl)phenyl]-1*H*-pyrrolo[2,3-*b*]pyridin-2-yl}methyl methanesulfonate (36mg, 0.083mmol). The reaction mixture was stirred at 20°C for 14 h prior to quenching with TFA and concentration by blowdown. Purification by mass directed HPLC gave the title compound.
LCMS rt= 2.91 min, MH+ = 449

### Example 185

### 1,1-Dimethylethyl {2-[({4-[2-(difluoromethyl)-1H-pyrrolo[2,3-b]pyridin-4-yl]phenyl}sulfonyl)amino]ethyl}carbamate

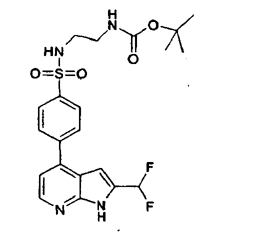

### Method A

A mixture of 1,1-dimethylethyl {2-[({4-[2-(difluoromethyl)-1-(phenylsulfonyl)-1H-pyrrolo[2,3-b]pyridin-4-yl]phenyl}sulfonyl)amino]ethyl}carbamate (221 mg, 0.365 mmol) and 1M tetrabutylammonium fluoride in THF (474 uL, 0.474 mmol) in THF (2 mL) was stirred at room temperature under nitrogen atmosphere for 3h 30 minutes. The reaction mixture was partitioned between DCM (60 mL) and water (30 mL). The organic layer was separated, dried (hydrophobic frit) and concentrated under vacuum to afford the title compound (266 mg).
LCMS rt = 3.14 mins, MH+ = 467

### Method B

To a solution of 1,1-dimethylethyl {2-[({4-[2-(difluoromethyl)-1-(phenylsulfonyl)-1*H-*pyrrolo[2,3-*b*]pyridin-4-yl]phenyl}sulfonyl)amino]ethyl}carbamate (250mg, 0.41 mmol) in THF (4 ml) was added TBAF (0.6 ml, 1M solution in THF, 0.6 mmol) dropwise. The solution was allowed to stand for 2h then poured onto a SCX column (20g) preconditioned with 50ml methanol. The column was eluted with methanol (100 ml) then 2M ammonia in methanol to elute the product. The appropriate fractions were combined and evaporated to dryness to give the title compound as a cream solid (160mg, 83%).
LCMS rt=3.14mins, MH+=467

### Example 186

### 1,1-Dimethylethyl [2-({[4-(2-ethyl-1H-pyrrolo[2,3-b]pyridin-4-yl)phenyl]sulfonyl}-amino)ethyl]carbamate

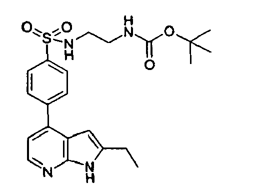

### Method A

4-Chloro-2-ethyl-1*H*-pyrrolo[2,3-*b*]pyridine (180mg, 1mmol) was added to a microwave vessel containing 2'(dimethylamino)-2-biphenyl-palladium II chloride dinorbornylphosphine complex (56mg, 0.5 mol), potassium phosphate (212mg, 0.3mmol), 1,1-dimethylethyl [2-({[4-(4,4,5,5-tetramethyl-1,3,2-dioxaborolan-2-yl)phenyl]sulfonyl}amino)ethyl]carbamate (640mg, 1.5mmol) and 5:1 dioxane-water (10ml). The reaction mixture was heated at 120°C in the microwave (Biotage Initiator) for 30 minutes. The reaction mixture was applied preadsorbed onto silica and then added to a silica SPE column and eluted with DCM to 30% ethyl acetate/DCM and then 9:1 DCM-methanol. The main fraction was evaporated to give a crude brown oil : 542mg.
LCMS rt= 1.08 min, MH+ 445

### Method B

Palladium acetate (63mg, 0.28mmol), n-butyl-di-1-adamantylphosphine (197mg, 0.55mmol), 4-chloro-2-ethyl-1*H*-pyrrolo[2,3-*b*]pyridine (1.00g, 5.554mmol), 1,1-dimethylethyl[2-({[4-(4,4,5,5-tetramethyl-1,3,2-dioxaborolan-2-yl)phenyl]sulfonyl}-amino)ethyl]carbamate (2.63g, 6.16mmol), potassium carbonate (1.07g, 7.76mmol) and 1,4-dioxane (15mL) was stirred at reflux under an atmosphere of nitrogen for 4.5h. The reaction was allowed to cool to ambient temperature and stirred overnight. The reaction was filtered through a pad of Celite®, washing with ethyl acetate (2 x 15mL). The combined filtrates were concentrated *in vacuo* and purified by chromatography (Silica 40g, 25 to 67% ethyl acetate in heptane). Trituration with ethyl acetate/heptane and drying *in vacuo* gave the title compound as a pale yellow solid (1.56g 70%).

### Example 187

### N-(2-Hydroxyethyl)-4-[2-(1-methylcyclopropyl)-1H-pyrrolo[2,3-b]pyridin-4-yl]benzenesulfonamide

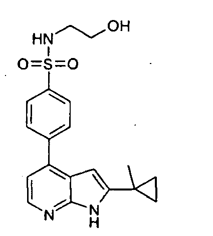

To a solution of 4-chloro-2-(1-methylcyclopropyl)-1*H*-pyrrolo[2,3-*b*]pyridine (248mg, 0.0012mole, ∼75%pure) in 1,2-dimethoxyethane (3ml) was added 10% sodium carbonate (0.25ml), *N*-(2-hydroxyethyl)-4-(4,4,5,5-tetramethyl-1,3,2-dioxaborolan-2-yl)benzenesulfonamide (392mg, 0.0012mole) and bis(diphenylphosphino)ferrocene palladium II chloride (15mg) and the mixture heated to 160 °C in a microwave for 1hr. The mixture was poured into dichloromethane (100ml) and water (20ml). The organic layer was separated, dried (phase separator) and evaporated to dryness. The residual gum was purified by MDAP and the appropriate fractions were combined and evaporated to dryness to afford the title compound as an off white solid (52mg, 11 %).
LCMS rt=0.91 minutes, MH+ 372

### Example 188

### Example 188a

### 4-(2-Cyclopropyl-1H-pyrrolo[2,3-b]pyridin-4-yl)-N-(1,1-dioxidotetrahydro-2H-thiopyran-4-yl)benzenesulfonamide

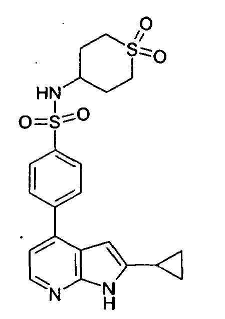

To a solution of 4-chloro-2-cyclopropyl-1*H*-pyrrolo[2,3-*b*]pyridine (193mg, 0.001mole) in 1,2-dimethoxyethane (2ml) was added 10% sodium carbonate (0.2ml), *N*-(1,1-dioxidotetrahydro-2H-thiopyran-4-yl)-4-(4,4,5,5-tetramethyl-1,3,2-dioxaborolan-2-yl)benzenesulfonamide (415mg, 0.001mole) and bis(diphenylphosphino)ferrocene palladium II chloride (20mg) and the resulting mixture heated to 160°C in a microwave for 1 hr. The reaction mixture was poured into dichloromethane (100ml) and water (20ml) and the resulting mixture stirred for 30 minutes. The mixture was then passed through a hydrophobic frit and evaporated to dryness. The residual gum was purified by chromatography (50g, bond elut) eluting with cyclohexane : ethyl acetate 20:1, 10:1, 4:1, 2:1, 1:1 (200ml of each) and ethyl acetate. The appropriate fractions were combined and evaporated to dryness to leave a solid. The solid was triturated under diethyl ether (stirring 2hr) filtered, washed well with diethyl ether and air dried to furnish the title compound as a yellow solid (205mg, 46%).
LCMS rt = 0.93 minutes, MH+ 446

### Example 188b

### 4-(2-Cyclopropyl-1H-pyrrolo[2,3-b]pyridin-4-yl)-N-(1,1-dioxidotetrahydro-2H-thiopyran-4-yl)benzenesulfonamide trifluoroacetate

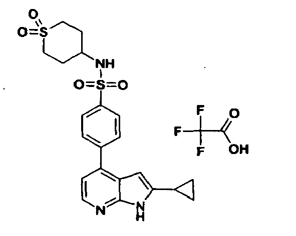

Example 188b was prepared similarly to Examples 111-135 at a temperature of 120 °C.
LCMS rt=2.83mins, MH+ 446

### Example 189

### 4-(2-Cyclopropyl-1H-pyrrolo[2,3-b]pyridin-4-yl)-N-(2-hydroxy-1,2-dimethylpropyl)benzenesulfonamide

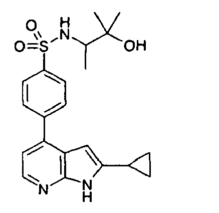

To a degassed suspension of (2-cyclopropyl-1H-pyrrolo[2,3-b]pyridin-4-yl)boronic acid (150 mg, 0.743 mmol), 4-bromo-N-(2-hydroxy-1,2-dimethylpropyl)benzenesulfonamide (263 mg, 0.817 mmol) and potassium phosphate (Tribasic) (158 mg, 0.743 mmol) in 5:1 dioxane:water (4.5 mL) was added solid chloro[2'-(dimethylamino)-2-biphenylyl]palladium - (1*R*,4*S*)-bicyclo[2.2.1]hept-2-yl[(1*S*,4*R*)-bicyclo[2.2.1]hept-2-yl]phosphane (1:1) (41.6 mg, 0.074 mmol). The reaction vessel was sealed and heated in Biotage Initiator using initial absorbtion setting very high to 120 °C for 30 min. The reaction was cooled to ambient temperature. The reaction mixture was partitioned between ethyl acetate:Chloroform (1:1) 25 mL and water 10 mL. The organic was separated and the aqueous extracted with ethyl acetate:chloroform (1:1) 25 mL. The combined organics were dried using a hydrophobic frit and concentrated *in vacuo* to give a brown oil (390 mg). The sample was loaded in dichloromethane and purified by chromatography silica (Si) 20g using a 0-25% methanol-dichloromethane over 60 mins. The appropriate product containing fractions were combined and concentrated *in vacuo* to give a yellow solid (167mg).

The sample was loaded in dichloromethane and purified by chromatography silica (Si) 50g using a 0-50% methanol-dichloromethane over 15 mins. The appropriate fractions were combined and evaporated *in vacuo* to give a yellow solid (120 mg).

The sample was loaded in dichloromethane and purified by chromatography silica (Si) 50g using a 0-25% methanol-dichloromethane over 60 mins. The appropriate fractions were combined and evaporated *in vacuo* and azeotroped with diethyl ether to give the title compound, as a very pale yellow solid (30 mg)

The remaining product containing fractions were concentrated *in vacuo* to a yellow solid. The sample was loaded in dichloromethane and purified by chromatography silica (Si) 20g using a 0-25% methanol-dichloromethane over 40 mins. The appropriate fractions were combined and evaporated *in vacuo* to give the crude title compound as a yellow gum (30 mg) the two Batches of compound were combined and the sample was loaded in chloroform and purified by chromatography silica (Si) 50g using a 0-10% methanol-dichloromethane over 60 mins. The appropriate fractions were combined and evaporated *in vacuo* to give the title compound, (30 mg) as a yellow solid.
LCMS: rt = 2.89 mins, MH+ = 400

### Example 190

### 4-(2-Cyclopropyl-1H-pyrrolo[2,3-b]pyridin-4-yl)-N-(2-hydroxy-1,1-dimethylpropyl)benzenesulfonamide

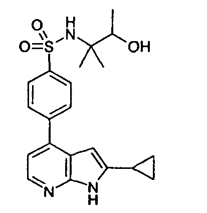

To a degassed suspension of (2-cyclopropyl-1H-pyrrolo[2,3-b]pyridin-4-yl)boronicacid (150 mg, 0.743 mmol), 4-bromo-N-(2-hydroxy-1,1-dimethylpropyl)benzenesulfonamide (215 mg, 0.668 mmol) and potassium phosphate (Tribasic) (155 mg, 0.730 mmol) in 5:1 dioxane:water (4.5 mL) was added solid chloro[2'-(dimethylamino)-2-biphenylyl]palladium - (1*R*,4*S*)-bicyclo[2.2.1]hept-2-yl[(1*S*,4*R*)-bicyclo[2.2.1]hept-2-yl]phosphane (1:1) (42 mg, 0.075 mmol). The reaction vessel was sealed and heated in a Biotage Initiator using absorbtion setting very high to 120 °C for 30 min. The reaction was cooled to ambient temperature. The reaction mixture was partitioned between ethyl acetate:chloroform (1:1) (25 mL) and water (10 mL). The organic was separated and the aqueous extracted with ethyl acetate:chloroform (1:1) (25 mL). The combined organics were dried using a hydrophobic frit and concentrated *in vacuo* to give a yellow gum (330 mg). The sample was loaded in dichloromethane and purified by chromatography silica (Si) 50g using a 0-100% ethyl acetate-dichloromethane over 60 mins. The appropriate fractions were combined and evaporated *in vacuo* to give a pale yellow solid (117mg). The sample was loaded in dichloromethane and purified by chromatography silica (Si) 70g using a 0-100% ethyl acetate-dichloromethane over 60 mins. The appropriate fractions were combined and evaporated *in vacuo* to give a yellow solid. The solid was triturated with diethyl ether (5 mL). The resulting solid was filtered through a medium fritted glass funnel, collected and dried *in vacuo* to give the title compound as a very pale yellow solid (75 mg).
LCMS: rt = 2.95 mins, MH+ = 400

### Example 191

### 4-(2-Cyclopropyl-1H-pyrrolo[2,3-b]pyridin-4-yl)-N-[(1S,2S)-2-hydroxycyclopentyl]-benzenesulfonamide

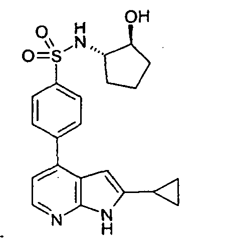

To a degassed suspension of (2-cyclopropyl-1H-pyrrolo[2,3-b]pyridin-4-yl)boronic acid (63 mg, 0.312 mmol), 4-bromo-N-[(1S,2S)-2-hydroxycyclopentyl]benzenesulfonamide (95 mg, 0.297 mmol) and potassium phosphate (Tribasic) (63 mg, 0.297 mmol) in 5:1 dioxane:water (3 mL) was added solid chloro[2'-(dimethylamino)-2-biphenylyl]palladium - (1*R*,4*S*)-bicyclo[2.2.1]hept-2-yl[(1*S*,4*R*)-bicyclo[2.2.1]hept-2-yl]phosphane (1:1) (17 mg, 0.030 mmol). The reaction vessel was sealed and heated in a Biotage Initiator using absorbtion setting very high to 120 °C for 30 min. The reaction was cooled to ambient temperature. The reaction mixture was partitioned between ethyl acetate:chloroform (1:1) (15 mL) and water (10 mL). The organic was separated and the aqueous extracted with ethyl acetate:chloroform (1:1) (15 mL). The combined organics were dried using a hydrophobic frit and concentrated *in vacuo* to give a yellow gum. The sample was loaded in dichloromethane and purified by chromatography: silica (Si) 20g using a 0-100% ethyl acetate-dichloromethane over 60 mins. The appropriate fractions were combined and evaporated *in vacuo* to give the title compound (50 mg) as a yellow solid.
LCMS: rt = 2.83 mins, MH+ = 398

### Example 192

### N-[(1S,2R)-2-Hydroxy-1-methylpropyl]-4-(2-methyl-1H-pyrrolo[2,3-b]pyridin-4-yl)benzenesulfonamide

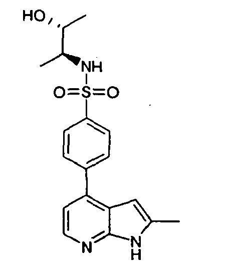

A mixture of (2-methyl-1*H*-pyrrolo[2,3-*b*]pyridin-4-yl)boronic acid (35 mg, 0.199 mmol), 4-bromo-*N*-[(1*S*,2*R*)-2-hydroxy-1-methylpropyl]benzenesulfonamide (61 mg, 0.198 mmol), chloro[2'-(dimethylamino)-2-biphenylyl]palladium-(1*R*,4*S*)-bicyclo[2.2.1]hept-2-yl[(1*S*,4*R*)-bicyclo[2.2.1]hept-2-yl]phosphane (1:1) (11.2 mg, 0.020 mmol) and potassium phosphate (129mg, 0.608mmol) in 1,4-dioxane (1.8 ml) and water (0.45ml) was heated in a sealed tube in a Biotage Initiator microwave using initial very high absorption level setting to 120 °C for 30 min. After cooling the reaction was purified by SPE on reverse phase (C18, 5g) eluted with water, 10%TFA/acetonitrile. The TFA/acetonitrile fractions were evaporated *in vacuo.* The sample was dissolved in DMSO 2x 1 ml and purified by MDAP. The solvent was evaporated *in vacuo* to give the title compound as a yellow gum (22mg).
LCMS rt = 2.70mins, MH+ = 360

### Example 193

### Example 193a

### N-[(1S)-2-Hydroxy-1-methylethyl]-4-[2-(trifluoromethyl)-1H-pyrrolo[2,3-b]pyridin-4-yl]benzenesulfonamide

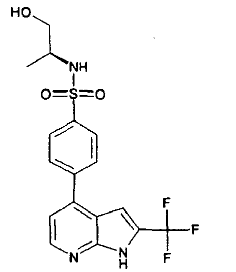

4-Bromo-*N*-[(1*S*)-2-hydroxy-1-methylethyl]benzenesulfonamide (300 mg, 1.3 mmol), [2-(trifluoromethyl)-1*H*-pyrrolo[2,3-*b*]pyridin-4-yl]boronic acid (385 mg, 1.3 mmol), chloro(di-2-norbornylphosphino)(2'-dimethylamino-1,1'-biphenyl-2-yl) palladium(II) (75 mg, 0.13 mmol) and potassium phosphate (275 mg, 1.3 mmol) were suspended in dioxane:water (5:1) (5 ml) and degassed for 10 mins. The reaction mixture was heated at 120 °C for 30 mins in a microwave. The reaction mixture was treated with water (20 ml) and a precipitate formed. The solid was filtered. The filtrate was extracted with ethyl acetate:chloroform (1:1) (2 x 20 ml), dried using a phase separator and concentrated *in vacuo* to afford a brown foam. The foam and the solid were taken up in DCM, combined and concentrated *in vacuo*. The resulting solid was pre-absorbed onto Florisil (60-100 mesh) and purified by flash column chromatography (silica, 50 g, 0-100% ethyl acetate:DCM, 60 mins). The relevant fractions were combined and concentrated *in vacuo* to afford the title compound as a cream solid (230 mg, 44%).
LCMS rt = 2.87 mins, MH+ = 400,

### Example 193b

### N-[(1S)-2-Hydroxy-1-methylethyl]-4-[2-(trifluoromethyl)-1H-pyrrolo[2,3-b]pyridin-4-yl]benzenesulfonamide hydrochloride

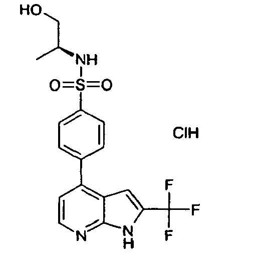

*N*-[(1*S*)-2-Hydroxy-1-methylethyl]-4-[2-(trifluoromethyl)-1*H*-pyrrolo[2,3-*b*]pyridin-4-yl]benzenesulfonamide (30 mg, 0.08 mmol) was dissolved in methanol (1.5 ml) and 1 M HCl in diethyl ether (90 µl, 0.09 mmol) was added and the reaction mixture blown down under nitrogen to afford the title compound as a yellow solid (32 mg, 91%).
LCMS rt = 2.93 mins, MH+ = 400

### Example 194

### N-(2-Aminoethyl)-4-{2-[(diethylamino)methyl]-1H-pyrrolo[2,3-b]pyridin-4-yl}benzenesulfonamide

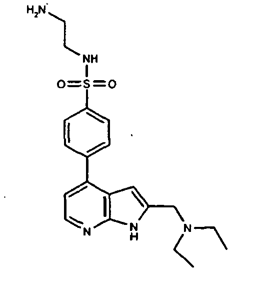

A solution of 1,1-dimethylethyl [2-({[4-(2-formyl-1*H*-pyrroolo[2,3-*b*]pyridin-4-yl)phenyl]sulfonyl}amino)ethyl]carbamate (35mg, 0.1mmol) in dry THF (1mL) was added to diethylamine (0.1 mmol). The reaction mixture was sonicated for 10 seconds prior to standing for 4h. prior to the addition of a suspension of sodium triacetoxyborohydride (106mg, 0.5mmol) in dry THF (1mL). The reaction mixture was sonicated for 10 seconds prior to standing for 16h. The reaction mixture was quenched with 2N HCl in MeOH and concentrated *in vacuo.* The reaction mixture was re-suspended in 1:1 CH₃Cl:MeOH and applied to an aminopropyl cartridge, pre-equilibrated with 1:1 CH₃Cl:MeOH. The sample was eluted with 1:1 CH₃Cl:MeOH. The sample was blown down and treated with 1:1 DCM:TFA and shaken briefly. Concentration by blow down and purification by high pH mass directed HPLC gave the title compound.
LCMS rt= 1.92 min, MH+ 403

### Example 195

### 4-(2-Cyclopropyl-1H-pyrrolo[2,3-b]pyridin-4-yl)-N-(2-hydroxy-2-methylpropyl)benzenesulfonamide

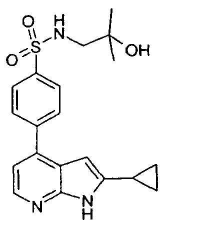

A solution of (2-cyclopropyl-1*H*-pyrrolo[2,3-*b*]pyridin-4-yl)boronic acid (20mg, 0.1 mmol) in dioxan (0.4mL) was added to a microwave vessel containing potassium phosphate (22mg, 0.1mmol) and 2'(dimethylamino)-2-biphenyl-palladium II chloride dinorbornylphosphine complex (0.04mg). A solution of 4-bromo-*N*-(2-hydroxy-2-methylpropyl)benzenesulfonamide (46.2mg, 0.15mmol) in dioxan (0.4mL) was added. The reaction mixture was heated at 110°C in the microwave for 30 minutes prior to cooling. The reaction mixture was applied directly to a C18 cartridge (500mg, pre equilibrated with 0.1% TFA in acetonitrile) and eluted with 0.1% TFA in acetonitrile (3 x 1mL). Concentration by blow down and concentration purification by high pH mass directed HPLC gave the title compound.
LCMS rt= 2.82min MH+ 386

Repurified samples were purified using low pH mass directed HPLC.

Examples 196 to 208 were similarly prepared:

| **Example** | Compound Structure | Compound Name | LCMS | MH+ |
|---|---|---|---|---|
| | | | rt, mins | |
| **196** | 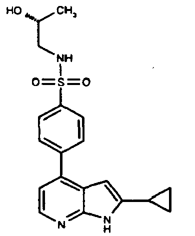 | 4-(2-cyclopropyl-1H-pyrrolo[2,3-b]pyridin-4-yl)-N-[(2R)-2-hydroxypropyl]benzene sulfonamide | 2.74 | 372 |
| **197** | 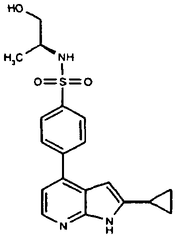 | 4-(2-cyclopropyl-1H-pyrrolo[2,3-b]pyridin-4-yl)-N-[(1S)-2-hydroxy-1-methylethyl]benzenesul fonamide | 2.72 | 372 |
| **198** | 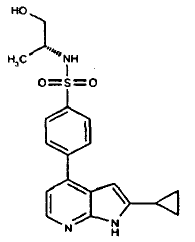 | 4-(2-cyclopropyl-1H-pyrrolo[2,3-b]pyridin-4-yl)-N-[(1R)-2-hydroxy-1-methylethyl]benzenesul fonamide | 2.72 | 372 |
| **199** | 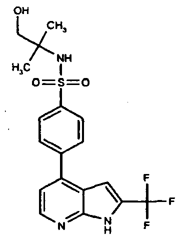 | N-(2-hydroxy-1,1-dimethylethyl)-4-[2-(trifluoromethyl)-1H-pyrrolo[2,3-b]pyridin-4-yl]benzenesulfonamide | 2.99 | 414 |
| **200** | 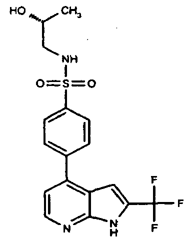 | N-[(2R)-2-hyd roxypropyl]-4-[2-(trifluoromethyl)-1H-pyrrolo[2,3-b]pyridin-4-yl]benzenesulfonamide | 2.88 | 400 |
| **201** | 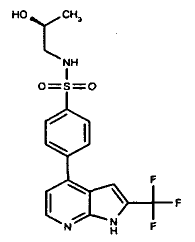 | N-[(2S)-2-hydroxypropyl]-4-[2-(trifluoromethyl)-1H-pyrrolo[2,3-b]pyridin-4-yl]benzenesulfonamide | 2.88 | 400 |
| **202** | 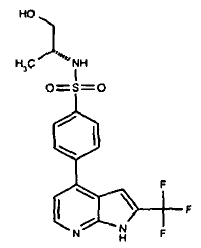 | N-[(1R)-2-hydroxy-1-methylethyl]-4-[2-(trifluoromethyl)-1H-pyrrolo[2,3-b]pyridin-4-yl]benzenesulfonamide | 2.87 | 400 |
| **203** | 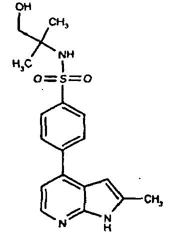 | N-(2-hydroxy-1,1-dimethylethyl)-4-(2-methyl-1H-pyrrolo[2,3-b]pyridin-4-yl)benzenesulfonamide | 2.65 | 360 |
| **204** | 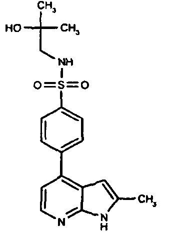 | N-(2-hydroxy-2-methylpropyl)-4-(2-methyl-1H-pyrrolo[2,3-b]pyridin-4-yl)benzenesulfonamide | 2.61 | 360 |
| **205** | 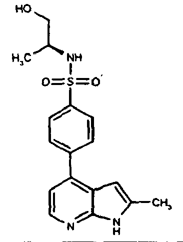 | N-[(1S)-2-hydroxy-1-methylethyl]-4-(2-methyl-1H-pyrrolo[2,3-b]pyridin-4-yl)benzenesulfonamide | 2.5 | 346 |
| **206** | 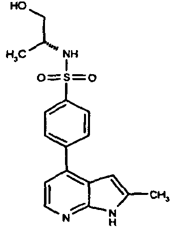 | N-[(1R)-2-hydroxy-1-methylethyl]-4-(2-methyl-1H-pyrrolo[2,3-b]pyridin-4-yl)benzenesulfonamide | 2.51 | 346 |
| **207** | 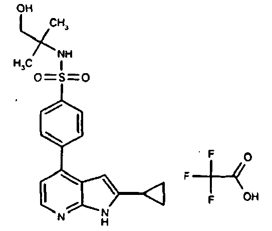 | 4-(2-cyclopropyl-1H-pyrrolo[2,3-b]pyridin-4-yl)-N-(2-hydroxy-1,1-dimethylethyl)benzenes ulfonamide trifluoroacetate (salt) | 0.9 | 501 |
| **208** | 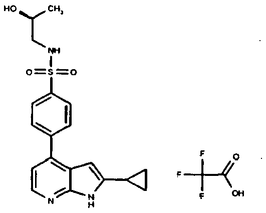 | 4-(2-cyclopropyl-1H-pyrrolo[2,3-b]pyridin-4-yl)-N-[(2S)-2-hydroxypropyl]benzene sulfonamide trifluoroacetate (salt) | 0.86 | 486 |

### Example 209

### 4-(2-Cyclopropyl-1H-pyrrolo[2,3-b]pyridin-4-yl)-N-[(1R)-1-(hydroxymethyl)propyl]-benzenesulfonamide

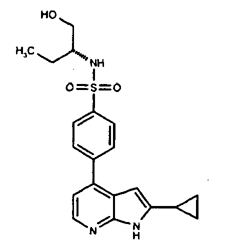

A solution of 4-bromobenzenesulfonyl chloride (51mg, 0.2mmol) in DCM (1mL) was added to a solution of (2*R*)-2-amino-1-butanol (0.3mmol), in dry DMF (1mL). DIPEA (200µL) was added and the reaction mixture sonicated for 15 seconds prior to standing for 16h. The reaction mixture was concentrated *in vacuo* and resuspended in dioxan (0.5mL) to afford a crude solution of 4-bromo-*N*-[1-(hydroxymethyl)cyclopentyl]benzenesulfonamide. A solution of (2-cyclopropyl-1*H*-pyrrolo[2,3-*b*]pyridin-4-yl)boronic acid (20mg, 0.1mmol) in dioxan (0.3mL) was added to a microwave vessel containing 2'(dimethylamino)-2-biphenyl-palladium II chloride dinorbornylphosphine complex (0.25mg, 0.5 mol%) and the crude solution of 4-bromo-*N*-[1-(hydroxymethyl)cyclopentyl]benzenesulfonamide. A solution of potassium phosphate (22mg, 0.1 mmol) in water (200uL) was added and the reaction mixture was heated at 110°C in the microwave for 30 minutes. The reaction mixture was applied directly to a C18 cartridge (500mg pre-equilibrated with acetonitrile) and eluted with 0.1% TFA in acetonitrile (2 x 1mL). Concentration by blow down afforded the crude product. Purification by high pH mass directed HPLC gave the title compound.
LCMS rt= 0.91 min, MH+ = 386

Deprotected samples were treated with a solution of DCM:TFA, 1:1 and re concentration by blow down prior to purification by high pH mass directed HPLC.

Examples 210 to 252 were similarly prepared:

| **Example** | Compound Structure | Compound Name | LCMS rt, | MH+ |
|---|---|---|---|---|
| | | | mins | |
| **210** | 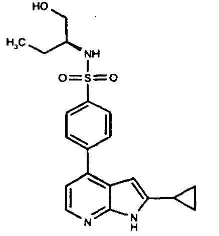 | 4-(2-cyclopropyl-1H-pyrrolo[2,3-b]pyridin-4-yl)-N-[(1S)-1-(hydroxymethyl)propyl ]benzenesulfonamide | 0.91 | 386 |
| **211** | 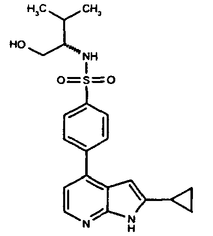 | 4-(2-cyclopropyl-1H-pyrrolo[2,3-b]pyridin-4-yl)-N-[(1S)-1-(hydroxymethyl)-2-methylpropyl]benzene sulfonamide | 0.97 | 401 |
| **212** | 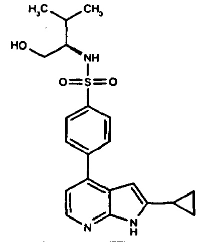 | 4-(2-cyclopropyl-1H-pyrrolo[2,3-b]pyridin-4-yl)-N-[(1R)-1-(hydroxymethyl)-2-methylpropyl]benzene sulfonamide | 0.97 | 401 |
| **213** | 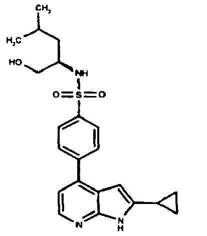 | 4-(2-cyclopropyl-1H-pyrrolo[2,3-b]pyridin-4-yl)-N-[(1R)-1-(hydroxymethyl)-3-methylbutyl]benzenes ulfonamide | 1.01 | 415 |
| **214** | 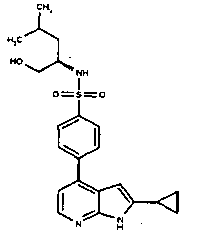 | 4-(2-cyclopropyl-1H-pyrrolo[2,3-b]pyridin-4-yl)-N-[(1S)-1-(hydroxymethyl)-3-methylbutyl]benzenes ulfonamide | 1.01 | 415 |
| **215** | 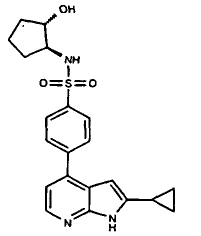 | 4-(2-cyclopropyl-1H-pyrrolo[2,3-b]pyridin-4-yl)-N-[(1S,2S)-2-hydroxycyclopentyl]be nzenesulfonamide | 0.91 | 399 |
| **216** | 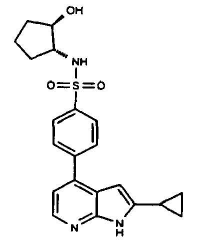 | 4-(2-cyclopropyl-1H-pyrrolo[2,3-b]pyridin-4-yl)-N-[(1R,2R)-2-hydroxycyclopentyl]be nzenesulfonamide | 0.91 | 399 |
| **217** | 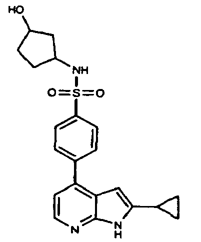 | 4-(2-cyclopropyl-1H-pyrrolo[2,3-b]pyridin-4-yl)-N-(3-hydroxycyclopentyl)be nzenesulfonamide | 0.91 | 399 |
| **218** | 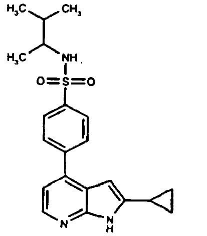 | 4-(2-cyclopropyl-1H-pyrrolo[2,3-b]pyridin-4-yl)-N-(1,2-dimethylpropyl)benze nesulfonamide | 1.17 | 385 |
| **219** | 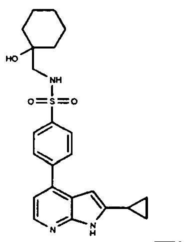 | 4-(2-cyclopropyl-1H-pyrrolo[2,3-b]pyridin-4-yl)-N-[(1-hydroxycyclohexyl)me thyl]benzenesulfonamide | 3.23 | 427 |
| **220** | 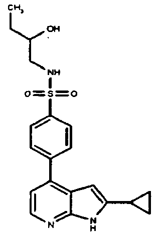 | 4-(2-cyclopropyl-1H-pyrrolo[2,3-b]pyridin-4-yl)-N-(2-hydroxybutyl)benzene sulfonamide | 0.94 | 386 |
| **221** | 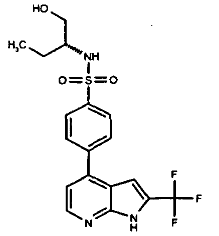 | N-[(1R)-1-(hydroxymethyl)propyl ]-4-[2-(trifluoromethyl)-1H-pyrrolo[2,3-b]pyridin-4-yl]benzenesulfonamide | 0.97 | 414 |
| **222** | 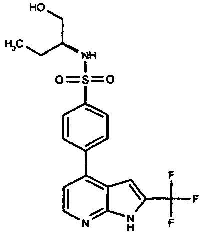 | N-[(1S)-1-(hydroxymethyl)propyl ]-4-[2-(trifluoromethyl)-1H-pyrrolo[2,3-b]pyridin-4-yl]benzenesulfonamide | 0.97 | 414 |
| **223** | 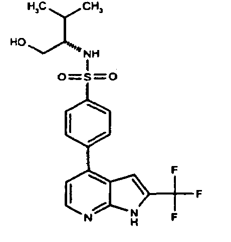 | N-[(1S)-1-(hydroxymethyl)-2-methylpropyl]-4-[2-(trifluoromethyl)-1H-pyrrolo[2,3-b]pyridin-4-yl]benzenesulfonamide | 1.02 | 428 |
| **224** | 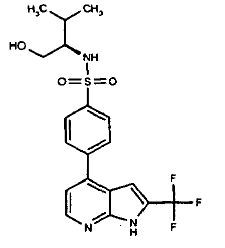 | N-[(1R)-1-(hydroxymethyl)-2-methylpropyl]-4-[2-(trifluoromethyl)-1H-pyrrolo[2,3-b]pyridin-4-yl]benzenesulfonamide | 1.02 | 428 |
| **225** | 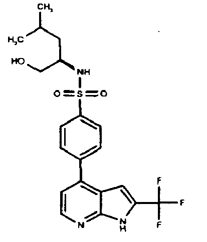 | N-[(1R)-1-(hydroxymethyl)-3-methylbutyl]-4-[2-(trifluoromethyl)-1H-pyrrolo[2,3-b]pyridin-4-yl]benzenesulfonamide | 1.06 | 442 |
| **226** | 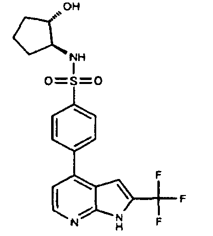 | N-[(1S,2S)-2-hydroxycyclopentyl]-4-[2-(trifluoromethyl)-1H-pyrrolo[2,3-b]pyridin-4-yl]benzenesulfonamide | 0.96 | 426 |
| **227** | 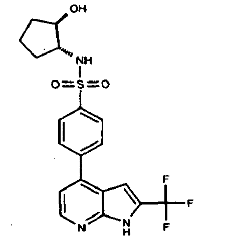 | N-[(1R,2R)-2-hydroxycyclopentyl]-4-[2-(trifluoromethyl)-1H-pyrrolo[2,3-b]pyridin-4-yl]benzenesulfonamide | 0.96 | 426 |
| **228** | 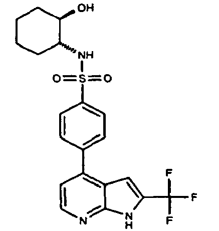 | N-[(1R,2R)-2-hydroxycyclohexyl]-4-[2-(trifluoromethyl)-1H-pyrrolo[2,3-b]pyridin-4-yl]benzenesulfonamide | 1.02 | 440 |
| **229** | 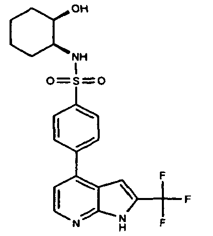 | rel-N-[(1R,2S)-2-hydroxycyclohexyl]-4-[2-(trifluoromethyl)-1H-pyrrolo[2,3-b]pyridin-4-yl]benzenesulfonamide | 1.05 | 440 |
| **230** | 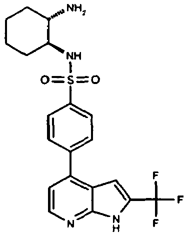 | rel-N-[(1R,2R)-2-aminocyclohexyl]-4-[2-(trifluoromethyl)-1H-pyrrolo[2,3-b]pyridin-4-yl]benzenesulfonamide | 0.79 | 439 |
| **231** | 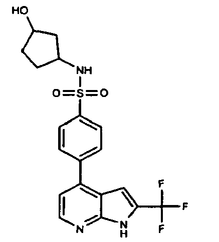 | N-(3-hydroxycyclopentyl)-4-[2-(trifluoromethyl)-1H-pyrrolo[2,3-b]pyridin-4-yl]benzenesulfonamide | 0.96 | 426 |
| **232** | 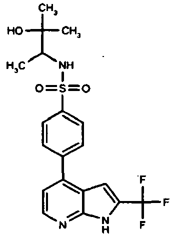 | N-(2-hydroxy-1,2-dimethylpropyl)-4-[2-(trifluoromethyl)-1H-pyrrolo[2,3-b]pyridin-4-yl]benzenesulfonamide | 0.99 | 428 |
| **233** | 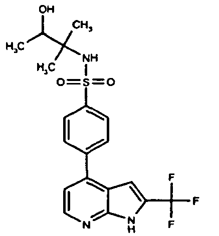 | N-(2-hydroxy-1,1-dimethylpropyl)-4-[2-(trifluoromethyl)-1H-pyrrolo[2,3-b]pyridin-4-yl]benzenesulfonamide | 1.02 | 428 |
| **234** | 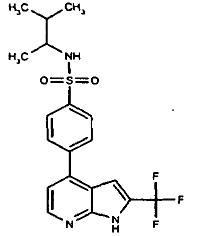 | N-(1,2-dimethylpropyl)-4-[2-(trifluoromethyl)-1H-pyrrolo[2,3-b]pyridin-4-yl]benzenesulfonamide | 1.2 | 412 |
| **235** | 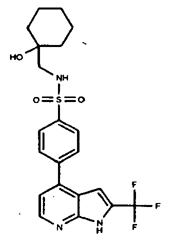 | N-[(1-hydroxycyclohexyl)me thyl]-4-[2-(trifluoromethyl)-1H-pyrrolo[2,3-b]pyridin-4-yl]benzenesulfonamide | 1.11 | 454 |
| **236** | 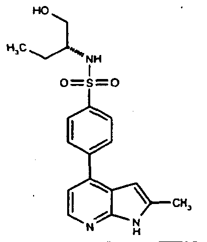 | N-[(1R)-1-(hydroxymethyl)propyl ]-4-(2-methyl-1H-pyrrolo[2,3-b]pyridin-4-yl)benzenesulfonamide | 0.84 | 360 |
| **237** | 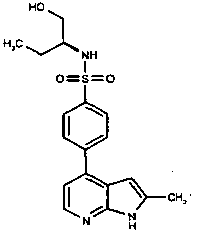 | N-[(1S)-1-(hydroxymethyl)propyl ]-4-(2-methyl-1H-pyrrolo[2,3-b]pyridin-4-yl)benzenesulfonamide | 0.83 | 360 |
| **238** | 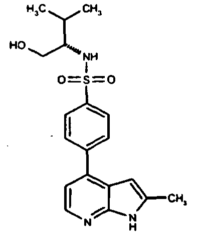 | N-[(1S)-1-(hydroxymethyl)-2-methylpropyl]-4-(2-methyl-1H-pyrrolo[2,3-b]pyridin-4-yl)benzenesulforiamide | 0.9 | 374 |
| **239** | 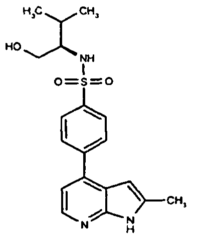 | N-[(1R)-1-(hydroxymethyl)-2-methylpropyl]-4-(2-methyl-1H-pyrrolo[2,3-b]pyridin-4-yl)benzenesulfonamide | 0.9 | 374 |
| **240** | 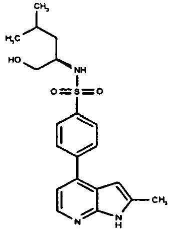 | N-[(1R)-1-(hydroxymethyl)-3-methylbutyl]-4-(2-methyl-1H-pyrrolo[2,3-b]pyridin-4-yl)benzenesulfonamide | 0.95 | 389 |
| **241** | 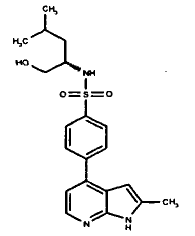 | N-[(1S)-1-(hydroxymethyl)-3-methylbutyl]-4-(2-methyl-1H-pyrrolo[2,3-b]pyridin-4-yl)benzenesulfonamide | 0.95 | 389 |
| **242** | 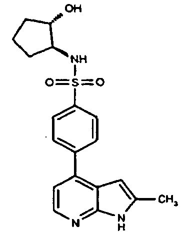 | N-[(1S,2S)-2-hydroxycyclopentyl]-4-(2-methyl-1H-pyrrolo[2,3-b]pyridin-4-yl)benzenesulfonamide | 0.83 | 372 |
| **243** | 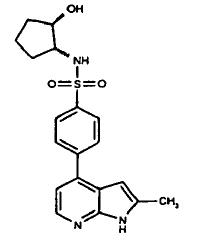 | N-[(1R,2R)-2-hyd roxycyclopentyl]-4-(2-methyl-1H-pyrrolo[2,3-b]pyridin-4-yl)benzenesulfonamide | 0.83 | 372 |
| **244** | 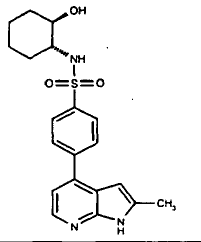 | N-[(1R,2R)-2-hydroxycyclohexyl]-4-(2-methyl-1H-pyrrolo[2,3-b]pyridin-4-yl)benzenesulfonamide | 0.9 | 386 |
| **245** | 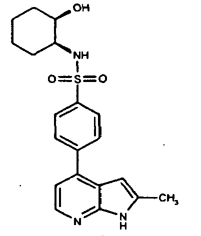 | rel-N-[(1R,2S)-2-hydroxycyclohexyl]-4-(2-methyl-1H-pyrrolo[2,3-b]pyridin-4-yl)benzenesulfonamide | 0.94 | 386 |
| **246** | 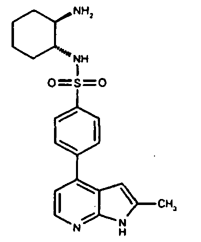 | rel-N-[(1R,2R)-2-aminocyclohexyl]-4-(2-methyl-1H-pyrrolo[2,3-b]pyridin-4-yl)benzenesulfonamide | 0.67 | 386 |
| **247** | 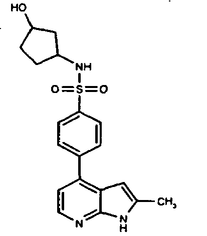 | N-(3-hydroxycyclopentyl)-4-(2-methyl-1H-pyrrolo[2,3-b]pyridin-4-yl)benzenesulfonamide | 0.67 | 372 |
| **248** | 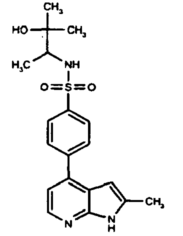 | N-(2-hydroxy-1,2-dimethylpropyl)-4-(2-methyl-1H-pyrrolo[2,3-b]pyridin-4-yl)benzenesulfonamide | 0.83 | 374 |
| **249** | 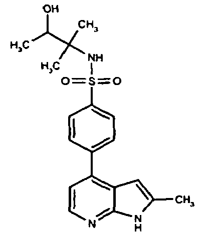 | N-(2-hydroxy-1,1-dimethylpropyl)-4-(2-methyl-1H-pyrrolo[2,3-b]pyridin-4-yl)benzenesulfonamide | 0.86 | 374 |
| **250** | 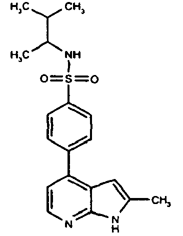 | N-(1,2-dimethylpropyl)-4-(2-methyl-1H-pyrrolo[2,3-b]pyridin-4-yl)benzenesulfonamide | 1.1 | 358 |
| **251** | 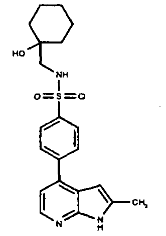 | N-[(1-hydroxycyclohexyl)me thyl]-4-(2-methyl-1H-pyrrolo[2,3-b]pyridin-4-yl)benzenesulfonamide | 1 | 401 |
| **252** | 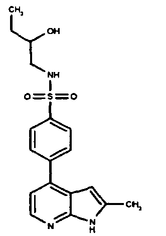 | N-(2-hydroxybutyl)-4-(2-methyl-1H-pyrrolo[2,3-b]pyridin-4-yl)benzenesulfonamide | 0.86 | 360 |

### Example 253

### 4-{2-[(Dimethylamino)methyl]-1H-pyrrolo[2,3-b]pyridin-4-yl}-N-[(3R)-1,1-dioxidotetrahydro-3-thienyl]benzenesulfonamide

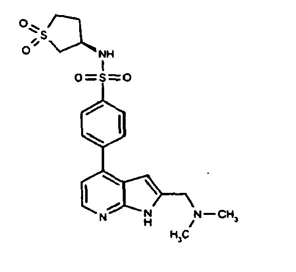

A solution of [4-({[(3*R*)-1,1-dioxidotetrahydro-3-thienyl]amino}sulfonyl)phenyl]-boronic acid (0.15 mmol) in dioxan (0.4mL) was added to a microwave vessel containing potassium phosphate (22mg, 0.1mmol) and 2'(dimethylamino)-2-biphenyl-palladium II chloride dinorbornylphosphine complex (0.04mg). A solution of [(4-bromo-1*H*-pyrrolo[2,3-*b*]pyridin-2-yl)methyl]dimethylamine (0.1 mmol) in dioxan (0.4mL) was added. The reaction mixture was heated at 110°C in the microwave for 30 minutes prior to cooling. The reaction mixture was applied directly to a C18 cartridge (500mg, pre equilibrated with acetonitrile) and eluted with 0.1% TFA in acetonitrile (3 x 1mL). Concentration by blow down and concentration purification by high pH mass directed HPLC gave the title compound.
LCMS rt= 0.61 min, MH+ = 450

Examples 254 to 259 were similarly prepared:

| **Example** | Compound Structure | Compound Name | LEMS rt, | MH+ |
|---|---|---|---|---|
| | | | mins | |
| **254** | 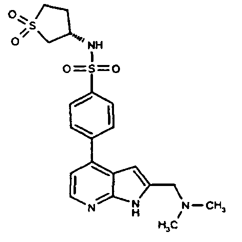 | 4-{2-[(dimethylamino)methyl ]-1H-pyrrolo[2,3-b]pyridin-4-yl}-N-[(3S)-1,1-dioxidotetrahydro-3-thienyl]benzenesulfonamide | 0.67 | 450 |
| **255** | 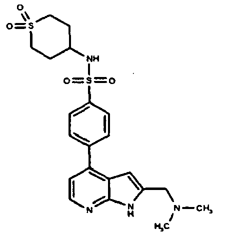 | 4-{2-[(dimethylamino)methyl ]-1H-pyrrolo[2,3-b]pyridin-4-yl}-N-(1,1-dioxidotetrahydro-2H-thiopyran-4-yl)benzenesulfonamide | 0.62 | 464 |
| **256** | 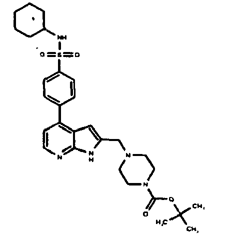 | 1,1-dimethylethyl 4-[(4-{4-[(cyclohexylamino)sulfo nyl]phenyl}-1H-pyrrolo[2,3-b]pyridin-2-yl)methyl]-1-piperazinecarboxylate | 1.07 | 555 |
| 257 | 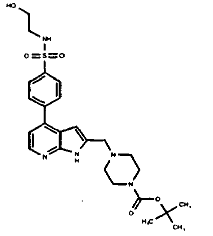 | 1,1-dimethylethyl 4-{[4-(4-{[(2-hydroxyethyl)amino]sulf onyl}phenyl)-1H-pyrrolo[2,3-b]pyridin-2-yl]methyl}-1-piperazinecarboxylate | 0.78 | 517 |
| 258 | 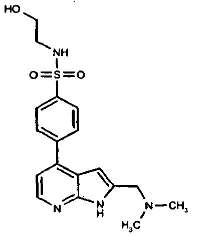 | 4-{2-[(dimethylamino)methyl ]-1H-pyrrolo[2,3-b]pyridin-4-y)}-N-(2-hydroxyethyl)benzenes ulfonamide | 0.6 | 375 |
| 259 | 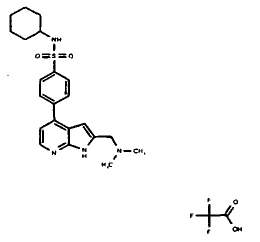 | N-cyclohexyl-4-{2-[(dimethylamino)methyl ]-1H-pyrrolo[2,3-b]pyridin-4-yl}benzenesulfonamide trifluoroacetate | 0.87 | 528 |

### Example 260

### N-(1,1-Dioxidotetrahydro-2H-thiopyran-4-yl)-4-[2-(1-methylcyclopropyl)-1H-pyrrolo[2,3-b]pyridin-4-yl]benzenesulfonamide

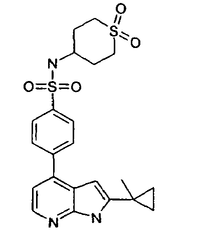

To a solution of *N*-(1,1-dioxidotetrahydro-2H-thiopyran-4-yl)-4-[2-(1-methylcyclopropyl)-1*H*-pyrrolo[2,3-b]pyridin-4-yl]benzenesulfonamide (∼70% pure, 248mg, 0.0012mole) in 1,2-dimethoxyethane (4ml) was added 10% sodium carbonate (0.5ml), *N*-(1,1-dioxidotetrahydro-2*H*-thiopyran-4-yl)-4-(4,4,5,5-tetramethyl-1,3,2-dioxaborolan-2-yl)benzenesulfonamide (451mg, 0.0012mole), and 1,1-bis(diphenylphosphino)ferrocene palladium II chloride (25mg) and the mixture heated to 160 °C in a microwave for 2hr. The reaction mixture was diluted with water (10ml) and dichloromethane (150ml). The organic layer was separated by filtration through a hydrophobic frit and evaporated to dryness to leave a black/red gum. The gum was purified by MDAP (4 runs of 90mg each) and the appropriate fractions were combined and evaporated to dryness to give the title compound as a yellow/brown solid (15mg).
LCMS rt = 2.96 minutes, MH+ 460

### POLYMORPH EXPERIMENTAL

### Example 145a Method B

### Differential Scanning Calorimetry (DSC)

The DSC thermogram of the product was obtained using a TA Q1000 calorimeter, serial number 1000-0126. The sample was weighed into an aluminium pan, a pan lid placed on top and lightly crimped without sealing the pan. The experiment was conducted using a heating rate of 10°C min⁻¹.

The data are illustrated in Figure 1. A melt onset temperature of ∼191.5°C was observed.

### X-Ray Powder Diffraction (XRPD)

The data were acquired on a PANalytical X'Pert Pro powder diffractometer, model PW3040/60, serial number DY1850 using an X'Celerator detector. The acquisition conditions were: radiation: Cu Kα, generator tension: 40 kV, generator current: 45 mA, start angle: 2.0 °2θ, end angle: 40.0 °2θ, step size: 0.0167 °2θ, time per step: 31.75 seconds. The sample was prepared by mounting a few milligrams of sample on a Si wafer (zero background) plates, resulting in a thin layer of powder.

The X-ray powder diffraction (XRPD) data are shown in Figure 2.

Characteristic peaks for the solid state form are summarised in Table 1 with calculated lattice spacings. Peak positions were measured using Highscore software.

**Table 1**

| **2θ/°** | **d-spacing** / **Å** |
|---|---|
| 9.5 | 9.4 |
| 10.5 | 8.4 |
| 11.1 | 7.9 |
| 12.0 | 7.4 |
| 13.8 | 6.4 |
| 14.4 | 6.2 |
| 15.3 | 5.8 |
| 16.1 | 5.5 |
| 16.7 | 5.3 |
| 17.8 | 5.0 |
| 18.1 | 4.9 |
| 18.9 | 4.7 |
| 21.0 | 4.2 |
| 21.6 | 4.1 |
| 22.4 | 4.0 |
| 22.6 | 3.9 |
| 23.7 | 3.7 |
| 24.0 | 3.7 |
| 24.8 | 3.6 |
| 31.5 | 2.8 |

### Example 146d

### Differential Scanning Calorimetry (DSC)

The DSC thermogram of the product was obtained using a similar method to that described above.

The data are illustrated in Figure 3. A melt onset temperature of 287.0°C was observed.

### X-Ray Powder Diffraction (XRPD)

The data were acquired using a simlar method to that described above.

The X-ray powder diffraction (XRPD) data are shown in Figure 4.

Characteristic peaks for the solid state form are summarised in Table 2 with calculated lattice spacings. Peak positions were measured using Highscore software.

**Table 2**

| **2θ/°** | **d-spacing** / **Å** |
|---|---|
| 6.5 | 13.6 |
| 7.2 | 12.3 |
| 10.1 | 8.8 |
| 12.9 | 6.9 |
| 14.1 | 6.3 |
| 14.4 | 6.2 |
| 15.2 | 5.8 |
| 16.9 | 5.3 |
| 18.3 | 4.8 |
| 21.2 | 4.2 |
| 22.0 | 4.0 |
| 23.3 | 3.8 |
| 25.0 | 3.6 |
| 25.9 | 3.4 |
| 27.5 | 3.2 |
| 28.4 | 3.1 |
| 28.4 | 3.1 |
| 30.4 | 2.9 |
| 31.1 | 2.9 |
| 31.9 | 2.8 |

### Example 146e

### Differential Scanning Calorimetry (DSC)

The DSC thermogram of the product was obtained using a similar method to that described above.

The data are illustrated in Figure 5. A melt onset temperature of 195.6°C was observed.

### X-Ray Powder Diffraction (XRPD)

The data were acquired using a simlar method to that described above.

The X-ray powder diffraction (XRPD) data are shown in Figure 6.

Characteristic peaks for the solid state form are summarised in Table 3 with calculated lattice spacings. Peak positions were measured using Highscore software.

**Table 3**

| **2θ/°** | **d-spacing / Å** |
|---|---|
| 8.8 | 10.0 |
| 9.0 | 9.8 |
| 11.2 | 7.9 |
| 13.8 | 6.4 |
| 15.4 | 5.8 |
| 17.4 | 5.1 |
| 17.6 | 5.0 |
| 18.0 | 4.9 |
| 18.3 | 4.8 |
| 20.9 | 4.3 |
| 21.2 | 4.2 |
| 21.4 | 4.2 |
| 21.6 | 4.1 |
| 23.0 | 3.9 |
| 24.0 | 3.7 |
| 24.8 | 3.6 |
| 26.7 | 3.3 |
| 29.1 | 3.1 |
| 29.8 | 3.0 |
| 32.1 | 2.8 |

### BIOLOGICAL DATA

### 1. In Vitro Data

### IKK2 Assay

Recombinant human IKKβ (residues 5-756) was expressed in baculovirus as a C-terminal GST-tagged fusion protein, and its activity was assessed using a time-resolved fluorescence resonance energy transfer (TR-FRET) assay. Briefly, IKKβ (0.5 - 4 nM final concentration) diluted in assay buffer (50 mM HEPES, 10 mM MgCl₂, 1 mM CHAPS pH 7.4 with 1 mM DTT and 0.01% w/v BSA) was added to wells containing various concentrations of compound or DMSO vehicle (1.7% v/v final). The reaction was initiated by the addition of GST-IkappaBalpha substrate (25 nM final)/ATP (1 µM final), in a total volume of 6 µl. The reaction was incubated for 15 mins at room temperature, then terminated by the addition of 3 µl of 50 mM EDTA in buffer (100 mM HEPES pH 7.4, 150 mM NaCl and 0.1% w/v BSA) containing antiphosphoserine-IkappaBalpha-32/36 monoclonal antibody clone 12C2 (Cell Signalling Technology, Beverly Massachusetts, USA) labelled with W-1024 europium chelate (Wallac OY, Turku, Finland), and an APC-labelled anti-GST antibody (Prozyme, San Leandro, California, USA). The reaction was further incubated for 60 mins at room temperature and the degree of phosphorylation of GST-IkappaBalpha measured using a Rubystar plate reader (BMG Instruments, Aylesbury, UK) as a ratio of specific 665 nm energy transfer signal to reference europium 620 nm signal.

### Human Peripheral Blood Mononuclear Cell Assay and Human Whole Blood Assay

### Human Peripheral Blood Mononuclear Cell Assay

The cellular potency of compounds was assessed in human peripheral blood mononuclear cells (PBMC) by measuring their impact on lipopolysaccharide (LPS) stimulated TNFa production. PBMCs were prepared from heparinised human blood from normal volunteers by centrifugation on hystopaque in Accuspin tubes at 800g for 20 minutes. The cells were collected from the interface, washed by centrifugation (1300g, 10 minutes) and resuspended in assay buffer (RPMI1640 containing 10% foetal calf serum, 1% L-glutamine and 1% penicillin/streptomycin) at 1x10⁶ cells/ml. 50µl cells were added to microtitre wells containing 1.0µl of an appropriately diluted compound solution which had been solvated and diluted in DMSO. 75µl LPS (s.typhosa Sigma Cat L6386, 1 ng/ml final) was added and the samples incubated at 37 °C, 5% CO₂ for 20 hours. The supernatant was removed and the concentrations of TNF determined by electrochemiluminescence assay using the MSD technology.

### Human Whole Blood Assay

Heparinised blood drawn from normal volunteers was dispensed (100µl) into microtitre plate wells containing 1.0µl of an appropriately diluted compound solution in DMSO. After 1hr incubation at 37 °C, 5% CO₂, 25µl LPS solution (S. typhosa) in RPMI 1640 (containing 1% L-glutamine and 1% Penicillin/ streptomycin) was added (50ng/ml final). The samples were incubated at 37°C, 5% CO₂ for 20 hours, 50µls physiological saline (0.138% NaCl) was added and diluted plasma was collected using a Biomek FX liquid handling robot after centrifugation at 1300 g for 10 min. Plasma TNFα content was determined by electrochemiluminescence assay using the Mesoscale (MSD) technology.

### TNFα Assay associated with PBMC and Whole Blood Assays

20µl supernatant from PBMC plates or 40µl from whole blood plates was transferred using the Biomek FX to a 96 well High-Bind MSD assay plate precoated with anti-hTNF alpha capture antibody and containing 25µl of MSD human serum cytokine assay diluent. Each plate also contained a TNFα standard curve (0-5000 pg/ml: R+D Systems, 210-TA). For the Whole blood assay, plates were sealed and shaken for 2 hours at room temperature after which they were washed and 40µl of MSD detection antibody was added. The plates were shaken at room temperature for a further 1 hour before washing again and adding 150µl of MSD Read Buffer T (2X). Plates were then read on the MSD Sector 6000 plate reader. For the PBMC assay, supernatant addition to the MSD plates was followed immediately by 20µl of MSD detection antibody, the plates were then sealed and shaken for 2 hours before addition of 90µl of MSD Read Buffer P (2.5X). Plates were read on the MSD Sector 6000.

TNF concentrations were derived from the standard curve run on the same plate and pIC50 values for inhibition of TNF production were derived from the compound dose response curves with non-linear least squares curve fitting using Activity base software.

### NFkB Reporter Assay

A 70% confluent T225 flask of A549 SPAP cells was harvested by centrifugation for 5 min at 200g, resuspended in assay buffer (DMEM supplemented with 10% FCS 2xHl, 2mM L-Glutamine,1% Pen/Strep and Non essential amino acids) and diluted to 0.16 x 10⁶/ml. 60µl of cell solution was dispensed to each well of clear Nunc 384-well plates, containing 0.5µl compound in neat DMSO at 140x the required final assay concentration. Plates were incubated for 1h at 37°C, 95% humidity, 5% CO2 before 10ml of TNF solution in assay buffer was added to give a final concentration of 3.2ng/ml and then returned to the cell incubator for 15h. Plates were equilibrated to room temperature for 1h prior to the addition of 25µl of pNPP buffer (1M Diethanolamine pH 9.8, 0.5mM MgCl₂, 0.28M NaCl, 2mg/ml pNPP) to each well of assay plates. The plates were covered to protect the reagents from light, and then incubated at room temperature for approximately 1 hour before reading them on an Ascent using a 405 nm single filter.

All data was normalized to the mean of 16 high and 16 low control wells on each plate. A four parameter curve fit of the following form was then applied

### 2. In Vivo Data

### Intranasally dosed LPS induced neutrophilia in the male CD rat

### Compound/Vehicle Pretreatment

Male CD rats (150-250g) were anaesthetised with isoflurane (5%, 2Umin O₂, 1L/min NO) and held vertically whilst being dosed with test compound or vehicle (0.2% Tween 80 in phosphate buffered saline or in a vehicle comprising an aqueous solution of 5% dextrose, 1.5% Avicel RC591, 0.15% EDTA, 0.025% polysorbate 80, 0.015% benzalkonium chloride) at a dose volume of 25µl per nostril, using a 100µl Gilson pipette. The tip of the pipette was inserted approximately 3mm into the nostril and the dosing substance instilled. After dosing, animals were placed in a supine position during recovery from anaesthesia.

### LPS Challenge Protocol

Approximately thirty minutes following dosing of compound or vehicle the rats were re-anaesthetised as above then dosed in the same manner with 25µl/nostril of either phosphate buffered saline vehicle, (PBS) or 10mg/ml lipopolysaccharide (LPS).

### Nasal Lavage Protocol

Four hours following the PBS/LPS challenge the animals were culled with an overdose of sodium pentobarbitone given intra peritoneally. The trachea was exposed and a small incision made, into which a tube was inserted orthograde towards the nasal cavity. The nose was then washed with 15mls of heparinised (10U/ml) PBS.

### Cell Counts

The NALF samples were centrifuged at 1300 rpm for 7minutes. The supernatant was removed and the resulting cell pellet resuspended in 0.5ml PBS. A cell slide of the resuspension fluid was prepared by placing 75µl of resuspended NAL fluid into cytospin holders and then spun at 500 rpm for 5minutes. The slides were allowed to air dry and then stained with Leishmans stain (20 minutes) to allow differential cell counting. The total cells were also counted from the resuspension using a Sysmex counter. From these two counts, the total numbers of neutrophils in the NALF were determined.

### LPS-induced TNFα Production in Rats

Male Lewis rats (180-200g) from Charles River Breeding Laboratories (Portage) ACUC Protocol# 05051 were pretreated orally with compound or vehicle. After a determined pretreatment time, the rats were given LPS (lipopolysaccharide from Escherichia coli Serotype 055-B5, Sigma Chemical Co., St Louis, MO) 30µg/rat in 0.5 ml saline, intraperitoneally. The rats were euthanized by CO₂ inhalation 90 minutes after the LPS injection and blood samples were collected by cardiac puncture, transferred into heparinized tubes and stored on ice. The blood samples were centrifuged at 2000 rpm for 10 minutes and the plasma collected for analysis by specific ELISA for TNFα levels.

The plasma samples were assayed for TNFα according to manufactures specifications. TNFα levels were expressed as pg/ml. Elisa kits were purchased from R&D Systems Inc. (Rat TNFα Quantikine Kit Catalog# RTA00).

### Results

The compounds of Examples 1-260 were tested for activity against IKK2 in the IKK2 assay and were found to be inhibitors of IKK2 with pIC₅₀ potency of 5.0 or greater.

Preferred compounds have pIC50 >6 in the human peripheral blood mononuclear cell assay.

Preferred compounds have pIC50 >5 in the human whole blood assay.

Preferred compounds have pIC50 >6 in the NFkB reporter assay.

The compounds of Examples 82, 84, 137b, 146b, 146c, 114, 118, 150 and 177 were tested in the *in vivo* model relating to intranasally dosed LPS induced neutrophilia in the male CD rat. The compounds of Examples 82, 146b, 146c, 118 and 177 showed a % inhibition of > 50 at 5µg/kg.

The compound of Example 145a was tested in the *in vivo* model relating to LPS-induced TNFα production in rats and showed an ED50 of < 20mg/kg.

## Claims

1. A compound according to formula (I): 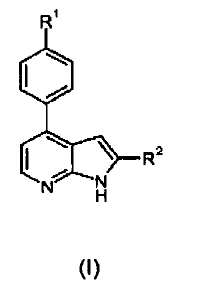 wherein
R¹ is -SO₂NR³R⁴ or -NR⁵SO₂CH₃;
R² is -CHR⁶R⁷, -CF₃ or-C(CH₃)₃;
R³ is hydrogen or methyl and R⁴ is hydrogen, C₁₋₆alkyl optionally substituted by hydroxy, - (CH₂)ₐC₃₋₅cycloalkyl wherein the cycloalkyl is optionally substituted by hydroxy or -NH₂, - (CH₂)_{b})NR⁸R⁹, piperidinyl optionally substituted by C₁₋₆alkyl, or 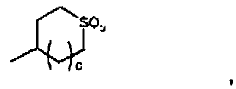 or
R³ and R⁴ are linked to form pyrrolidinyl, or piperidinyl optionally substituted by -NH₂;
R⁵ is hydrogen or methyl;
R⁶ is hydrogen and R⁷ is hydrogen, C₁₋₆alkyl, -(CH₂)_{d}OR¹⁰, -NR¹¹R¹², -CO₂C₁₋₆alkyl. - CONR¹³R¹⁴, phenyl, or 5-membered heteroaryl containing from one to four nitrogen atoms wherein the heteroaryl is optionally substituted by one or two substituents independently selected from C₁₋₆alkyl, -COC₁₋₆alkyl, -(CH₂)ₑphenyl and thienyl,
R⁶ and R⁷ are each fluorine,
R⁶ is methyl and R⁷ is methyl or hydroxyl, or
R⁶ and R⁷ are linked to form C₃₋₆cycloalkyl optionally substituted by methyl;
R⁸ is hydrogen;
R⁹ is hydrogen, C₁₋₆alkyl or -CO₂C₁₋₆alkyl;
R¹⁰ is hydrogen, phenyl optionally substituted by -(CH₂)_{f}CO₂R¹⁶, or pyridyl optionally substituted by one or two substituents independently selected from chlorine and C₁₋₆alkyl;
R¹¹ is hydrogen and R¹² is hydrogen, -(CH₂)_{g}NR¹⁶R¹⁷, -(CH₂)ₕNCOC₁₋₆alkyl, -(CH₂)ᵢC₃₋₆ cycloalkyl, -(CH₂)ⱼphenyl, -(CH₂)ₖpyridyl, or -(CH₂)ₘheterocydyl wherein the heterocyclyl is optionally substituted by C₁₋₆alkyl.
R¹¹ is C₁₋₆alkyl and R¹² is C₁₋₅alkyl or -SO₂phenyl,
R¹¹ and R¹² are linked to form a 6-membered heterocyclyl optionally containing one further nitrogen wherein the heterocyclyl is optionally substituted by -CO₂C₁₋₆alkyl or piperidinyl, or
R¹¹ and R¹² are linked to form 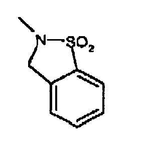
R¹³ is hydrogen and R¹⁴ is hydrogen, C₁₋₆alkyl, -(CH₂)ₙOR¹⁸, -(CH₂)ₚNR¹⁹R²⁰, - (CH₂)_{q}CO₂R²¹, -(CH₂)ᵣSO₂NH₂, C₃₋₆cycloalkyl, or phenyl optionally substituted by chlorine or -OC₁₋₆alkyl,
R¹³ and R¹⁴ are each independently C₁₋₆alkyl, or
R¹³ and R¹⁴ are linked to form pyrrolidinyl;
R¹⁵, R¹⁶, R¹⁷, R¹⁸, R¹⁹, R²⁰ and R²¹ are each independently hydrogen or C₁₋₆alkyl;
a, d, e, f, i, j, k and m are each independently an integer selected from 0 to 4;
b, g, h, n, p, q and r are each independently an integer selected from 1 to 4; and
c is 0 or 1;
or a salt thereof.

2. A compound according to claim 1 wherein R¹ is -SO₂NR³R⁴.

3. A compound according to claim 1 or claim 2 wherein R² is -CHR⁶R⁷.

4. A compound according to any one of the preceding claims wherein R³ is hydrogen and R⁴ is C₁₋₆alkyl substituted by hydroxy.

5. A compound according to any one of claims 1 to 3 wherein R³ is hydrogen and R⁴ is -(CH₂)_{b}NR⁸R⁹.

6. A compound according to any one of the preceding claims wherein R⁶ is hydrogen and R⁷ is methyl.

7. A compound according to any one of claims 1 to 5 wherein R⁶ and R⁷ are linked to form C₃₋₆cycloalkyl.

8. A compound, according to claim 1, which is substantially as described in any one of Examples 1 to 260, or a salt thereof.

9. A compound, according to claim 1, which is:
4-(2-cyclopropyl-1*H*-pyrrolo[2,3-b]pyridin-4-yl)-*N*-(2-hydroxyethyl)-benzenesulfonamide;
*N*-(2-aminoethyl)-4-(2-ethyl-1*H*-pyrrolo[2,3-*b*]pyridin-4-yl)benzenesulfonamide;
(2-cyclopropyl-1*H-*pyrrolo[2,3-*b*]pyridin-4-yl)-N*-*(1,1-dioxidotetrahydro-2*H*-opyran-4-yl)benzenesulfonamide;
*N*-[(1*S*)-2-hydroxy-1-methylethyl]-4-[2-(trifluoromethyl)-1*H*-pyrrolo[2,3-*b*]pyridin-4-yl]benzenesulfonamide;
4-(2-cyclopropyl-1*H-*pyrrolo[2,3-*b*]pyridin-4-yl)-*N*-(2-hydroxy-2-methylpropyl)benzenesulfonamide;
4-(2-cyclopropyl-1H-pyrrolo[2,3-b]pyridin-4-yl)-N-[(1S)-2-hydroxy-1-methylethyl]benzenesulfonamide;
4-[2-[(dimethylamino)methyl]-1*H*-pyrrolo[2,3-*b*]pyridin-4-yl]-*N*-[(3*R*)1,1-dioxidotetrahydro-3-thienyl]benzenesulfonamide; or
a salt thereof.

10. A compound according to any one of claims 1 to 9 in the form of a pharmaceutically acceptable salt thereof.

11. A pharmaceutical composition comprising a compound as claimed in any one of claims 1 to 9, or a pharmaceutically acceptable salt thereof, and one or more pharmaceutically acceptable excipients.

12. A compound as claimed in any one of claims 1 to 9, or a pharmaceutically acceptable salt thereof, for use in medical therapy

13. A compound as claimed in any one of claims 1 to 9, or a pharmaceutically acceptable salt thereof, for use in the treatment of disorders mediated by IKK2 activity.

14. Use of a compound as claimed in any one of claims 1 to 9, or a pharmaceutically acceptable salt thereof, in the manufacture of a medicament for use in the treatment of a disorder **characterized by** inappropriate IKK2 activity.

## Patentansprüche

1. Verbindung gemäss Formel (I): 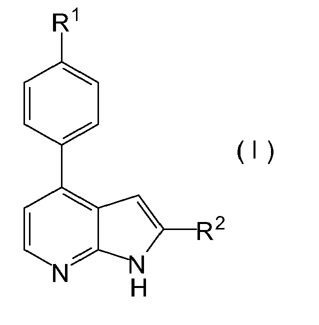 worin
R¹ -SO₂NR³R⁴ oder -NR⁵SO₂CH₃ ist;
R² -CHR⁶R⁷, -CF₃ oder -C(CH₃)₃ ist;
R³ Wasserstoff oder Methyl ist und R⁴ Wasserstoff, C₁₋₆-Alkyl, gegebenenfalls substituiert mit Hydroxy, -(CH₂)ₐC₃₋₆-Cycloalkyl, worin das Cycloalkyl gegebenenfalls mit Wasserstoff oder -NH₂ substituiert ist, -(CH₂)_{b}NR⁸R⁹, Piperidinyl, gegebenenfalls substituiert mit C₁₋₆-Alkyl, oder 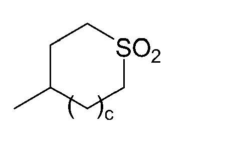 ist, oder
R³ und R⁴ unter Bildung von Pyrrolidinyl oder gegebenenfalls mit -NH₂ substituiertem Piperidinyl verknüpft sind;
R⁵ Wasserstoff oder Methyl ist;
R⁶ Wasserstoff ist und R⁷ Wasserstoff, C₁₋₆-Alkyl, -(CH₂)_{d}OR¹⁰, -NR¹¹R¹², -CO₂C₁₋₆-Alkyl, -CONR¹³R¹⁴, Phenyl oder 5-gliedriges Heteroaryl mit ein bis vier Stickstoffatomen ist, wobei das Heteroaryl gegebenenfalls mit ein oder zwei Substituenten, unabhängig voneinander ausgewählt aus C₁₋₆-Alkyl, -COC₁₋₆-Alkyl, -(CH₂)ₑPhenyl und Thienyl, substituiert ist,
R⁶ und R⁷ jeweils Fluor sind;
R⁶ Methyl ist und R⁷ Methyl oder Hydroxyl ist, oder
R⁶ und R⁷ verknüpft sind, um gegebenenfalls mit Methyl substituiertes C₃₋₆-Cycloalkyl zu bilden;
R⁸ Wasserstoff ist;
R⁹ Wasserstoff, C₁₋₆-Alkyl oder -CO₂C₁₋₆-Alkyl ist;
R¹⁰ Wasserstoff, gegebenenfalls mit -(CH₂)_{f}CK₂R¹⁵ substituiertes Phenyl oder gegebenenfalls mit ein oder zwei Substituenten, unabhängig voneinander ausgewählt aus Chlor und C₁₋₆-Alkyl, substituiertes Pyridyl ist;
R¹¹ Wasserstoff ist und R¹² Wasserstoff, -(CH₂)_{g}NR¹⁶R¹⁷, -(CH₂)ₕNCOC₁₋₆-Alkyl, -(CH₂)ᵢC₃₋₆-Cycloalkyl, -(CH₂)ⱼPhenyl, -(CH₂)ₖPyridyl oder -(CH₂)ₘHeterocyclyl ist, wobei das Heterocyclyl gegebenenfalls mit C₁₋₆-Alkyl substituiert ist,
R¹¹ C₁₋₆-Alkyl ist und R¹² C₁₋₆-Alkyl oder -SO₂-Phenyl ist,
R¹¹ und R¹² verknüpft sind, um 6-gliedriges Heterocyclyl mit gegebenenfalls einem weiteren Stickstoff zu bilden, wobei das Heterocyclyl gegebenenfalls mit -CO₂C₁-₆-Alkyl oder Piperidinyl substituiert ist,
oder
R¹¹ und R¹² verknüpft sind, um 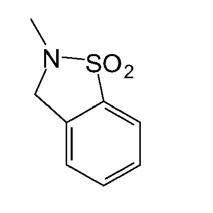
zu bilden;
R¹³ Wasserstoff ist und R¹⁴ Wasserstoff, C₁₋₆-Alkyl, -(CH₂)ₙOR¹⁸, -(CH₂)ₚNR¹⁹R²⁰, -(CH₂)_{q}CO₂R²¹, -(CH₂)ᵣSO₂NH₂, C₃₋₆-Cycloalkyl oder gegebenenfalls mit Chlor oder -OC₁₋₆-Alkyl substituiertes Phenyl ist,
R¹³ und R¹⁴ jeweils unabhängig voneinander C₁₋₆-Alkyl sind, oder
R¹³ und R¹⁴ verknüpft sind, um Pyrrolidinyl zu bilden;
R¹⁵, R¹⁶, R¹⁷, R¹⁸, R¹⁹, R²⁰ und R²¹ jeweils unabhängig voneinander Wasserstoff oder C₁₋₆-Alkyl sind;
a, d, e, f, i, j, k und m jeweils unabhängig voneinander eine ganze Zahl, ausgewählt aus 0 bis 4, sind;
b, g, h, n, p, q und r jeweils unabhängig voneinander eine ganze Zahl, ausgewählt aus 1 bis 4, sind; und
c 0 oder 1 ist;
oder ein Salz davon.

2. Verbindung gemäss Anspruch 1, worin R¹ -SO₂NR³R⁴ ist.

3. Verbindung gemäss Anspruch 1 oder Anspruch 2, worin R² -CHR⁶R⁷ ist.

4. Verbindung gemäss irgendeinem der vorhergehenden Ansprüche, worin R³ Wasserstoff ist und R⁴ mit Hydroxy substituiertes C₁₋₆-Alkyl ist.

5. Verbindung gemäss irgendeinem der Ansprüche 1 bis 3, worin R³ Wasserstoff ist und R⁴ -(CH₂)_{b}NR⁸R⁹ ist.

6. Verbindung gemäss irgendeinem der vorhergehenden Ansprüche, worin R⁶ Wasserstoff ist und R⁷ Methyl ist.

7. Verbindung gemäss irgendeinem der Ansprüche 1 bis 5, worin R⁶ und R⁷ verknüpft sind, um C₃₋₆-Cycloalkyl zu bilden.

8. Verbindung gemäss Anspruch 1, die im wesentlichen wie in irgendeinem der Beispiele 1 bis 260 beschrieben ist, oder ein Salz davon.

9. Verbindung gemäss Anspruch 1, die ist:
4-(2-Cyclopropyl-1H-pyrrolo[2,3-b]pyridyl-4-yl)-N-(2-hydroxyethyl)benzolsulfonamid;
N-(2-Aminoethyl)-4-(2-ethyl-1H-pyrrolo[2,3-b]pyridin-4-yl)benzolsulfonamid;
4-(2-Cyclopropyl-1H-pyrrolo[2,3-b]pyridin-4-yl)-N-(1,1-dioxidotetrahydro-2H-thiopyran-4-yl)benzolsulfonamid;
N-[(1S)-2-Hydroxy-1-methylethyl]-4-[2-(trifluormethyl)-1H-pyrrolo[2,3-b]pyridin-4-yl]benzolsulfonamid;
4-(2-Cyclopropyl-1H-pyrrolo[2,3-b]pyridin-4-yl)-N-(2-hydroxy-2-methylpropyl)benzolsulfonamid;
4-(2-Cyclopropyl-1H-pyrrolo[2,3-b]pyridin-4-yl)-N-[(1S)-2-hydroxy-1-methylethyl]benzolsulfonamid;
4-{2-[(Dimethylamino)methyl]-1H-pyrrolo[2,3-b]-pyridin-4-yl}-N-[(3R)-1,1-dioxidotetrahydro-3-thienyl]benzolsulfonamid
oder ein Salz davon.

10. Verbindung gemäss irgendeinem der Ansprüche 1 bis 9 in Form eines pharmazeutisch annehmbaren Salzes davon.

11. Pharmazeutische Zusammensetzung, umfassend eine Verbindung gemäss irgendeinem der Ansprüche 1 bis 9 oder ein pharmazeutisch annehmbares Salz davon und einen oder mehrere pharmazeutisch annehmbare(n) Exzipienten.

12. Verbindung gemäss irgendeinem der Ansprüche 1 bis 9 oder ein pharmazeutisch annehmbares Salz davon zur Verwendung in der medizinischen Therapie.

13. Verbindung gemäss irgendeinem der Ansprüche 1 bis 9 oder ein pharmazeutisch annehmbares Salz davon zur Verwendung bei der Behandlung von durch IKK2-Aktivität vermittelten Störungen.

14. Verwendung einer Verbindung gemäss irgendeinem der Ansprüche 1 bis 9 oder eines pharmazeutisch annehmbaren Salzes davon bei der Herstellung eines Medikaments zur Verwendung bei der Behandlung einer Störung, die durch unangemessene IKK2-Aktivität **gekennzeichnet** ist.

## Revendications

1. Composé selon la formule (I) : 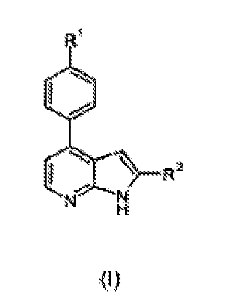 dans laquelle
R¹ représente -SO₂NR³R⁴ ou -NR⁵SO₂CH₃ ;
R² représente -CHR⁶R⁷, -CF₃ ou -C(CH₃)₃ ;
R³ représente un atome d'hydrogène ou un groupe méthyle et R⁴ représente un atome d'hydrogène, un groupe alkyle en C₁ à C₆ éventuellement substitué par un groupe hydroxy, -(CH₂)ₐ-cycloalkyle en C₃ à C₆ où le groupe cycloalkyle est éventuellement substitué par un groupe hydroxy ou -NH₂, -(CH₂)_{b}NR⁸R⁹, pipéridinyle éventuellement substitué par un groupe alkyle en C₁ à C₆, ou 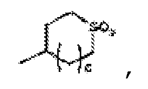 ou
R³ et R⁴ sont liés pour former un groupe pyrrolidinyle ou pipéridinyle éventuellement substitué par -NH₂ ;
R⁵ représente un atome d'hydrogène ou un groupe méthyle ;
R⁶ représente un atome d'hydrogène et R⁷ représente un atome d'hydrogène, un groupe alkyle en C₁ à C₆, -(CH₂)_{d}OR¹⁰, -NR¹¹R¹², -CO₂-alkyle en C₁ à C₆, -CONR¹³R¹⁴, phényle, ou hétéroaryle de 5 chaînons contenant d'un à quatre atomes d'azote, où le groupe hétéroaryle est éventuellement substitué par un ou deux substituants choisis indépendamment parmi les groupes alkyle en C₁ à C₆, -CO-alkyle en C₁ à C₆, -(CH₂)ₑ-phényle et thiényle,
R⁶ et R⁷ représentent chacun un atome de fluor,
R⁶ représente un atome de méthyle et R⁷ représente un groupe méthyle ou hydroxyle, ou
R⁶ et R⁷ sont liés pour former un groupe cycloalkyle en C₃ à C₆ éventuellement substitué par un groupe méthyle ;
R⁸ représente un atome d'hydrogène ;
R⁹ représente un atome d'hydrogène, un groupe alkyle en C₁ à C₆ ou -CO₂-alkyle en C₁ à C₆ ;
R¹⁰ représente un atome d'hydrogène, un groupe phényle éventuellement substitué par -(CH₂)_{f}CO₂R¹⁵ ou pyridyle éventuellement substitué par un ou deux substituants choisis indépendamment parmi un atome de chlore et un groupe alkyle en C₁ à C₆ ;
R¹¹ représente un atome d'hydrogène et R¹² représente un atome d'hydrogène, -(CH₂)_{g}NR¹⁶R¹⁷, -(CH₂)ₕNCO-alkyle en C₁ à C₆, -(CH₂)ᵢ-cycloalkyle en C₃ à C₆, -(CH₂)ⱼ-phényle, -(CH₂)ₖ-pyridyle ou -(CH₂)ₘ-hétérocyclyle, où le groupe hétérocyclyle est éventuellement substitué par un groupe alkyle en C₁ à C₆,
R¹¹ représente un groupe alkyle en C₁ à C₆ et R¹² représente un groupe alkyle en C₁ à C₆ ou -SO₂-phényle,
R¹¹ et R¹² sont liés pour former un groupe hétérocyclyle de 6 chaînons contenant éventuellement un autre atome d'azote où le groupe hétérocyclyle est éventuellement substitué par un groupe -CO₂-alkyle en C₁ à C₆ ou pipéridinyle, ou
R¹¹ et R¹² sont liés pour former un groupe 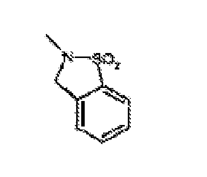
R¹³ représente un atome d'hydrogène et R¹⁴ représente un atome d'hydrogène, un groupe alkyle en C₁ à C₆, -(CH₂)ₙOR¹⁸, -(CH₂)ₚNR¹⁹R²⁰, -(CH₂)_{q}CO₂R²¹, -(CH₂)ᵣSO₂NH₂, cycloalkyle en C₃ à C₆ ou phényle éventuellement substitué par un atome de chlore ou un groupe -O-alkyle en C₁ à C₆,
R¹³ et R¹⁴ représentent chacun indépendamment un groupe alkyle en C₁ à C₆, ou
R¹³ et R¹⁴ sont liés pour former un groupe pyrrolidinyle ;
R¹⁵, R¹⁶, R¹⁷, R¹⁸, R¹⁹, R²⁰ et R²¹ représentent chacun indépendamment un atome d'hydrogène ou un groupe alkyle en C₁ à C₆ ;
a, d, e, f, i, j, k et m représentent chacun indépendamment un nombre entier choisi parmi 0 à 4 ;
b, g, h, n, p, q et r représentent chacun indépendamment un nombre entier choisi parmi 1 à 4 ; et
c vaut 0 ou 1 ;
ou un sel de celui-ci.

2. Composé selon la revendication 1, dans lequel R¹ représente -SO₂NR³R⁴.

3. Composé selon la revendication 1 ou la revendication 2, dans lequel R² représente -CHR⁶R⁷.

4. Composé selon l'une quelconque des revendications précédentes, dans lequel R³ représente un atome d'hydrogène et R⁴ représente un groupe alkyle en C₁ à C₆ substitué par un groupe hydroxy.

5. Composé selon l'une quelconque des revendications 1 à 3, dans lequel R³ représente un atome d'hydrogène et R⁴ représente -(CH₂)_{b}NR⁸R⁹.

6. Composé selon l'une quelconque des revendications précédentes, dans lequel R⁶ représente un atome d'hydrogène et R⁷ représente un groupe méthyle.

7. Composé selon l'une quelconque des revendications 1 à 5, dans lequel R⁶ et R⁷ sont liés pour former un groupe cycloalkyle en C₃ à C₆.

8. Composé selon la revendication 1 tel que décrit dans l'un quelconque des exemples 1 à 260, ou un sel de celui-ci.

9. Composé selon la revendication 1, qui est :
le 4-(2-cyclopropyl-1*H*-pyrrolo[2,3-*b*]pyridin-4-yl)-*N*-(2-hydroxyéthyl)-benzènesulfonamide ;
le *N*-(2-aminoéthyl)-4-(2-éthyl-1*H*-pyrrolo[2,3-*b*]-pyridin-4-yl)benzènesulfonamide ;
le 4-(2-cylopropyl-1*H*-pyrrolo[2,3-*b*]pyridin-4-yl)-*N*-(1,1-dioxydotétrahydro-2*H*-thiopyran-4-yl)-benzènesulfonamide ;
le *N*-[(1*S*)-2-hydroxy-1-méthyléthyl]-4-[2-(trifluorométhyl)-1*H*-pyrrolo[2,3-*b*]pyridin-4-yl]-benzènesulfonamide ;
le 4-(2-cyclopropyl-1*H*-pyrrolo[2,3-*b*]pyridin-4-yl)-*N*-(2-hydroxy-2-méthylpropyl)benzènesulfonamide ;
le 4-(2-cyclopropyl-1H-pyrrolo[2,3-b]pyridin-4-yl)-N-[(1S)-2-hydroxy-1-méthyléthyl]benzènesulfonamide ;
le 4-{2-[(diméthylamino)méthyl]-1*H*-pyrrolo[2,3-*b*]-pyridin-4-yl}-*N*-[(3R)-1,1-dioxydotétrahydro-3-thiényl]-benzènesulfonamide ; ou
un sel de celui-ci.

10. Composé selon l'une quelconque des revendications 1 à 9, sous la forme d'un sel pharmaceutiquement acceptable de celui-ci.

11. Composition pharmaceutique comprenant un composé selon l'une quelconque des revendications 1 à 9, ou un sel pharmaceutiquement acceptable de celui-ci, et un ou plusieurs excipients pharmaceutiquement acceptables.

12. Composé selon l'une quelconque des revendications 1 à 9, ou un sel pharmaceutiquement acceptable de celui-ci, pour une utilisation en thérapie médicale.

13. Composé selon l'une quelconque des revendications 1 à 9, ou un sel pharmaceutiquement acceptable de celui-ci, pour une utilisation dans le traitement de troubles médiés par l'activité IKK2.

14. Utilisation d'un composé selon l'une quelconque des revendications 1 à 9, ou d'un sel pharmaceutiquement acceptable de celui-ci, pour une utilisation dans le traitement d'un trouble **caractérisé par** une activité IKK2 inappropriée.
